# EUROPEAN PATENT SPECIFICATION

(11) **EP 3 904 346 B1**
(45) Date of publication and mention of the grant of the patent: **28.08.2024**
(21) Application number: 20763578.0
(22) Date of filing: 24.02.2020
(51) Int. Cl.: C07D 307/91, C07D 209/82, C07D 405/04, C07D 493/04, C07D 495/04, C07F 7/08, H10K 50/11, C07C 211/61, C07D 209/86, C07D 307/93, C07D 333/78, H10K 85/60, C09K 11/06, H10K 85/40

(54) **NOVEL COMPOUND AND ORGANIC LIGHT EMITTING DEVICE COMPRISING SAME**
NEUARTIGE VERBINDUNG UND ORGANISCHE LICHTEMITTIERENDE VORRICHTUNG DAMIT
NOUVEAU COMPOSÉ ET DISPOSITIF ÉLECTROLUMINESCENT ORGANIQUE LE COMPRENANT

(30) Priority: 28.02.2019 KR 20190024296; 21.02.2020 KR 20200021512
(43) Date of publication of application: 03.11.2021
(73) Proprietor: LG Chem, Ltd., Seoul 07336, (KR)
(72) Inventor: KIM, Yongwook, Daejeon 34122 (KR); BAE, Jaesoon, Daejeon 34122 (KR); LEE, Jaechol, Daejeon 34122 (KR); KIM, Young Kwang, Daejeon 34122 (KR); KIM, Juhwan, Daejeon 34122 (KR); KHIM, Dongyoon, Daejeon 34122 (KR); CHO, Beomshin, Daejeon 34122 (KR)
(74) Representative: Hoffmann Eitle
(86) International application number: PCT/KR2020/002606
(87) International publication number: WO 2020/175867

(56) References cited:
- EP-A1- 3 269 790
- WO-A1-2017/023021
- WO-A1-2019/013556
- KR-A- 20070 119 470
- KR-A- 20180 042 513
- KR-A- 20180 050 891
- KR-A- 20180 080 603

## Description

### [TECHNICAL FIELD]

This application claims the benefit of Korean Patent Application No. 10-2019-0024296 filed on February 28, 2019 and Korean Patent Application No. 10-2020-0021512 filed on February 21, 2020 in the Korean Intellectual Property Office.

The present disclosure relates to a novel compound and an organic light emitting device comprising the same.

### [BACKGROUND OF ART]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

Materials used in the organic light emitting devices are described in numerous documents, including the Patent Literatures indicated below. As one example, Patent Literature 2 discloses the following compounds:

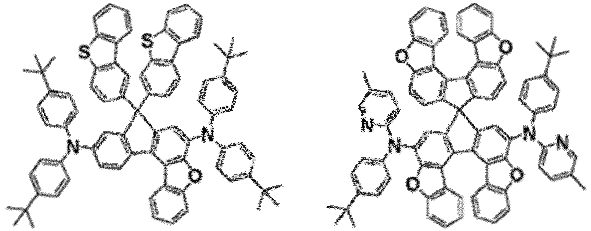

There is a continuing need for the development of new materials for the organic materials used in the organic light emitting devices as described above.

Meanwhile, recently, in order to reduce process costs, an organic light emitting device using a solution process, particularly an inkjet process, has been developed instead of a conventional deposition process. In the initial stage of development, attempts have been made to develop organic light emitting devices by coating all organic light emitting device layers by a solution process, but current technology has limitations. Therefore, only HIL, HTL, and EML are processed in a layer device structure by a solution process, and a hybrid process utilizing traditional deposition processes as a subsequent process is being studied.

In this regard, the present disclosure provides novel materials for organic light emitting devices that can be used for an organic light emitting device and simultaneously, can be deposited by a solution process.

### [PRIOR ART DOCUMENTS]

### [Patent Documents]

(Patent Literature 0001) Korean Unexamined Patent Publication No. 10-2000-0051826
(Patent Literature 0002) WO 2019/013556
(Patent Literature 0003) EP 3 269 790
(Patent Literature 0004) WO 2017/023021
(Patent Literature 0005) KR 2018 0050891
(Patent Literature 0006) KR 2018 0080603
(Patent Literature 0007) US 2016/200080
(Patent Literature 0008) EP 3 266 848
(Patent Literature 0009) WO 2016/126022
(Patent Literature 0010) US 2016/204355
(Patent Literature 0011) WO 2016/10841
(Patent Literature 0012) KR 201434884
(Patent Literature 0013) KR 201412887
(Patent Literature 0014) KR 2014078096

### [DETAILED DESCRIPTION OF THE INVENTION]

### [Technical Problem]

It is an object of the present disclosure to provide a novel compound and an organic light emitting device.

### [Technical Solution]

According to an aspect of the present disclosure, there is provided a compound represented by the following Chemical Formula 1: 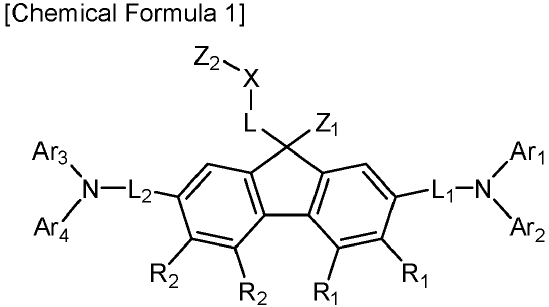 in the Chemical Formula 1,
R₁ and R₂ are each independently hydrogen, or two R₁s or two R₂s are joined together to form a substituted or unsubstituted Ce-eo aromatic ring; a substituted or unsubstituted C₆₋₆₀ non-aromatic ring; or a substituted or unsubstituted C₂₋₆₀ heterocyclic ring containing any one or more heteroatoms selected from the group consisting of N, O, S and Si,
at least one of the two R₁s or the two R₂s are joined together to form the C₆₋₆₀ non-aromatic ring; or the C₂₋₆₀ heterocyclic ring,
L is a substituted or unsubstituted C₆₋₆₀ arylene; or a substituted or unsubstituted C₂₋₆₀ heteroarylene containing any one or more heteroatoms selected from the group consisting of N, O and S,
X is O, S, NZ₃, or SiZ₄Z₅,
Z₁ is a substituted or unsubstituted C₆₋₆₀ aryl,
Z₂ to Z₅ are each independently a substituted or unsubstituted C₁₋₆₀ alkyl; a substituted or unsubstituted C₁₋₆₀ haloalkyl; a substituted or unsubstituted C₃₋₆₀ cycloalkyl; a substituted or unsubstituted C₇₋₆₀ aralkyl; a substituted or unsubstituted C₆₋₆₀ aryl; or a substituted or unsubstituted C₂₋₆₀ heteroaryl containing any one or more heteroatoms selected from the group consisting of N, O and S,
the L and Z₂; Z₂ and Z₃; or Z₄ and Z₅ may be connected to each other to form a 5-membered-heterocyclic ring, with the proviso that Z₁ and LX-Z₂ are different from each other and are not connected to each other,
L₁ and L₂ are each independently a single bond; a substituted or unsubstituted C₆₋₆₀ arylene; or a substituted or unsubstituted C₂₋₆₀ heteroarylene containing any one or more heteroatoms selected from the group consisting of N, O and S, and
Ar₁ to Ar₄ are each independently a substituted or unsubstituted C₆₋₆₀ aryl; or a substituted or unsubstituted C₂₋₆₀ heteroaryl containing any one or more heteroatoms selected from the group consisting of N, O and S.

According to another aspect of the present disclosure, there is provided an organic light emitting device comprising: a first electrode; a second electrode that is provided opposite to the first electrode; and a light emitting layer that is provided between the first electrode and the second electrode, wherein the light emitting layer includes the above-mentioned compound represented by Chemical Formula 1.

### [ADVANTAGEOUS EFFECTS]

The above-mentioned compound represented by the Chemical Formula 1 can be used as a material of an organic material layer of an organic light emitting device, can be subjected to a solution process, and can improve the efficiency, achieve low driving voltage and/or improve lifetime characteristics in the organic light emitting device.

### [BRIEF DESCRIPTION OF THE DRAWINGS]

FIG. 1 shows an example of an organic light emitting device comprising a substrate 1, an anode 2, a light emitting layer 3, and a cathode 4.
FIG. 2 shows an example of an organic light emitting device comprising a substrate 1, an anode 2, a hole injection layer 5, a hole transport layer 6, a light emitting layer 7, an electron transport layer 8, an electron injection layer 9 and a cathode 4.

### [DETAILED DESCRIPTION OF THE EMBODIMENTS]

Hereinafter, embodiments of the present disclosure will be described in more detail to facilitate understanding of the invention.

### (Definition of Terms)

As used herein, the notation 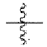 means a bond linked to another substituent group, Ph means a phenyl group, D means deuterium, and t-Bu means tert-butyl group.

As used herein, the term "substituted or unsubstituted" means being unsubstituted or substituted with one or more substituents selected from the group consisting of deuterium; a halogen group; a cyano group; a nitro group; a hydroxy group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heteroaryl group containing at least one of N, O and S atoms, or being unsubstituted or substituted with a substituent in which two or more substituents of the above-exemplified substituents are connected. For example, "a substituent in which two or more substituents are connected" may be a biphenyl group. Namely, a biphenyl group may be an aryl group, or it may also be interpreted as a substituent in which two phenyl groups are connected.

In the present disclosure, the carbon number of a carbonyl group is not particularly limited, but is preferably 1 to 40. Specifically, the carbonyl group may be a group having the following structural formulas, but is not limited thereto.

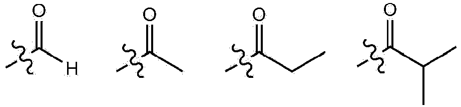

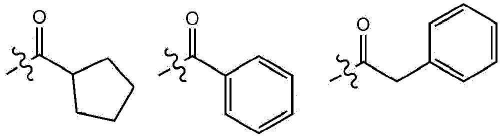

In the present disclosure, an ester group may have a structure in which oxygen of the ester group may be substituted by a straight-chain, branched-chain, or cyclic alkyl group having 1 to 25 carbon atoms, or an aryl group having 6 to 25 carbon atoms. Specifically, the ester group may be a gruop having the following structural formulas, but is not limited thereto.

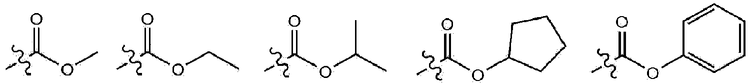

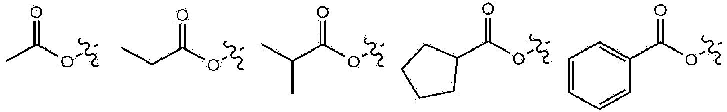

In the present disclosure, the carbon number of an imide group is not particularly limited, but is preferably 1 to 25. Specifically, the imide group may be a group having the following structural formulas, but is not limited thereto.

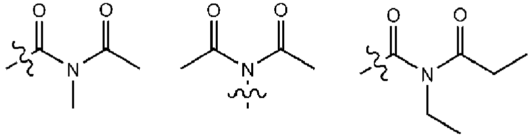

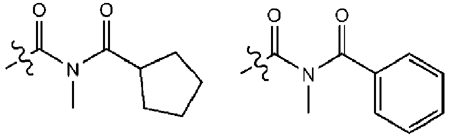

In the present disclosure, a silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but is not limited thereto.

In the present disclosure, a boron group specifically includes a trimethylboron group, a triethylboron group, a t-butyldimethylboron group, a triphenylboron group, and a phenylboron group, but is not limited thereto.

In the present disclosure, examples of a halogen group include fluorine, chlorine, bromine, or iodine.

In the present disclosure, the alkyl group may be straight-chain or branched-chain, and the carbon number thereof is not particularly limited, but is preferably 1 to 40. According to one embodiment, the carbon number of the alkyl group is 1 to 20. According to another embodiment, the carbon number of the alkyl group is 1 to 10. According to another embodiment, the carbon number of the alkyl group is 1 to 6. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl,4-methylhexyl, 5-methylhexyl, and the like, but are not limited thereto.

In the present disclosure, the alkenyl group may be straight-chain or branched-chain, and the carbon number thereof is not particularly limited, but is preferably 2 to 40. According to one embodiment, the carbon number of the alkenyl group is 2 to 20. According to another embodiment, the carbon number of the alkenyl group is 2 to 10. According to still another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present disclosure, a cycloalkyl group is not particularly limited, but the carbon number thereof is preferably 3 to 60. According to one embodiment, the carbon number of the cycloalkyl group is 3 to 30. According to another embodiment, the carbon number of the cycloalkyl group is 3 to 20. According to still another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present disclosure, an aralkyl group is not particularly limited, but the carbon number thereof is preferably 7 to 60. According to one embodiment, the carbon number of the aralkyl group is 7 to 30 . According to another embodiment, the carbon number of the aralkyl group is 7 to 20. Specific examples thereof include phenylmethyl, 2-methylphenylmethyl, 3-methylphenylmethyl, 4-methylphenylmethyl, 2-tert-butylphenylmethyl, 3-tert-butylphenylmethyl, 4-tert-butylphenylmethyl, and the like, but are not limited thereto.

In the present disclosure, an aryl group is not particularly limited, but the carbon number thereof is preferably 6 to 60, and it may be a monocyclic aryl group or a polycyclic aryl group. According to one embodiment, the carbon number of the aryl group is 6 to 30. According to one embodiment, the carbon number of the aryl group is 6 to 20. The aryl group may be a phenyl group, a biphenyl group, a terphenyl group or the like as the monocyclic aryl group, but is not limited thereto. The polycyclic aryl group includes a naphthyl group, an anthracenyl group, a phenanthrenyl group, a pyrenyl group, a perylenyl group, a chrysenyl group, a fluorenyl group or the like, but is not limited thereto.

In the present disclosure, the fluorenyl group may be substituted, and two substituents may be linked with each other to form a spiro structure. In the case where the fluorenyl group is substituted, 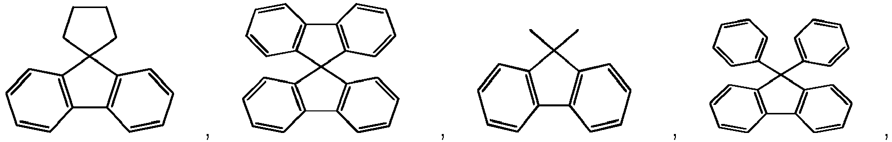 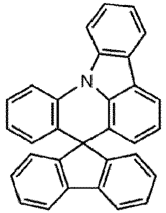 and the like can be formed. However, the structure is not limited thereto.

In the present disclosure, a heteroaryl group is a heteroaryl group containing one or more of O, N, Si and S as a heteroatom, and the carbon number thereof is not particularly limited, but is preferably 2 to 60. Examples of the heteroaryl group include a xanthene group, a thioxanthene group, a thiophene group, a furan group, a pyrrole group, an imidazole group, a thiazole group, an oxazol group, an oxadiazol group, a triazol group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzoimidazole group, a benzothiazol group, a benzocarbazole group, a benzothiophene group, a dibenzothiophene group, a benzofuranyl group, a phenanthroline group, an isoxazolyl group, a thiadiazolyl group, a phenothiazinyl group, a dibenzofuranyl group, and the like, but are not limited thereto.

In the present disclosure, the aryl group in the aralkyl group, the aralkenyl group, the alkylaryl group, the arylamine group and the arylsily group is the same as the aforementioned examples of the aryl group. In the present disclosure, the alkyl group in the aralkyl group, the alkylaryl group and the alkylamine group is the same as the aforementioned examples of the alkyl group. In the present disclosure, the heteroaryl in the heteroarylamine can apply the aforementioned description of the heteroaryl group. In the present disclosure, the alkenyl group in the aralkenyl group is the same as the aforementioned examples of the alkenyl group. In the present disclosure, the aforementioned description of the aryl group may be applied except that the arylene is a divalent group. In the present disclosure, the aforementioned description of the heteroaryl group can be applied except that the heteroarylene is a divalent group. In the present disclosure, the aforementioned description of the aryl group or cycloalkyl group can be applied except that the hydrocarbon ring is not a monovalent group but formed by combining two substituent groups. In the present disclosure, the aforementioned description of the heteroaryl group can be applied, except that the heterocycle is not a monovalent group but formed by combining two substituent groups.

In the present disclosure, an aromatic ring means a condensed monocyclic or condensed polycyclic ring in which the entire molecule has aromaticity while containing only carbon as a ring-forming atom. The carbon number of the aromatic ring is 6 to 60, or 6 to 30, or 6 to 20, but is not limited thereto. In addition, the aromatic ring may include a benzene ring, a naphthalene ring, an anthracene ring, a phenanthrene ring, a pyrene ring, and the like, but is not limited thereto.

In the present disclosure, a non-aromatic ring means a condensed monocyclic or condensed polycyclic ring in which the entire molecule has no aromaticity while containing only carbon as a ring-forming atom. The carbon number of the non-aromatic ring is 6 to 60, or 6 to 30, or 6 to 20, but is not limited thereto. In addition, the non-aromatic ring may include an indene ring, a fluorene ring, and the like, but is not limited thereto. At this time, the indene ring, the fluorene ring, and the like may be substituted or unsubstituted, and as for the type of the substituent, refer to those described above.

In the present disclosure, the heterocyclic ring means a condensed monocyclic or condensed polycyclic ring in which the entire molecule has aromaticity or does not have aromaticity, while containing one or more heteroatoms selected among O, N, Si and S in addition to carbon as a ring-forming atom. In the present disclosure, a divalent non-aromatic condensed polycyclic group means a divalent group having the same structure as the above-mentioned monovalent non-aromatic hetero-condensed polycyclic group. The carbon number of the heterocyclic ring is 2 to 60, or 2 to 30, or 2 to 20, but is not limited thereto. In addition, the heterocyclic ring includes a benzofuran ring, a benzothiophene ring, a benzosilole ring, a dibenzofuran ring, a dibenzothiophene ring, a dibenzosilole ring, a silole ring, and the like, but is not limited thereto. At this time, the benzosilole ring, the dibenzosilole ring, etc. may be substituted or unsubstituted, and as for the type of the substituent, refer to those described above.

### (Compound)

Meanwhile, the present disclosure provides the diamine compound represented by Chemical Formula 1.

The compound represented by Chemical Formula 1 has a structure in which the fluorene-based core contains 'Z₁' and 'LX-Z₂' as substituents, and 'Z₁' and 'LX-Z₂' are different from each other and are not connected to each other, as described in detail below. Specifically, the compound having a structure in which the substituents of the fluorene-based core are identical to each other, or the substituents are connected to each other, has a high crystallinity, which cause the problem of low solubility in an organic solvent, whereas the compound represented by Chemical Formula 1 has a low crystallinity compared to the above, and exhibits increased solubility in an organic solvent used in a solution process, and thus, is preferably used in a solution process during the manufacture of an organic light emitting device.

Preferably, in Chemical Formula 1,
at least one of the two R₁s or the two R₂s are joined together to form a C₆₋₆₀ alicyclic ring; or a C₂₋₆₀ heterocycle containing any one or more heteroatoms selected from the group consisting of N, O, S and Si, the rest are hydrogen, or R₁ or R₂ are joined together to form a C₆₋₆₀ aromatic ring; a C₆₋₆₀ alicyclic ring; or a C₂₋₆₀ heterocyclic ring containing any one or more heteroatoms selected from the group consisting of N, O, S and Si.

In this case, the C₆₋₆₀ aromatic ring, the C₆₋₆₀ alicyclic ring and the C₂₋₆₀ heterocyclic ring may be each independently substituted with a substituent group selected from the group consisting of hydrogen, C₁₋₁₀ alkyl and C₆₋₂₀ aryl.

In other words,
R₁ groups are joined together to form the C₆₋₆₀ alicyclic ring or the C₂₋₆₀ heterocyclic ring, and R₂ groups are all hydrogen, or are joined together to form the C₆₋₆₀ aromatic ring, the C₆₋₆₀ alicyclic ring, or the C₂₋₆₀ heterocyclic ring; or
R₂ groups are joined together to form the C₆₋₆₀ alicyclic ring or the C₂₋₆₀ heterocyclic ring, and R₁ groups are all hydrogen, or are joined together to form the C₆₋₆₀ aromatic ring, the C₆₋₆₀ alicyclic ring, or the C₂₋₆₀ heterocyclic ring; or
both R₁ and R₂ may be joined together to form the C₆₋₆₀ aromatic ring, the C₆₋₆₀ alicyclic ring, or the C₂₋₆₀ heterocyclic ring.

Preferably, at least one of the two R₁s or the two R₂s are joined together, together with the carbon atoms to which R₁ or R₂ is attached, to form any one of the structures represented by the following Chemical Formulas 2d to 2g, the rest are hydrogen, or are joined together to form any one of the structures represented by the following Chemical Formulas 2a to 2g: 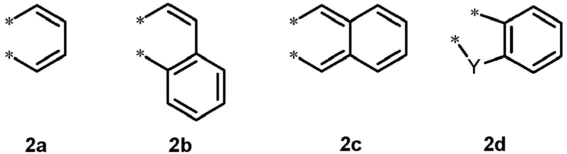 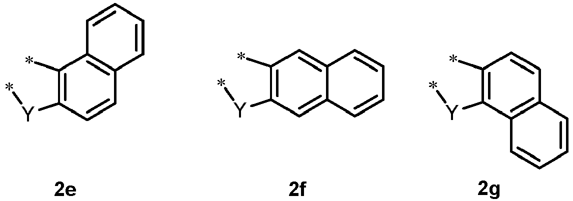 in the Chemical Formulas 2a to 2g,
Y is O, S, CZ₆Z₇, or SiZ₈Z₉,
Z₆ to Z₉ are each independently a substituted or unsubstituted C₁₋₆₀ alkyl; a substituted or unsubstituted C₁₋₆₀ haloalkyl; a substituted or unsubstituted C₃₋₆₀ cycloalkyl; a substituted or unsubstituted C₆₋₆₀ aryl; a substituted or unsubstituted C₂₋₆₀ heteroaryl containing any one or more heteroatoms selected from the group consisting of N, O and S, preferably, Z₆ to Z₉ are each independently C₁₋₁₀ alkyl, or C₆₋₂₀ aryl, and
* means a bonding position with the carbon bonded to R₁ or R₂.

In other words,
R₁ groups are joined together to form any one of the structures represented by Chemical Formulas 2d to 2g, and R₂ groups are all hydrogen, or are joined together to form any one of the structures represented by Chemical Formulas 2a to 2g, or
R₂ groups are joined together to form any one of the structures represented by Chemical Formulas 2d to 2g, and R₁ groups are all hydrogen, or are joined together to form any one of the structures represented by Chemical Formulas 2a to 2g, or
both R₁ and R₂ may be, R₁s or R₂s joined together to form any one of the structures represented by the Chemical Formulas 2d to 2g.

Most preferably, at least one of R₁ and R₂ is joined together to form a structure represented by Chemical Formula 2d, and the rest are hydrogen or are joined together to form a structure represented by Chemical Formula 2a or 2d.

For example, the compound represented by Chemical Formula 1,
is represented by the following Chemical Formula 1-1 when R₁ groups are all hydrogen, and when R₂ groups are joined together to form a structure represented by Chemical Formula 2d,
is represented by the following Chemical Formula 1-2 when R₂ groups are all hydrogen, and when R₁ groups are joined together to form a structure represented by Chemical Formula 2d,
is represented by the following Chemical Formula 1-3 when R₁ groups are joined together to form a structure represented by Chemical Formula 2a, and R₂ groups are joined together to form a structure represented by Chemical Formula 2d,
is represented by the following Chemical Formula 1-4 when R₁ groups are joined together to form a structure represented by Chemical Formula 2d, and R₂ groups are joined together to form a structure represented by Chemical Formula 2a, and
is represented by the following Chemical Formula 1-5 when R₁ and R₂ are each joined together to form a structure represented by Chemical Formula 2d. 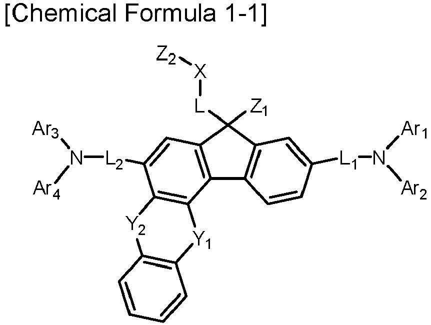 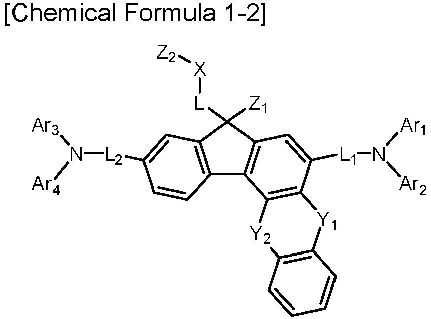 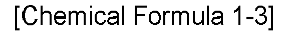 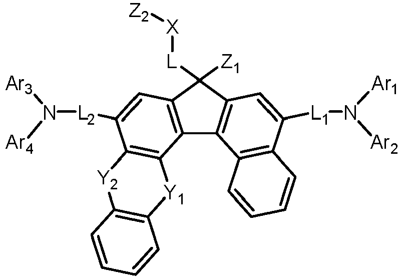 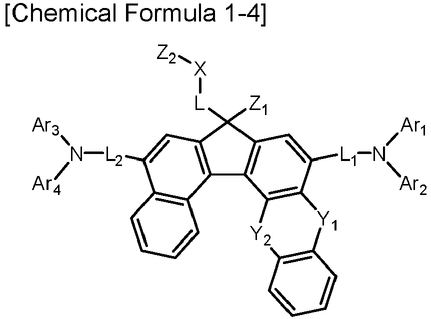 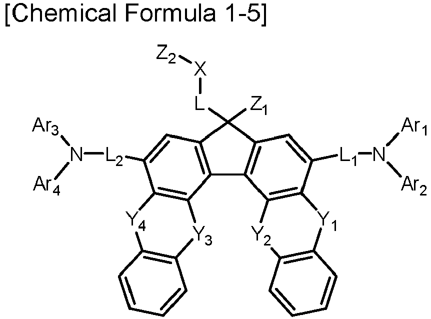 in the Chemical Formulas 1-1 to 1-5,
   L, X, Z₁, Z₂, L₁, L₂ and Ar₁ to Ar₄ are the same as defined in Chemical Formula 1,
   one of Y₁ and Y₂ is a single bond, and the other one is O, S, C(C₁₋₄ alkyl)₂, or Si(C₁₋₄ alkyl)₂,
   one of Y₃ and Y₄ is a single bond, and the other one is O, S, C(C₁₋₄ alkyl)₂, or Si(C₁₋₄ alkyl)₂.

Preferably, one of Y₁ and Y₂ is a single bond, and the other one is O, S, C(methyl)₂, or Si(methyl)₂,
one of Y₃ and Y₄ is a single bond, and the other one is O, S, C(methyl)₂, or Si(methyl)₂.

Preferably, Z₁ is phenyl or biphenylyl, wherein Z₁ is unsubstituted, or substituted with 1 to 5 substituents each independently selected from the group consisting of deuterium, halogen, cyano, C₁₋₁₀ alkyl, C₃₋₁₀ cycloalkyl, Si(C₁₋₁₀ alkyl)₃ and Si(C₆₋₂₀ aryl)₃.

More preferably, Z₁ is phenyl or biphenylyl, wherein Z₁ is unsubstituted, or substituted with 1 to 5 substituents each independently selected from the group consisting of deuterium, halogen, cyano, methyl, ethyl, propyl, tert-butyl, cyclopentyl, Si(methyl)₃ and Si(phenyl)₃.

Most preferably, Z₁ is any one selected from the group consisting of:

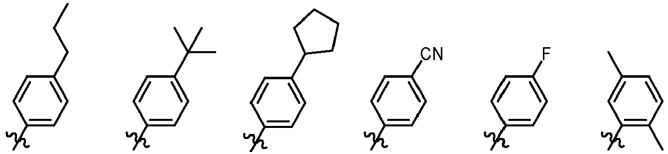

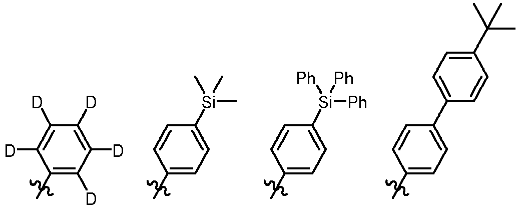

Preferably, L is phenylene unsubstituted or substituted with C₁₋₁₀ alkyl. More preferably, L is phenylene. Most preferably, L is 1,4-phenylene.

Preferably, Z₂ to Z₅ are each independently C₁₋₄ alkyl; C₆₋₂₀ aryl; or C₇₋₂₀ aralkyl, wherein Z₂ to Z₅ are unsubstituted or substituted with C₁₋₁₀ alkyl or Si(C₁₋₁₀ alkyl)₃.

More preferably, Z₂ and Z₃ are each independently C₁₋₄ alkyl; C₆₋₂₀ aryl unsubstituted or substituted with C₁₋₁₀ alkyl; or C₇₋₂₀ aralkyl unsubstituted or substituted with C₁₋₁₀ alkyl. In addition, Z₄ and Z₅ are each independently C₁₋₄ alkyl; or C₆₋₆₀ aryl unsubstituted or substituted with C₁₋₁₀ alkyl.

At this time, 'the L and Z₂; Z₂ and Z₃; or Z₄ and Z₅ may be connected to each other to form a 5-membered-heterocyclic ring' means that L and Z₂, Z₂ and Z₃, or Z₄ and Z₅ may be connected to each other to form a 5-membered-heterocyclic ring containing O, S, N, or Si, which is a hetero atom of X.

For example, in Chemical Formula 1, when L is 1,4-phenylene, Z₂ is phenyl, and X is O, the above-mentioned compound means not only a structure represented by the following Chemical Formula 3a but also a structure of the following Chemical Formula 3b wherein L and Z₂ are connected to each other to form a 5-membered-heterocyclic ring containing heteroatom O of X.

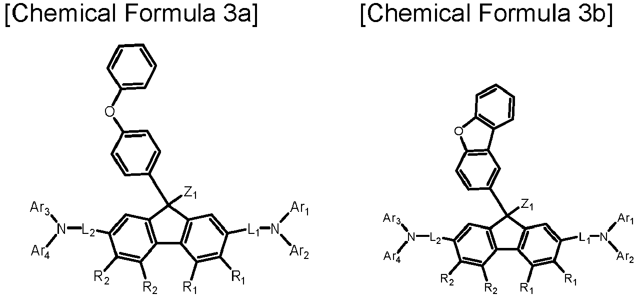

Further, for example, in Chemical Formula 1, when Z₂ groups are phenyl, and X is N(phenyl), that is, when both Z₂ and Z₃ are phenyl, the above-mentioned compound means not only a structure represented by the following Chemical Formula 3c, but also a structure of the following Chemical Formula 3d wherein Z₂ and Z₃ are connected to each other to form a 5-membered-heterocyclic ring containing heteroatom N of X.

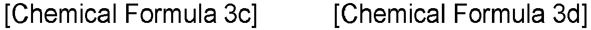

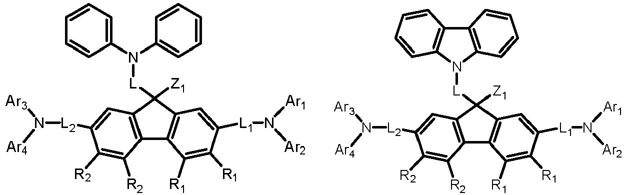

Further, for example, in Chemical Formula 1, when both Z₄ and Z₅ are ethyl, the above-mentioned compound means not only a structure represented by the following Chemical Formula 3e, but also a structure of the following Chemical Formula 3f wherein Z₄ and Z₅ are connected to each other to form a 5-membered-heterocyclic ring containing heteroatom Si of X.

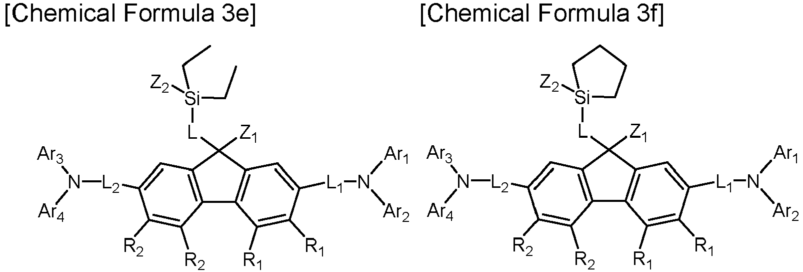

For example, L-X-Z₂ is any one selected from the group consisting of: 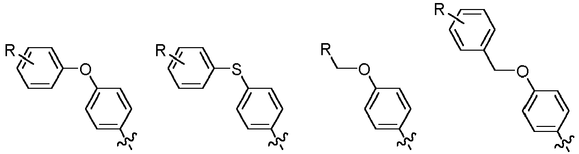 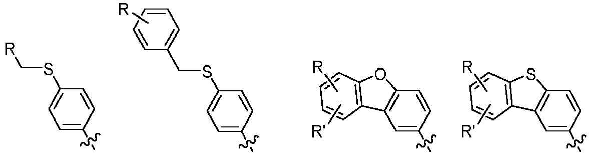 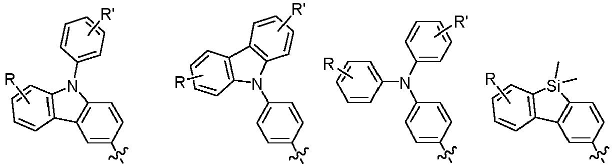 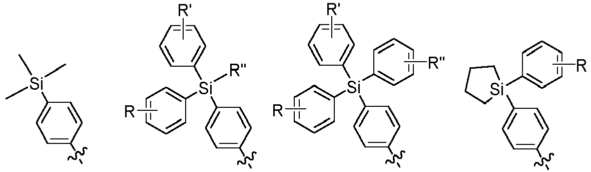 wherein,
R, R' and R" are each independently hydrogen, methyl, tert-butyl, or Si(methyl)₃.

Preferably, L₁ and L₂ are a single bond.

Preferably, Ar₁ to Ar₄ are each independently phenyl, naphthyl, biphenylyl, or dibenzofuranyl,
wherein Ar₁ to Ar₄ are unsubstituted, or substituted with 1 to 5 substituents each independently selected from the group consisting of deuterium, halogen, C₁₋₁₀ alkyl and -Si(C₁₋₁₀ alkyl)₃.

More preferably, Ar₁ to Ar₄ are each independently phenyl, naphthyl, biphenylyl, or dibenzofuranyl,
wherein Ar₁ to Ar₄ are unsubstituted, or substituted with 1 to 5 substituents each independently selected from the group consisting of deuterium, halogen, methyl, tert-butyl and Si(methyl)₃.

Most preferably, Ar₁ to Ar₄ are each independently one selected from the group consisting of:

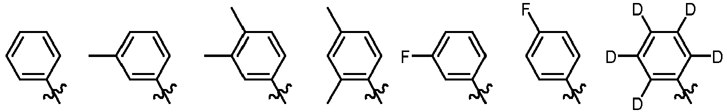

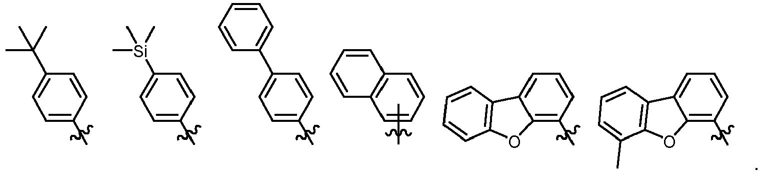

At this time, preferably, Ar₁ and Ar₄ are identical to each other, and Ar₂ and Ar₃ are identical to each other.

Further, preferably, the compound represented by Chemical Formula 1 is represented by any one of the following Chemical Formulas 2-1 to 2-10: 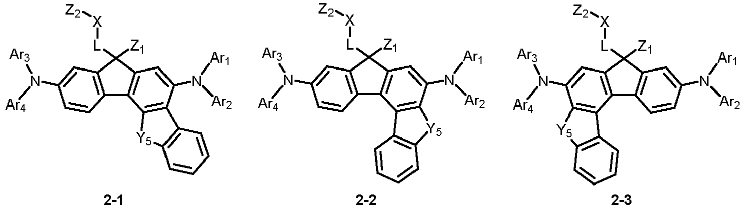 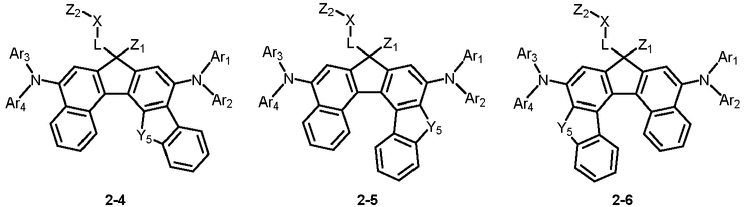 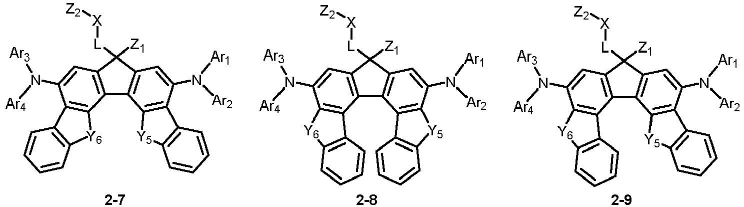 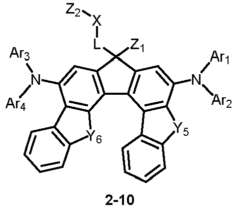 in the Chemical Formulas 2-1 to 2-10,
L, X, Z₁, Z₂ and Ar₁ to Ar₄ are as described above, and
Y₅ and Y₆ are each independently O, S, C(methyl)₂, or Si(methyl)₂.

More preferably, the compound represented by Chemical Formula 1 may be selected from the group consisting of: 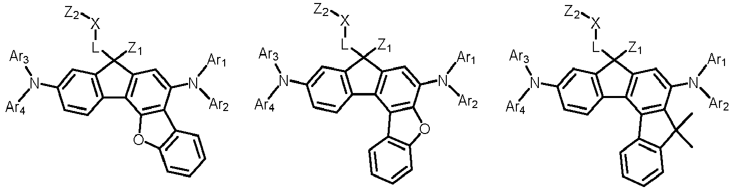 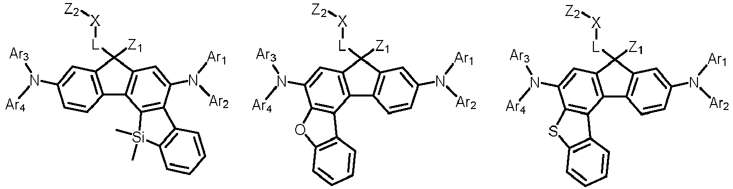 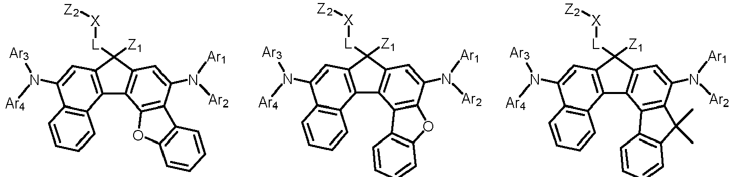 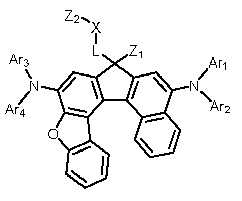 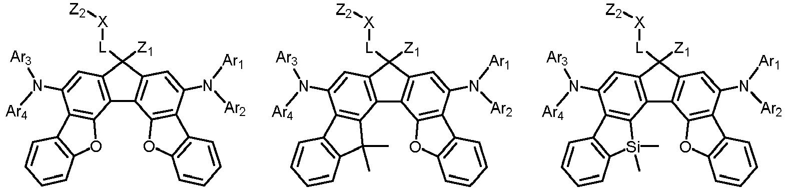 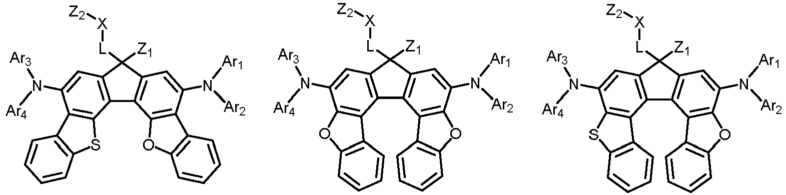 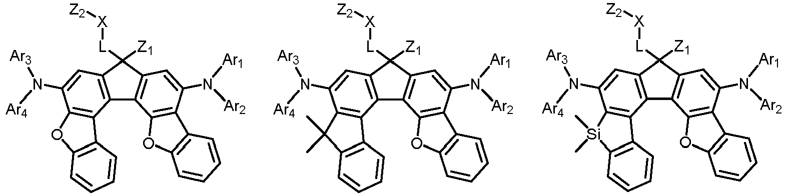 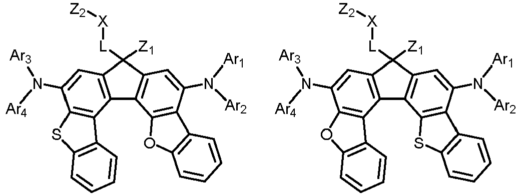 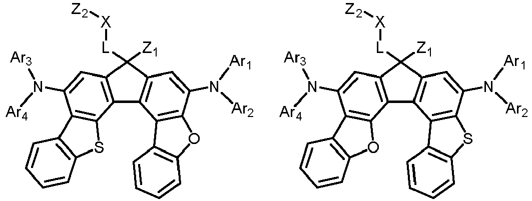 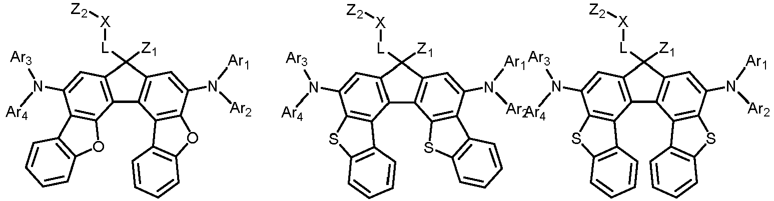 wherein, the definition of each substituent is the same as described above.

For example, the compound represented by Chemical Formula 1 is any one selected from the group consisting of the following compounds:

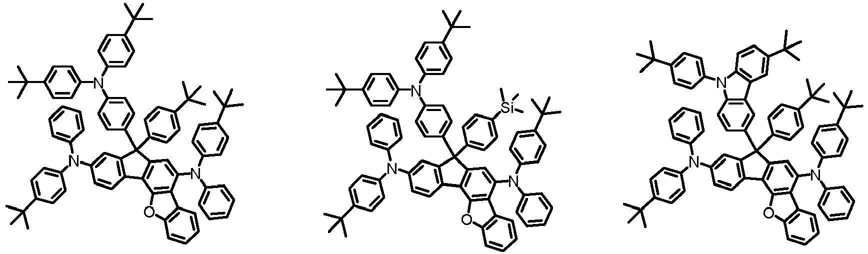

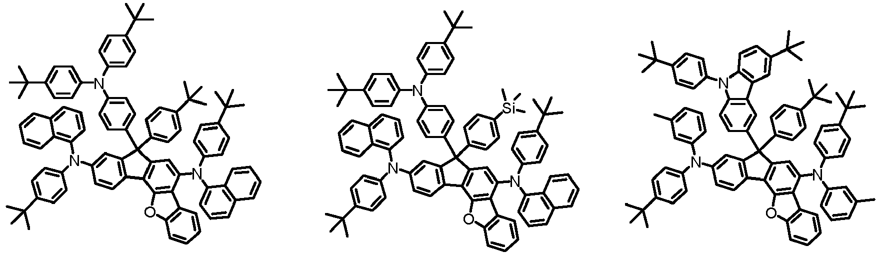

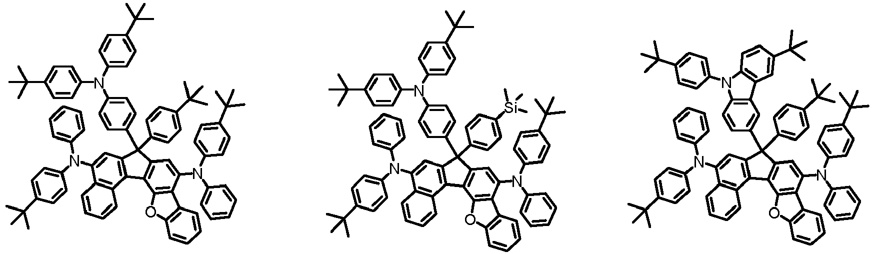

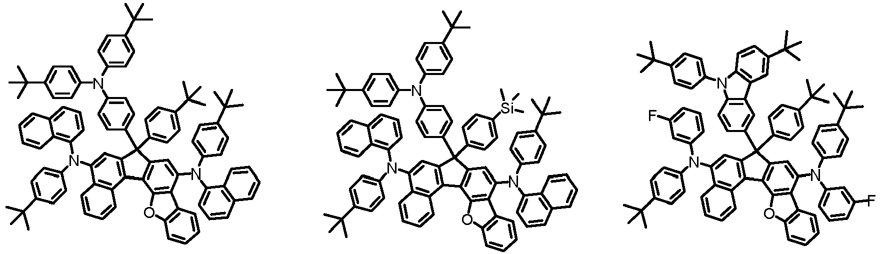

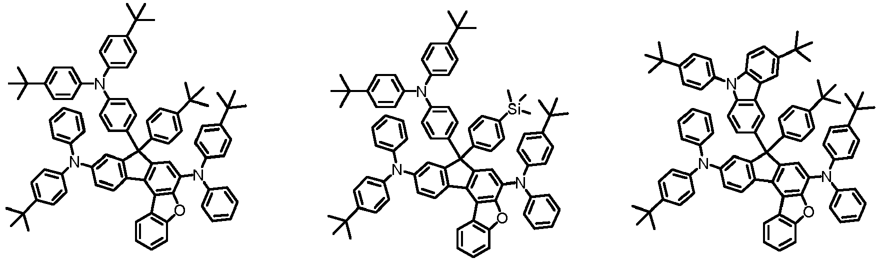

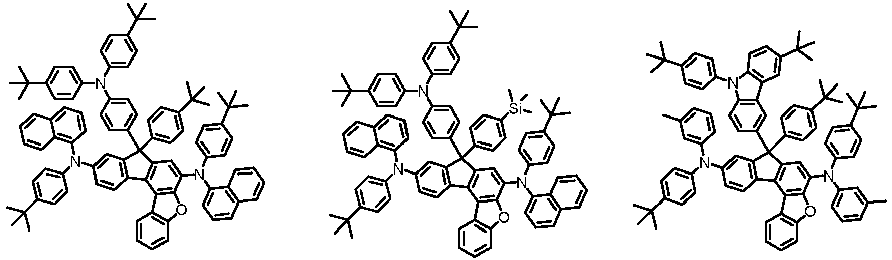

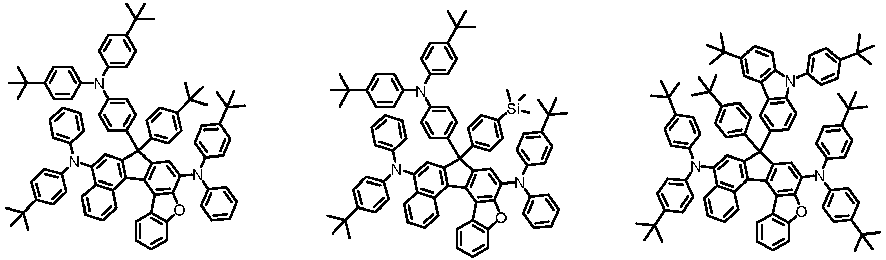

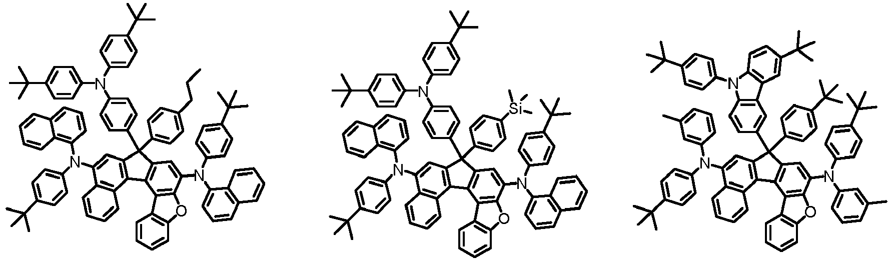

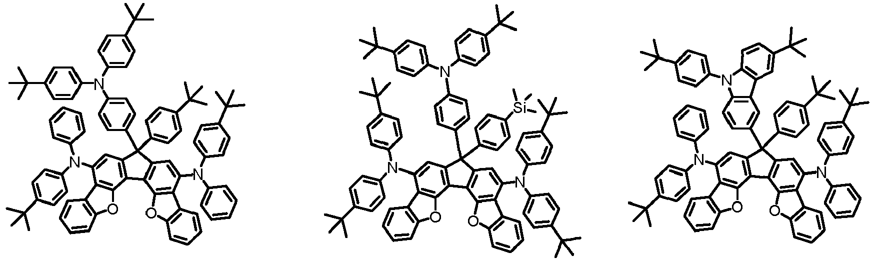

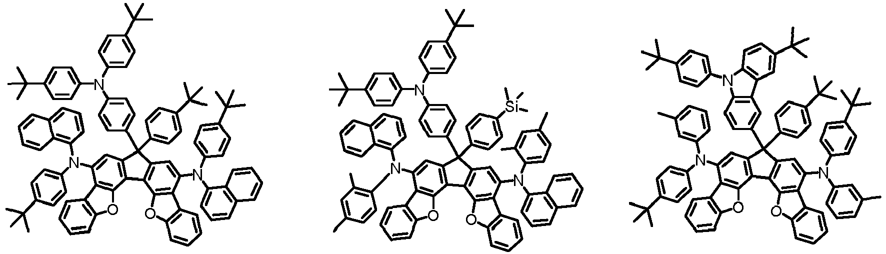

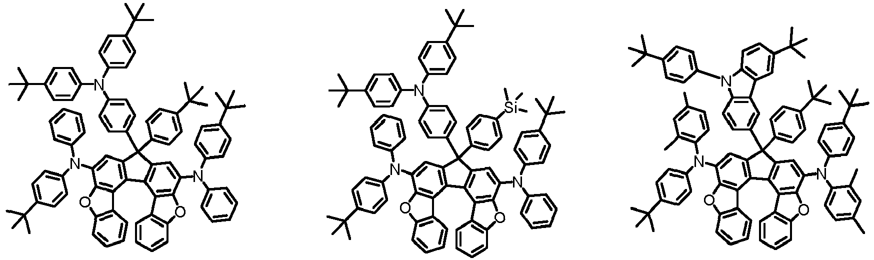

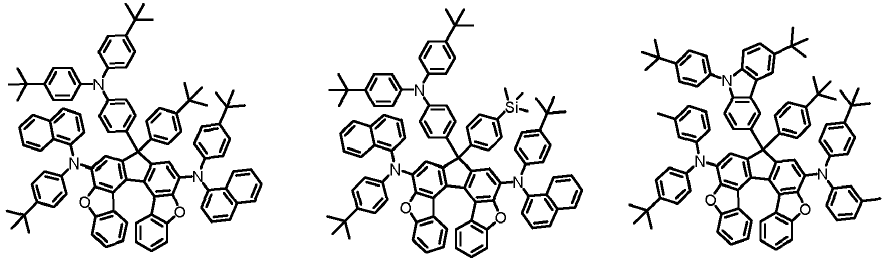

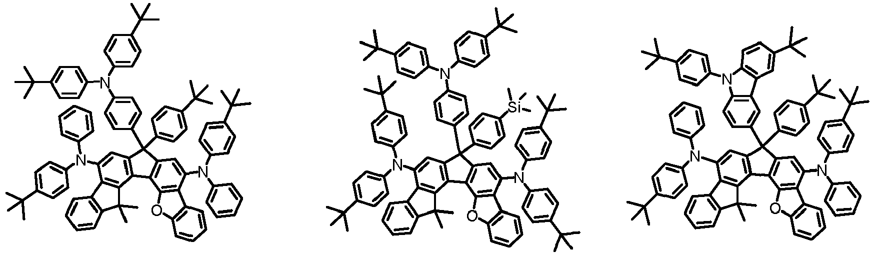

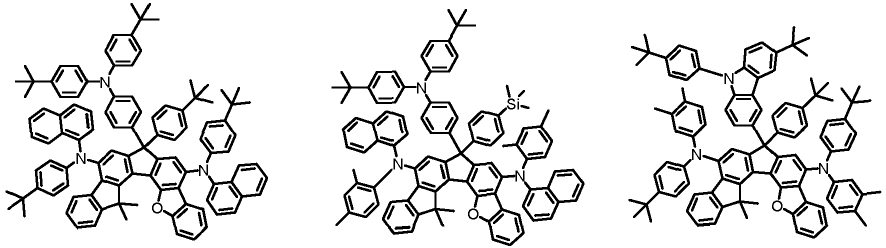

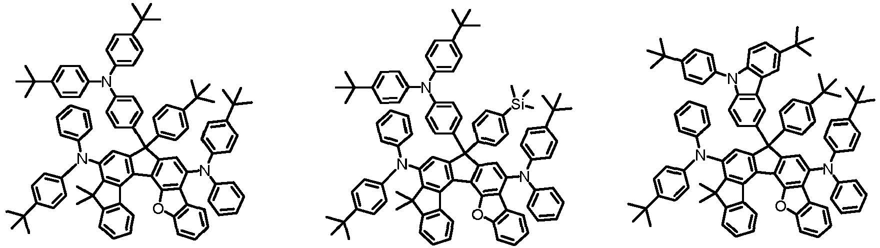

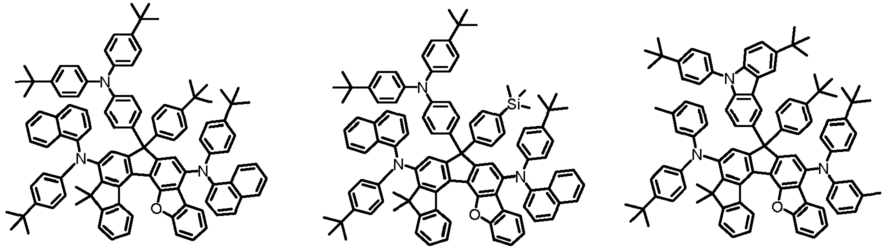

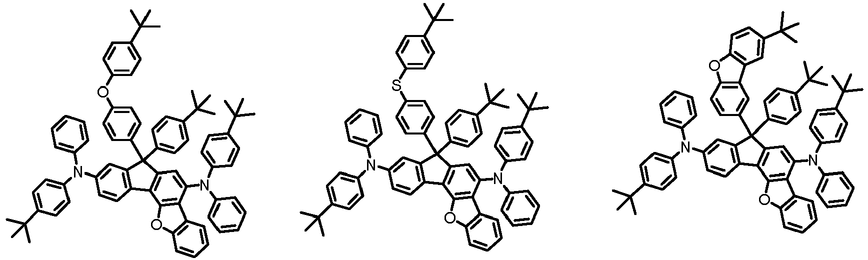

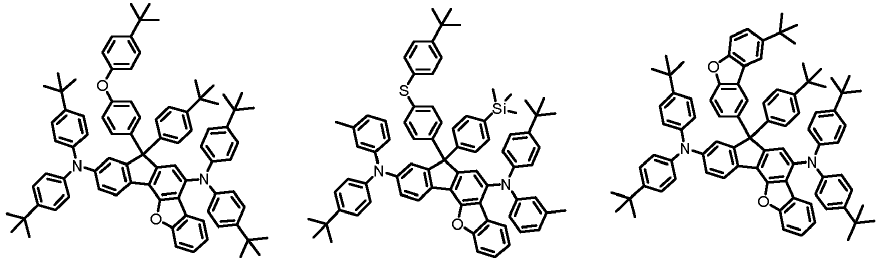

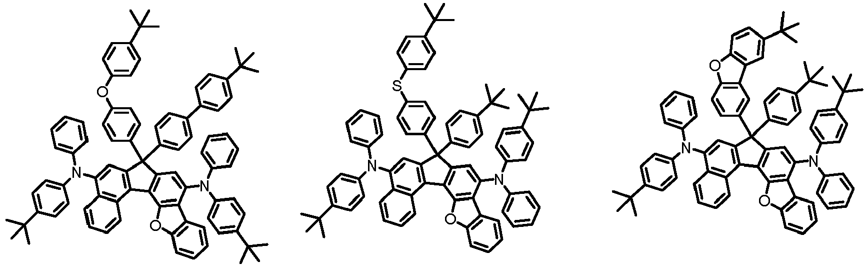

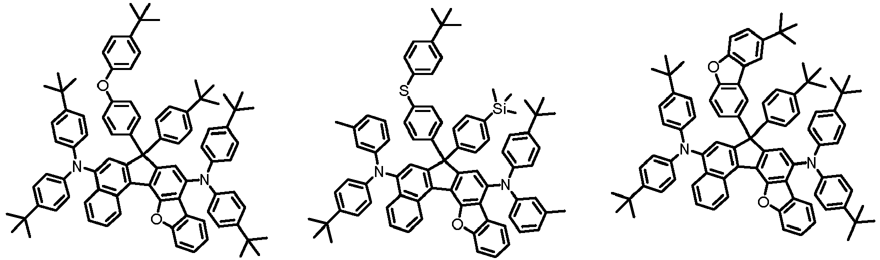

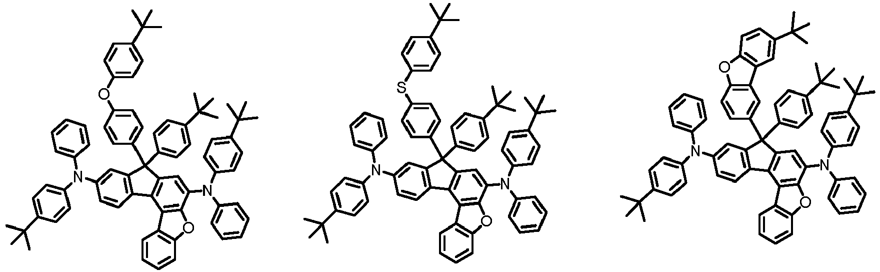

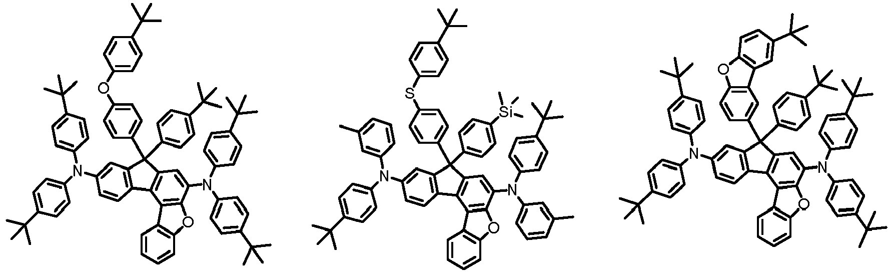

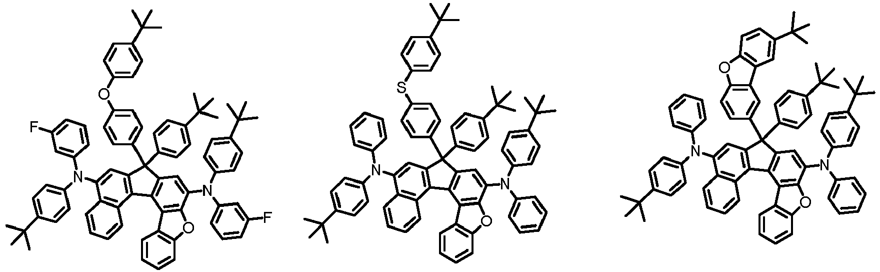

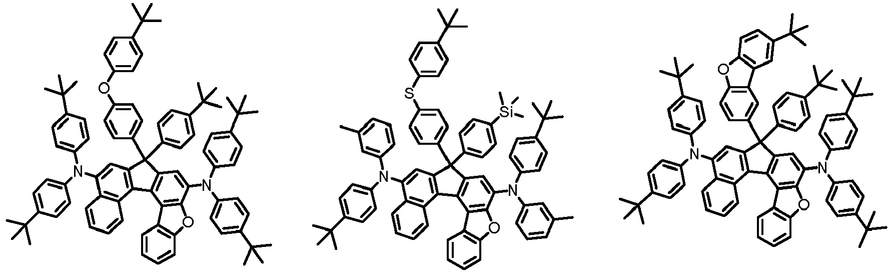

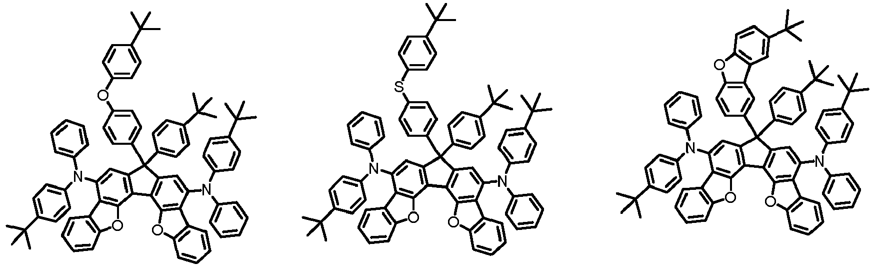

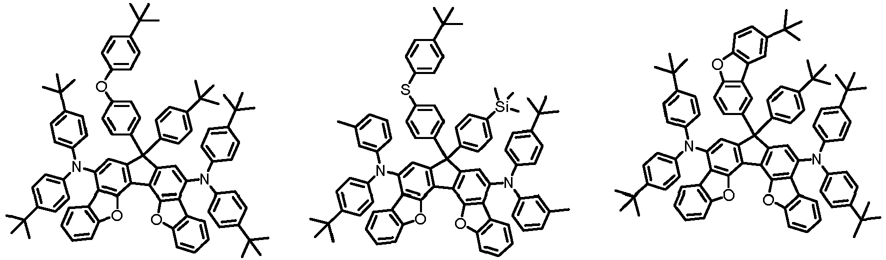

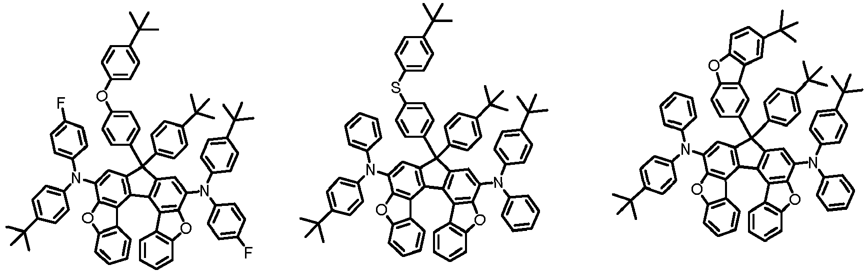

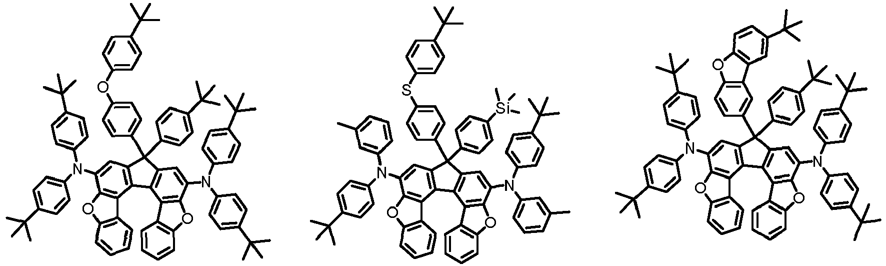

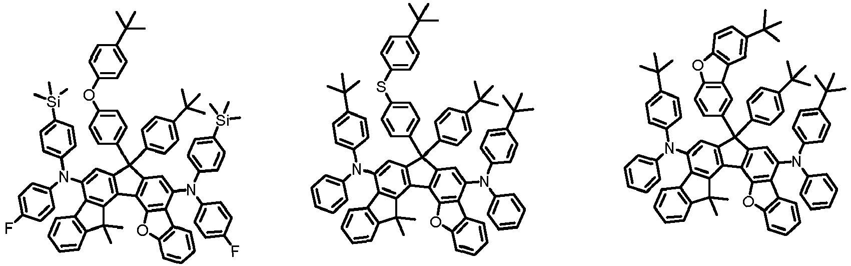

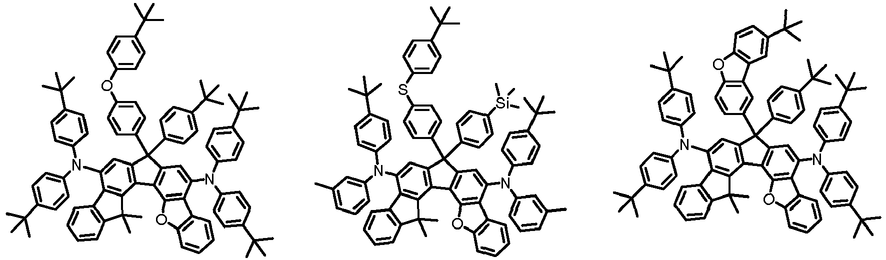

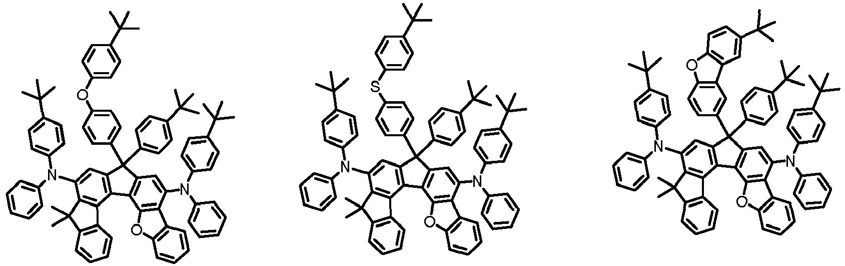

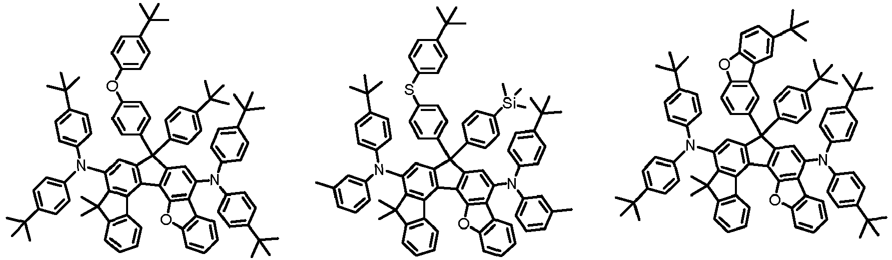

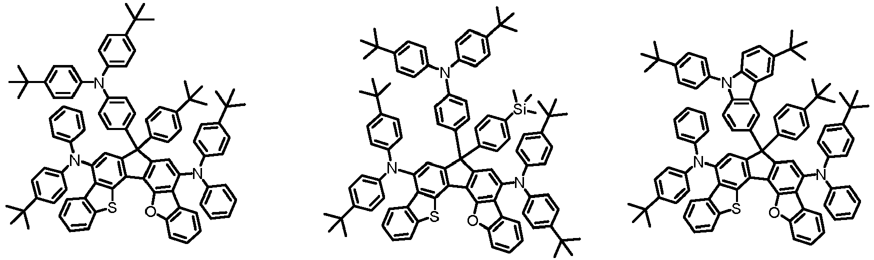

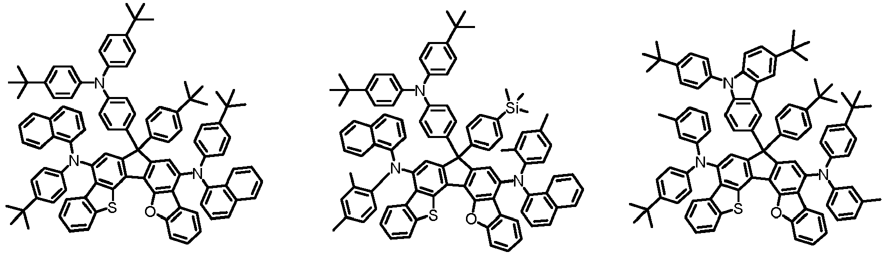

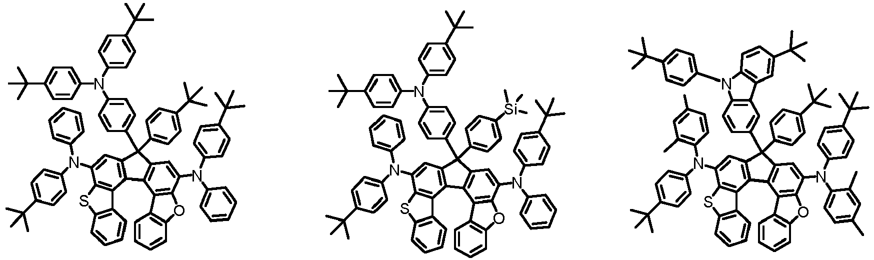

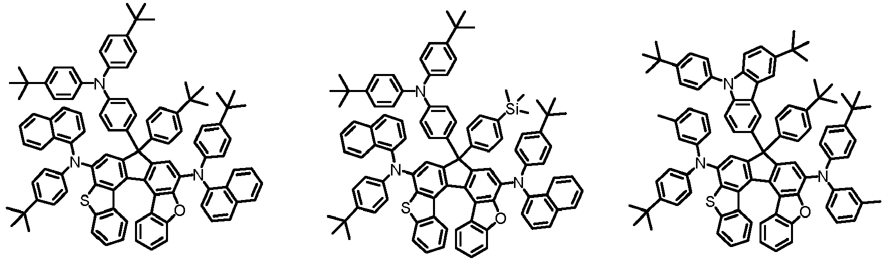

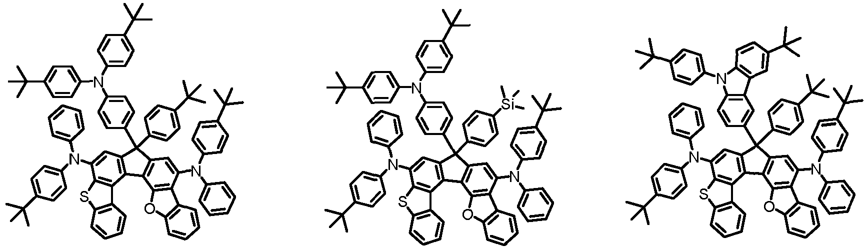

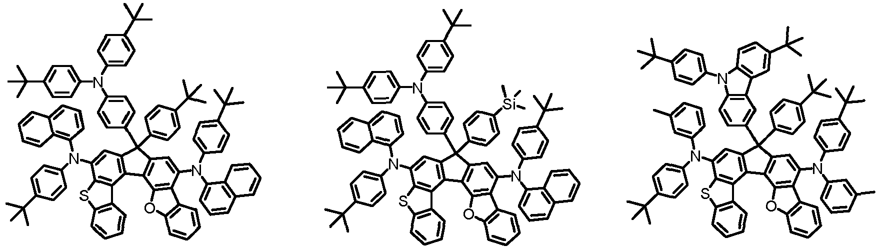

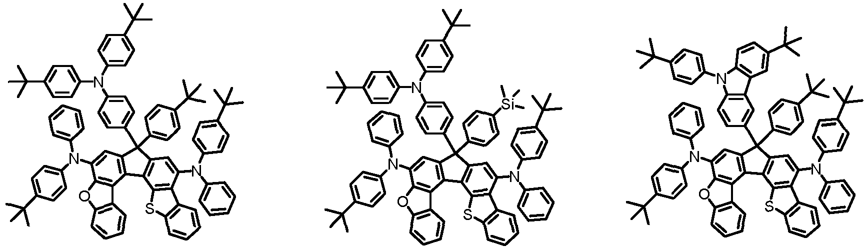

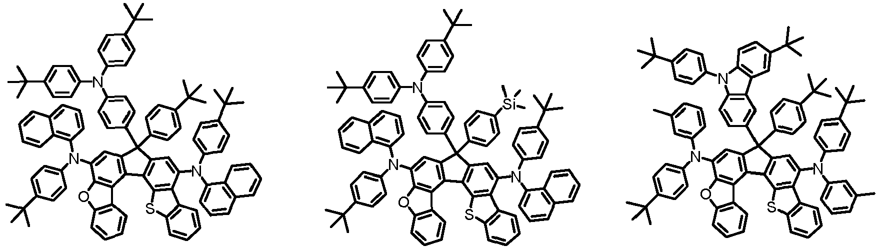

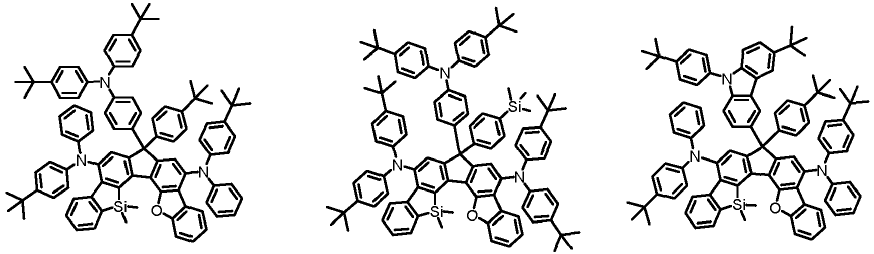

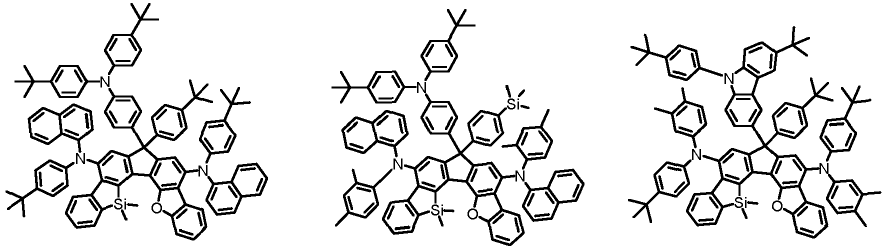

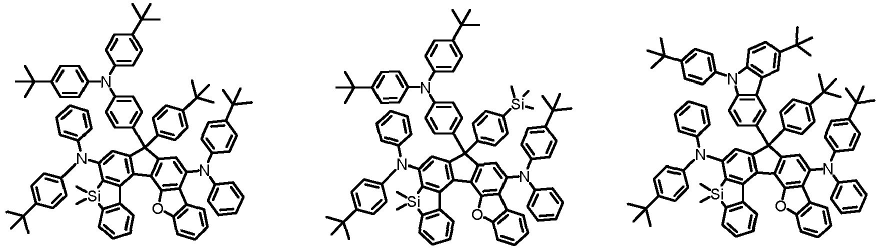

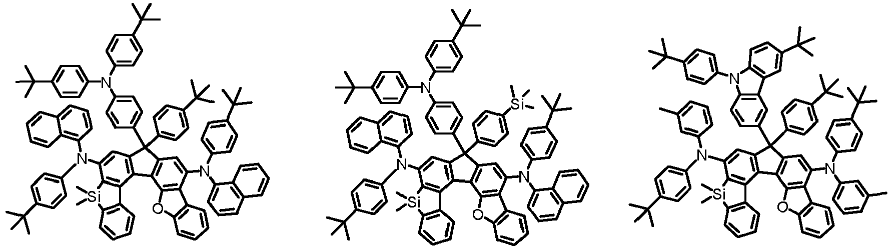

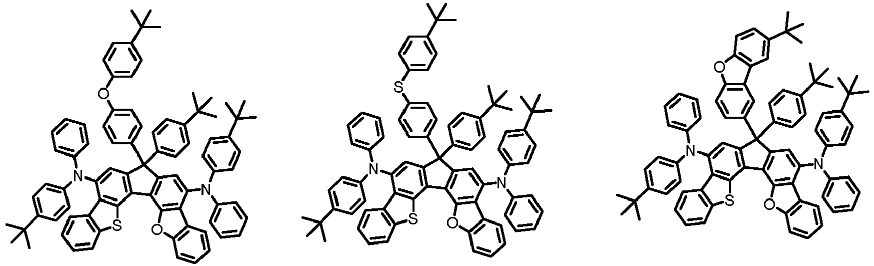

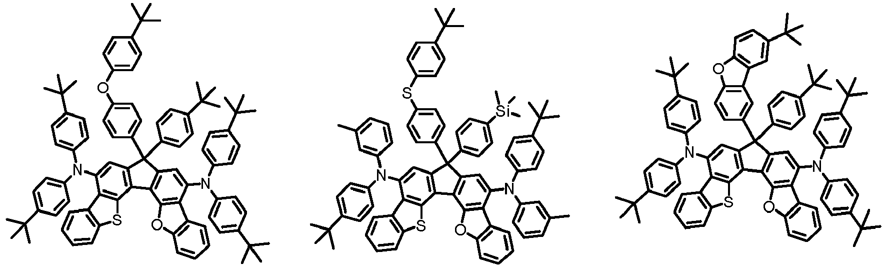

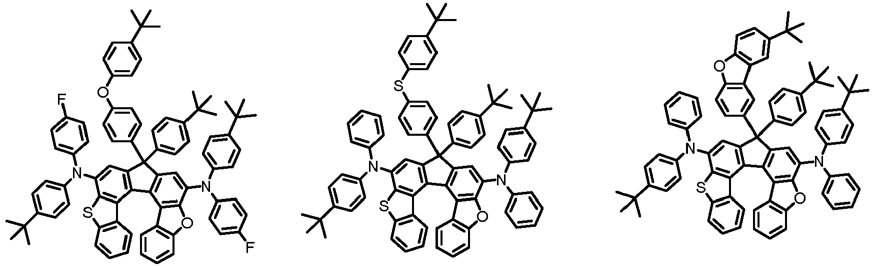

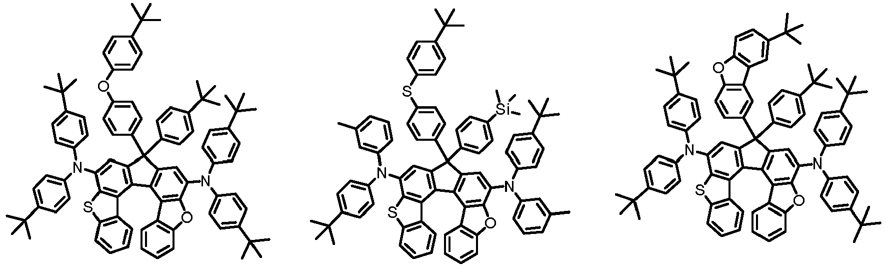

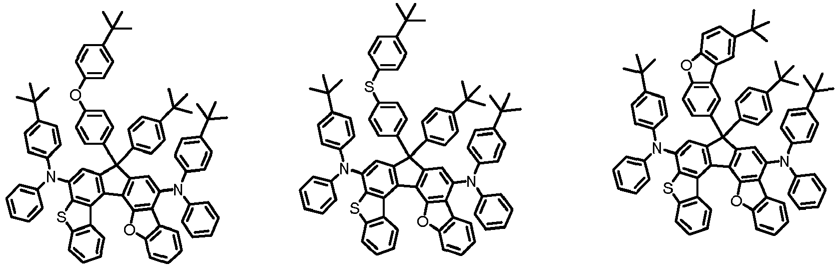

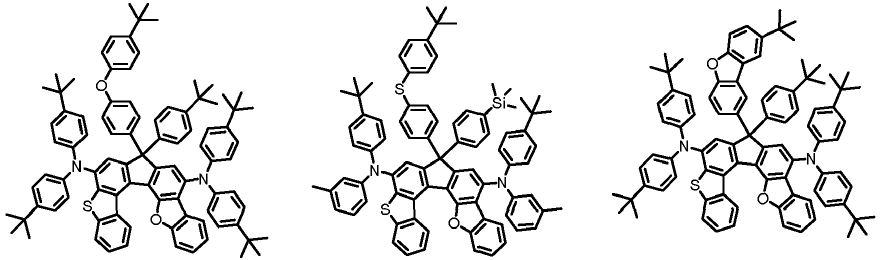

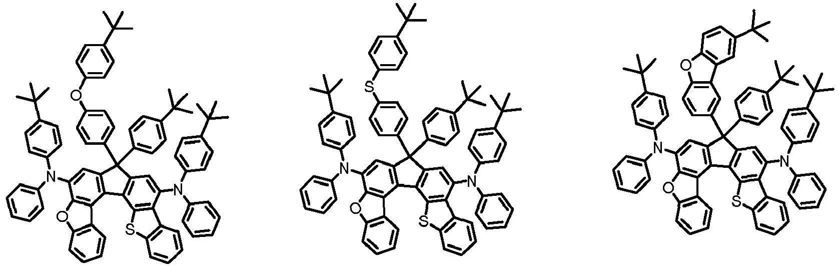

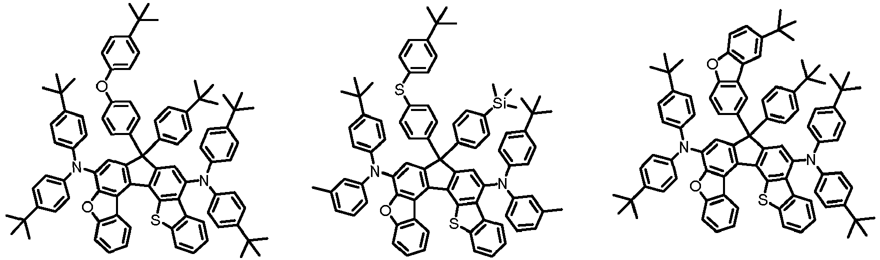

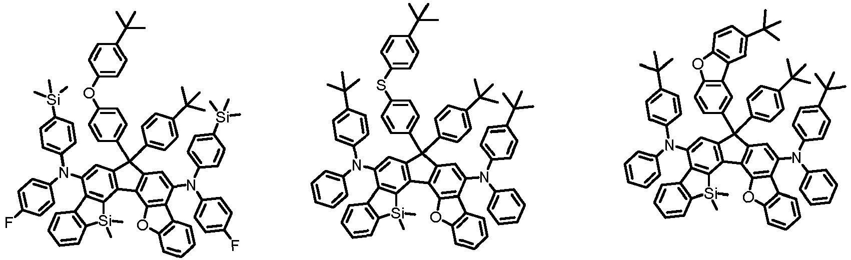

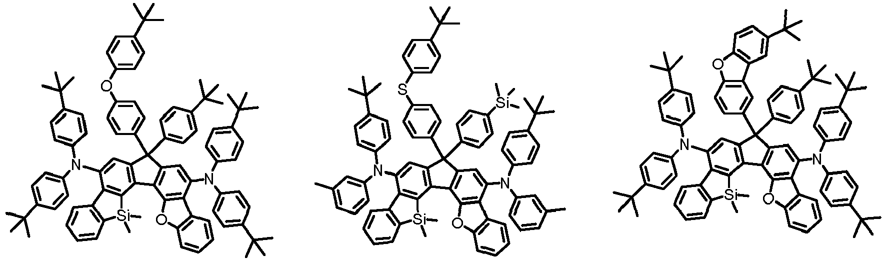

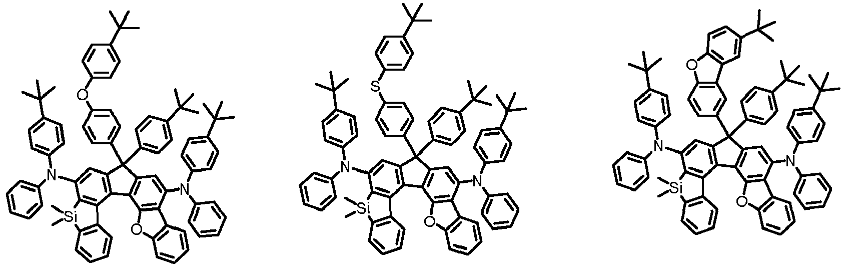

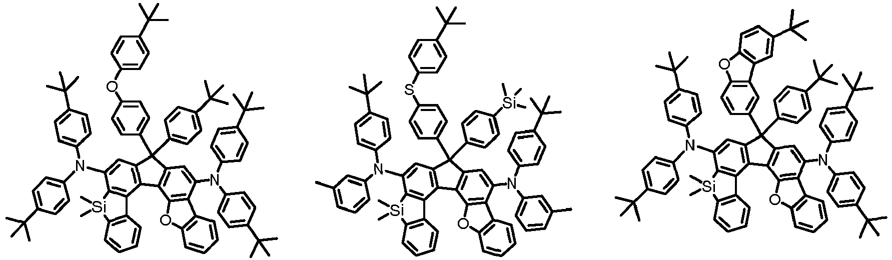

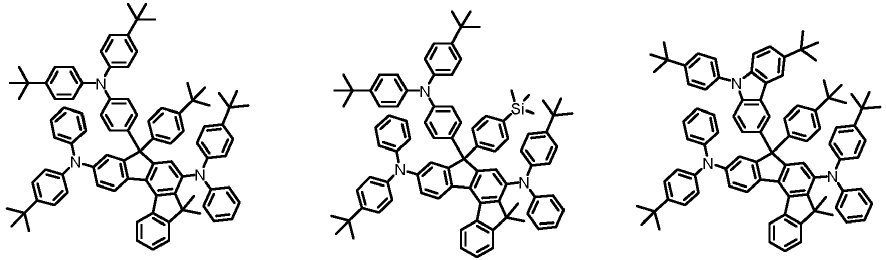

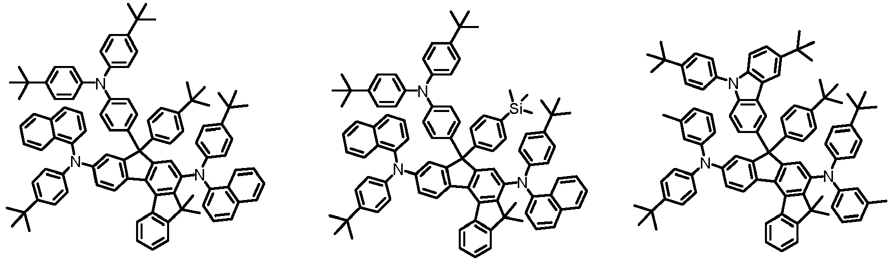

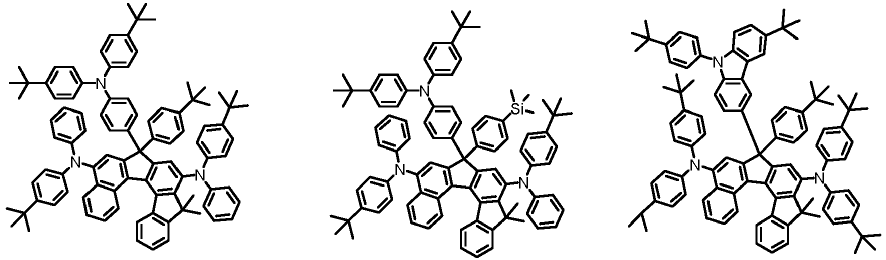

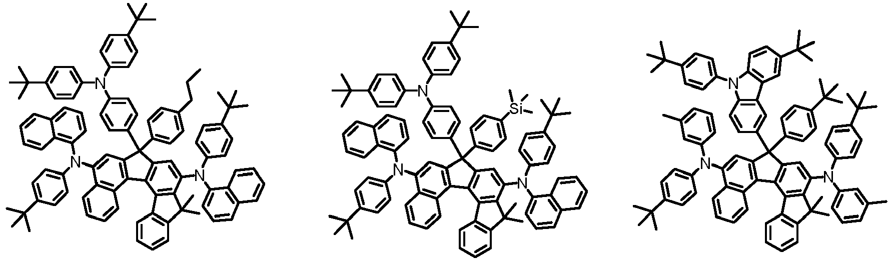

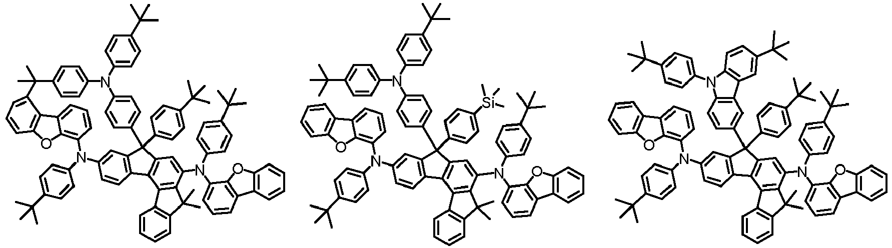

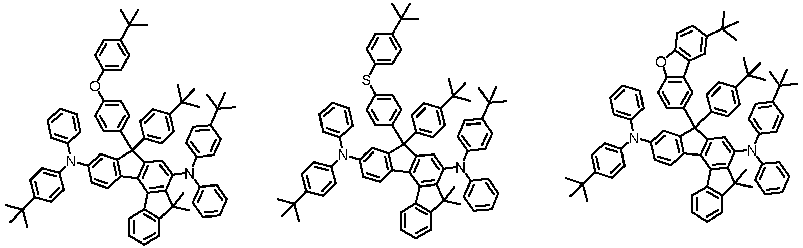

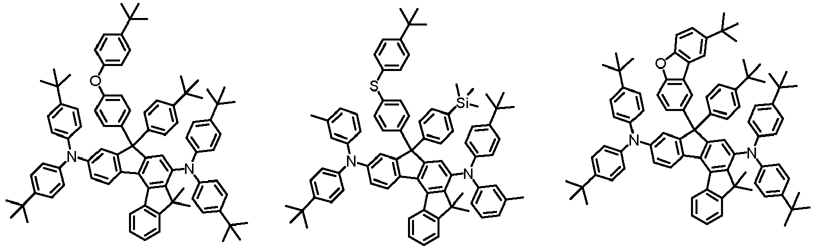

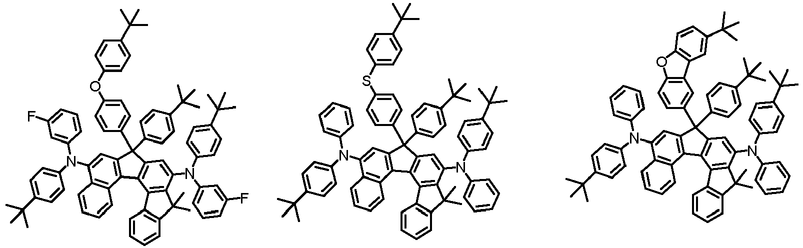

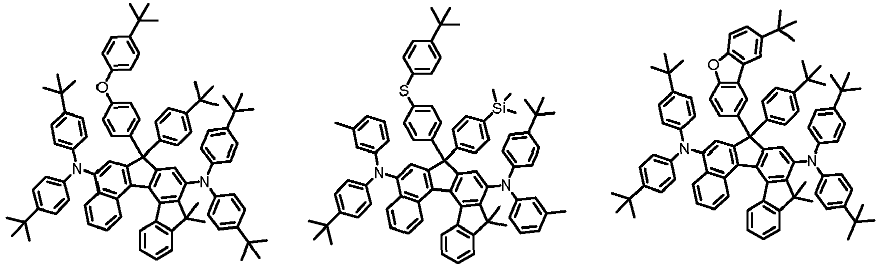

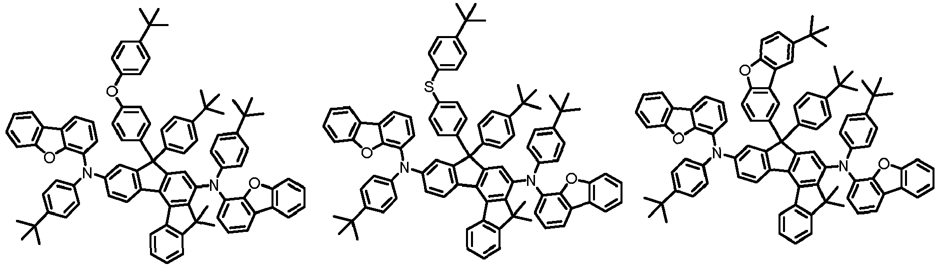

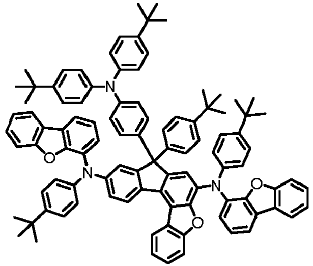

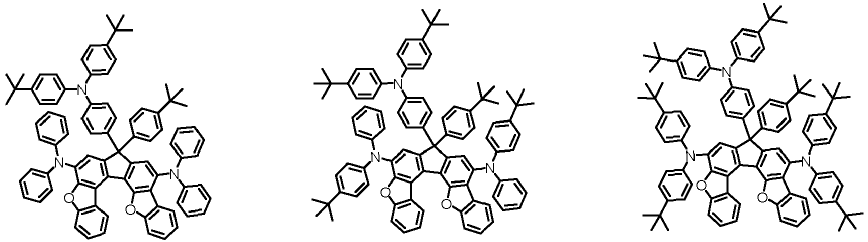

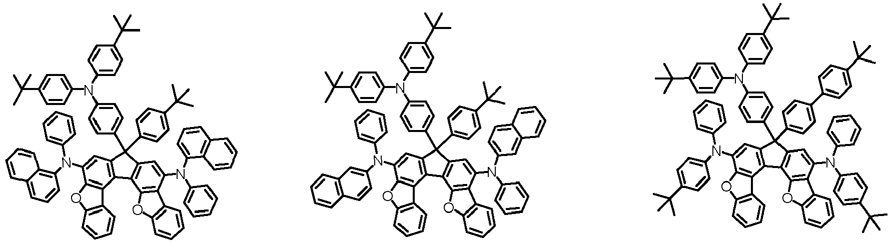

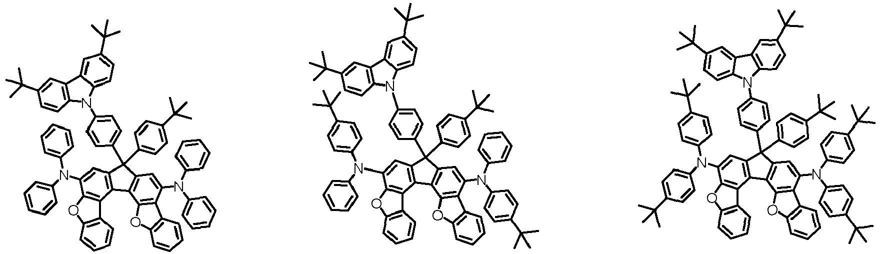

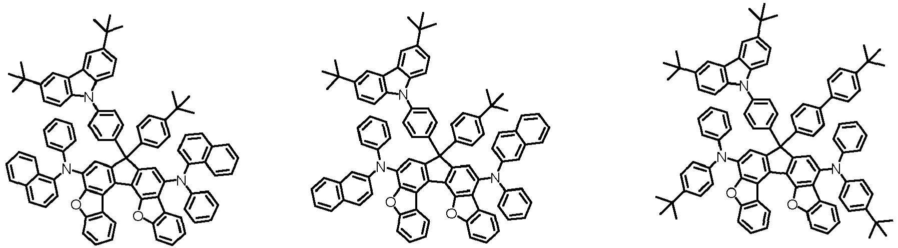

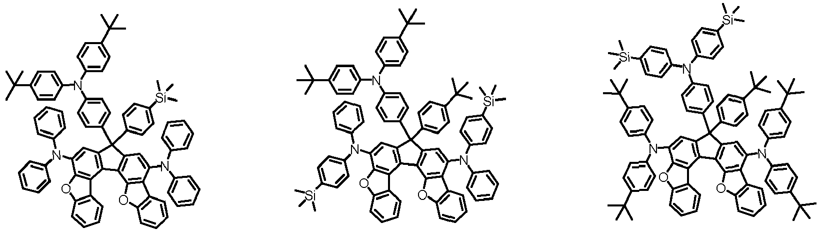

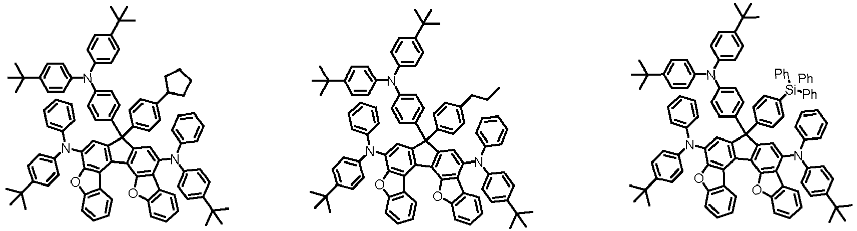

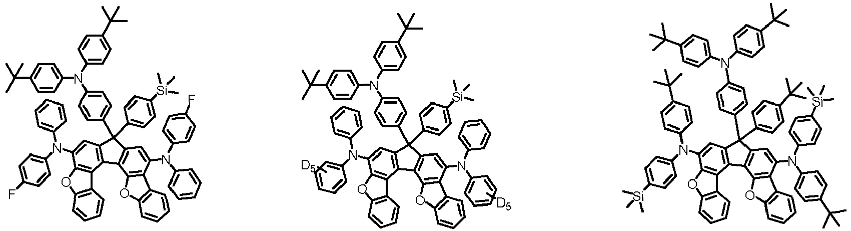

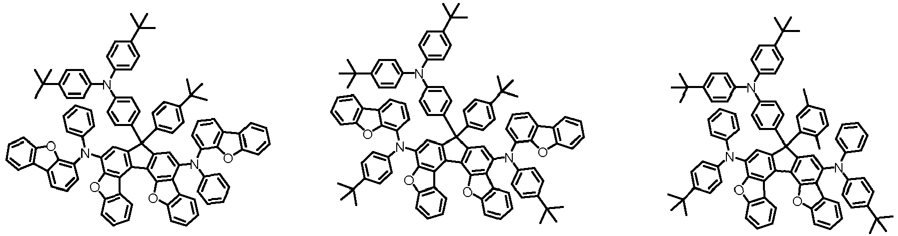

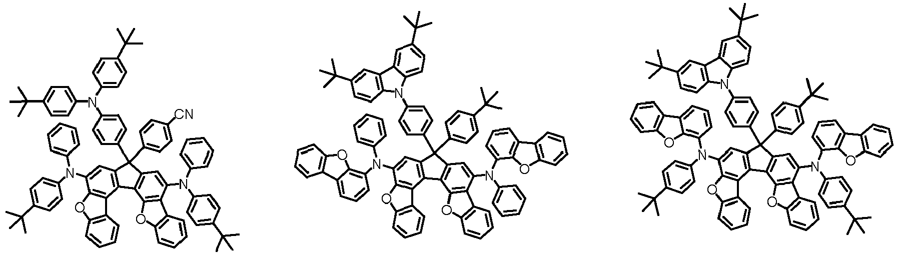

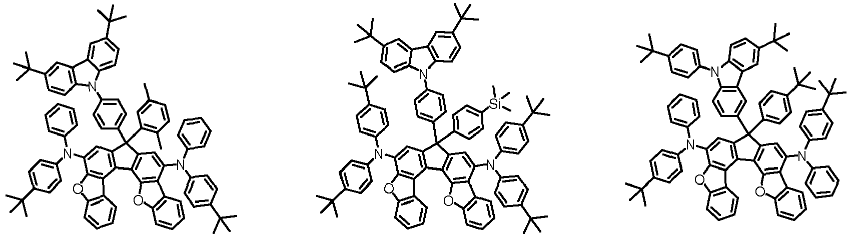

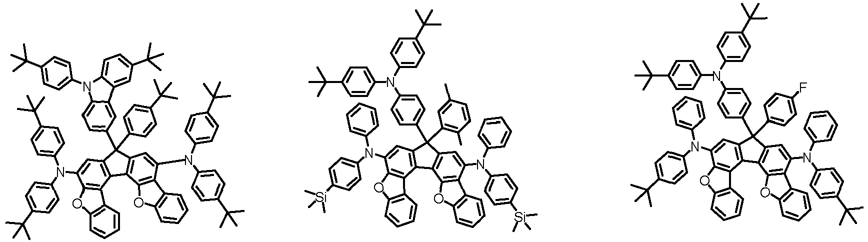

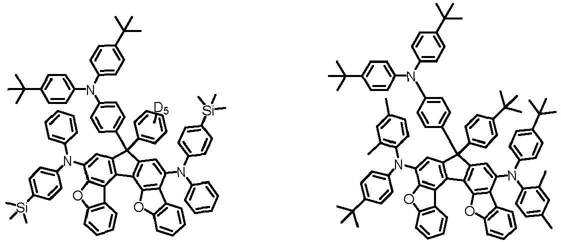

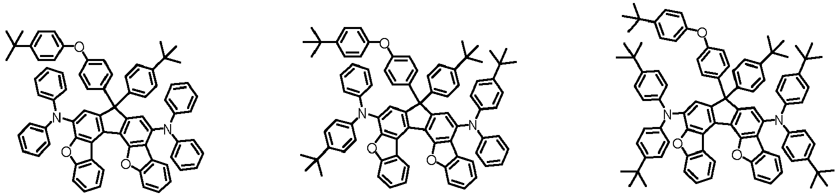

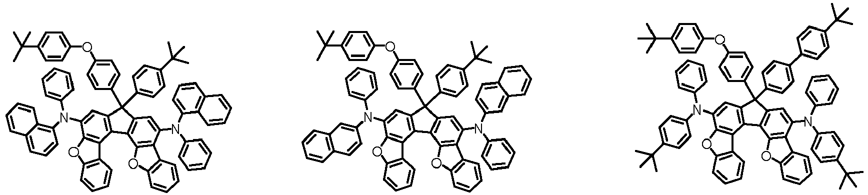

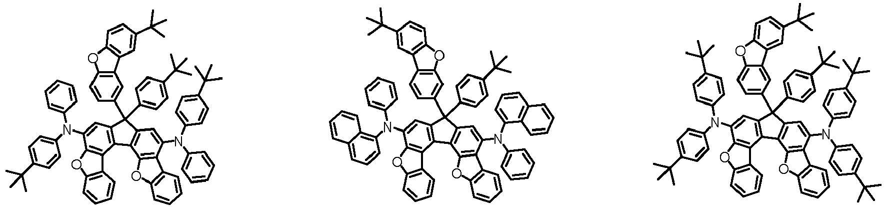

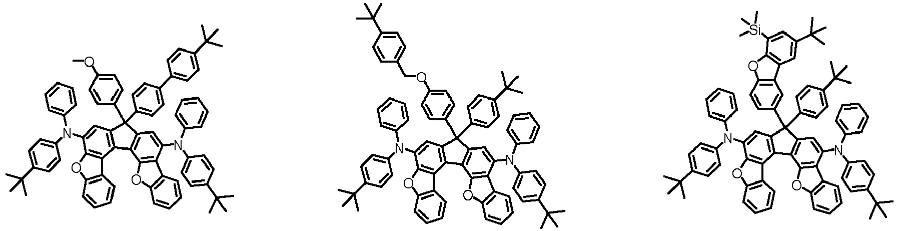

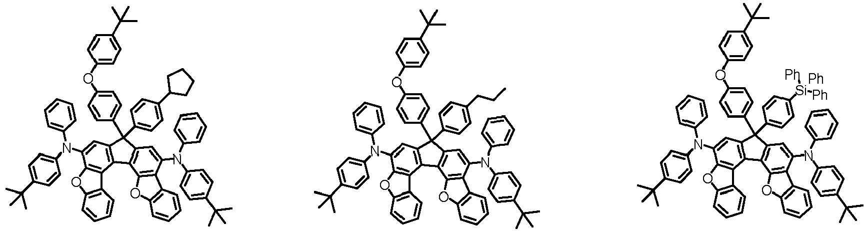

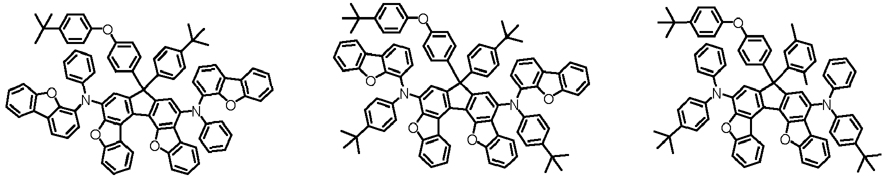

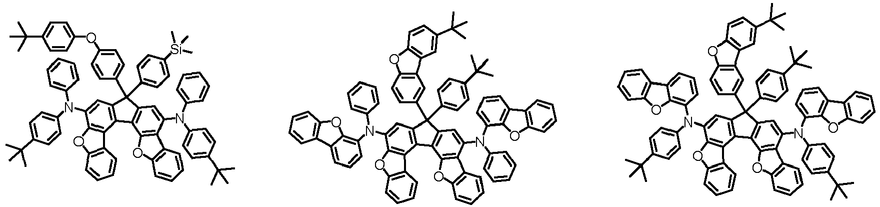

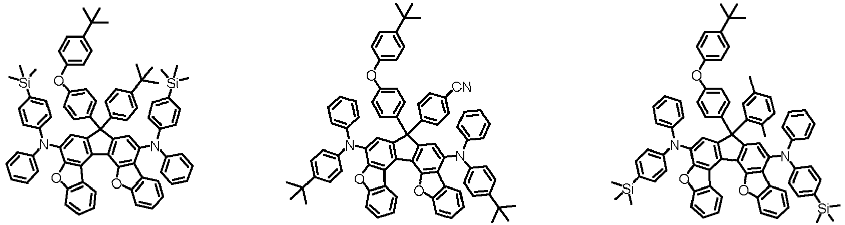

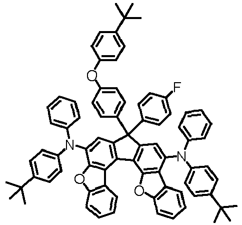

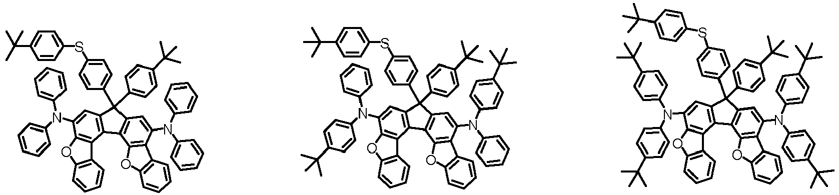

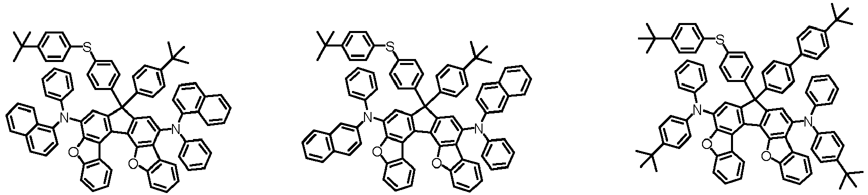

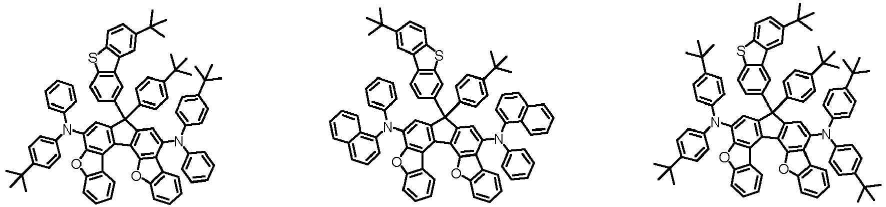

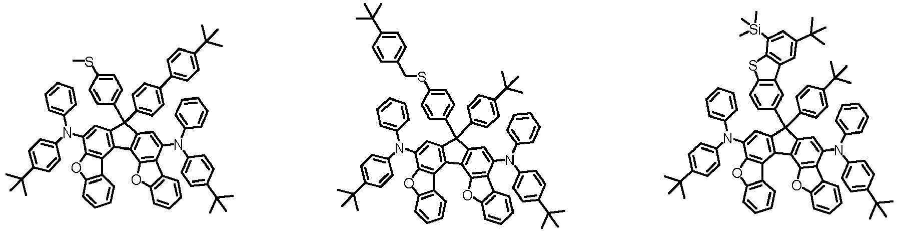

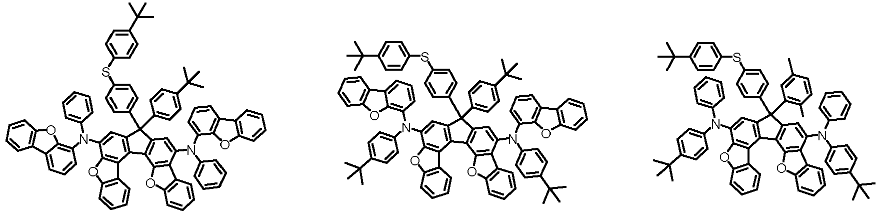

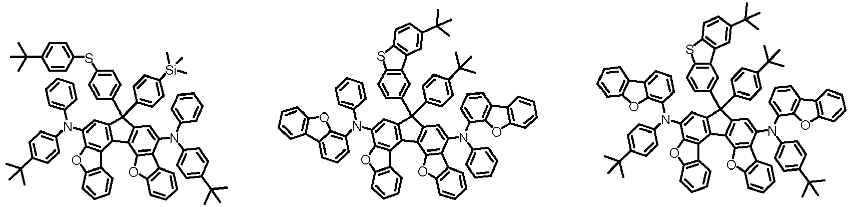

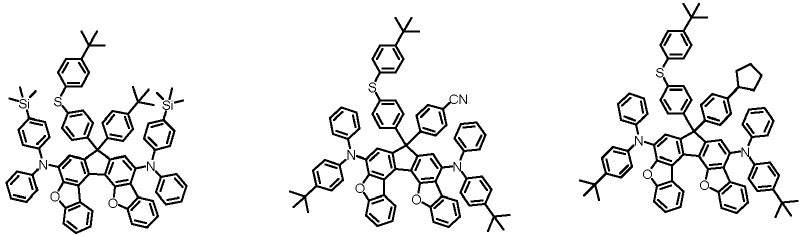

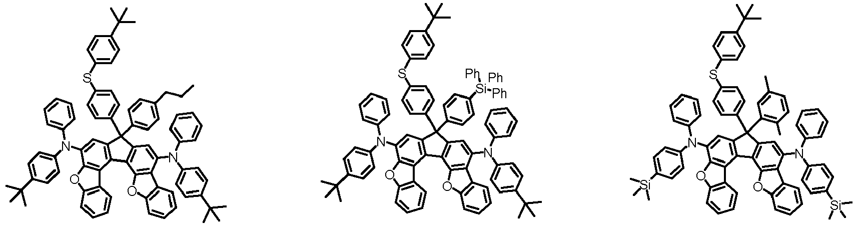

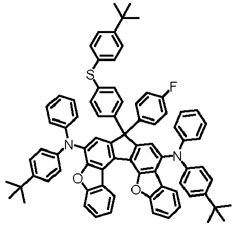

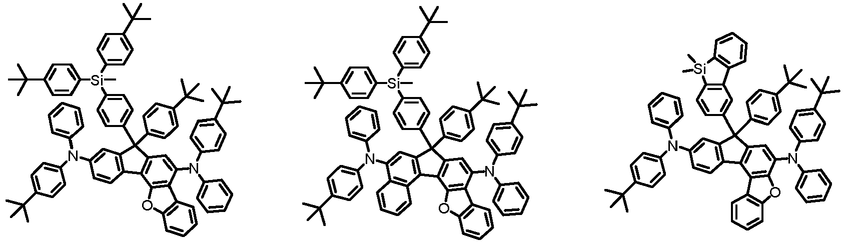

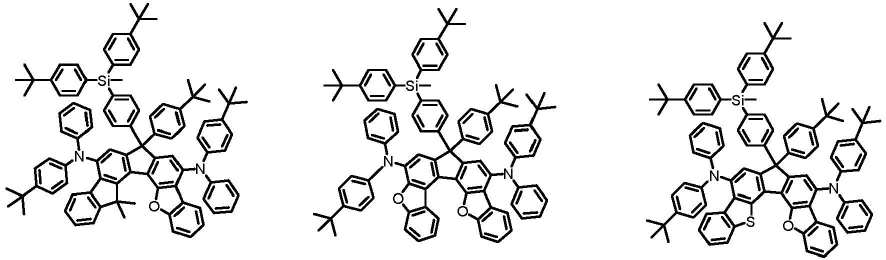

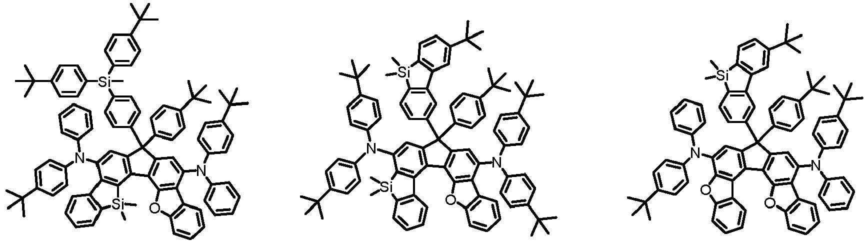

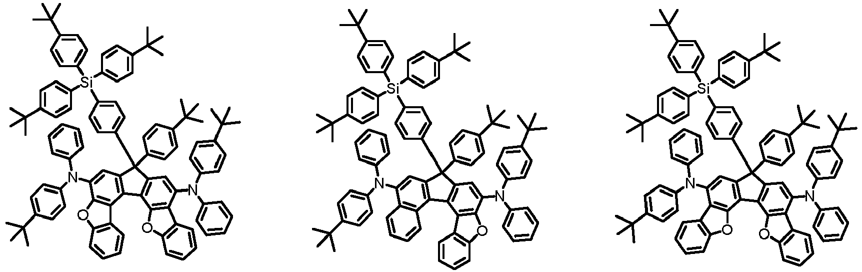

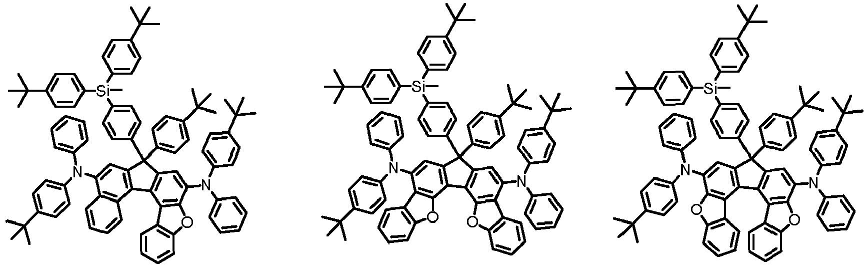

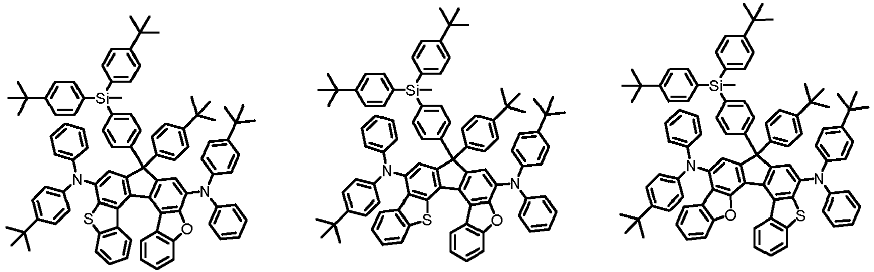

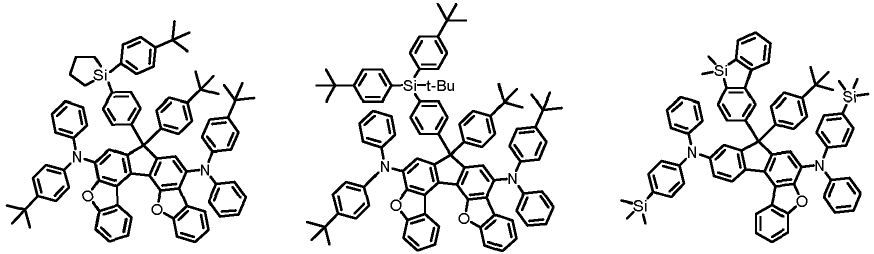

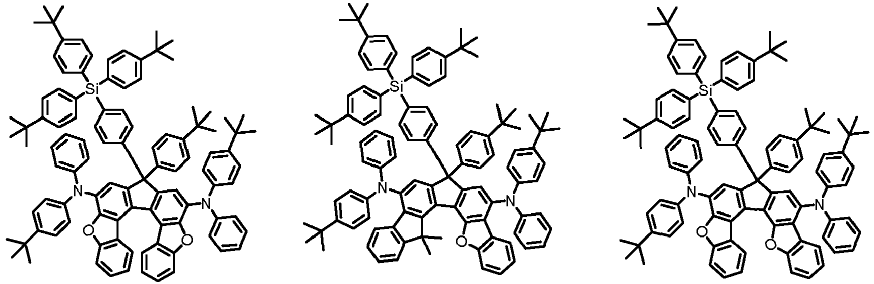

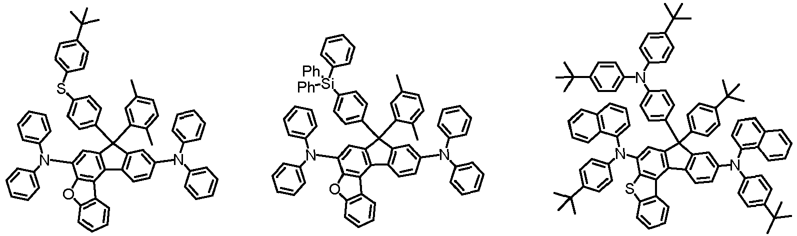

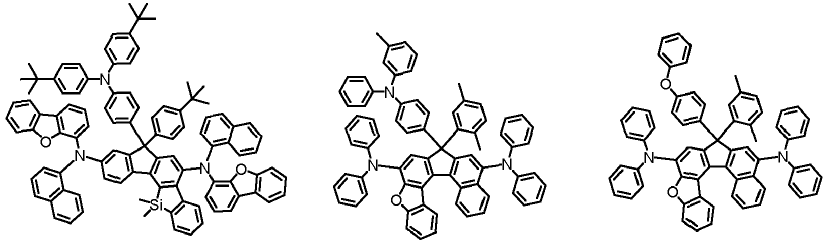

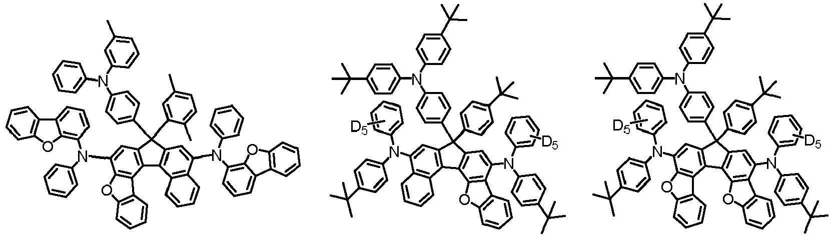

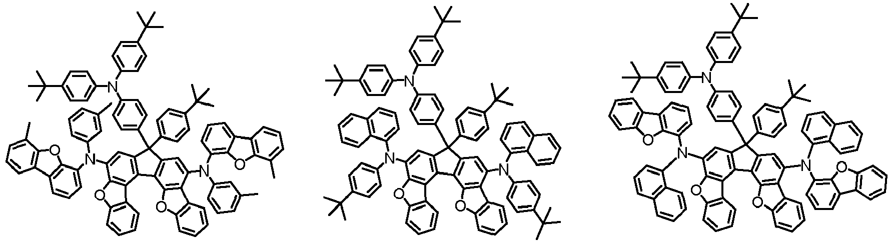

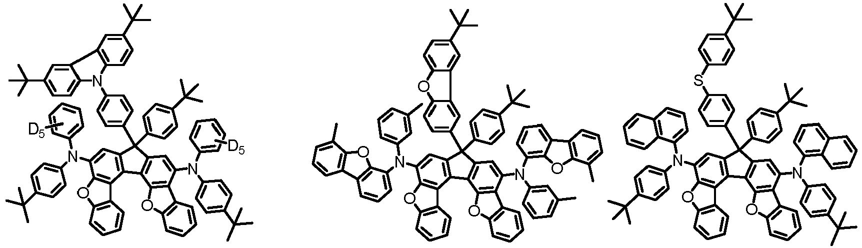

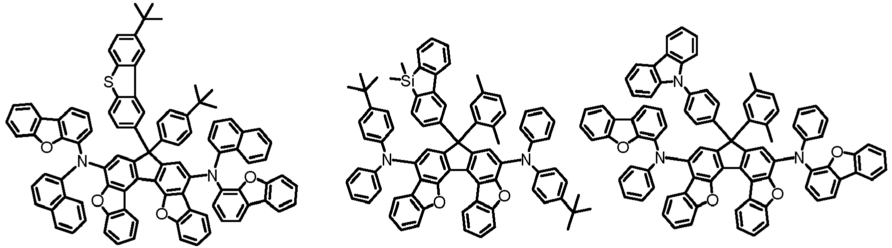

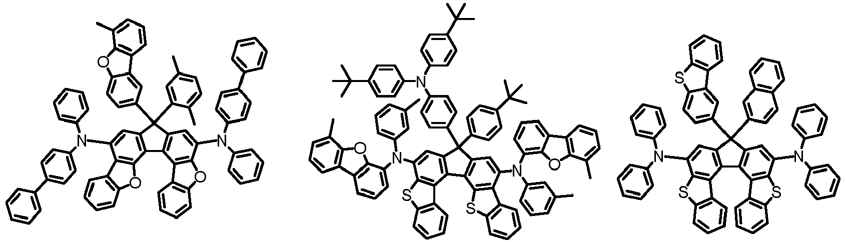

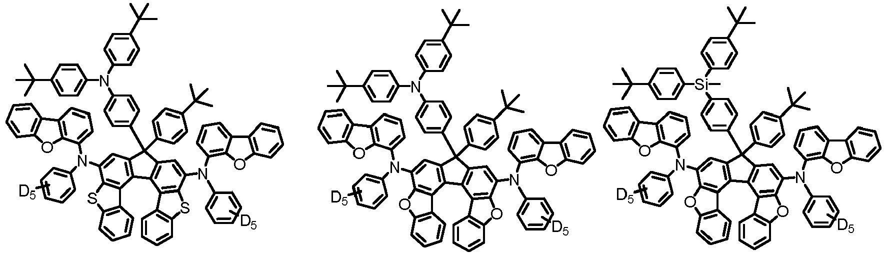

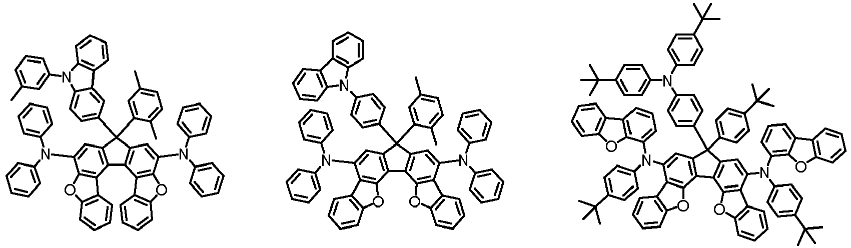

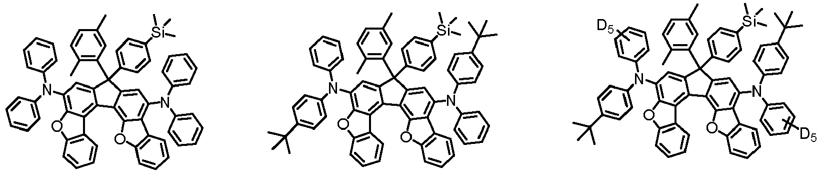

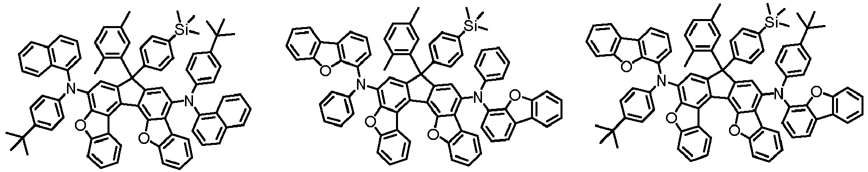

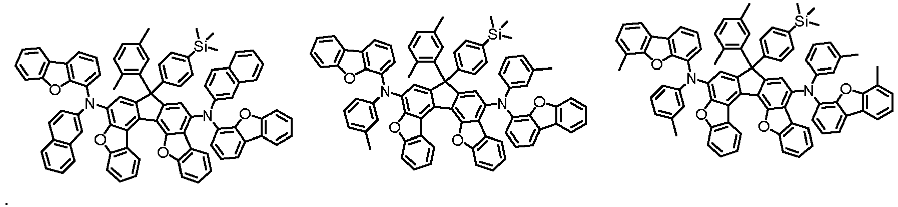

At this time, among the substituents of the compounds,

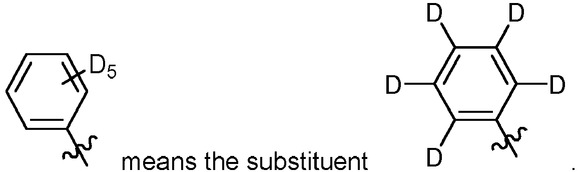

Meanwhile, the compound represented by Chemical Formula 1 may be prepared, for example, by a method as shown in the following Reaction Scheme 1. The preparation method may be more specifically described in the Preparation Examples described below. 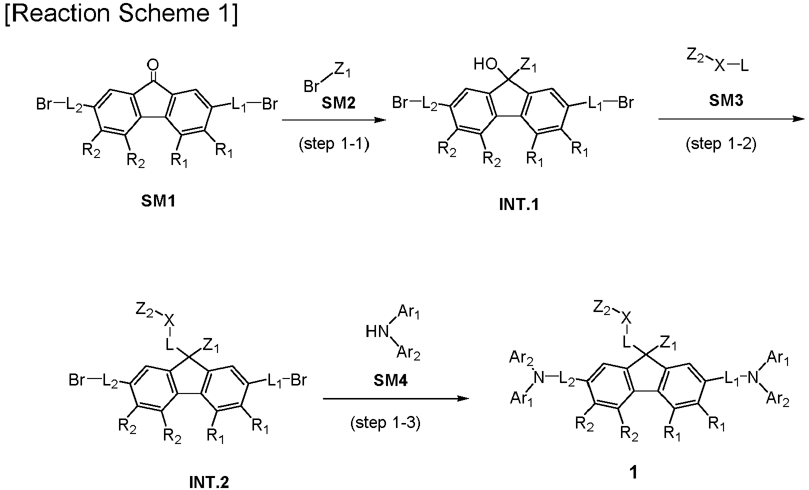 in the Reaction Schemes 1, the definition of each substituent is the same as described above.

Step 1-1 is a step of introducing a hydroxy group and a compound SM2 into the starting material SM1 by a reduction reaction of a carbonyl group by a strong base to prepare an intermediate compound INT.1. Step 1-2 is a step of introducing a compound SM3 into the hydroxyl group of the intermediate compound INT.1 by Friedel-Crafts type electrophilic substitution reactions to prepare an intermediate compound INT.2. Step 1-3 is a step of reacting the intermediate compound INT.2 with a secondary amine SM4 to prepare the compound represented by Chemical Formula 1. Such a preparation method will be more specifically described in the Preparation Examples described hereinafter.

### (Coating composition)

Meanwhile, the compound according to the present disclosure can form an organic material layer, particularly a light emitting layer, of an organic light emitting device by a solution process. Specifically, the above-mentioned compound can be used as a dopant material of the light emitting layer. For this purpose, the present disclosure provides a coating composition comprising the above-mentioned compound according to the present disclosure and a solvent.

The solvent is not particularly limited as long as it is a solvent capable of dissolving or dispersing the compound according to the present disclosure. Examples of the solvent may include chlorine-based solvents such as chloroform, methylene chloride, 1,2-dichloroethane, 1,1,2-trichloroethane, chlorobenzene and o-dichlorobenzene; ether-based solvents such as tetrahydrofuran and dioxane; aromatic hydrocarbon-based solvents such as toluene, xylene, trimethylbenzene and mesitylene; aliphatic hydrocarbon-based solvents such as cyclohexane, methylcyclohexane, n-pentane, n-hexane, n-heptane, n-octane, n-nonane and n-decane; ketone-based solvents such as acetone, methyl ethyl ketone, and cyclohexanone; ester-based solvents such as ethyl acetate, butyl acetate and ethyl cellosolve acetate; polyalcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin and 1,2-hexanediol, and derivatives thereof; alcohol-based solvents such as methanol, ethanol, propanol, isopropanol and cyclohexanol; sulfoxide-based solvents such as dimethyl sulfoxide; amide-based solvents such as N-methyl-2-pyrrolidone and N,N-dimethylformamide; benzoate-based solvents such as butyl benzoate and methyl-2-methoxybenzoate; tetraline; 3-phenoxy-toluene, and the like. In addition, the above-mentioned solvents may be used singly or in combination of two or more solvents. Preferably, toluene can be used as the solvent.

Further, the coating composition may further include the compound used as the host material, and the details concerning the compound used as a host material will be described hereinafter.

Further, the viscosity (25°C) of the coating composition is preferably 1 cP to 10 cP, and coating is easy within the above range. Further, in the coating composition, the concentration of the compound according to the present disclosure is preferably 0.1 wt/v% to 20 wt/v%.

Further, the solubility (wt%) of the coating composition in a solvent is 2.5 wt% to 10 wt% based on the solvent toluene, and thus the coating composition comprising the compound represented by Chemical Formula 1 is suitable for use in a solution process.

In another embodiment of the present disclosure, there is provided a method for forming a light emitting layer using the above-mentioned coating composition. Specifically, the method includes the steps of: coating the above-mentioned coating composition according to the present disclosure on the anode or on the hole transport layer formed on the anode by a solution process; and heat treating the coated coating composition.

The solution process uses the coating composition according to the present disclosure, and refers to spin coating, dip coating, doctor blading, inkjet printing, screen printing, spray method, roll coating, and the like, but is not limited thereto.

The heat treatment temperature in the heat treatment is preferably from 150 to 230°C. In another embodiment, a heat treatment time may be from 1 minute to 3 hours, more preferably 10 minutes to 1 hour. In another embodiment, the heat treatment is preferably carried out in an inert gas atmosphere such as argon and nitrogen.

### (Organic Light Emitting Device)

According to yet another aspect of the present disclosure, there is provided an organic light emitting device comprising the compound represented by Chemical Formula 1. As an example, the present disclosure provides an organic light emitting device comprising: a first electrode; a second electrode that is provided opposite to the first electrode; and a light emitting layer that is provided between the first electrode and the second electrode, wherein the light emitting layer includes the above-mentioned compound represented by Chemical Formula 1.

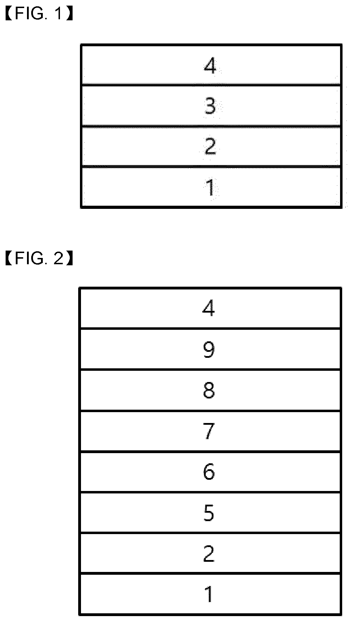

Further, the organic light emitting device according to the present disclosure may be a normal type organic light emitting device in which an anode, one or more organic material layers, and a cathode are sequentially stacked on a substrate. Further, the organic light emitting device according to the present disclosure may be an inverted type organic light emitting device in which a cathode, one or more organic material layers, and an anode are sequentially stacked on a substrate. For example, the structure of an organic light emitting device according to an embodiment of the present disclosure is illustrated in FIGS. 1 and 2.

FIG. 1 shows an example of an organic light emitting device comprising a substrate 1, an anode 2, a light emitting layer 3, and a cathode 4. In such a structure, the compound represented by Chemical Formula 1 may be included in the light emitting layer.

FIG. 2 shows an example of an organic light emitting device comprising a substrate 1, an anode 2, a hole injection layer 5, a hole transport layer 6, a light emitting layer 7, an electron transport layer 8, an electron injection layer 9 and a cathode 4. In such a structure, the compound represented by Chemical Formula 1 may be included in the light emitting layer.

The organic light emitting device according to the present disclosure may be manufactured by materials and methods known in the art, except that the light emitting layer includes the compound according to the present disclosure.

For example, the organic light emitting device according to the present disclosure can be manufactured by sequentially stacking an anode, an organic material layer and a cathode on a substrate. In this case, the organic light emitting device may be manufactured by depositing a metal, metal oxides having conductivity, or an alloy thereof on the substrate using a PVD (physical vapor deposition) method such as a sputtering method or an e-beam evaporation method to form an anode, forming organic material layers including the hole injection layer, the hole transport layer, the light emitting layer and the electron transport layer thereon, and then depositing a material that can be used as the cathode thereon.

In addition to such a method, the organic light emitting device may be manufactured by sequentially depositing a cathode material, an organic material layer and an anode material on a substrate (International Publication WO2003/012890). However, the manufacturing method is not limited thereto.

As an example, the first electrode is an anode, and the second electrode is a cathode, or alternatively, the first electrode is a cathode and the second electrode is an anode.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:Al or SnO₂:Sb; conductive compounds such as poly(3-methylthiophene), poly[3,4-(ethylene-1,2-dioxy)thiophene](PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

The hole injection layer is a layer for injecting holes from the electrode, and the hole injection material is preferably a compound which has a capability of transporting the holes, thus has a hole injecting effect in the anode and an excellent hole injecting effect to the light emitting layer or the light emitting material, prevents excitons produced in the light emitting layer from moving to a electron injection layer or the electron injection material, and further is excellent in the ability to form a thin film. It is preferable that a HOMO (highest occupied molecular orbital) of the hole injection material is between the work function of the anode material and a HOMO of a peripheral organic material layer. Specific examples of the hole injection material include metal porphyrine, oligothiophene, an arylamine-based organic material, a hexanitrilehexaazatriphenylene-based organic material, a quinacridone-based organic material, a perylene-based organic material, anthraquinone, polyaniline and polythiophene-based conductive compound, and the like, but are not limited thereto.

The hole transport layer is a layer that receives holes from a hole injection layer and transports the holes to the light emitting layer. The hole transport material is suitably a material having large mobility to the holes, which may receive holes from the anode or the hole injection layer and transfer the holes to the light emitting layer. Specific examples thereof include an arylamine-based organic material, a conductive compound, a block copolymer in which a conjugate portion and a non-conjugate portion are present together, and the like, but are not limited thereto.

The light emitting layer may include a host material and a dopant material. As the dopant material, a compound represented by Chemical Formula 1 may be used. The host material may be a fused aromatic ring derivative, a heterocycle-containing compound or the like. Specific examples of the fused aromatic ring derivatives include anthracene derivatives, pyrene derivatives, naphthalene derivatives, pentacene derivatives, phenanthrene compounds, and fluoranthene compounds. Examples of the heterocycle-containing compound include carbazole derivatives, dibenzofuran derivatives, ladder-type furan compounds, pyrimidine derivatives, and the like, but are not limited thereto.

The electron transport layer is a layer which receives electrons from an electron injection layer and transports the electrons to a light emitting layer, and an electron transport material is suitably a material which may receive electrons well from a cathode and transfer the electrons to a light emitting layer, and has a large mobility for electrons. Specific examples of the electron transport material include: an Al complex of 8-hydroxyquinoline; a complex including Alqs; an organic radical compound; a hydroxyflavone-metal complex, and the like, but are not limited thereto. The electron transport layer may be used with any desired cathode material, as used according to the related art. In particular, appropriate examples of the cathode material are a typical material which has a low work function, followed by an aluminum layer or a silver layer. Specific examples thereof include cesium, barium, calcium, ytterbium, and samarium, in each case followed by an aluminum layer or a silver layer.

The electron injection layer is a layer which injects electrons from an electrode, and is preferably a compound which has a capability of transporting electrons, has an effect of injecting electrons from a cathode and an excellent effect of injecting electrons into a light emitting layer or a light emitting material, prevents excitons produced from the light emitting layer from moving to a hole injection layer, and is also excellent in the ability to form a thin film. Specific examples of the electron injection layer include LiF, NaF, NaCl, CsF, Li₂O, BaO, fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like, but are not limited thereto.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

The organic light emitting device according to the present disclosure may be a front side emission type, a back side emission type, or a double side emission type according to the used material.

In addition, the compound according to the present disclosure may be included in an organic solar cell or an organic transistor in addition to an organic light emitting device.

The preparation of the compound represented by Chemical Formula 1 according to the present disclosure and the organic light emitting device containing the same will be described in detail in the following examples. However, these examples are presented for illustrative purposes only, and are not intended to limit the scope of the present disclosure.

### Preparation Example 1: Preparation of Compound 1

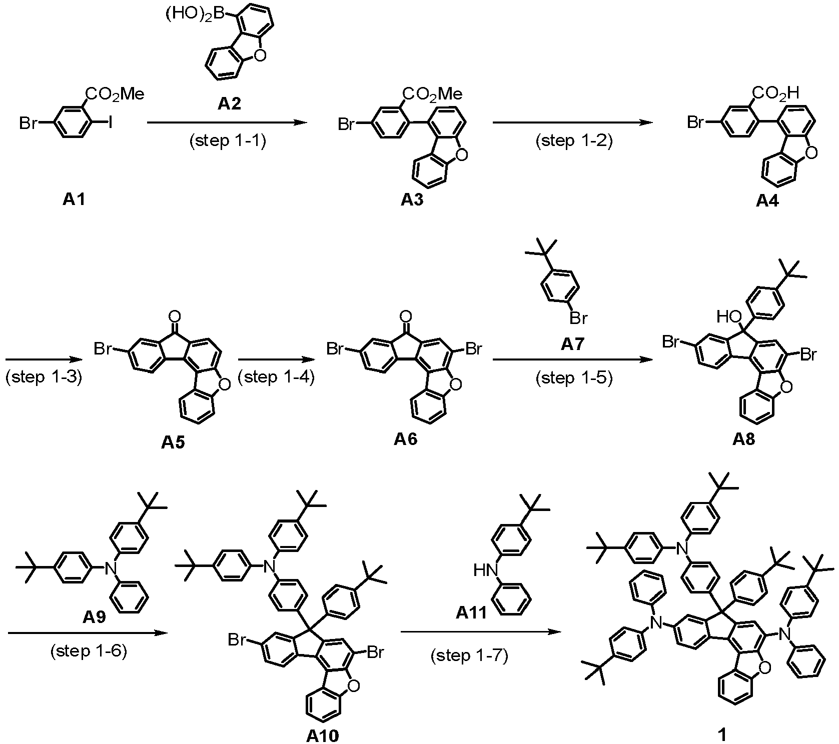

### Step 1-1: Synthesis of Intermediate Compound A3

Compound A1(17 g, 50 mmol), A2(15.9 g, 75 mmol), Pd(PPh₃)₄(5.8 g, 5 mmol) and K₂CO₃(20.7 g, 150 mmol) were dissolved in toluene (500 ml) and distilled water (150 ml), and then the mixture was stirred at 90°C for 15 hours. The organic layer was separated and then water was removed using MgSO₄, which was then subjected to reduced pressure to remove the solvent. The resulting material was subjected to column chromatography to separate and purify Compound A3 (12 g, 63%).
MS: [M+H]⁺ = 381

### Step 1-2: Synthesis of Intermediate Compound A4

Compound A3 (1.52 g, 4 mmol) prepared in step 1-1, sodium hydroxide (0.2 g, 5 mmol), and 30 ml of ethanol were added thereto, and the mixture was stirred at reflux for 48 hours, and then cooled to room temperature. 2-Normal hydrochloric acid was added dropwise thereto, and the reaction mixture was stirred for 30 minutes, and then filtered. Recrystallization from dichloromethane and n- hexane gave A4 (1.2 g, yield: 79%).
MS: [M+H]⁺ = 367

### Step 1-3: Synthesis of Intermediate Compound A5

Compound A4 (1.47 g, 4 mmol) prepared in step 1-2, and 20 ml of methanesulfonic acid was added, and the mixture was heated to 80°C, stirred for 3 hours, and then cooled to room temperature. The reaction solution was slowly added dropwise to 20 ml of ice water and stirred for 30 minutes. The resulting solid was filtered and washed with water and methanol to give Compound A5 (1.24 g, yield: 89%).
MS: [M+H]⁺ = 349

### Step 1-4: Synthesis of Intermediate Compound A6

Compound A5 (1.05 g, 3 mmol) prepared in step 1-3 was added to 30 ml of dichloromethane and stirred at room temperature. Bromine (0.96 g, 6 mmol) was diluted with 5 ml of dichloromethane and added dropwise thereto, and the reaction solution was stirred at room temperature for 8 hours. After completion of the reaction, 30 ml of acetone was added to a reaction vessel and stirred. The resulting solid was filtered and then washed with acetone. The solid was recrystallized from monochlorobenzene to give Compound A6 (0.85 g, yield: 66%).
MS: [M+H]⁺ = 427

### Step 1-5: Synthesis of Intermediate Compound A8

Compound A7 (2.13 g, 10 mmol) was added to 400 ml of tetrahydrofuran, and the mixture was cooled to -78°C while stirring. N-butyllithium 1.6 M (5.35 ml, 8.5 mmol) was added to the solution prepared above, and then stirred for 1 hour. Next, Compound A6 (3.21 g, 7.5 mmol) prepared in step 1-4 was added thereto, and the reaction mixture was stirred for 1 hour, then heated to room temperature, and then stirred for 2 hours. Then, an aqueous solution of ammonium chloride was added and stirred for 30 minutes, and then the organic layer was separated using a separatory funnel, and then water was removed using MgSO₄, which was then subjected to reduced pressure to remove the solvent. The resulting material was subjected to column chromatography to separate and purify Compound A8 (2.81 g, yield: 50%).
MS: [M+H]⁺ = 561

### Step 1-6: Synthesis of Intermediate Compound A10

Compound A8 (2.81 g, 5 mmol) prepared in step 1-5 was added to 200 mL of dichloromethane, and the mixture was cooled to 0°C with stirring, and then methanesulfonic acid (1.2 g, 20 mmol) was added for 10 minutes and heated to room temperature. Then, Compound A9 (2.68 g, 7.5 mmol) was added thereto, stirred for 30 minutes, and then an aqueous sodium bicarbonate solution was added and stirred for 30 minutes. Then, the organic layer was separated using a separatory funnel, and then water was removed using MgSO₄, which was then subjected to reduced pressure to remove the solvent. The resulting material was subjected to column chromatography to separate and purify Compound A10 (4.7 g, yield: 94%).
MS: [M+H]⁺ = 901

### Step 1-7: Synthesis of Compound 1

Compound A10(2.03 g, 2.25 mmol) prepared in step 1-6, Compound A11(1.13 g, 5 mmol), Pd₂(dba)₃ (110 mg, 0.12 mmol), P(t-Bu)₃(73 mg, 0.36 mmol) and Nat-BuO(0.71 g, 7.43 mmol) were disssolved in toluene (50 ml) and then stirred at 100°C for 15 hours. The mixture was cooled to room temperature and distilled water was added. Then, the organic layer was separated using a separatory funnel, and then water was removed using MgSO₄, which was then subjected to reduced pressure to remove the solvent. The resulting material was subjected to column chromatography to separate and purify Compound 1 (1.1 g, yield: 41%).
MS: [M+H]⁺ = 1191

### Preparation Example 2: Preparation of Compound 2

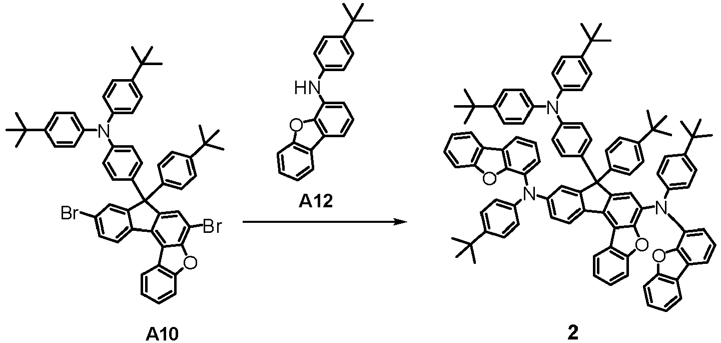

Compound 2 (1.51 g, yield: 49%) was prepared in the same manner as in Preparation Example 1, except that Compound A12 was used instead of Compound A11 in Preparation Example 1.
MS: [M+H]⁺ = 1371

### Preparation Example 3: Preparation of Compound 3

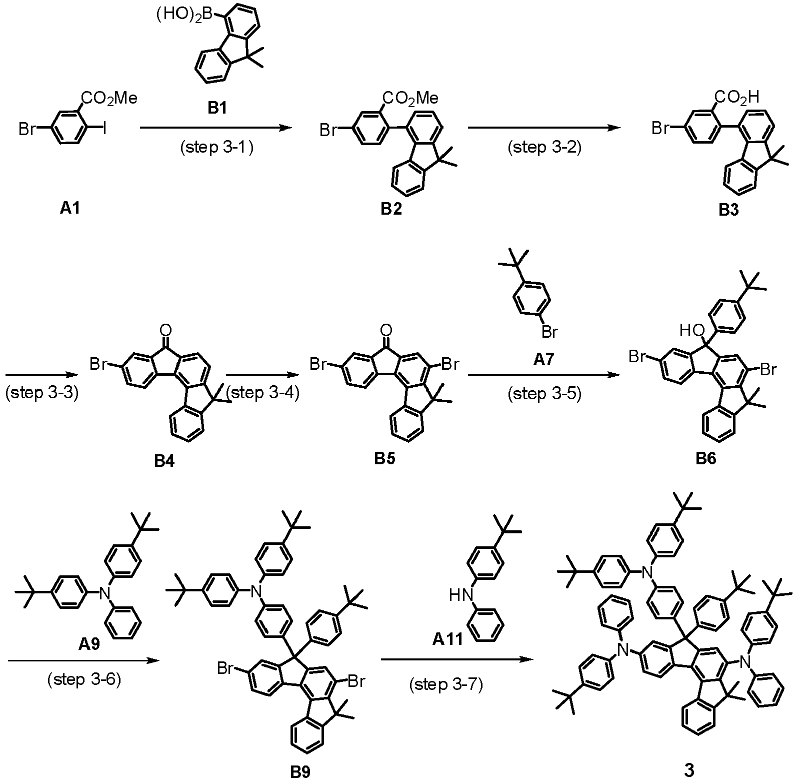

Compound 3 was prepared in the same manner as in Preparation Example 1, except that Compound B1 was used instead of Compound A2 in Preparation Example 1.
MS: [M+H]⁺ = 1217

### Preparation Example 4: Preparation of Compound 4

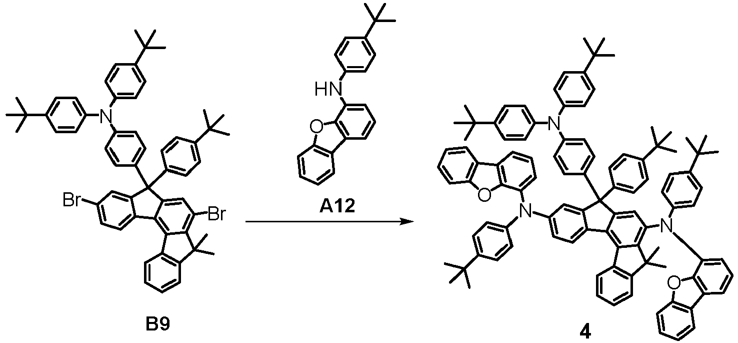

Compound 4(1.19 g, yield: 38%) was prepared in the same manner as in Preparation Example 3, except that Compound A12 was used instead of Compound A11 in Preparation Example 3.
MS: [M+H]⁺ = 1397

### Preparation Example 5: Preparation of Compound 5

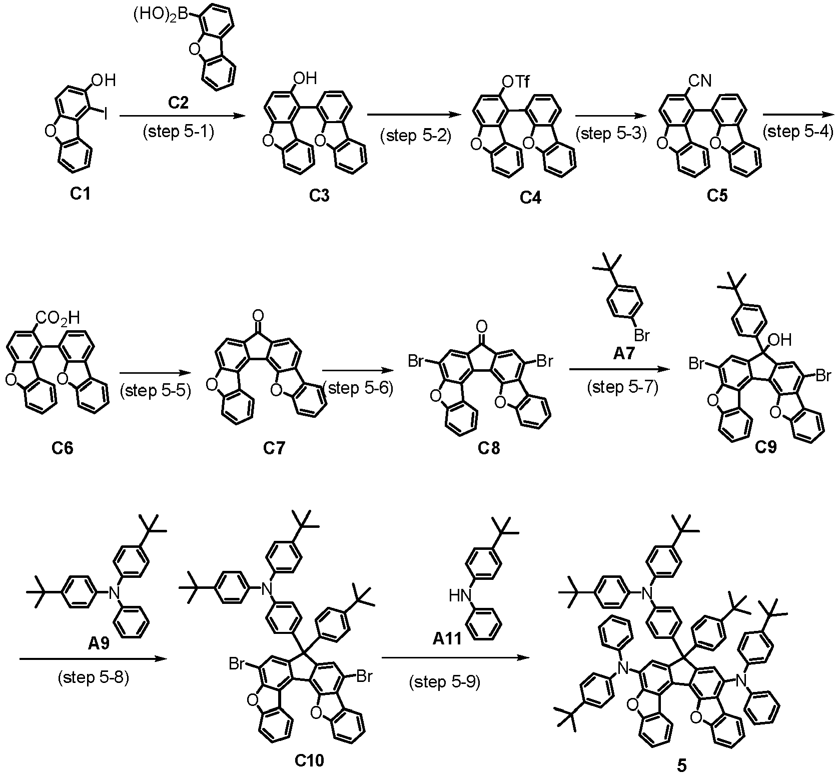

### Step 5-1: Synthesis of Intermediate Compound C3

Compound C1(15.5g, 50mmol), C2 (15.9g, 75mmol), Pd(PPh₃)₄(5.8g, 5mmol) and K₂CO₃(20.7g, 150mmol) were dissolved in toluene (500 ml) and distilled water (150 ml), and then the mixture was stirred at 90°C for 15 hours. The organic layer was separated and then water was removed using MgSO₄, which was then subjected to reduced pressure to remove the solvent. The resulting material was subjected to column chromatography to separate and purify Compound C3 (13.1 g, 75%).
MS: [M+H]⁺ = 351

### Step 5-2: Synthesis of Intermediate Compound C4

Compound C3(7.0 g, 20 mmol) prepared in step 5-1 was added, dissolved in dichloromethane (100 mL), and then cooled to 0°C. Pyridine (1.9 g, 24 mmol) was added followed by trifluoromethanesulfonic anhydride (5.92 g, 21 mmol), and the mixture was stirred at room temperature for 3 hours. Then, the organic layer was separated using a separatory funnel, and then water was removed using MgSO₄, which was then subjected to reduced pressure to remove the solvent. The resulting material was subjected to column chromatography to separate and purify Compound C4 (7.8 g, yield: 81%).
MS: [M+H]⁺ = 483

### Step 5-3: Synthesis of Intermediate Compound C5

Compound C4 (4.82 g, 10 mmol) prepared in step 5-2, potassium cyanide (1.3 g, 20 mmol) and Pd(PPh₃)₄ (1.16 g, 1 mmol) were added, and dissolved in N,N-dimethylformamide (50 mL), and then the mixture was heated to 130°C, and stirred for 18 hours. Then, the organic layer was separated using a separatory funnel, and then water was removed using MgSO₄, which was then subjected to reduced pressure to remove the solvent. The resulting material was subjected to column chromatography to separate and purify Compound C5 (2.3 g, yield: 65%).
MS: [M+H]⁺ = 360

### Step 5-4: Synthesis of Intermediate Compound C6

Compound C5 (1.8 g, 5 mmol) prepared in step 5-3 and potassium hydroxide (0.56 g, 10 mmol) were added to ethanol (30 ml) and water (10 mL), and the mixture was stirred at reflux for 24 hours. The mixture was cooled to room temperature, acidified with 0.2 normal hydrochloric acid, and the resulting solid was filtered, washed with hexane, and dried to give C6 (1.36 g, yield: 72%).
MS: [M+H]⁺ = 379

### Step 5-5: Synthesis of Intermediate Compound C7

Compound C6 (1.9 g, 5 mmol) prepared in step 5-4 and methane sulfonic acid (20 mL) were added, and stirred at 120°C for 4 hours. After cooling, the reaction solution was added to 200 ml of water, and the resulting precipitate was filtered, washed with toluene, and then dried to give C7 (0.92 g, yield: 51%).
MS:[M+H]⁺=361

### Step 5-6: Synthesis of Intermediate Compound C8

Compound C7 (3.0 g, 8.32 mmol) prepared in step 5-5 was added to 100 mL of dichloromethane, and bromine (4.0 g, 25 mmol) dissolved in 30 mL of dichloromethane was slowly added, followed by stirring at room temperature for 60 hours. The precipitate was filtered, washed with dichloromethane and hexane, and then recrystallized from toluene and N-methylpyrrolidone to give C8 (1.4 g, yield: 32%).
MS: [M+H]⁺ = 517

### Step 5-7: Synthesis of Intermediate Compound C9

Compound A7 (1.07 g, 5 mmol) was added to 200 mL of tetrahydrofuran, and the mixture was cooled to -78°C while stirring. N-butyllithium 1.6 M (2.8 ml, 4.5 mmol) was added to the solution prepared above, and then stirred for 1 hour. Next, Compound C8(2.07 g, 4 mmol) prepared in step 5-6 was added thereto, and the reaction mixture was stirred for 1 hour, then heated to room temperature, and then stirred for 2 hours. Then, an aqueous solution of ammonium chloride was added and stirred for 30 minutes, and then the organic layer was separated using a separatory funnel, and then water was removed using MgSO₄, which was then subjected to reduced pressure to remove the solvent. The resulting material was subjected to column chromatography to separate and purify Compound C9 (1.1 g, yield: 42%).
MS: [M+H]⁺ = 651

### Step 5-8: Synthesis of Intermediate Compound C10

Compound C9 (3.26 g, 5 mmol) prepared in step 5-7 was added to 200 mL of dichloromethane, and the mixture was cooled to 0°C while stirring. Methanesulfonic acid (1.2 g, 20 mmol) was added for 10 minutes and heated to room temperature. Then, Compound A9 (2.68 g, 7.5 mmol) was added thereto, stirred for 30 minutes, and then an aqueous sodium bicarbonate solution was added and stirred for 30 minutes. Then, the organic layer was separated using a separatory funnel, and then water was removed using MgSO₄, which was then subjected to reduced pressure to remove the solvent. The resulting material was subjected to column chromatography to separate and purify Compound C10 (4.36 g, yield: 88%).
MS: [M+H]⁺ = 990

### Step 5-9: Synthesis of Compound 5

Compound C10 (2.23 g, 2.25 mmol) prepared in step 5-8, Compound A11 (1.13 g, 5 mmol), Pd₂(dba)₃ (110 mg, 0.12 mmol), P(t-Bu)3 (73 mg) , 0.36 mmol) and Nat-BuO (0.71 g, 7.43 mmol) were dissolved in toluene (50 ml), followed by stirring at 100°C for 15 hours. The mixture was cooled to room temperature and distilled water was added. The organic layer was separated using a separatory funnel, and then water was removed using MgSO₄, which was then subjected to reduced pressure to remove the solvent. The resulting material was subjected to column chromatography to separate and purify Compound 5 (1.4 g, yield: 49%).
MS: [M+H]⁺ = 1281

### Preparation Example 6: Preparation of Compound 6

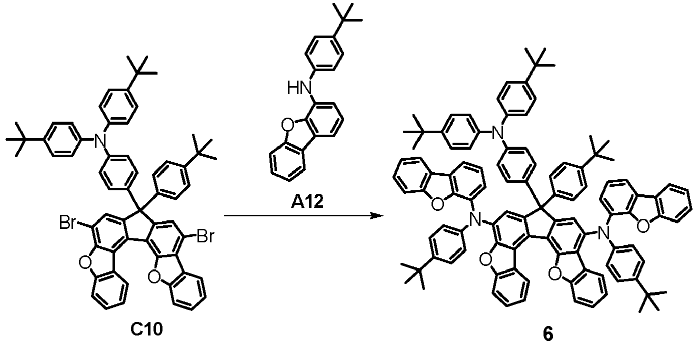

Compound 6(1.48 g, yield: 45%) was prepared in the same manner as in Preparation Example 5, except that Compound A12 was used instead of Compound A11 in Preparation Example 5.
MS: [M+H]⁺ = 1461

### Preparation Example 7: Preparation of Compound 7

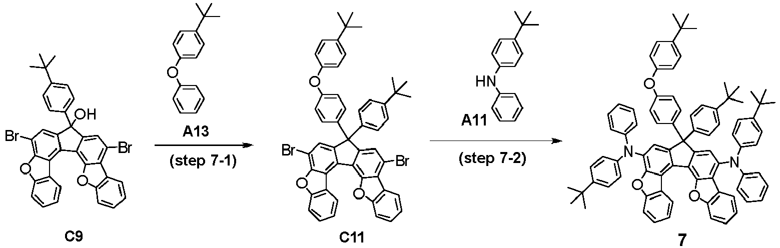

### Step 7-1: Synthesis of Compound C11

Compound C11(2.24 g, yield: 52%) was prepared in the same manner as in step 5-8, except that Compound A13 was used instead of Compound A9 in step 5-8.
MS: [M+H]⁺ = 859

### Step 7-2: Preparation of Compound 7

Compound 7(1.68 g, yield: 56 %) was prepared in the same manner as in step 5-9, except that Compound C11 was used instead of Compound C10 in step 5-9.
MS: [M+H]⁺ = 1150

### Preparation Example 8: Preparation of Compound 8

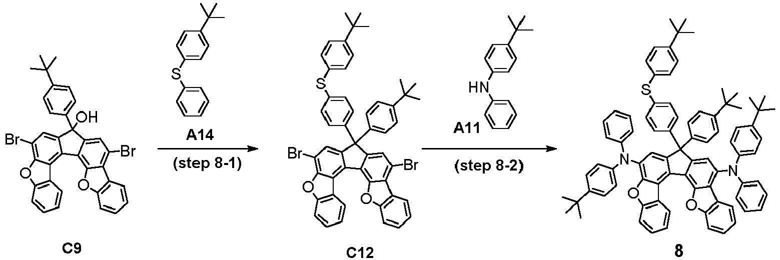

### Step 8-1: Synthesis of Compound C12

Compound C12(2.67 g, yield: 61%) was prepared in the same manner as in step 5-8, except that Compound A14 was used instead of Compound A9 in step 5-8.
MS: [M+H]⁺ = 875

### Step 8-2: Preparation of Compound 8

Compound 8(1.95 g, yield: 55 %) was prepared in the same manner as in step 5-9, except that Compound C12 was used instead of Compound C10 in step 5-9.
MS: [M+H]⁺ = 1166

### Preparation Example 9: Preparation of Compound 9

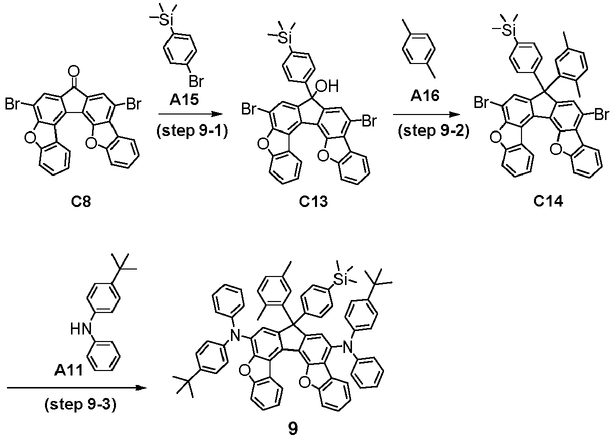

### Step 9-1: Synthesis of Compound C13

Compound C13(1.27 g, yield: 38 %) was prepared in the same manner as in step 5-7, except that Compound A15 was used instead of Compound A7 in step 5-7.
MS: [M+H]⁺ = 667

### Step 9-2: Preparation of Compound C14

Compound C13(3.34 g, 5 mmol) prepared in step 9-1 was added to A16(200 mL), and the mixture was cooled to 0°C with stirring, and then methanesulfonic acid (1.2 g, 20 mmol) was added for 10 minutes and heated to 80°C. After stirring for 12 hours, the mixture was cooled to room temperature, and then an aqueous sodium bicarbonate solution was added and stirred for 30 minutes. Then, the organic layer was separated using a separatory funnel, and then water was removed using MgSO₄, which was then subjected to reduced pressure to remove the solvent. The resulting material was subjected to column chromatography to separate and purify Compound C14 (1.32 g, yield: 59%).
MS: [M+H]⁺ = 745

### Step 9-3: Synthesis of Compound 9

Compound 9 (1.13 g, yield: 62 %) was prepared in the same manner as in step 5-9, except that Compound C14 was used instead of Compound C10 in step 5-9.
MS: [M+H]⁺ = 1046

### Experimental Example 1: Solubility Experiment

Compounds 1 to 9 prepared in the Preparation Examples and Compound BD below were respectively dissolved in toluene, and the solubility in toluene at room temperature/normal pressure was measured. The results are shown in Table 1 below.

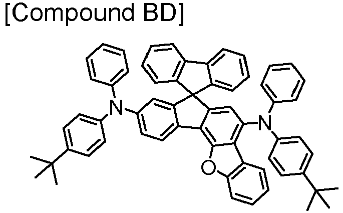

**[Table 1]**

| Compound | Solubility (wt%) |
|---|---|
| Compound 1 | 4.5 |
| Compound 2 | 4.7 |
| Compound 3 | 5.8 |
| Compound 4 | 5.5 |
| Compound 5 | 3.9 |
| Compound 6 | 4.1 |
| Compound 7 | 3.8 |
| Compound 8 | 3.9 |
| Compound 9 | 3.4 |
| Compound BD | 2.0 |

As shown in Table 1, it can be seen that the compound represented by Chemical Formula 1 of the present disclosure in which the substituent of the fluorene core is asymmetric has a significantly higher solubility in toluene compared to Compound BD.

### Example 1: Manufacture of organic light emitting device

A glass substrate on which ITO (indium tin oxide) was coated as a thin film to a thickness of 500 Å was put into distilled water in which a detergent was dissolved, and ultrasonically cleaned. At this time, a product manufactured by Fischer Co. was used as the detergent, and as the distilled water, distilled water filtered twice using a filter manufactured by Millipore Co. was used. After the ITO was cleaned for 30 minutes, ultrasonic cleaning was repeated twice using distilled water for 10 minutes. After the cleaning with distilled water was completed, the substrate was ultrasonically cleaned with solvents of isopropyl alcohol, acetone and methanol, dried, and thereby, the cleaned ITO glass substrate with a thickness of 500 Å was prepared.

A composition in which the following compound Z-1 and the following compound Z-2 were mixed in a weight ratio of 8:2 and dissolved in toluene at a weight ratio of 2% was spin-coated onto the ITO transparent electrode, and cured at 220°C for 30 minutes on a hot plate under a nitrogen atmosphere to form a hole injection layer with a thickness of 400 Å. A composition in which the following compound Z-3 was dissolved in toluene at a weight ratio of 1% was spin-coated onto the hole injection layer, and heat-treated at 200°C for 30 minutes on a hot plate to form a hole transport layer with a thickness of 200 Å.

A 2 wt% toluene solution in which the following compound Z-4 and the compound 1 prepared in Preparation Example 1 were dissolved in a weight ratio of 94:6, spin-coated on the hole transport layer at 5000 rpm, baked at 80°C for 2 minutes and baked at 120°C for 30 minutes to form a light emitting layer with a thickness of 550 Å. This was dried at 130°C for 10 minutes under a nitrogen atmosphere, and lithium fluoride (LiF) was deposited to a thickness of 10 Å on the light emitting layer to form an electron transport and injection layer, and finally, aluminum was deposited to a thickness of 1000Å to form a cathode.

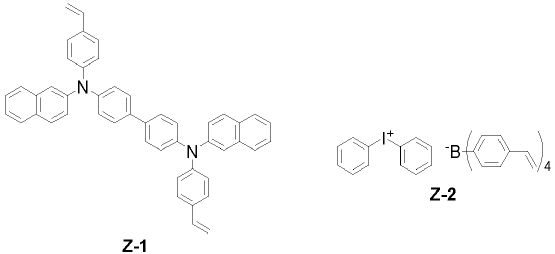

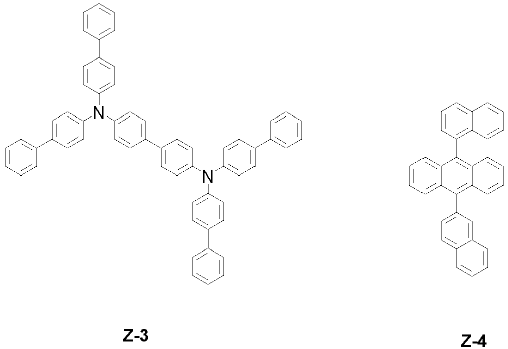

In the above-mentioned process, the deposition rate of lithium fluoride was maintained at 0.3 Å/sec, the deposition rate of aluminum was maintained at 2 Å/sec, and the degree of vacuum during the deposition was maintained at 2×10⁻⁷ to 5×10⁻⁶ torr, thereby manufacturing the organic light emitting device.

### Examples 2 to 9 and Comparative Example 1

An organic light emitting device was manufactured in the same manner as in Example 1, except that the compounds shown in Table 2 below were used instead of the compound 1.

The compounds used in Examples 1 to 9 and Comparative Example 1 are summarized as follows:

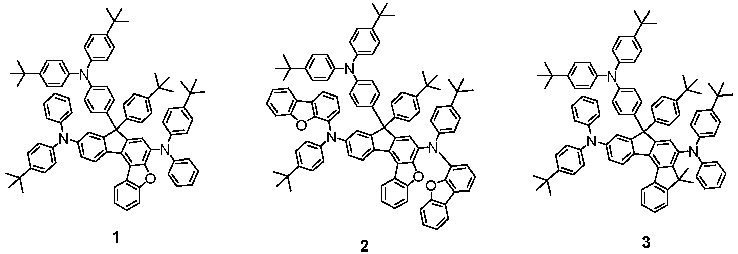

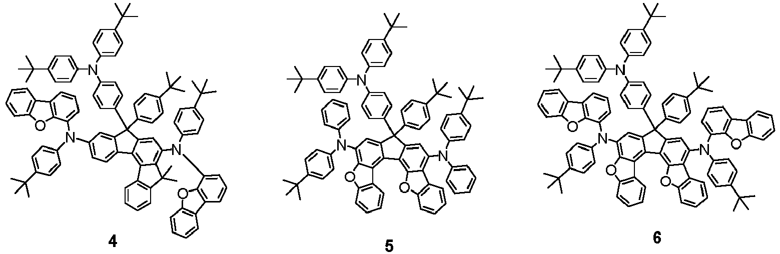

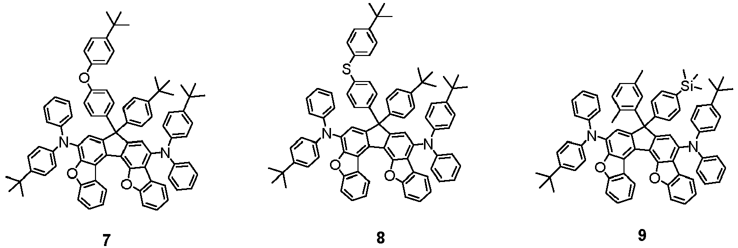

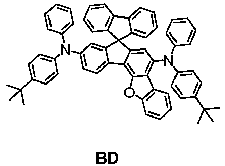

### Experimental Example 2: Evaluation of characteristics of organic light emitting device

When a current was applied to the organic light emitting devices manufactured in Examples 1 to 9 and Comparative Example 1, the driving voltage, power efficiency, luminous efficiency, quantum efficiency, and lifetime at a current density of 10 mA/cm² were measured, and the results are shown in Table 2 below. Lifetime T90 means the time required for the luminance to be reduced to 90% of the initial luminance (1000 nit).

**[Table 2]**

| | Compound (light emitting layer dopant) | Driving voltage (V @10mA/cm²) | Power efficiency (lm/W @10mA/cm²) | Luminous efficiency (cd/A @10mA/cm²) | Lifetime (hr @1000 nit) |
|---|---|---|---|---|---|
| Example 1 | Compound 1 | 4.37 | 3.75 | 5.29 | 351 |
| Example 2 | Compound 2 | 4.31 | 3.77 | 5.20 | 340 |
| Example 3 | Compound 3 | 4.28 | 3.95 | 5.37 | 365 |
| Example 4 | Compound 4 | 4.29 | 3.90 | 5.35 | 366 |
| Example 5 | Compound 5 | 4.35 | 3.69 | 5.26 | 339 |
| Example 6 | Compound 6 | 4.38 | 3.81 | 5.51 | 347 |
| Example 7 | Compound 7 | 4.27 | 3.81 | 5.30 | 335 |
| Example 8 | Compound 8 | 4.32 | 3.75 | 5.24 | 342 |
| Example 9 | Compound 9 | 4.33 | 3.77 | 5.29 | 340 |
| Comparative Example 1 | Compound BD | 4.40 | 3.61 | 5.17 | 312 |

As shown in Table 2, it was confirmed that the organic light emitting device using the compound of the present disclosure as a dopant of a light emitting layer exhibits very excellent characteristics in terms of driving voltage, efficiency and lifetime as compared with the organic light emitting device using the compound BD of Comparative Example as a dopant of the light emitting layer. This means that, considering that the luminous efficiency and lifetime characteristics of the organic light emitting device generally have a trade-off relationship with each other, the organic light emitting device employing the compound of the present disclosure as a dopant of the light emitting layer is significantly improved as compared with the device of the Comparative Example.

**<Description of Symbols>**

| | | | |
|---|---|---|---|
| 1: | substrate | 2: | anode |
| 3: | light emitting layer | 4: | cathode |
| 5: | hole injection layer | 6: | hole transport layer |
| 7: | light emitting layer | 8. | electron transport layer |
| 9: | electron injection layer | | |

## Claims

1. A compound represented by the following Chemical Formula 1: 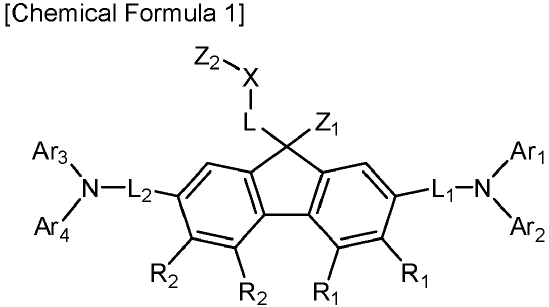 in the Chemical Formula 1,
R₁ and R₂ are each independently hydrogen, or two R₁s or two R₂s are joined together to form a substituted or unsubstituted C₆₋₆₀ aromatic ring; a substituted or unsubstituted C₆₋₆₀ non-aromatic ring; or a substituted or unsubstituted C₂₋₆₀ heterocyclic ring containing any one or more heteroatoms selected from the group consisting of N, O, S and Si,
at least one of the two R₁s or the two R₂s are joined together to form the C₆₋₆₀ non-aromatic ring; or the C₂₋₆₀ heterocyclic ring,
L is a substituted or unsubstituted C₆₋₆₀ arylene; or a substituted or unsubstituted C₂₋₆₀ heteroarylene containing any one or more heteroatoms selected from the group consisting of N, O and S,
X is O, S, NZ₃, or SiZ₄Z₅,
Z₁ is a substituted or unsubstituted C₆₋₆₀ aryl,
Z₂ to Z₅ are each independently a substituted or unsubstituted C₁₋₆₀ alkyl; a substituted or unsubstituted C₁₋₆₀ haloalkyl; a substituted or unsubstituted C₃₋₆₀ cycloalkyl; a substituted or unsubstituted C₇₋₆₀ aralkyl; a substituted or unsubstituted C₆₋₆₀ aryl; or a substituted or unsubstituted C₂₋₆₀ heteroaryl containing any one or more heteroatoms selected from the group consisting of N, O and S,
the L and Z₂; Z₂ and Z₃; or Z₄ and Z₅ are optionally connected to each other to form a 5-membered-heterocyclic ring,
with the proviso that Z₁ and LX-Z₂ are different from each other and are not connected to each other,
L₁ and L₂ are each independently a single bond; a substituted or unsubstituted C₆₋₆₀ arylene; or a substituted or unsubstituted C₂₋₆₀ heteroarylene containing any one or more heteroatoms selected from the group consisting of N, O and S, and
Ar₁ to Ar₄ are each independently a substituted or unsubstituted C₆₋₆₀ aryl; or a substituted or unsubstituted C₂₋₆₀ heteroaryl containing any one or more heteroatoms selected from the group consisting of N, O and S.

2. The compound of claim 1,
wherein at least one of the two R₁s or the two R₂s are joined together to form any one of the structures represented by the following Chemical Formulas 2d to 2g, the rest are hydrogen, or are joined together to form any one of the structures represented by the following Chemical Formulas 2a to 2g: 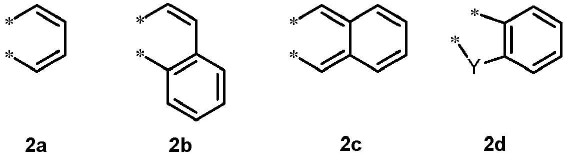 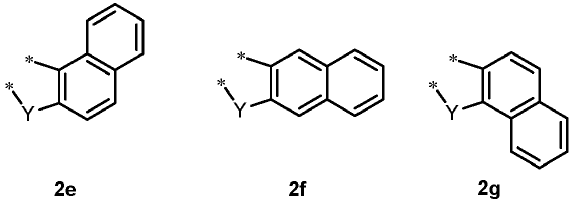 in the Chemical Formulas 2a to 2g,
Y is O, S, CZ₆Z₇, or SiZ₈Z₉,
Z₆ to Z₉ are each independently C₁₋₁₀ alkyl, or C₆₋₂₀ aryl, and
* means a bonding position with the carbon bonded to R₁ or R₂.

3. The compound of claim 2,
wherein the compound represented by Chemical Formula 1 is represented by any one of the following Chemical Formulas 1-1, 1-3, and 1-5. 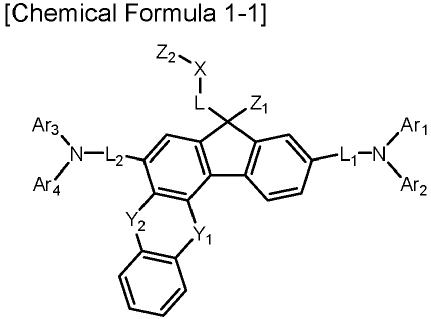 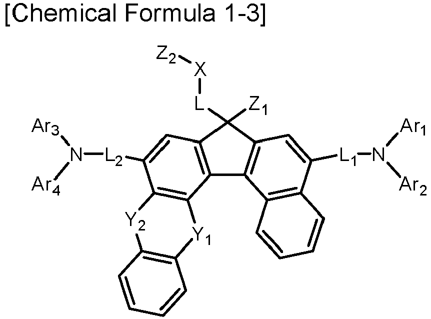 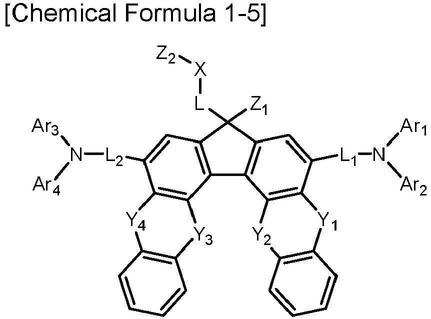 in the Chemical Formulas 1-1, 1-3, and 1-5,
L, X, Z₁, Z₂, L₁, L₂ and Ar₁ to Ar₄ are as defined in claim 1,
one of Y₁ and Y₂ is a single bond, and the other one is O, S, C(C₁₋₄ alkyl)₂, or Si(C₁₋₄ alkyl)₂, and
one of Y₃ and Y₄ is a single bond, and the other one is O, S, C(C₁₋₄ alkyl)₂, or Si(C₁₋₄ alkyl)₂.

4. The compound of claim 1,
wherein Z₁ is phenyl or biphenylyl,
wherein Z₁ is unsubstituted, or substituted with 1 substituent to 5 substituents independently selected from the group consisting of deuterium, halogen, cyano, C₁₋₁₀ alkyl, C₃₋₁₀ cycloalkyl, Si(C₁₋₁₀ alkyl)₃ and Si(C₆₋₂₀ aryl)₃.

5. The compound of claim 1,
wherein Z₁ is any one selected from the group consisting of:

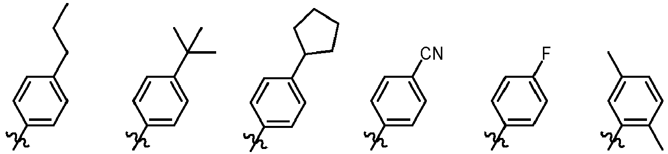

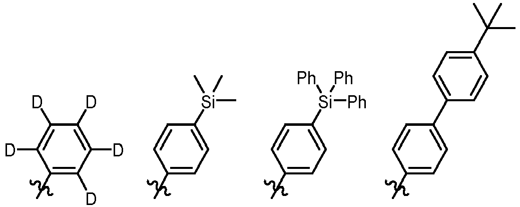

6. The compound of claim 1,
wherein L-X-Z₂ is any one selected from the group consisting of: 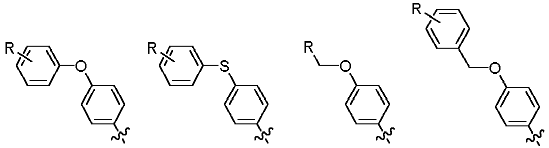 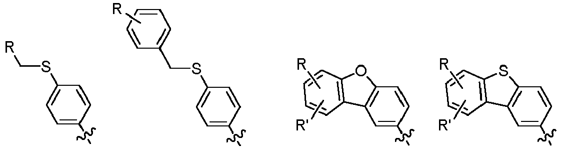 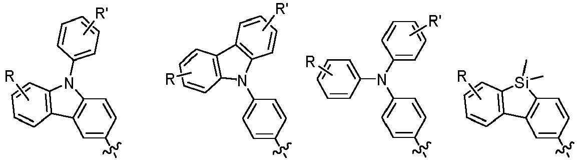 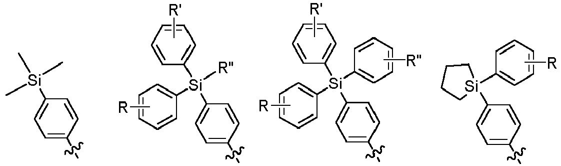 wherein,
R, R' and R" are each independently hydrogen, methyl, tert-butyl, or Si(methyl)₃.

7. The compound of claim 1,
wherein L₁ and L₂ are a single bond.

8. The compound of claim 1,
wherein Ar₁ to Ar₄ are each independently phenyl, naphthyl, biphenylyl, or dibenzofuranyl,
wherein Ar₁ to Ar₄ are unsubstituted, or substituted with 1 substituent to 5 substituents each independently selected from the group consisting of deuterium, halogen, C₁₋₁₀alkyl and Si(C₁₋₁₀ alkyl)₃.

9. The compound of claim 1,
wherein Ar₁ to Ar₄ are each independently any one selected from the group consisting of:

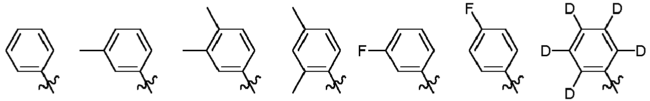

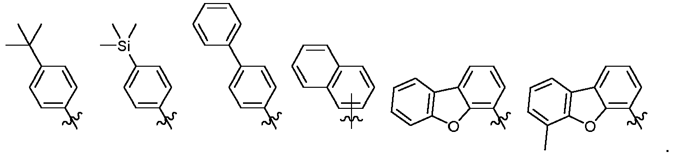

10. The compound of claim 1,
wherein Ar₁ and Ar₄ are identical to each other, and
Ar₂ and Ar₃ are identical to each other.

11. The compound of claim 1,
wherein the compound represented by Chemical Formula 1 is represented by any one of the following Chemical Formulas 2-1, 2-2, 2-4, 2-5, 2-7, 2-8, and 2-10: 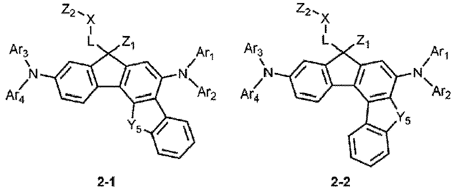 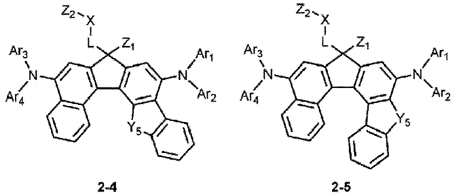 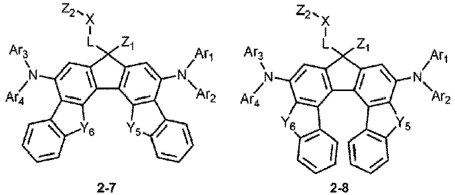 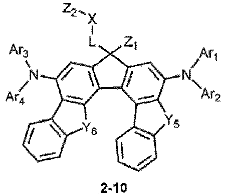 in the Chemical Formulas 2-1, 2-2, 2-4, 2-5, 2-7, 2-8, and 2-10,
L, X, Z₁, Z₂ and Ar₁ to Ar₄ are as defined in claim 1, and
Y₅ and Y₆ are each independently O, S, C(methyl)₂, or Si(methyl)₂.

12. The compound of claim 1,
wherein the compound is any one of the following compounds:

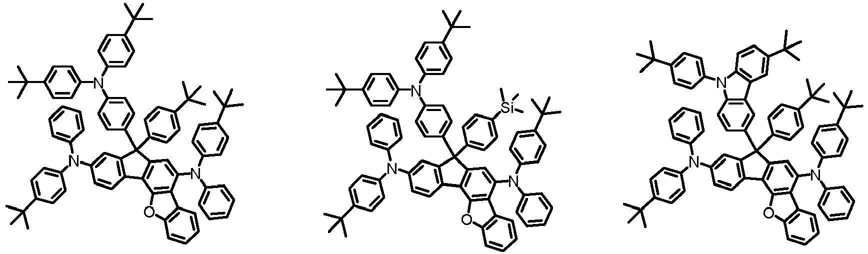

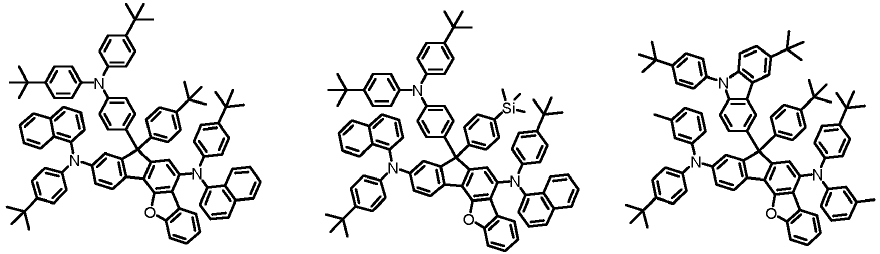

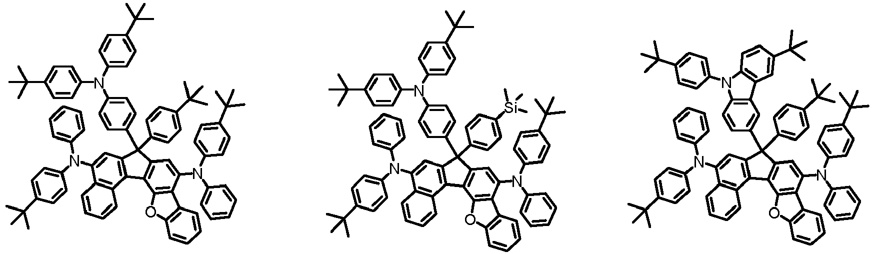

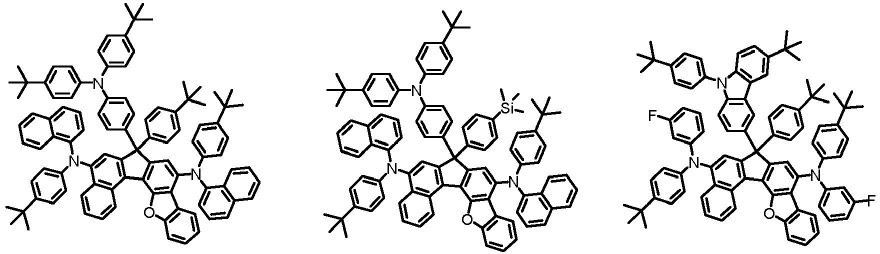

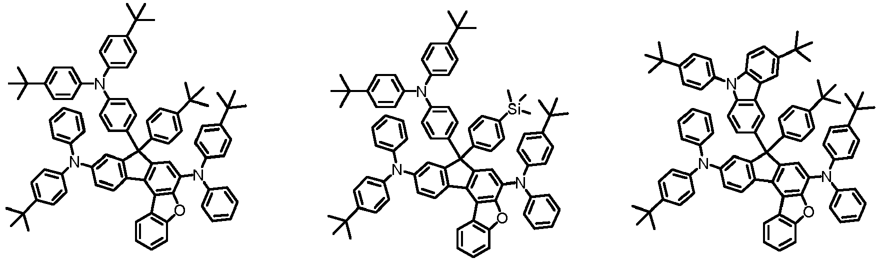

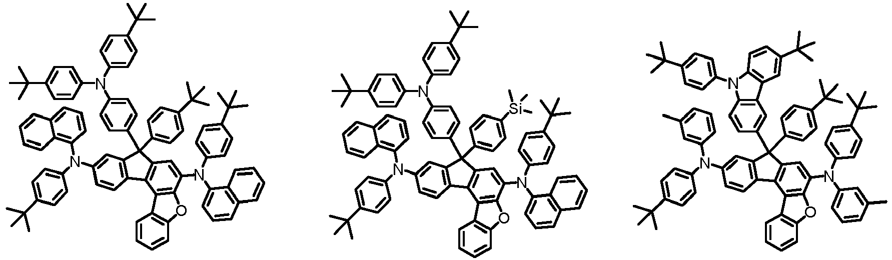

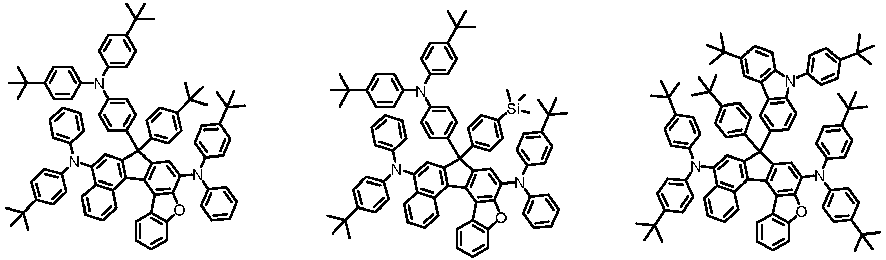

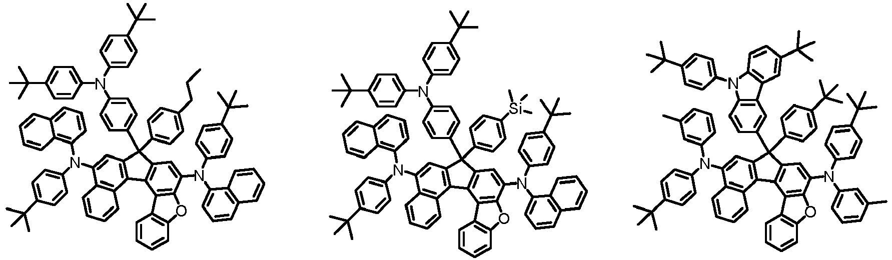

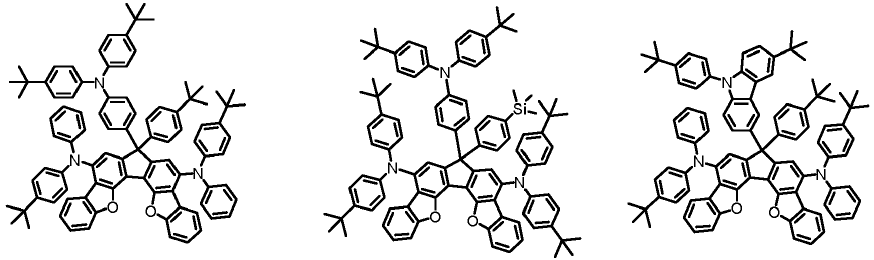

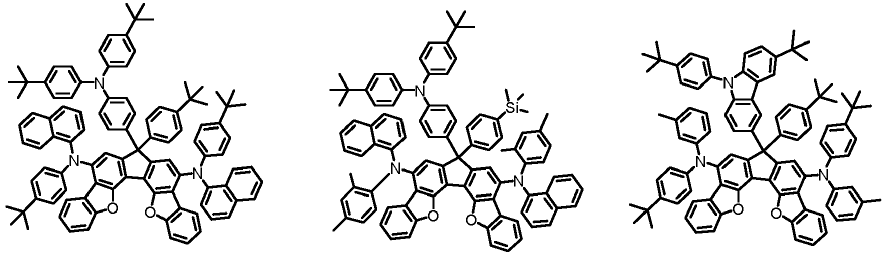

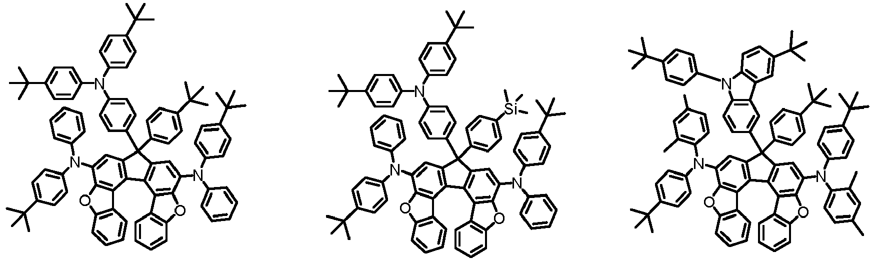

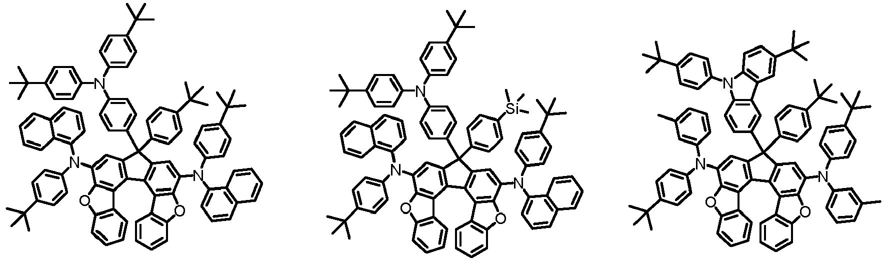

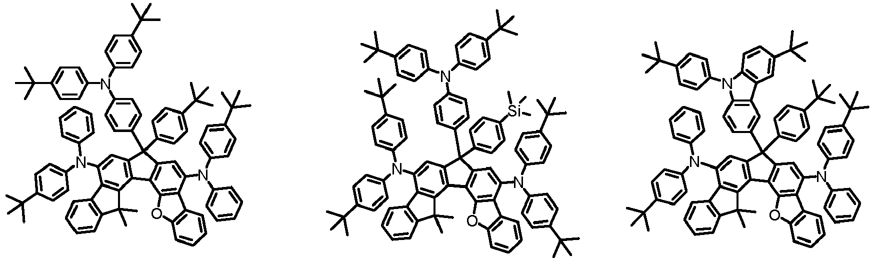

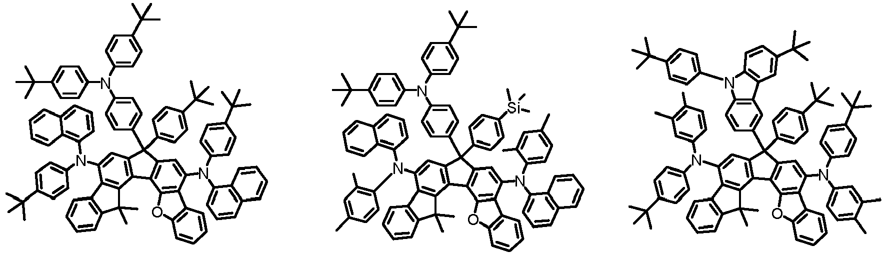

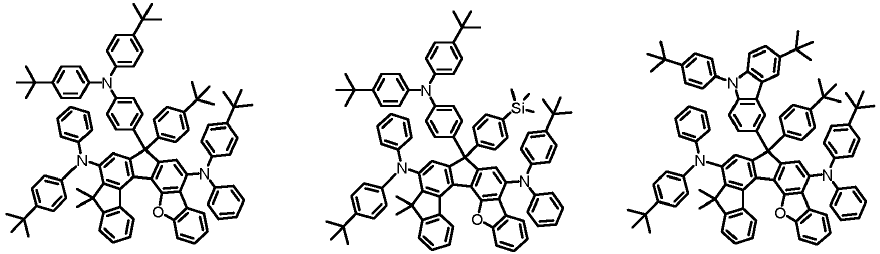

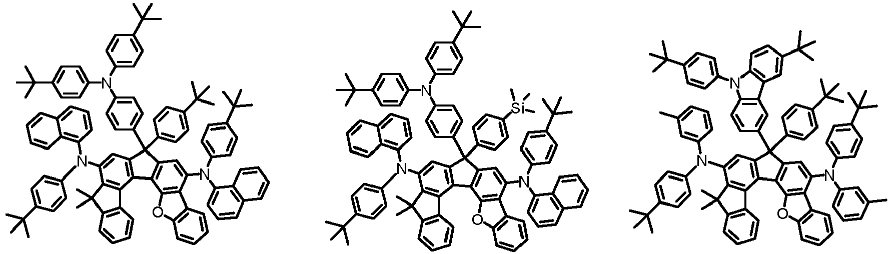

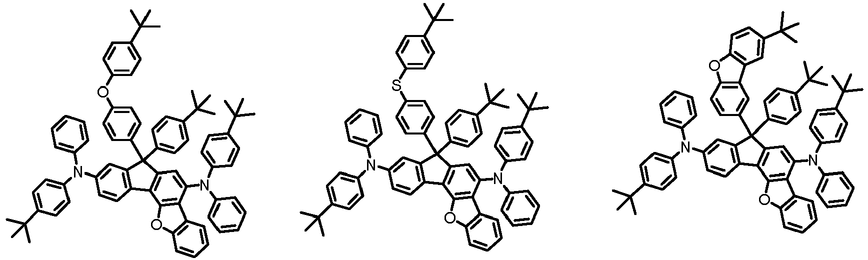

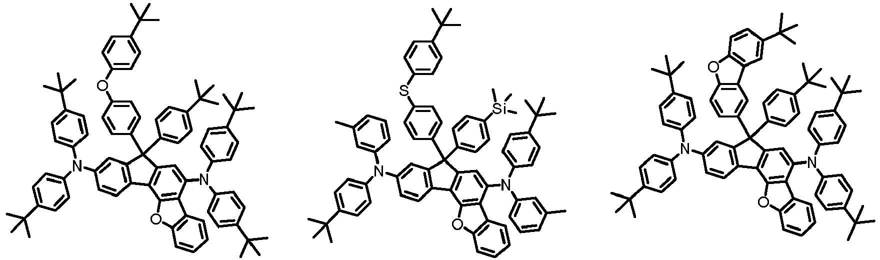

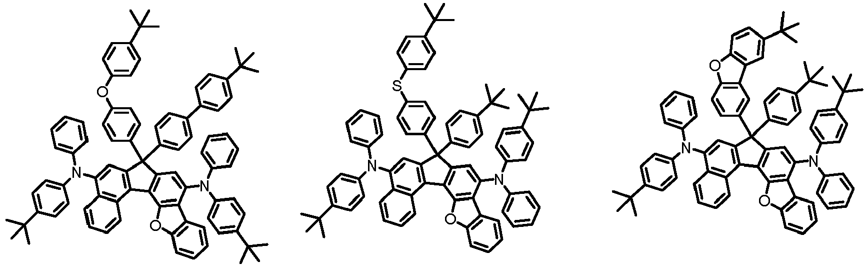

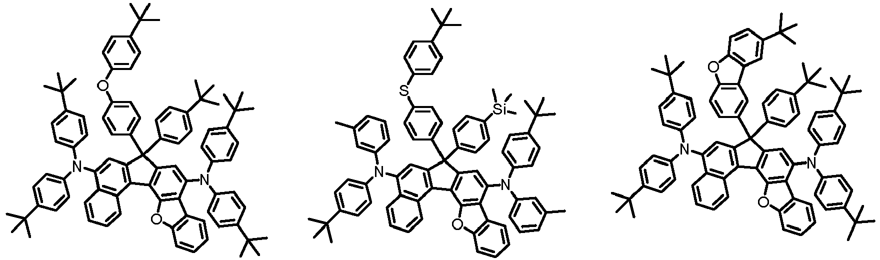

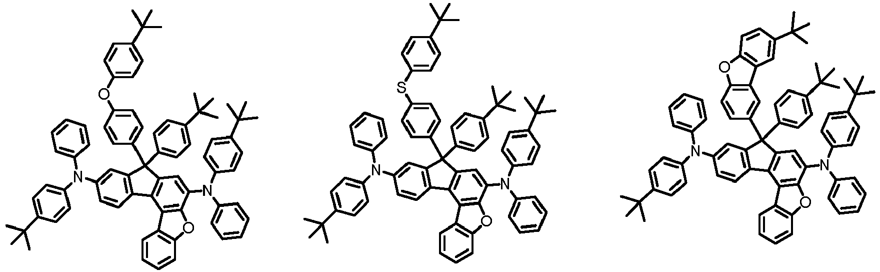

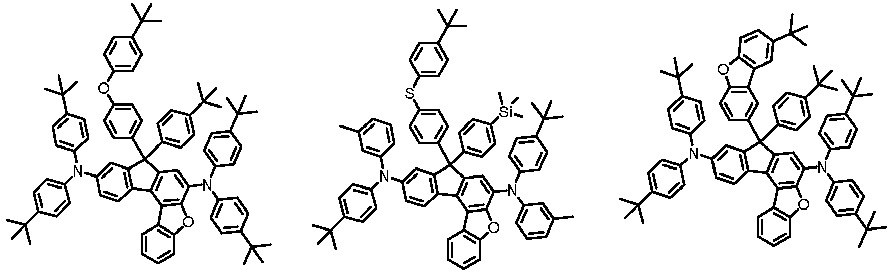

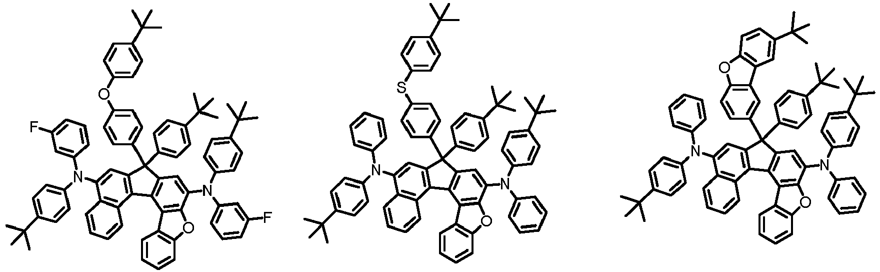

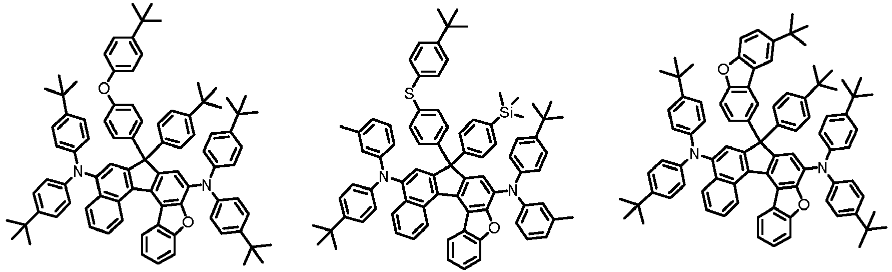

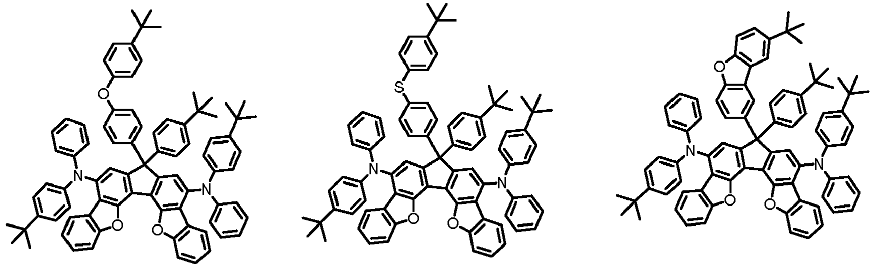

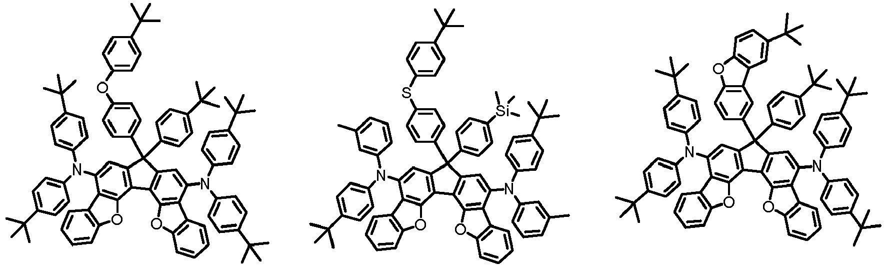

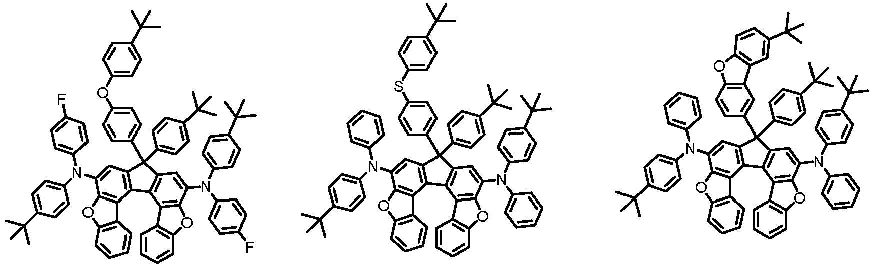

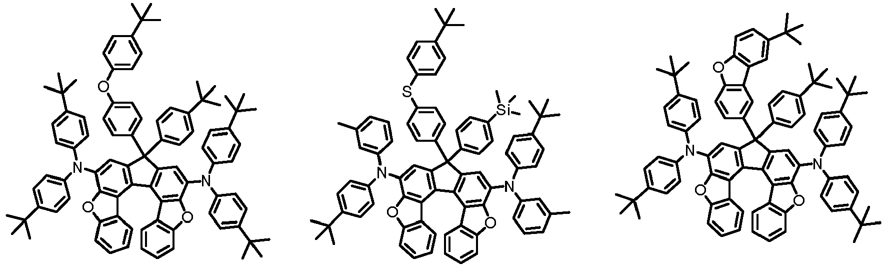

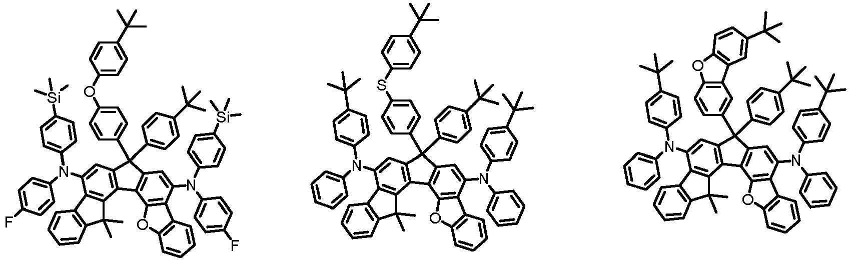

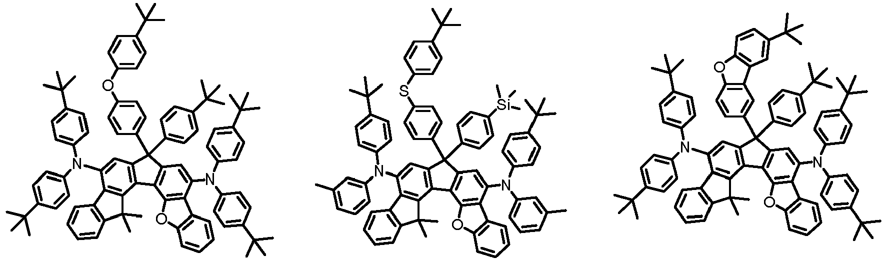

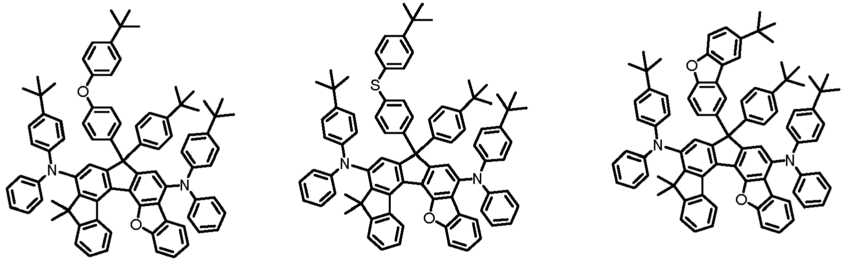

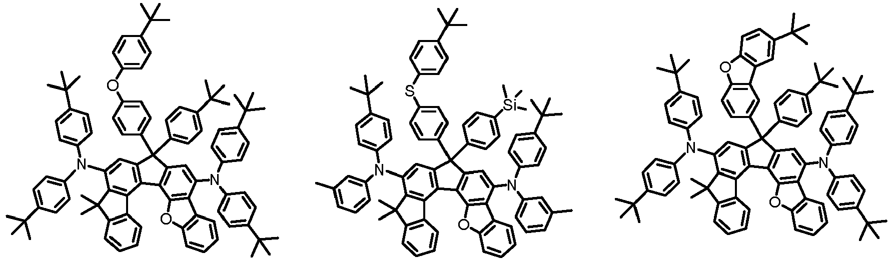

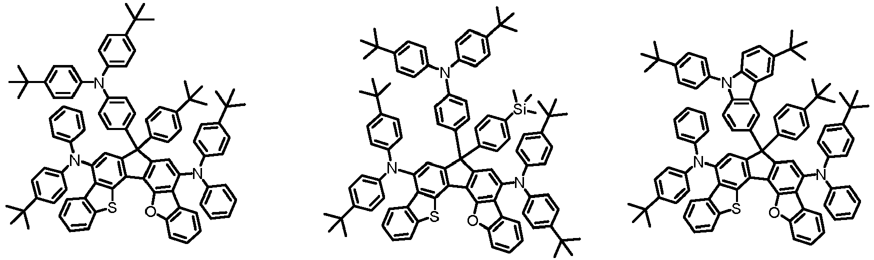

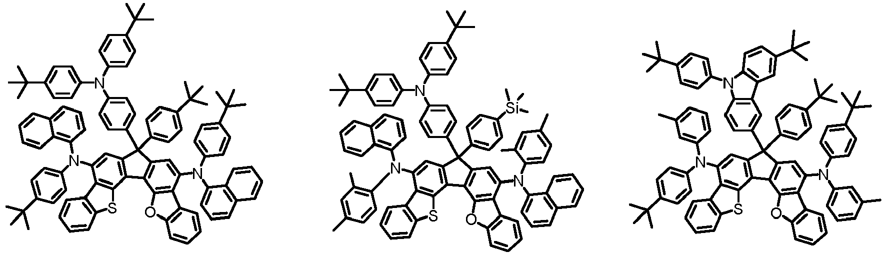

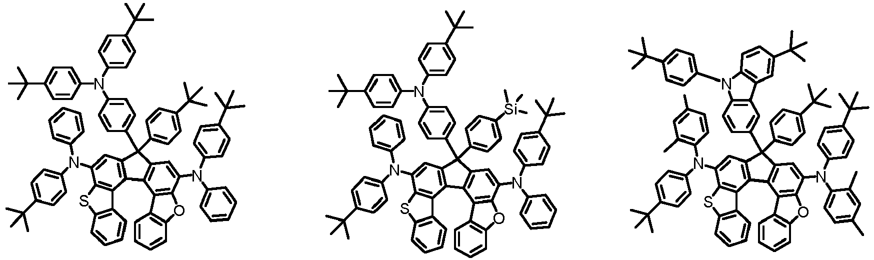

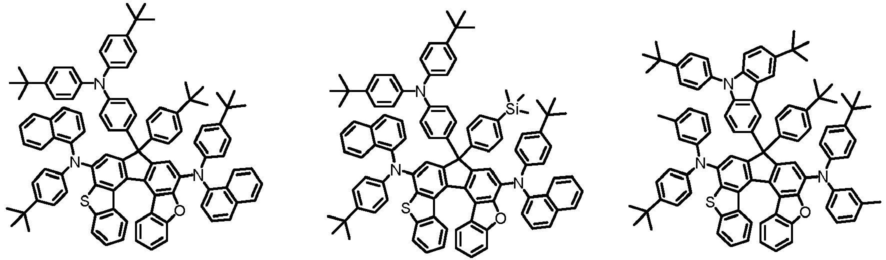

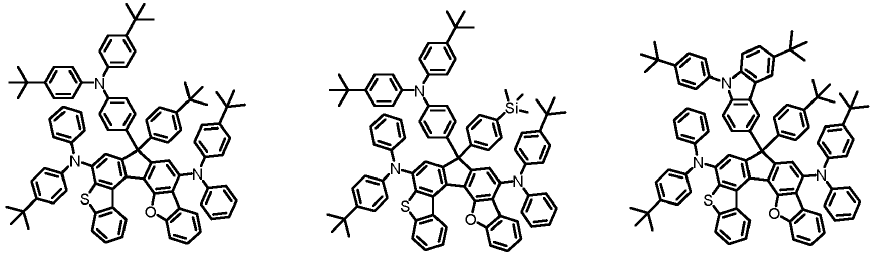

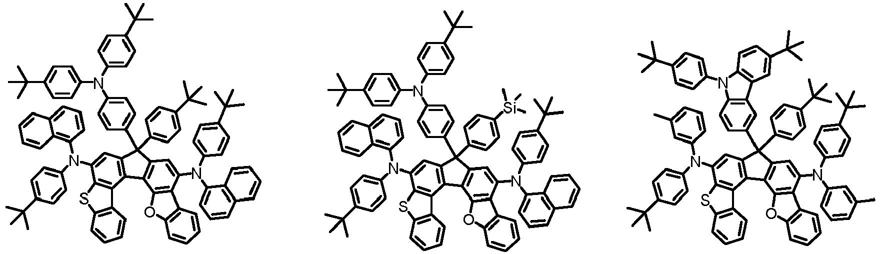

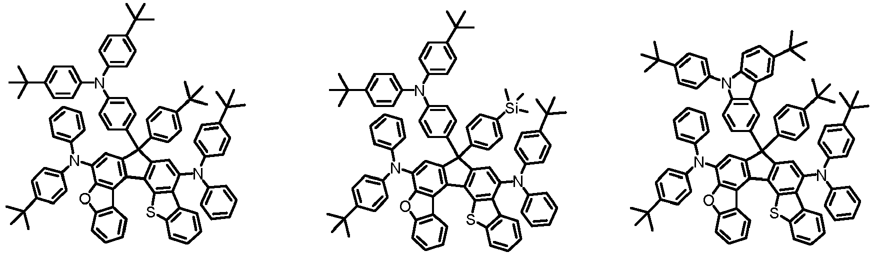

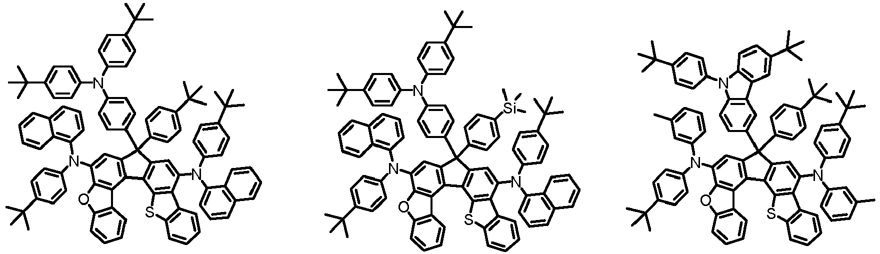

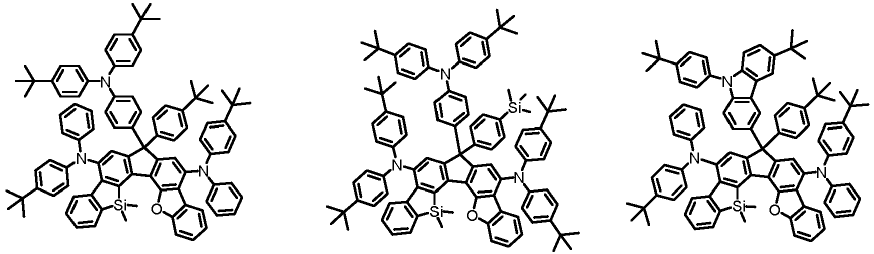

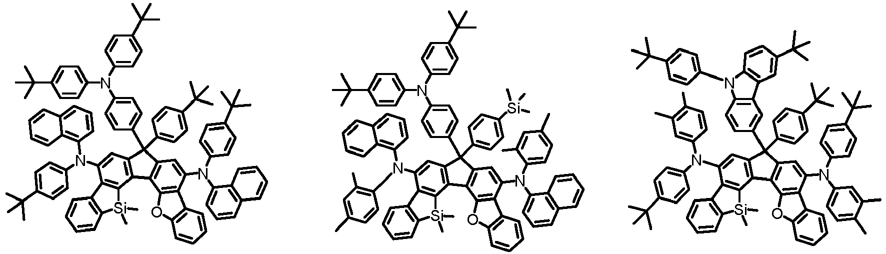

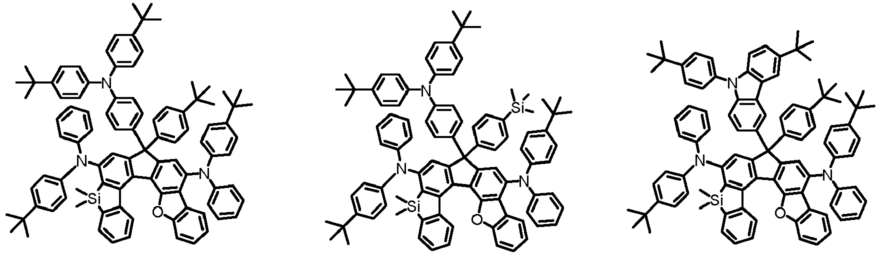

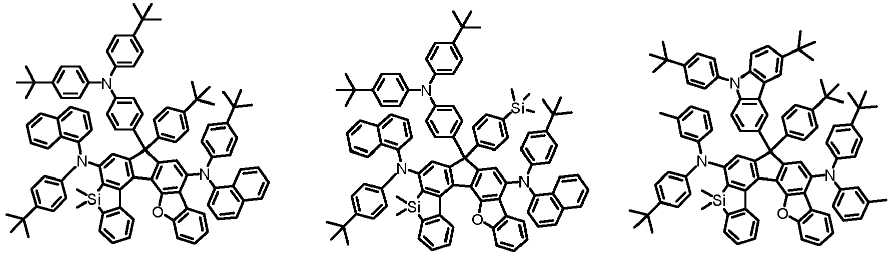

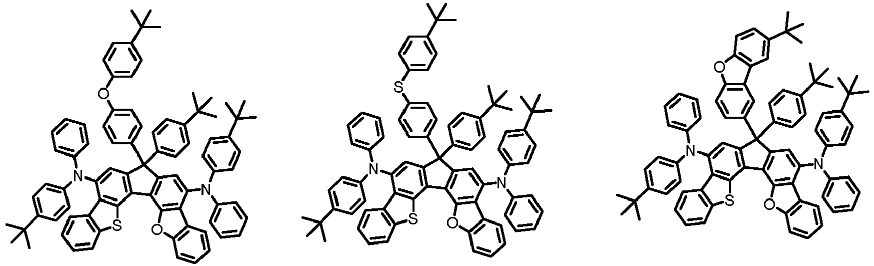

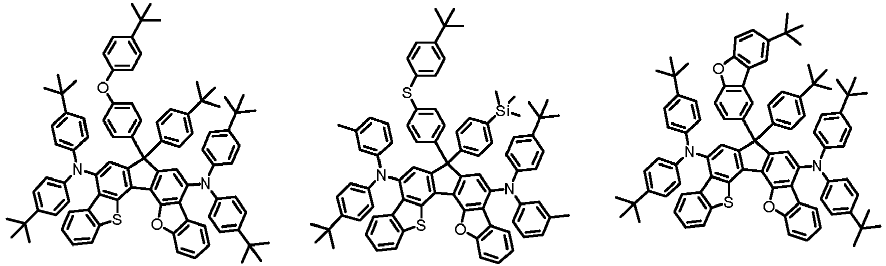

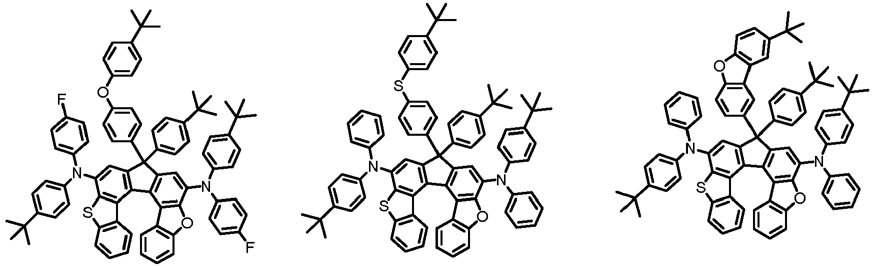

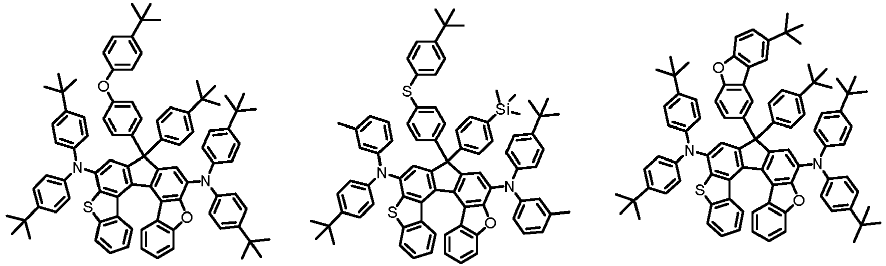

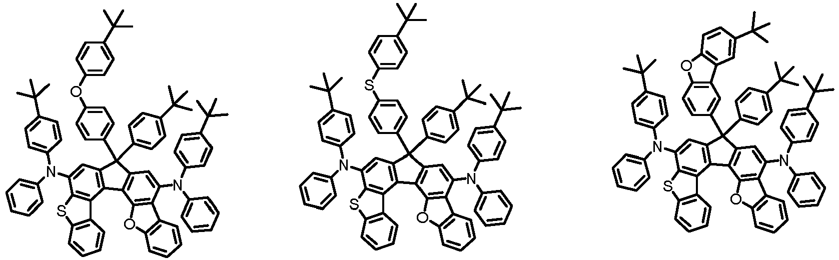

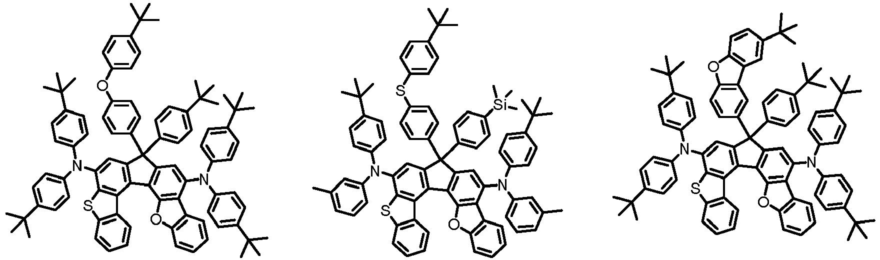

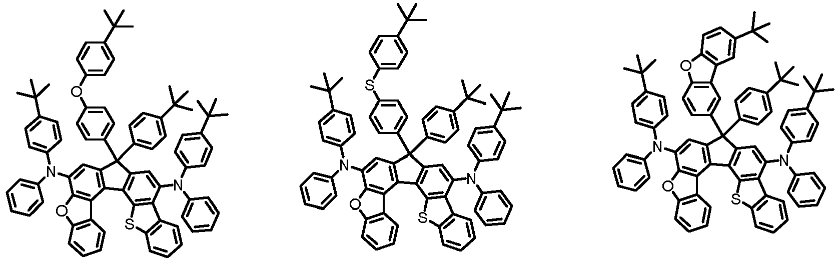

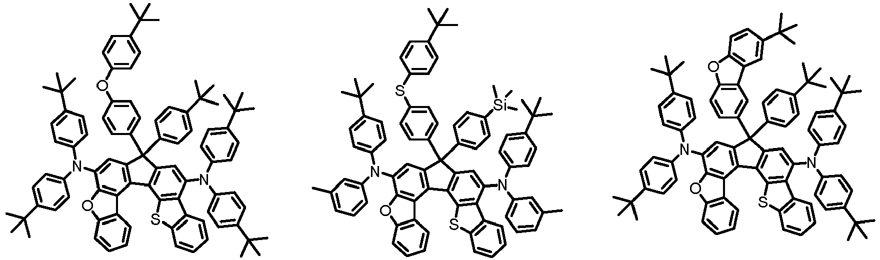

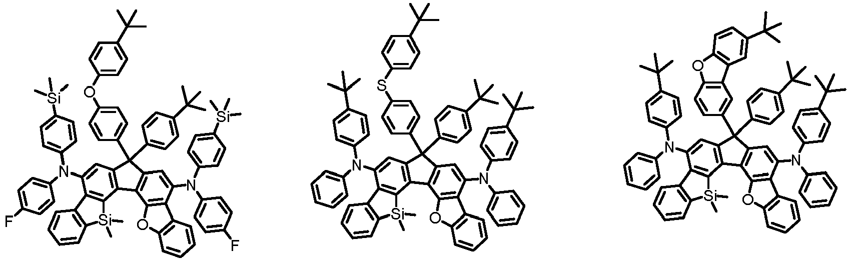

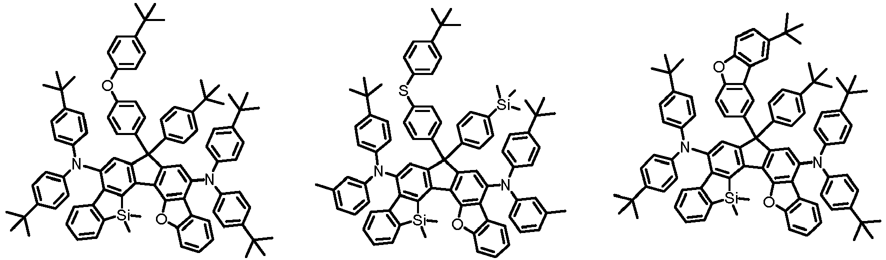

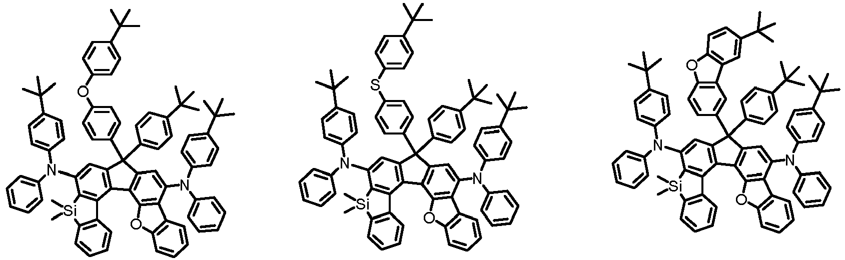

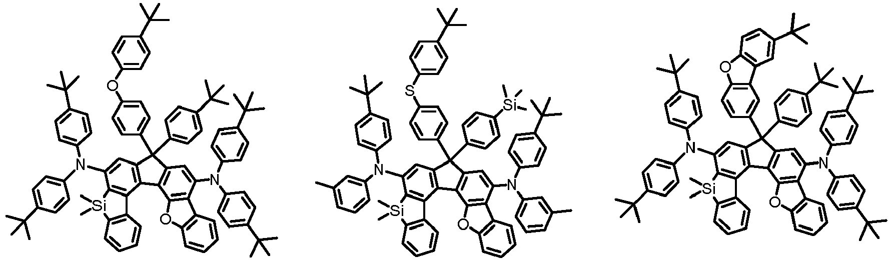

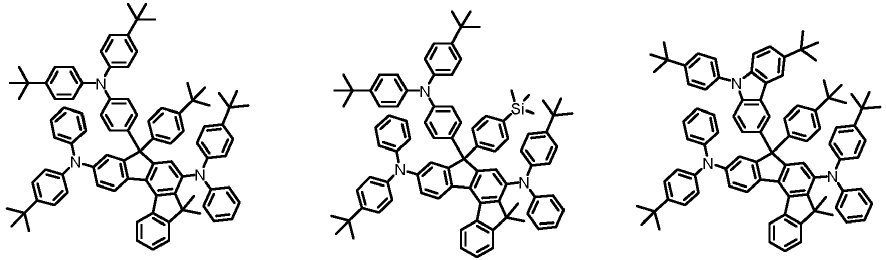

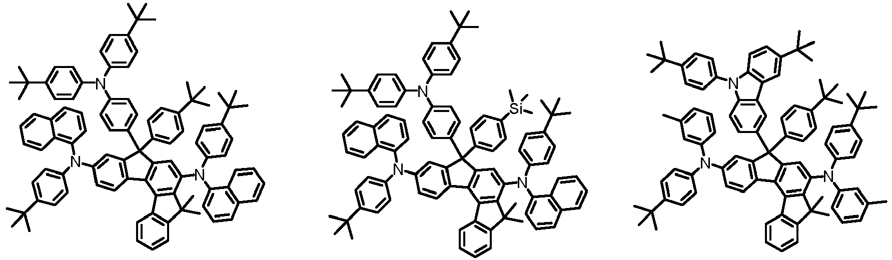

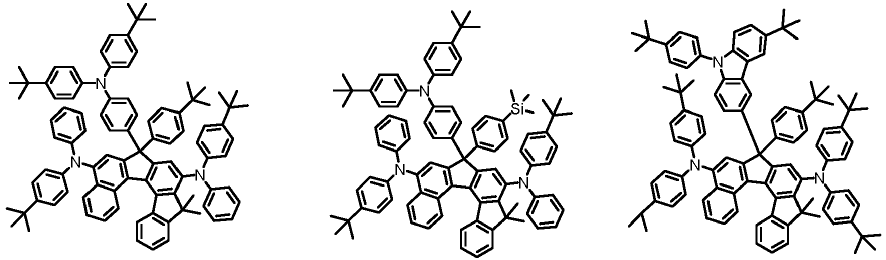

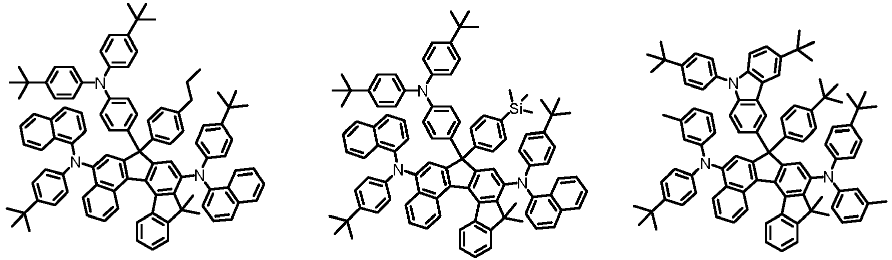

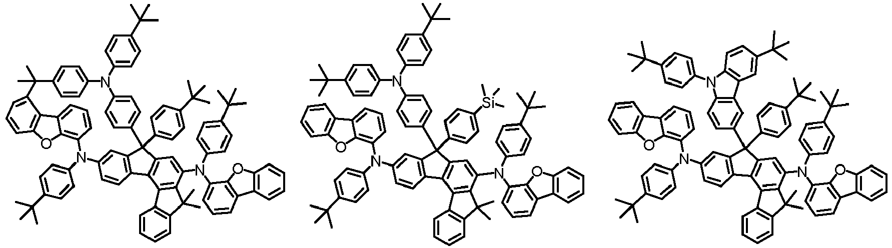

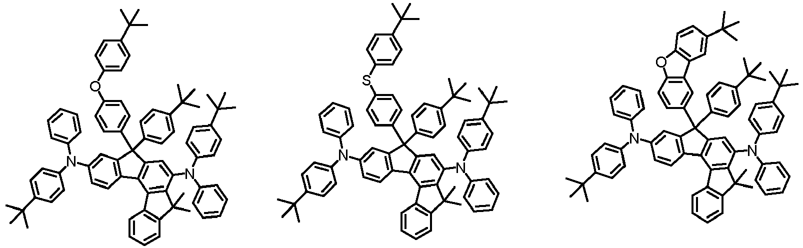

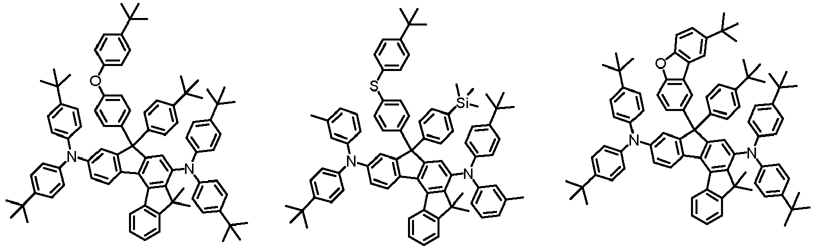

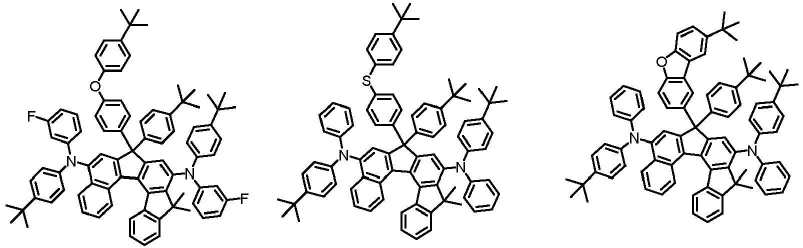

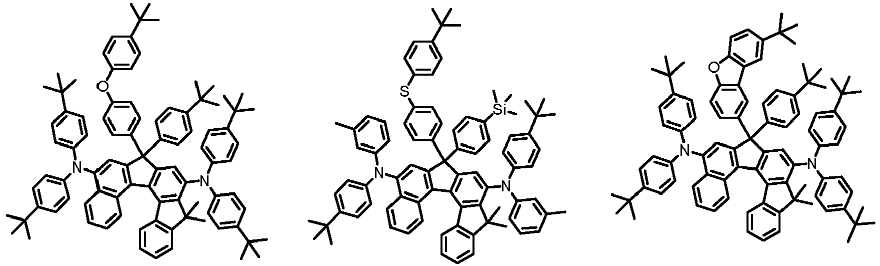

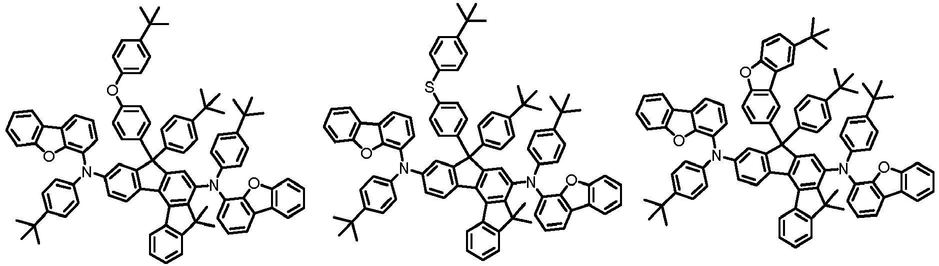

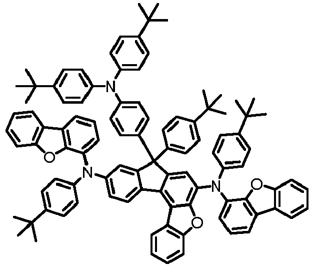

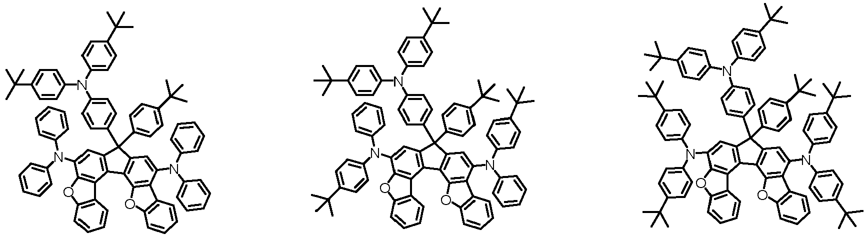

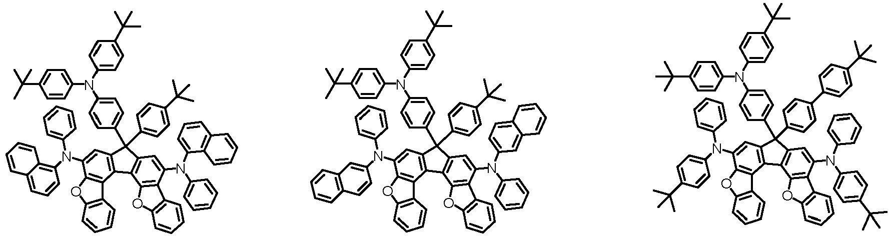

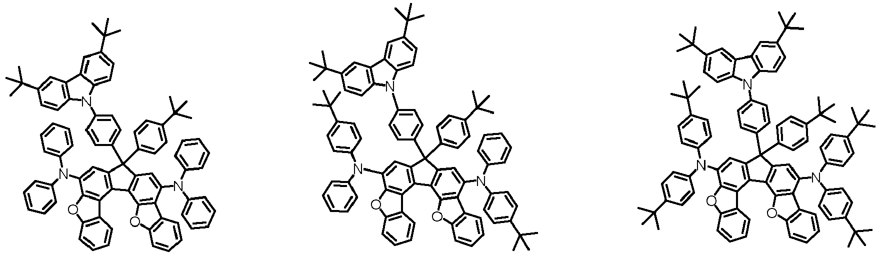

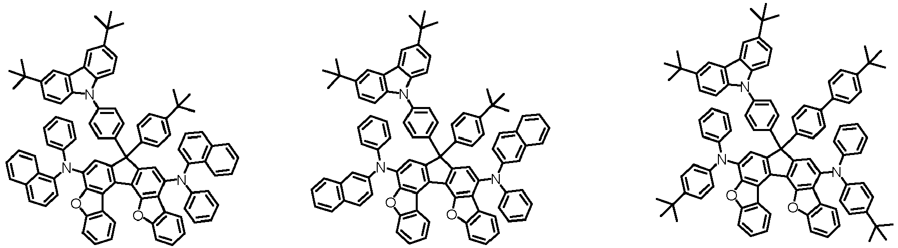

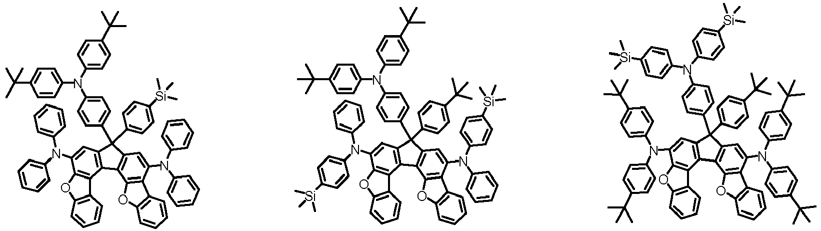

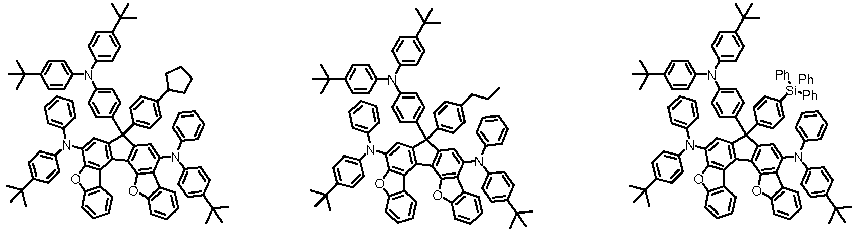

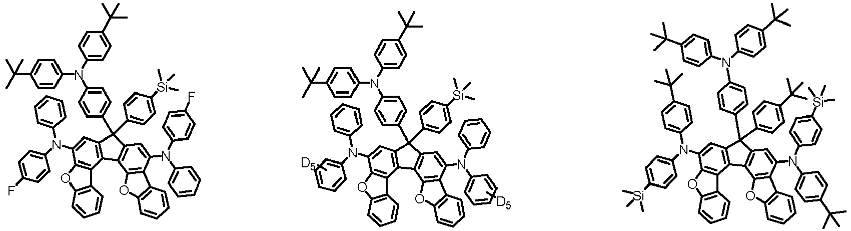

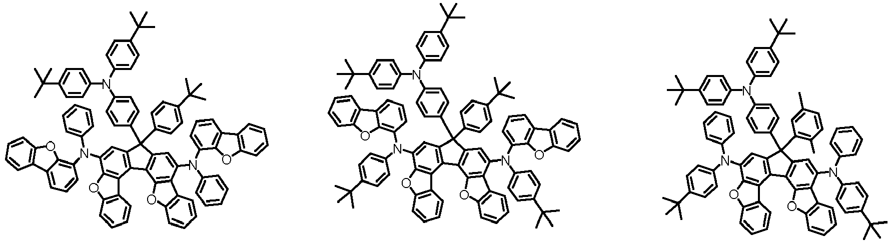

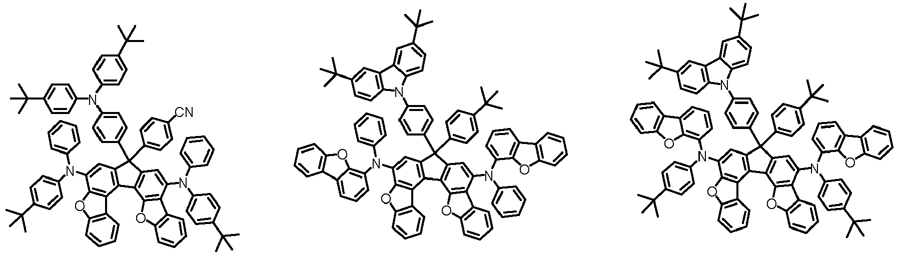

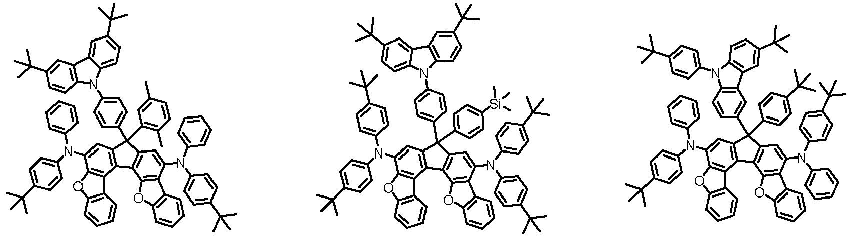

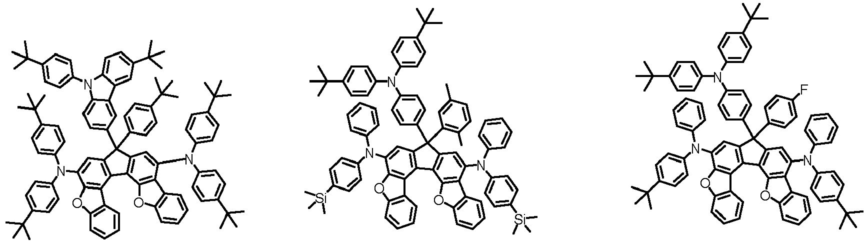

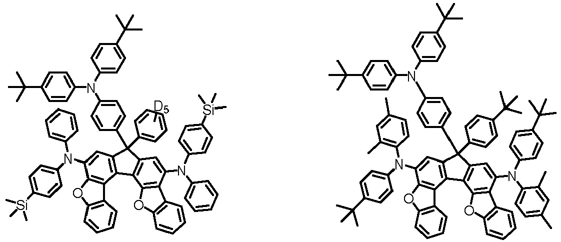

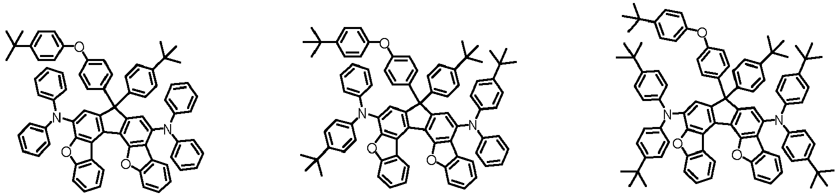

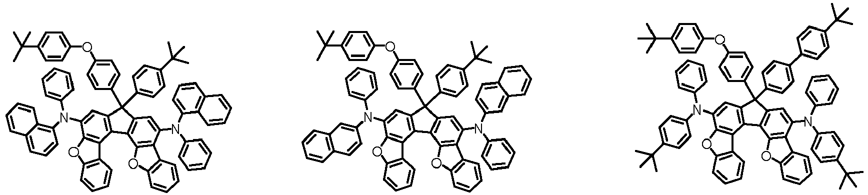

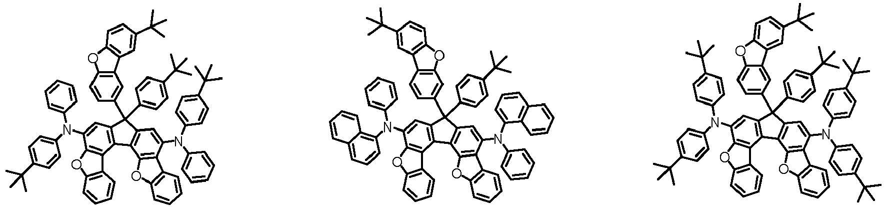

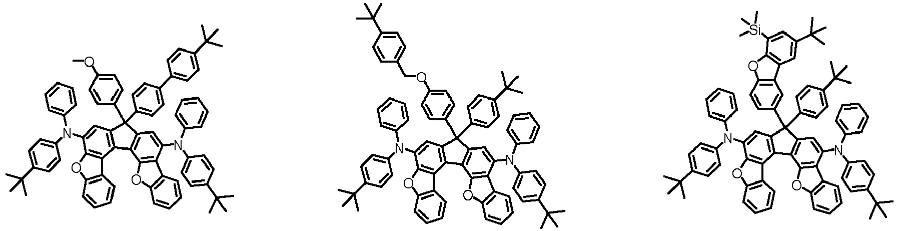

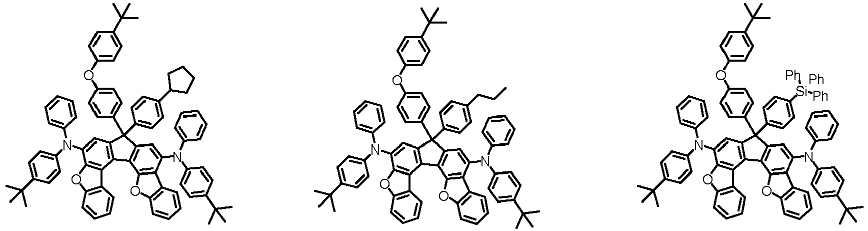

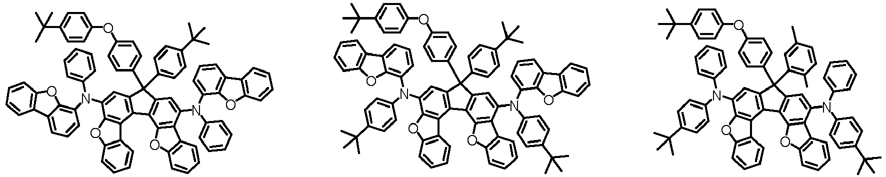

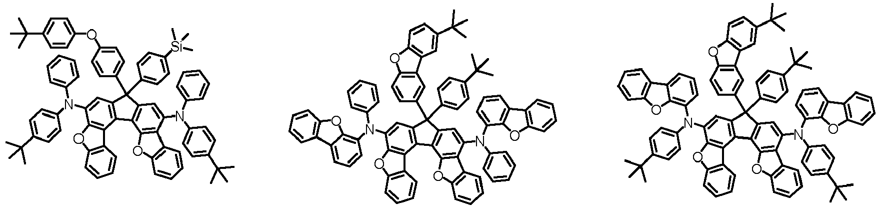

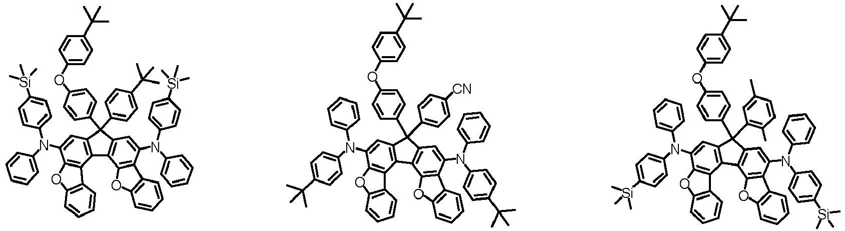

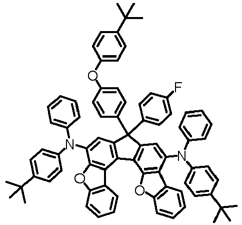

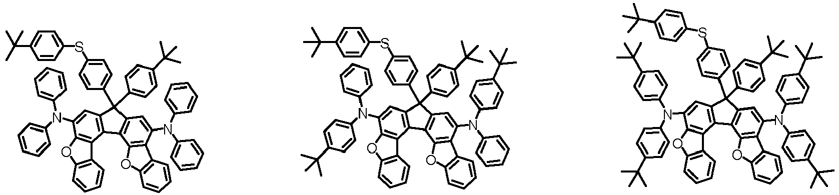

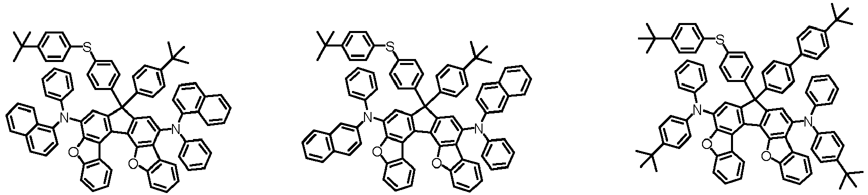

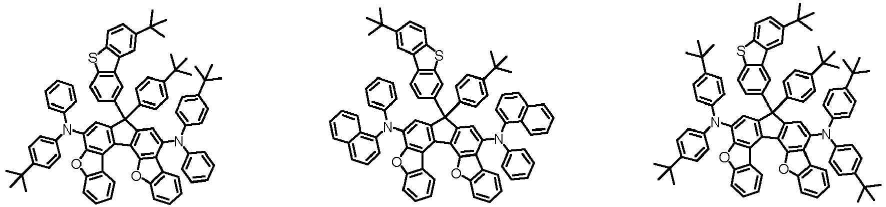

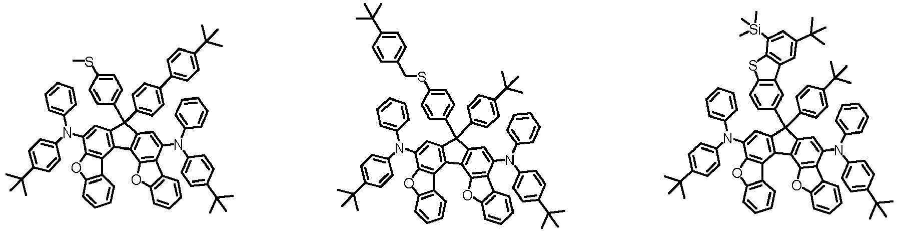

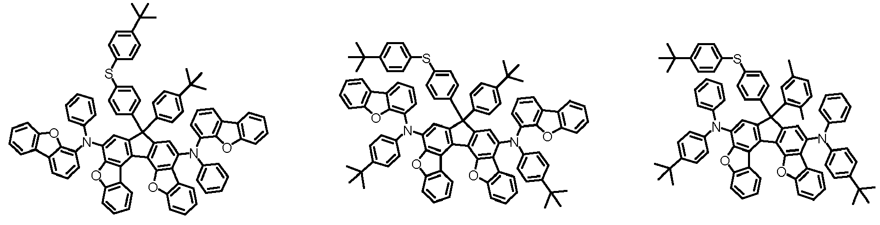

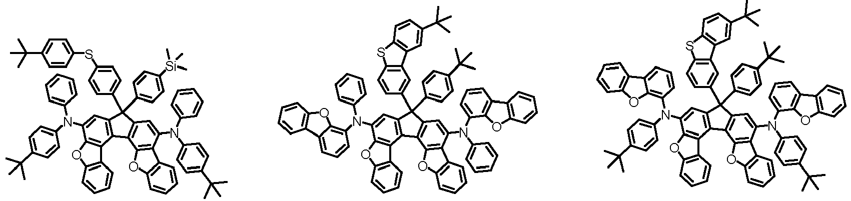

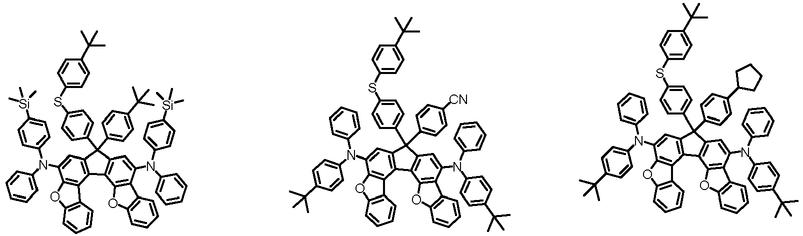

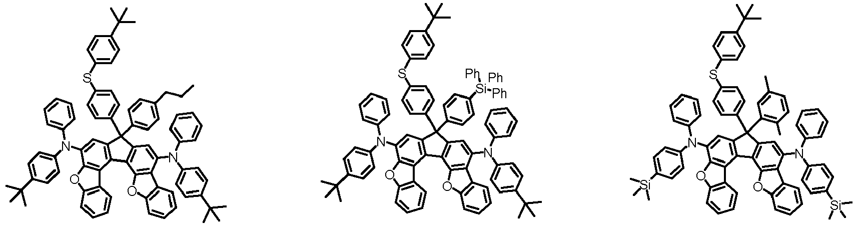

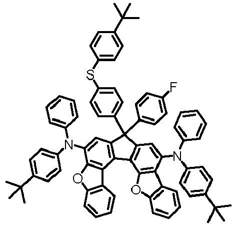

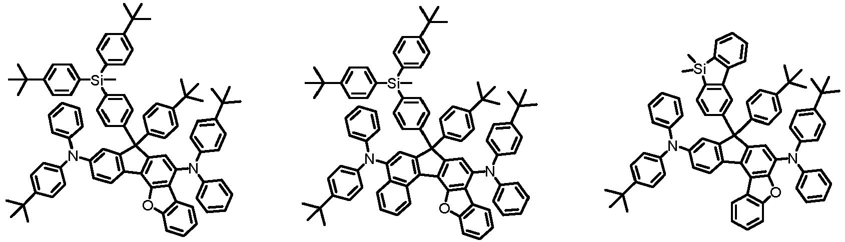

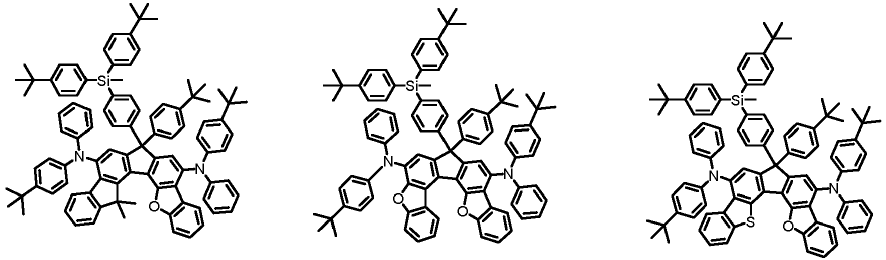

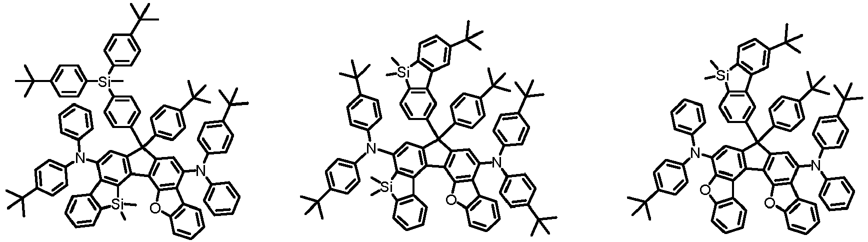

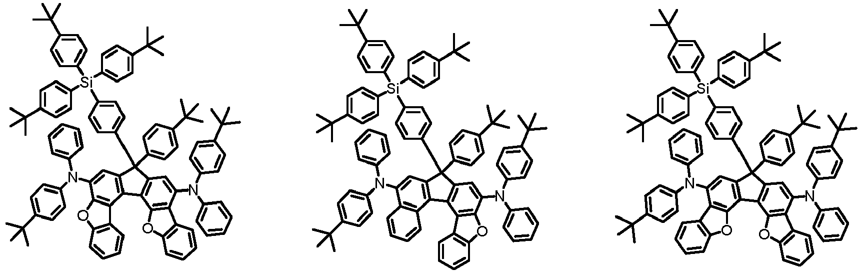

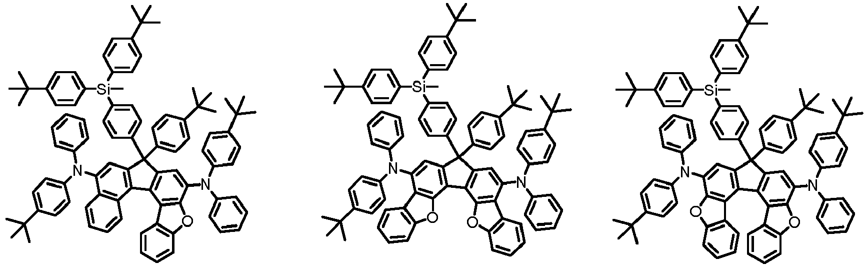

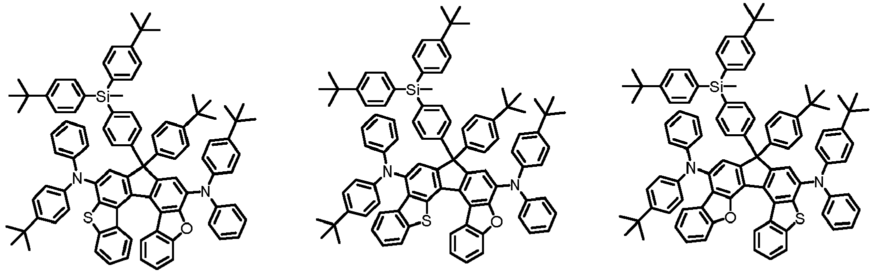

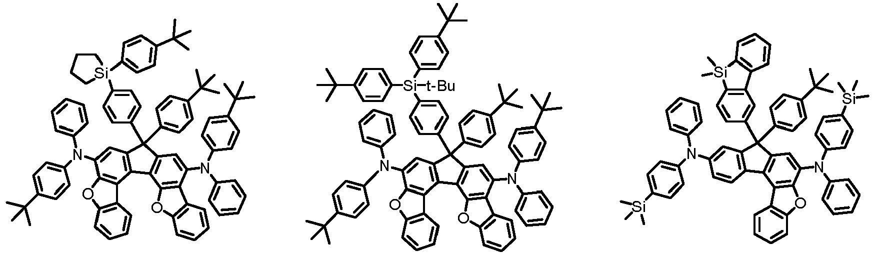

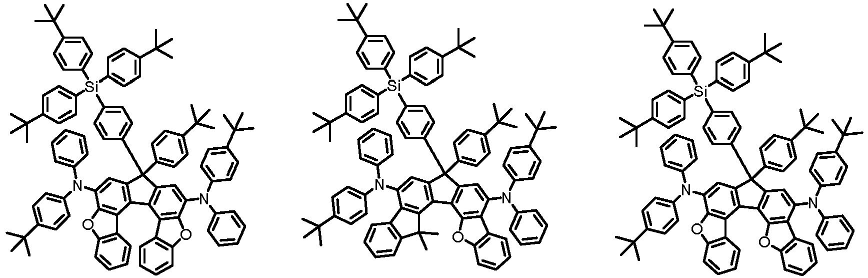

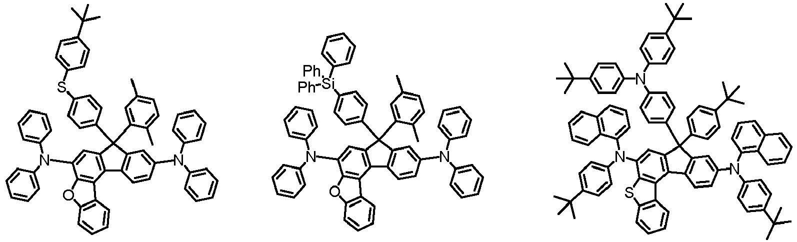

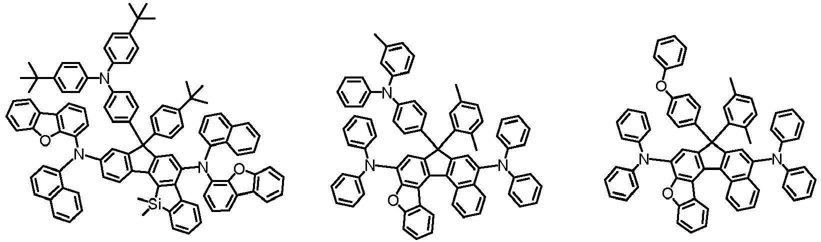

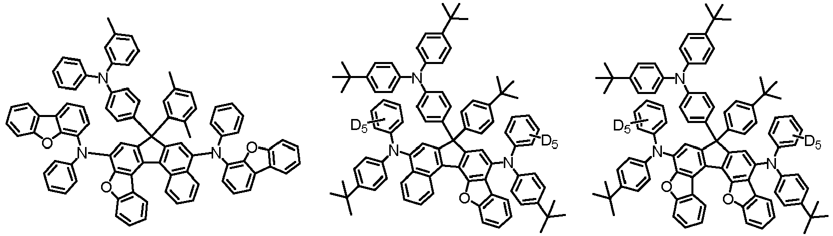

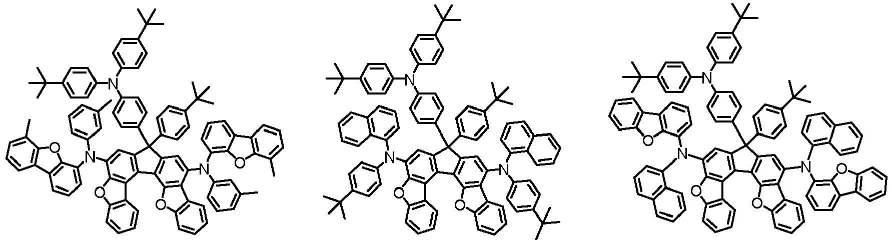

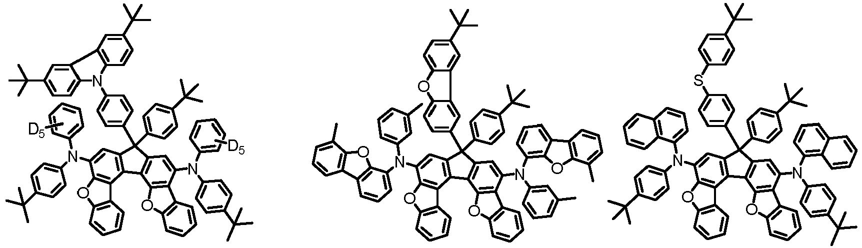

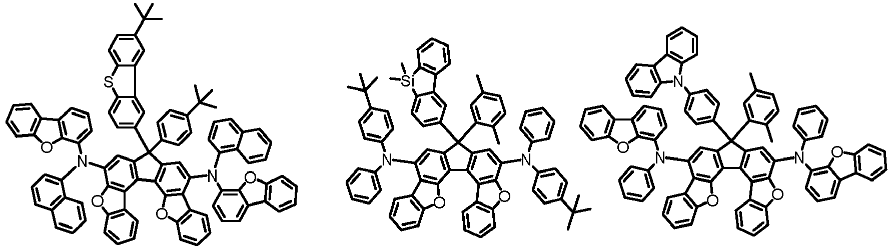

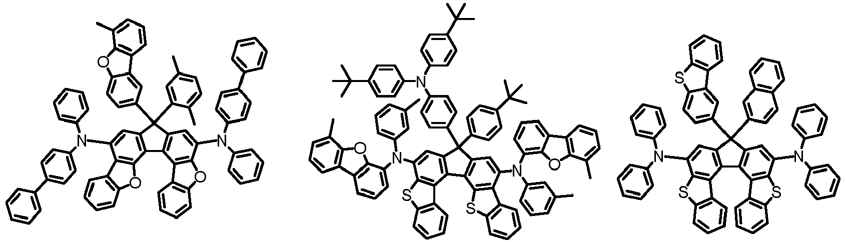

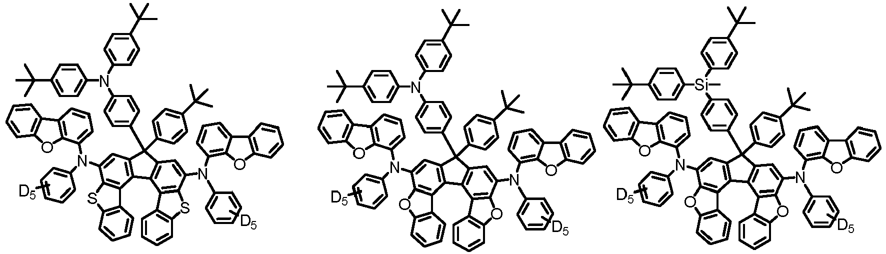

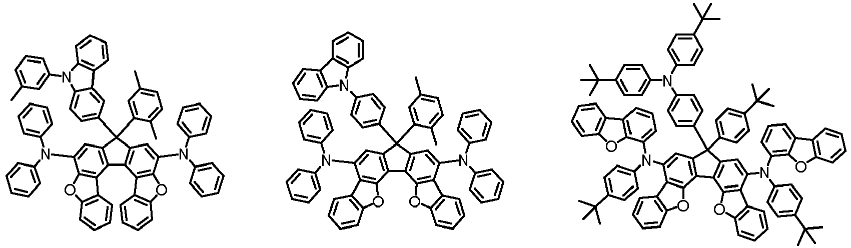

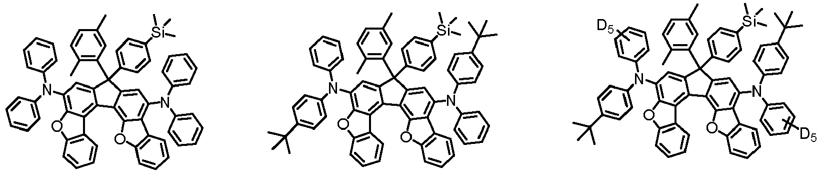

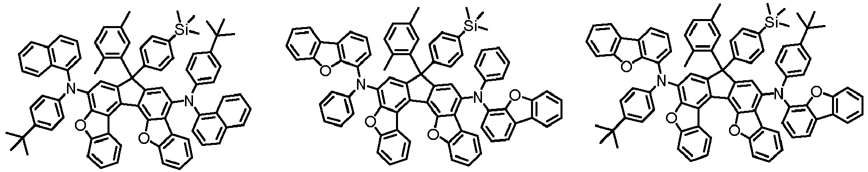

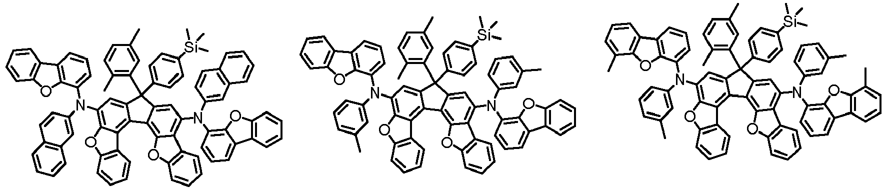

13. An organic light emitting device comprising: a first electrode; a second electrode that is provided opposite to the first electrode; and a light emitting layer that is provided between the first electrode and the second electrode, wherein the light emitting layer includes the compound according to any one of claims 1 to 12.

## Patentansprüche

1. Verbindung, dargestellt durch die folgende chemische Formel 1: 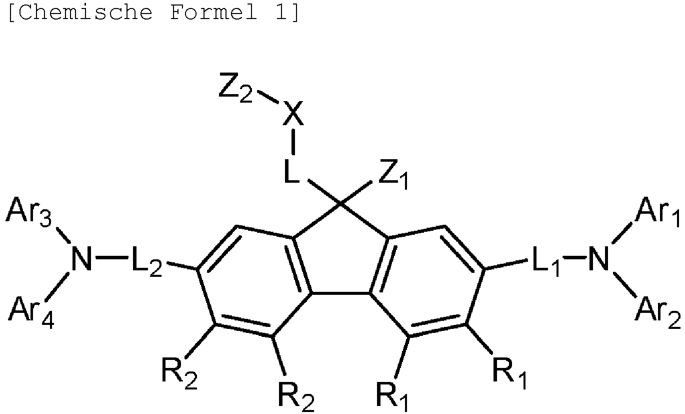 worin in der chemischen Formel 1
R₁ und R₂ jeweils unabhängig Wasserstoff sind, oder zwei R₁ oder zwei R₂ miteinander verbunden sind, um einen substituierten oder unsubstituierten aromatischen C₆₋₆₀-Ring; einen substituierten oder unsubstituierten nicht-aromatischen C₆₋₆₀-Ring; oder einen substituierten oder unsubstituierten heterocyclischen C₂₋₆₀-Ring, der irgendeines oder mehrere Heteroatome enthält, ausgewählt aus der Gruppe bestehend aus N, O, S und Si, zu bilden,
zumindest eines der zwei R₁ oder der zwei R₂ miteinander verbunden sind, um den nicht-aromatischen C₆₋₆₀-Ring; oder den heterocyclischen C₂₋₆₀-Ring zu bilden,
L ein substituiertes oder unsubstituiertes C₆₋₆₀-Arylen oder ein substituiertes oder unsubstituiertes C₂₋₆₀-Heteroarylen, das irgendeines oder mehr Heteroatome umfasst, ausgewählt aus der Gruppe, bestehend aus N, O und S, ist,
X O, S, NZ₃ oder SiZ₄Z₅ ist,
Z₁ substituiertes oder unsubstituiertes C₆₋₆₀-Aryl ist,
Z₂ bis Z₅ jeweils unabhängig ein substituiertes oder unsubstituiertes C₁₋₆₀-Alkyl, ein substituiertes oder unsubstituiertes C₁₋₆₀-Haloalkyl, ein substituiertes oder unsubstituiertes C₃₋₆₀-Cycloalkyl, ein substituiertes oder unsubstituiertes C₇₋₆₀-Aralkyl, ein substituiertes oder unsubstituiertes C₆₋₆₀-Aryl oder ein substituiertes oder unsubstituiertes C₂₋₆₀-Heteroaryl, das irgendeines oder mehr Heteroatome enthält, ausgewählt aus der Gruppe, bestehend aus N, O und S, sind,
die L und Z₂; Z₂ und Z₃; oder Z₄ und Z₅ optional miteinander verbunden sind, um einen 5-gliedrigen heterocyclischen Ring zu bilden,
mit der Maßgabe, dass Z₁ und LX-Z₂ sich voneinander unterscheiden und nicht miteinander verbunden sind,
L₁ und L₂ jeweils unabhängig eine Einfachbindung; ein substituiertes oder unsubstituiertes C₆₋₆₀-Arylen; oder ein substituiertes oder unsubstituiertes C₂₋₆₀-Heteroarylen, das irgendeines oder mehr Heteroatome enthält, ausgewählt aus der Gruppe, bestehend aus N, O und S, sind, und
Ar₁ bis Ar₄ jeweils unabhängig ein substituiertes oder unsubstituiertes C₆₋₆₀-Aryl; oder ein substituiertes oder unsubstituiertes C₂₋₆₀-Heteroaryl, das irgendeines oder mehr Heteroatome enthält, ausgewählt aus der Gruppe bestehend aus N, O und S, sind.

2. Verbindung gemäß Anspruch 1,
worin zumindest eines von den zwei R₁ oder den zwei R₂ miteinander verbunden sind, um irgendeine der durch die folgenden chemischen Formeln 2d bis 2g dargestellten Strukturen zu bilden, der Rest Wasserstoff sind oder miteinander verbunden sind, um irgendeine der durch die folgenden chemischen Formeln 2a bis 2g dargestellten Strukturen zu bilden: 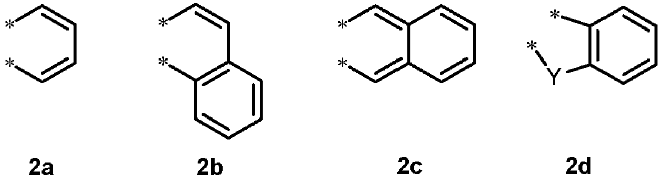 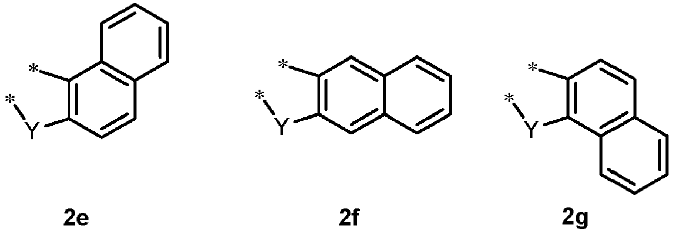 worin in den chemischen Formeln 2a bis 2g
Y O, S, CZ₆Z₇, oder SiZ₈Z₉ ist,
die Z₆ bis Z₉ jeweils unabhängig C₁₋₁₀-Alkyl oder C₆₋₂₀-Aryl sind und
* eine Bindungsposition an den an R₁ oder R₂ gebundenen Kohlenstoff bezeichnet.

3. Verbindung gemäß Anspruch 2,
worin die durch die chemische Formel 1 dargestellte Verbindung durch irgendeine der folgenden chemischen Formeln 1-1, 1-3 und 1-5 dargestellt ist: 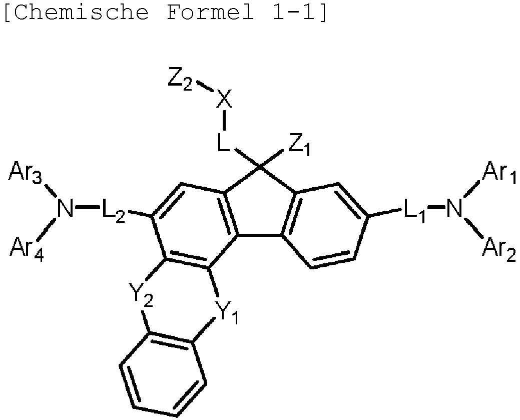 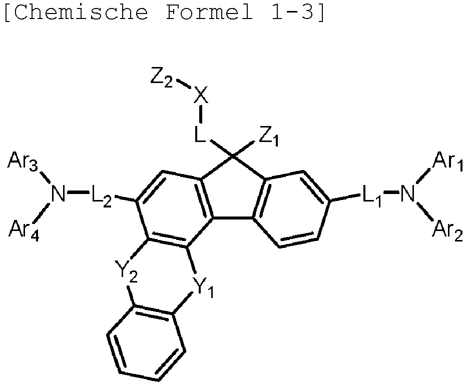 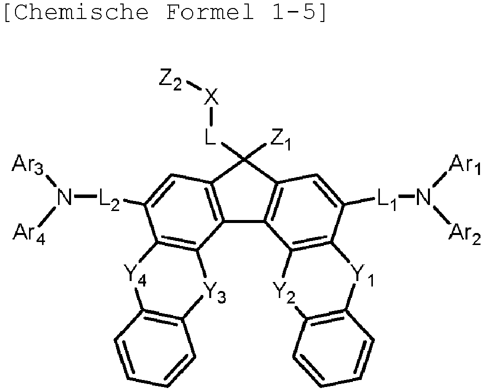 worin in den chemischen Formeln 1-1, 1-3 und 1-5
L, X, Z₁, Z₂, L₁, L₂ und Ar₁ bis Ar₄ wie in Anspruch 1 definiert sind,
eines von Y₁ und Y₂ eine Einfachbindung ist und das andere O, S, C(C₁₋₄-Alkyl)₂ oder Si(C₁₋₄-Alkyl)₂ ist; und
eines von Y₃ und Y₄ eine Einfachbindung ist und das andere eines von O, S, C(1-₄-Alkyl)₂ oder Si(C₁₋₄-Alkyl)₂ ist.

4. Verbindung gemäß Anspruch 1,
worin Z₁ Phenyl oder Biphenylyl ist,
worin Z₁ unsubstituiert ist oder substituiert ist mit 1 Substituent bis 5 Substituenten, unabhängig ausgewählt aus der Gruppe bestehend aus Deuterium, Halogen, Cyano, C₁₋₁₀-Alkyl, C₃₋₁₀-Cycloalkyl, Si(C₁₋₁₀-Alkyl)₃ und Si(C₆₋₂₀-Aryl)₃.

5. Verbindung gemäß Anspruch 1,
worin Z₁ irgendeines ist, das ausgewählt ist aus der Gruppe bestehend aus:

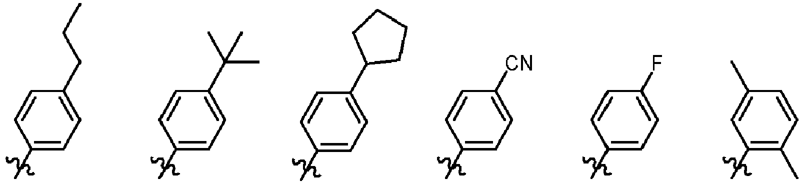

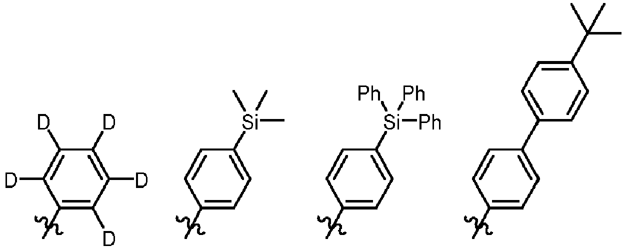

6. Verbindung gemäß Anspruch 1,
worin L-X-Z₂ irgendeines ist, das ausgewählt ist aus der Gruppe bestehend aus: 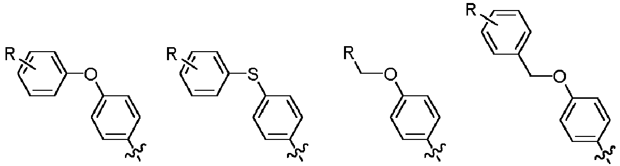 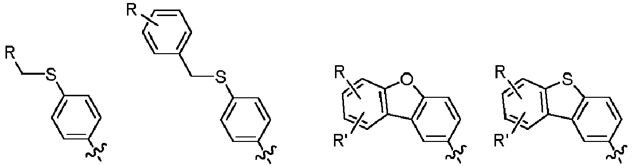 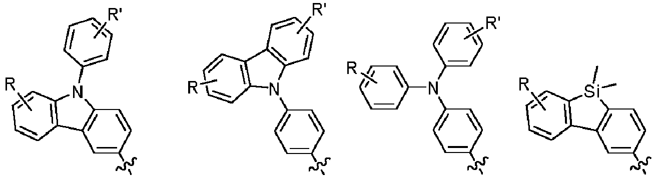 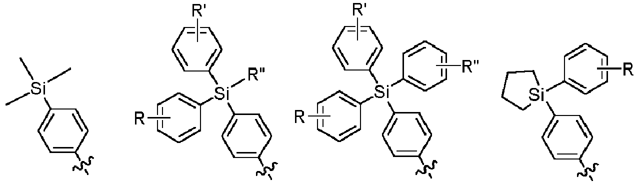 worin
R, R' und R" jeweils unabhängig Wasserstoff, Methyl, tert-Butyl oder Si(Methyl)₃ sind.

7. Verbindung gemäß Anspruch 1
worin L₁ und L₂ eine Einfachbindung sind.

8. Verbindung gemäß Anspruch 1,
worin Ar₁ bis Ar₄ jeweils unabhängig Phenyl, Naphthyl, Biphenylyl oder Dibenzofuranyl sind,
worin Ar₁ bis Ar₄ unsubstituiert sind oder mit 1 Substituent bis 5 Substituenten substituiert sind, jeweils unabhängig ausgewählt aus der Gruppe bestehend aus Deuterium, Halogen, C₁₋₁₀-Alkyl und Si(C₁₋₁₀-Alkyl)₃.

9. Verbindung gemäß Anspruch 1,
worin Ar₁ bis Ar₄ jeweils unabhängig irgendeines sind, das ausgewählt ist aus der Gruppe bestehend aus:

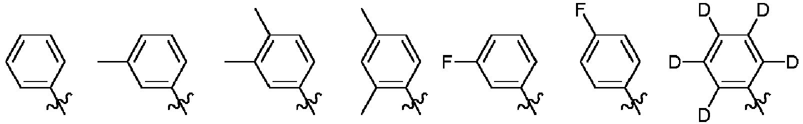

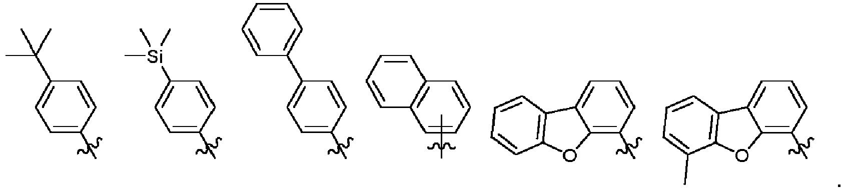

10. Verbindung gemäß Anspruch 1,
worin Ar₁ bis Ar₄ zueinander identisch sind und
Ar₂ und Ar₃ zueinander identisch sind.

11. Verbindung gemäß Anspruch 1,
worin die durch die chemische Formel 1 dargestellte Verbindung durch irgendeine der folgenden chemischen Formeln 2-1, 2-2, 2-4, 2-5, 2-7, 2-8 und 2-10 dargestellt ist: 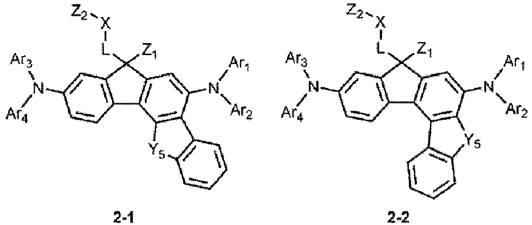 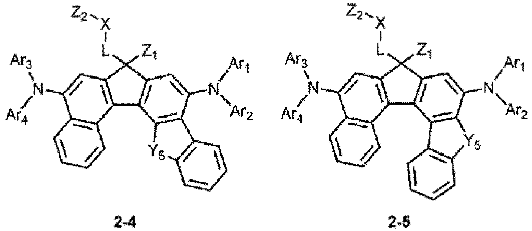 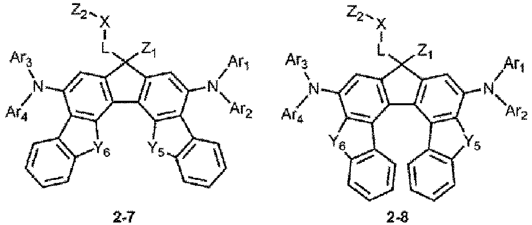 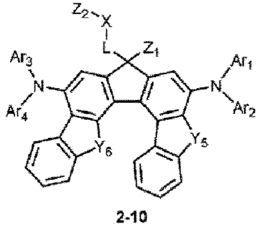 worin in den chemischen Formeln 2-1, 2-2, 2-4, 2-5, 2-7, 2-8 und 2-10
L, X, Z₁, Z₂ und Ar₁ bis Ar₄ wie in Anspruch 1 definiert sind und
Y₅ und Y₆ jeweils unabhängig O, S, C(Methyl)₂ oder Si(Methyl)₂ sind.

12. Verbindung gemäß Anspruch 1,
worin die Verbindung irgendeine der folgenden Verbindungen ist:

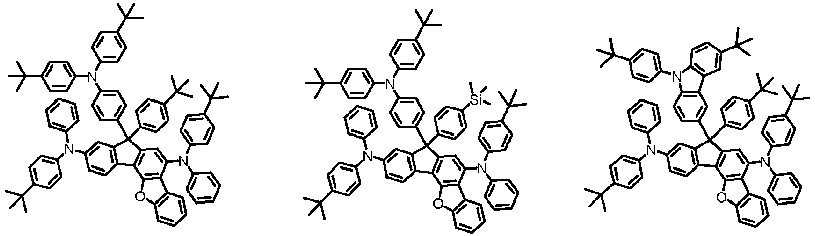

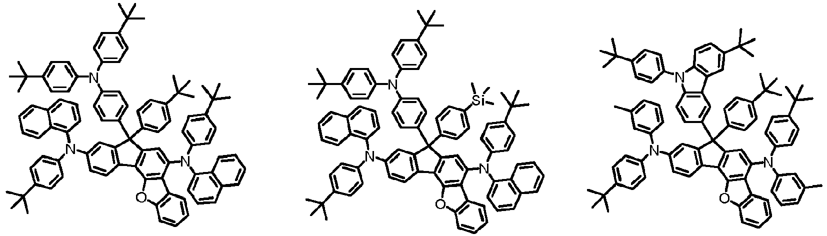

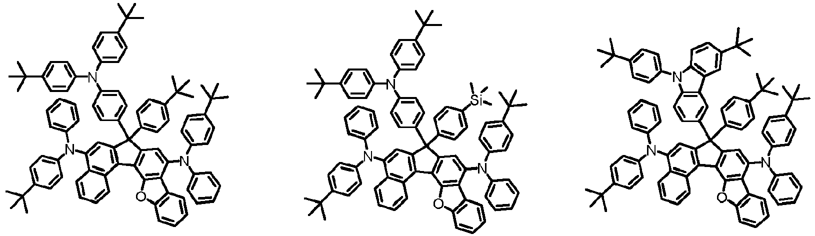

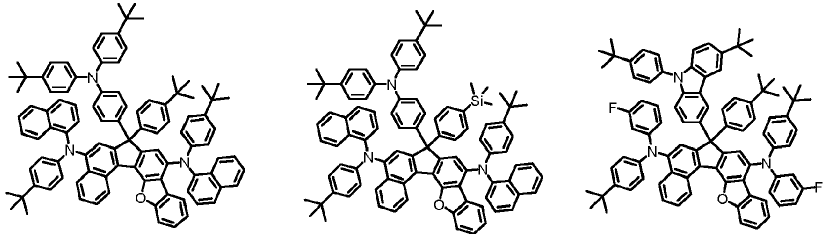

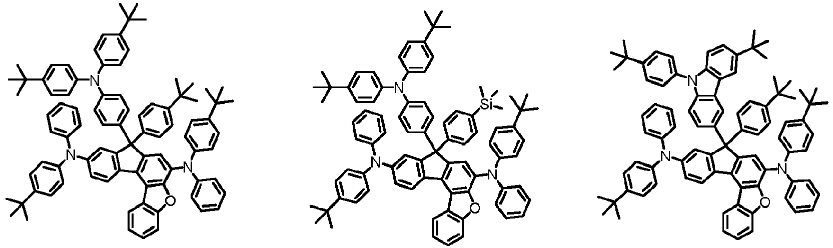

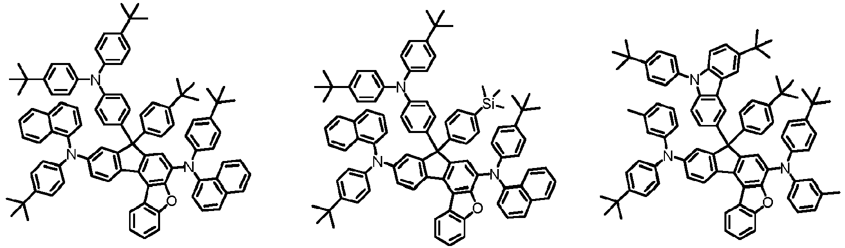

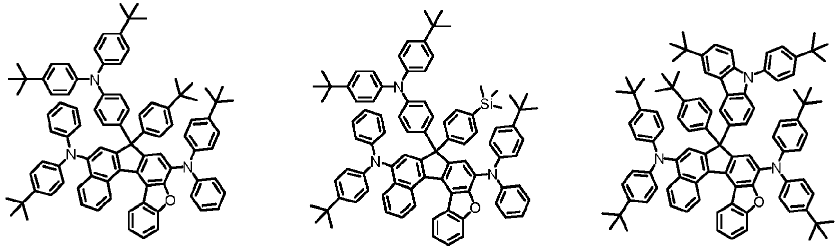

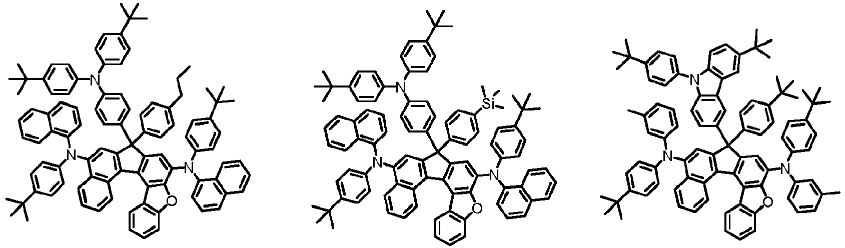

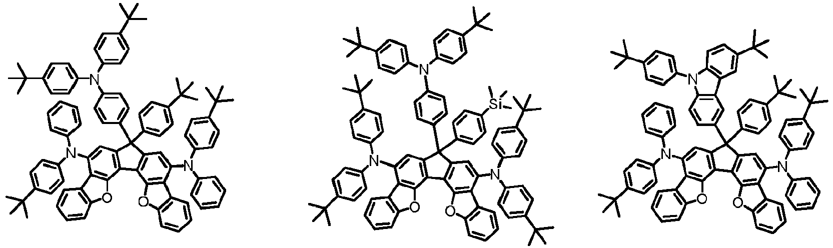

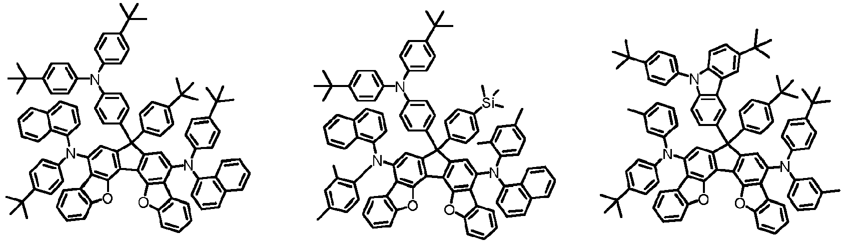

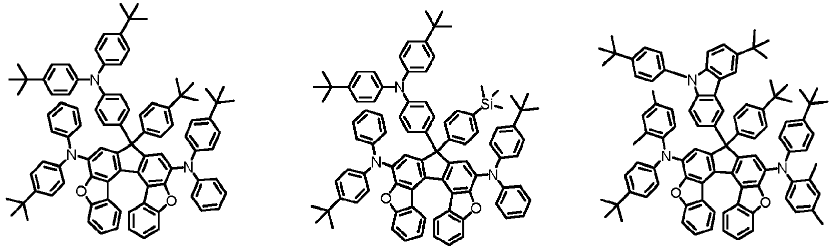

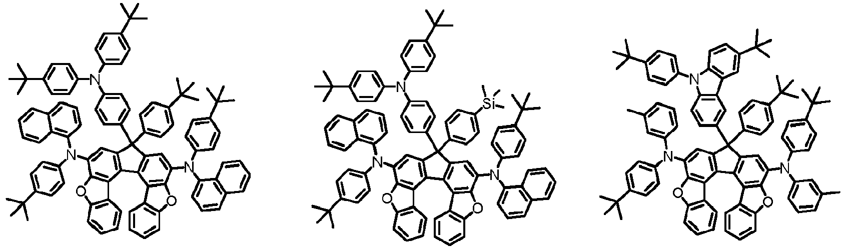

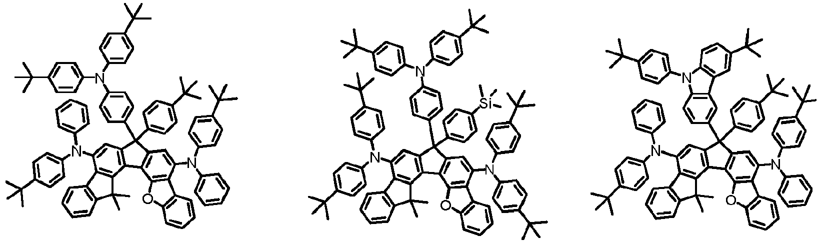

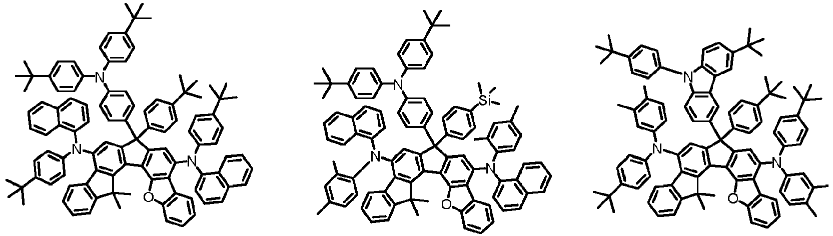

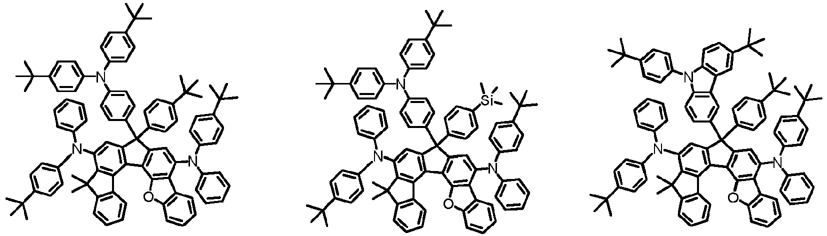

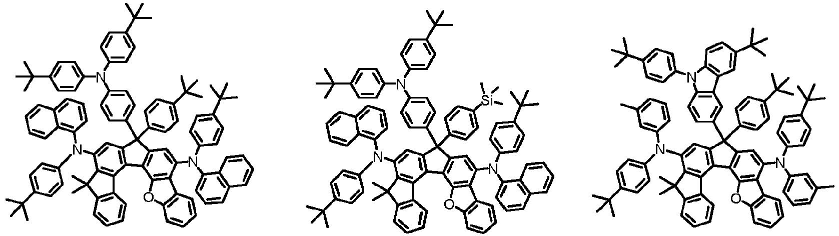

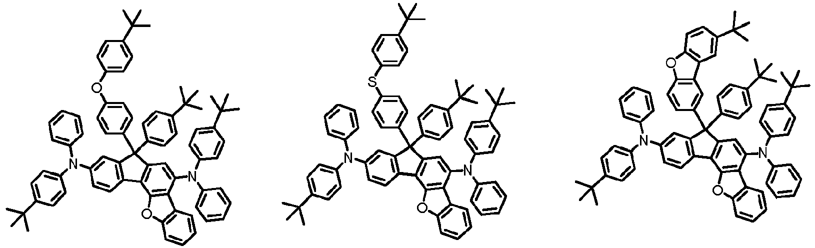

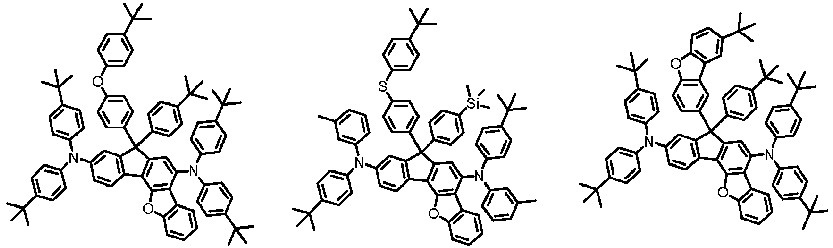

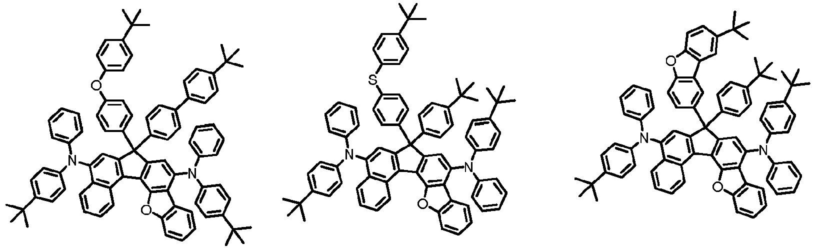

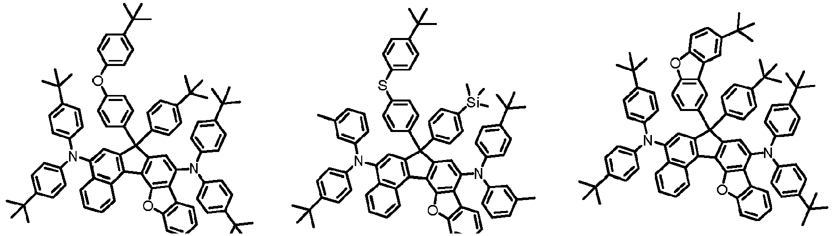

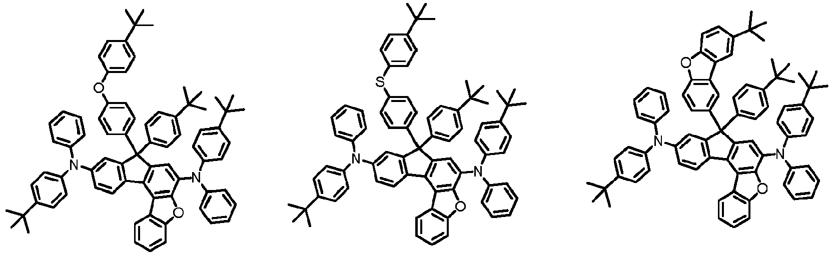

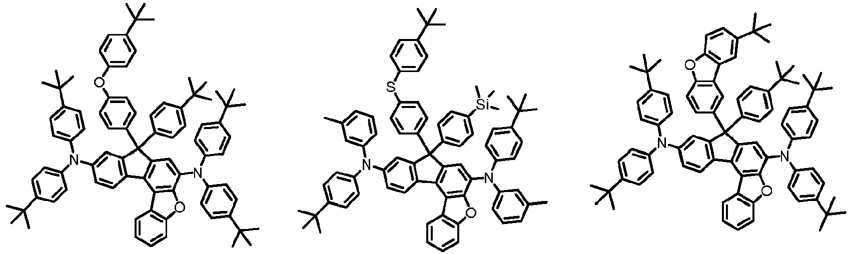

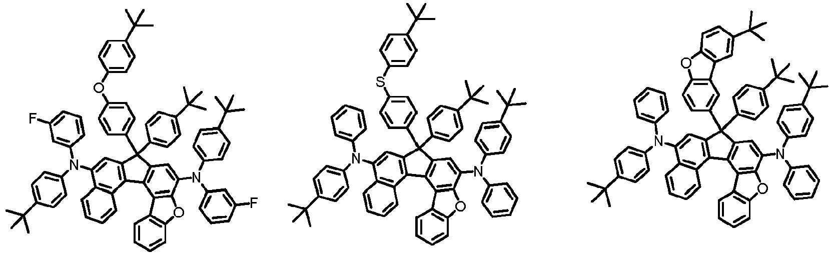

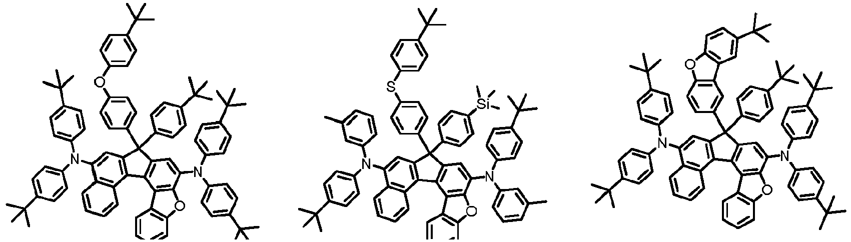

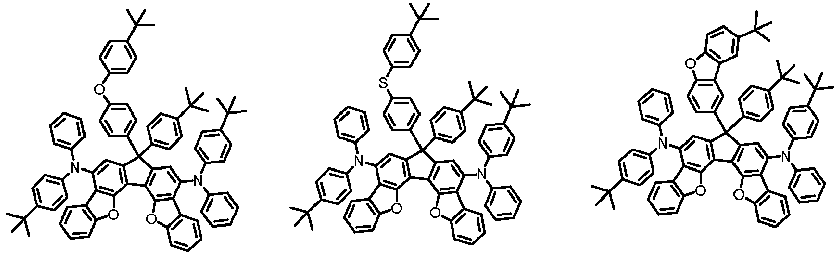

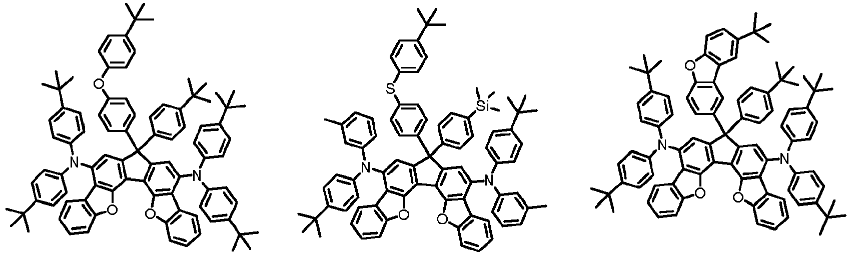

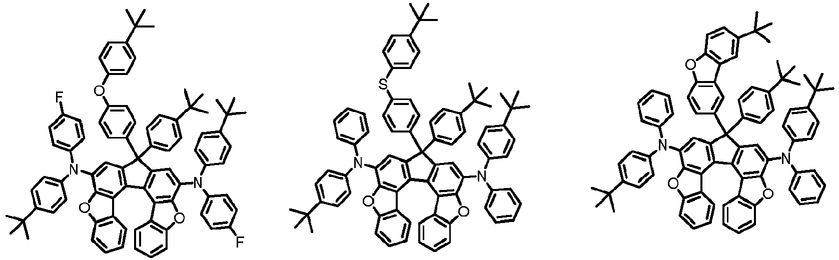

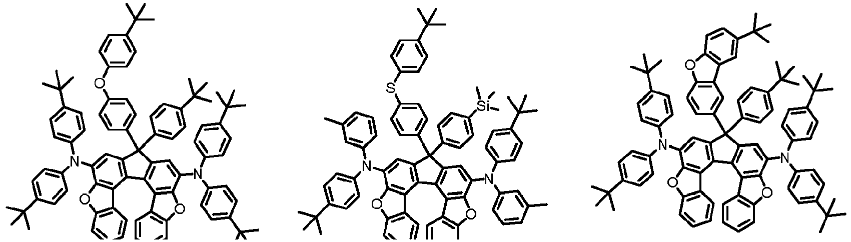

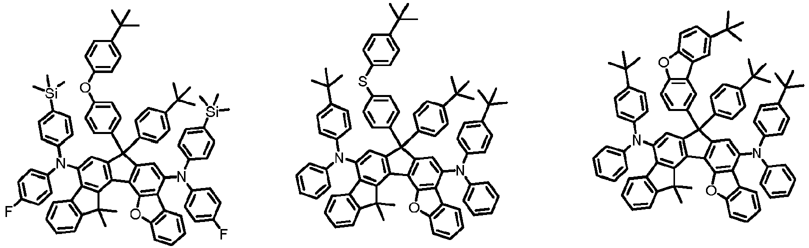

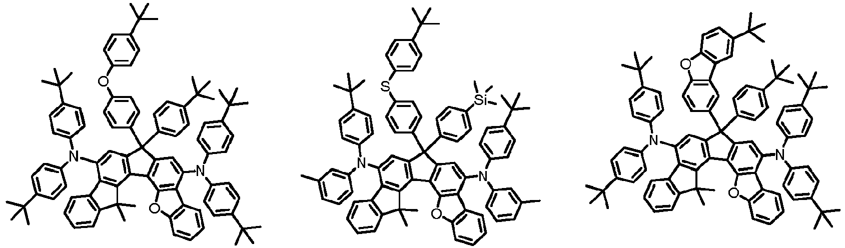

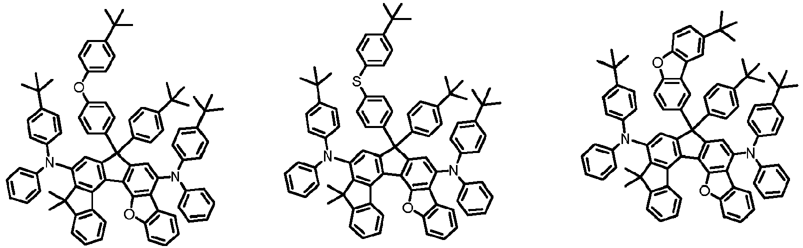

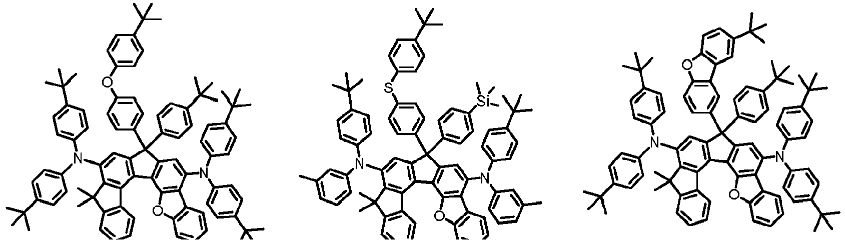

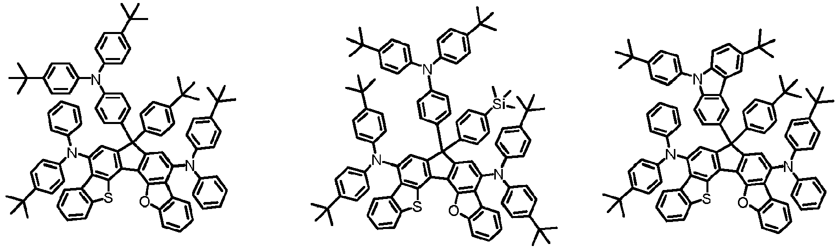

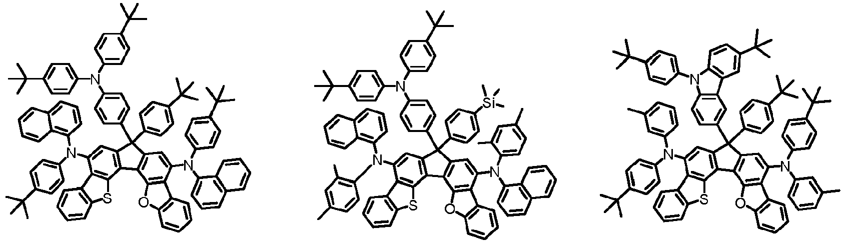

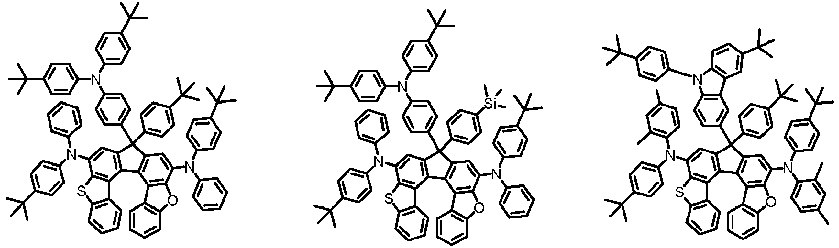

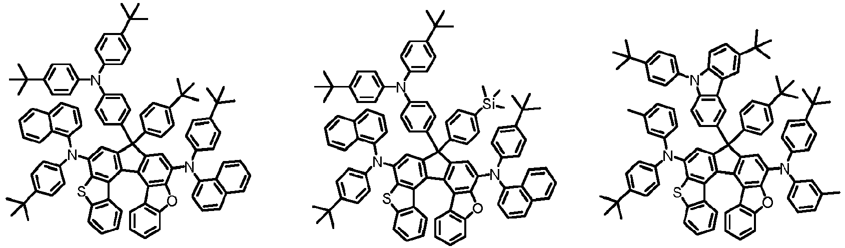

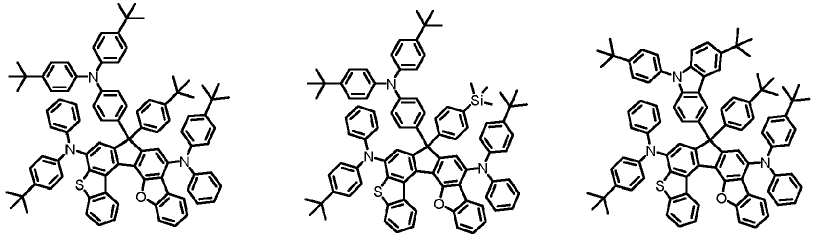

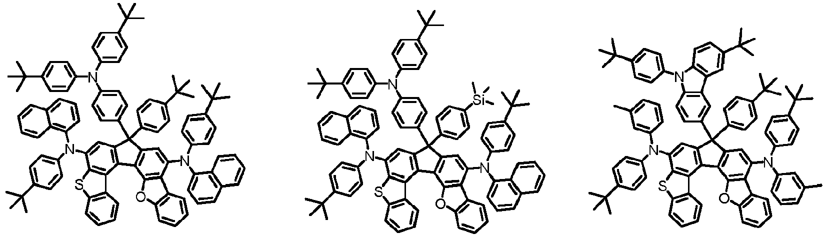

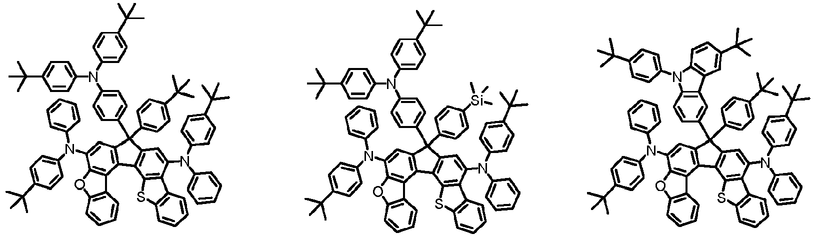

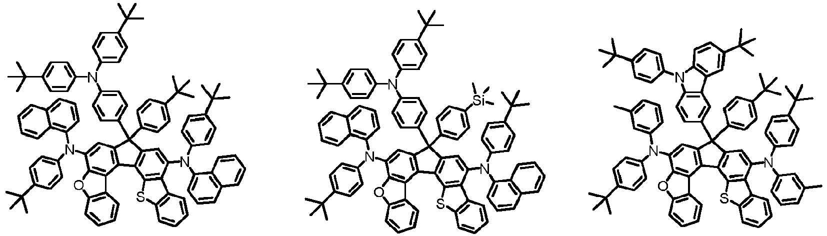

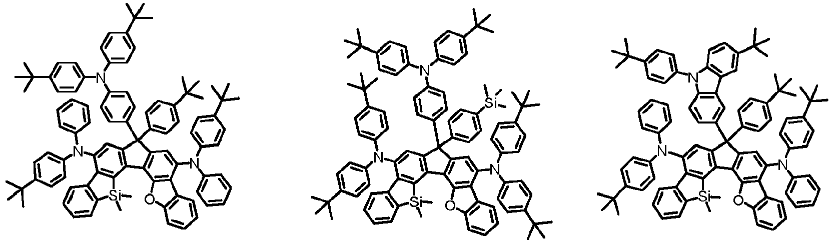

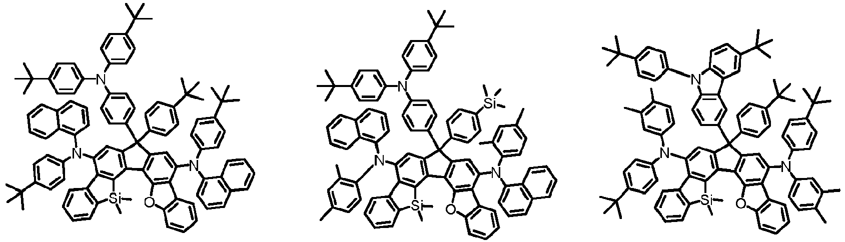

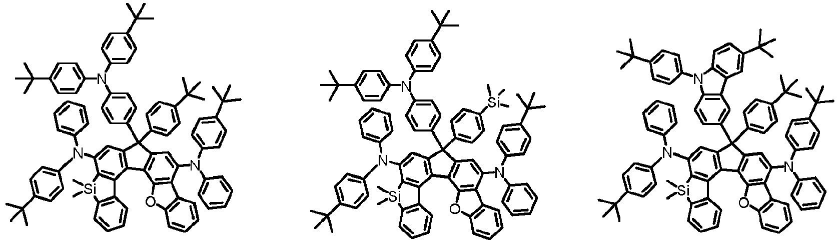

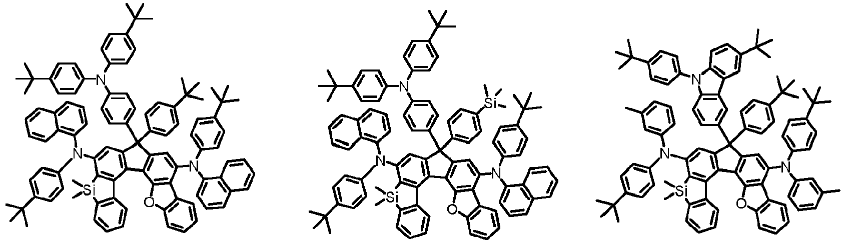

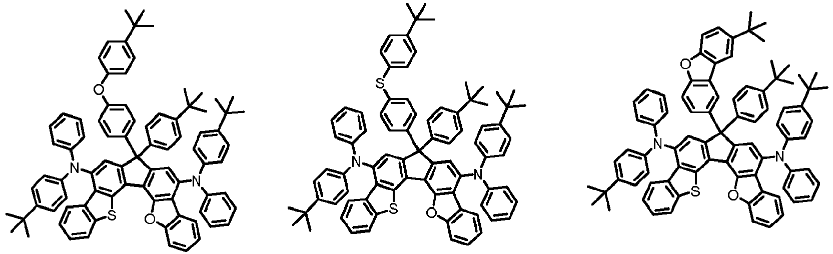

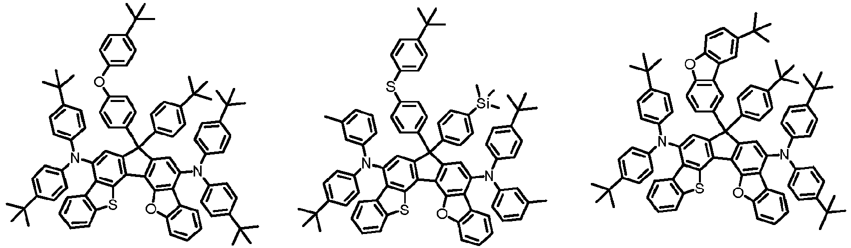

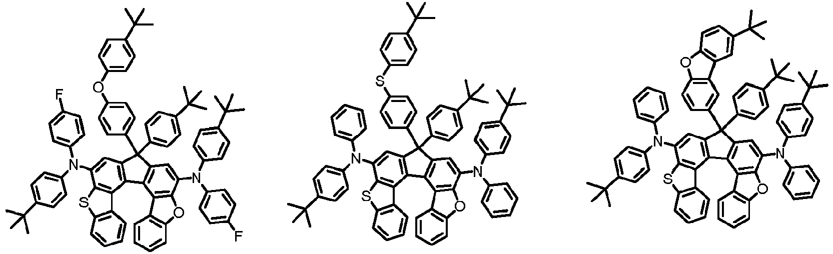

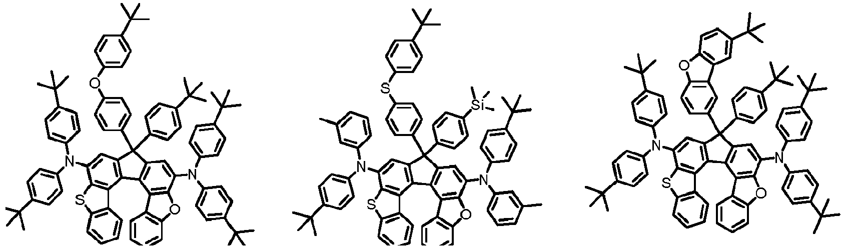

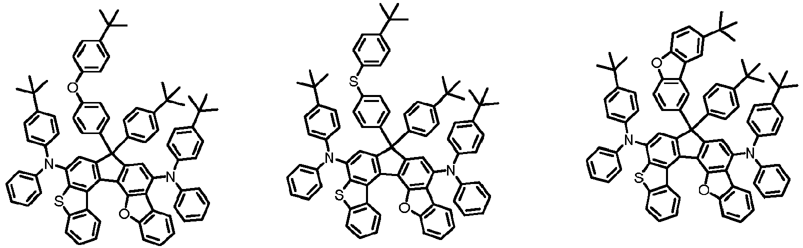

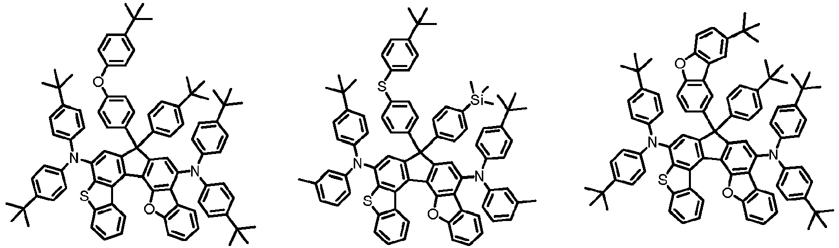

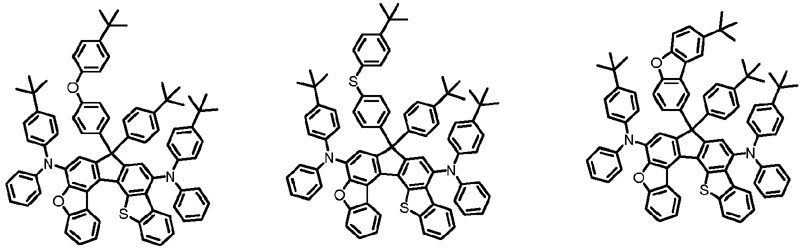

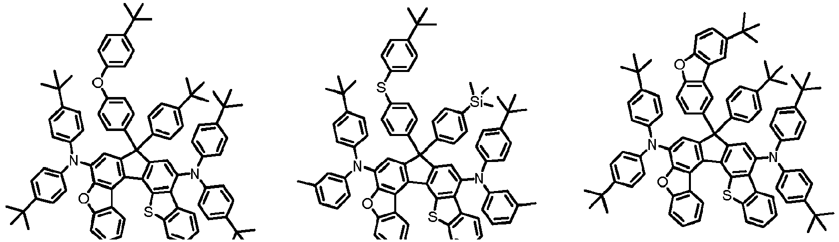

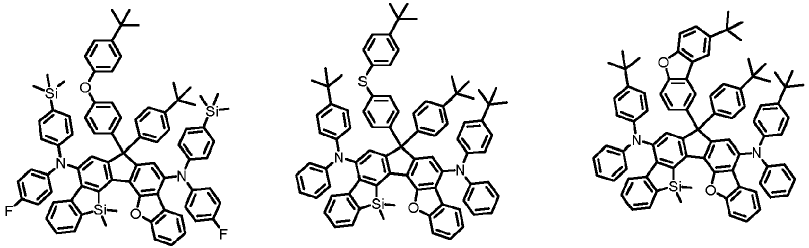

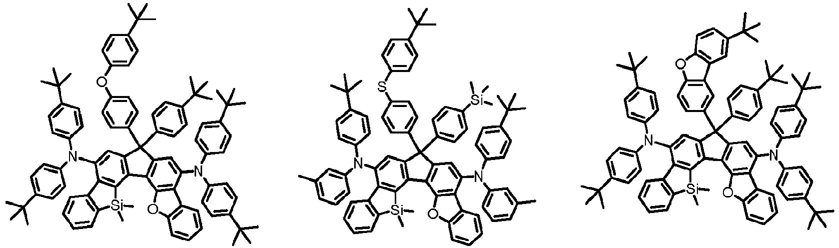

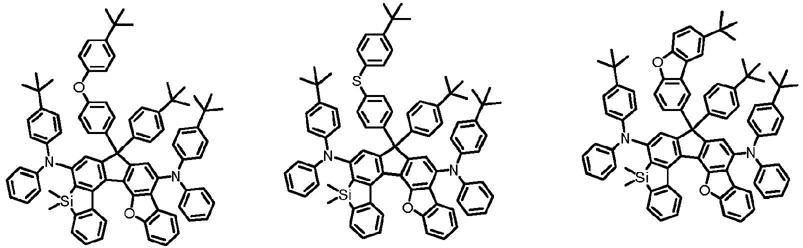

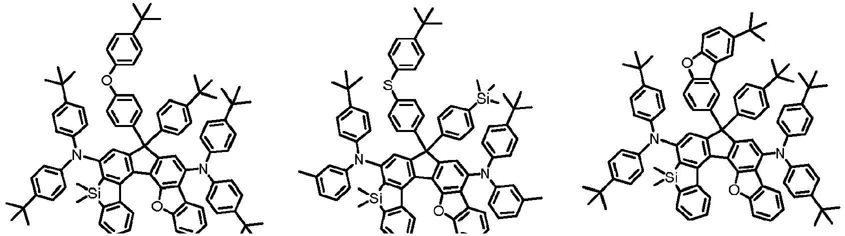

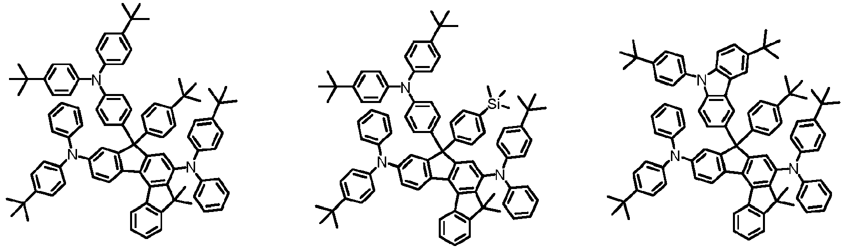

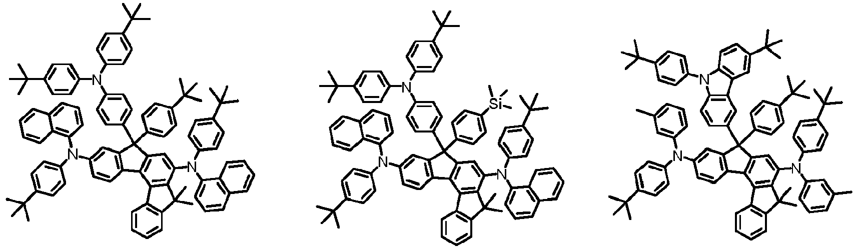

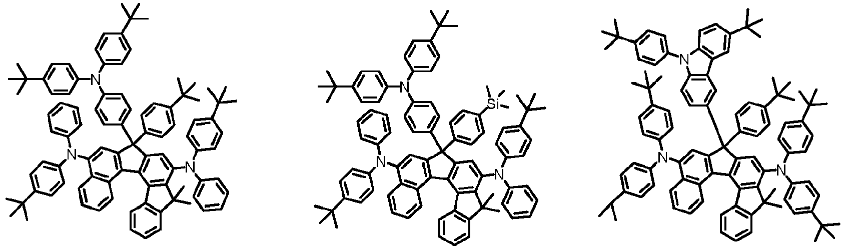

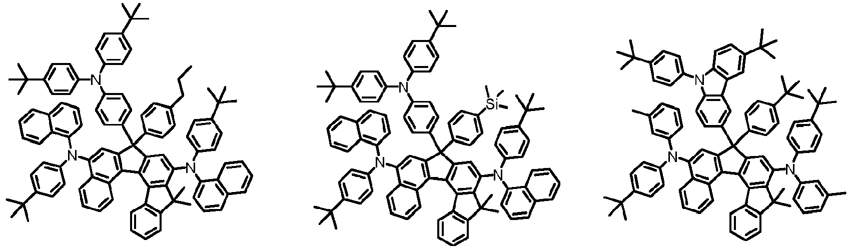

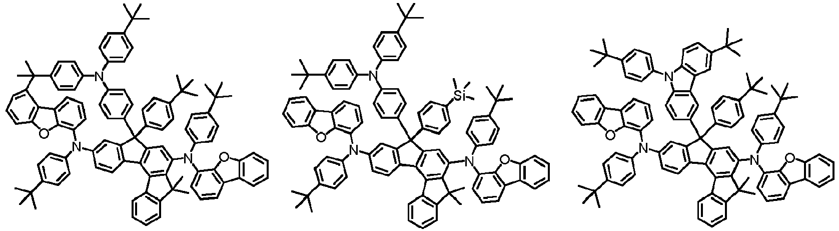

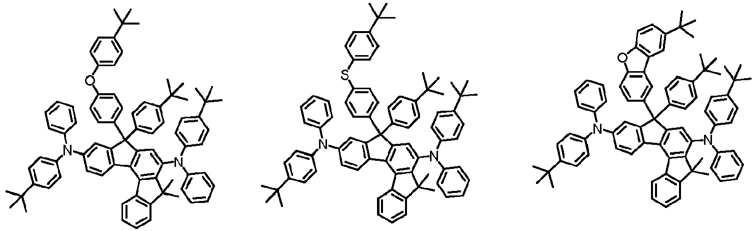

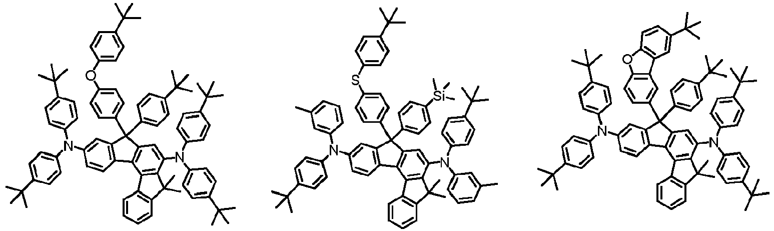

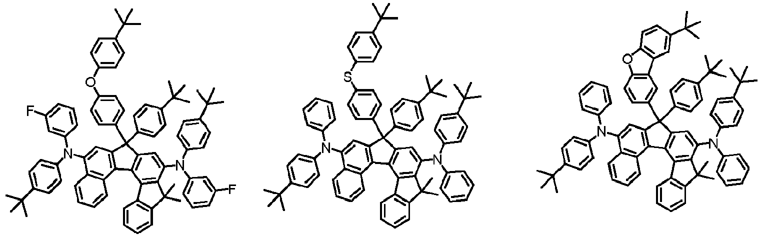

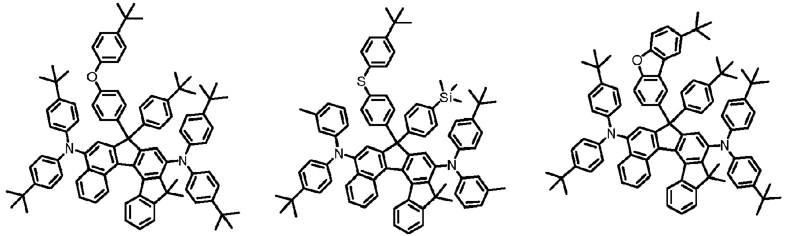

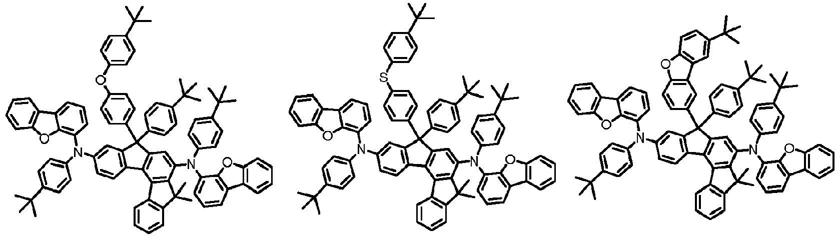

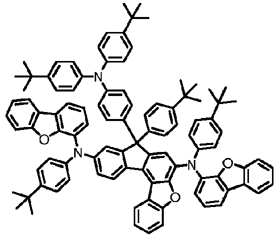

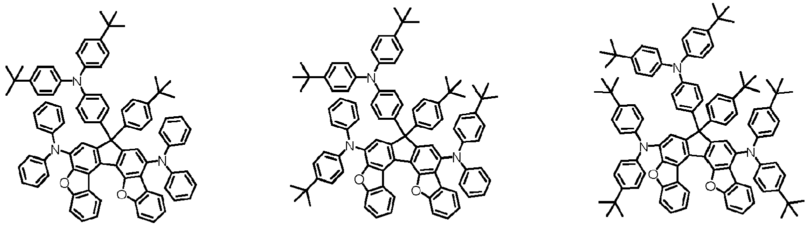

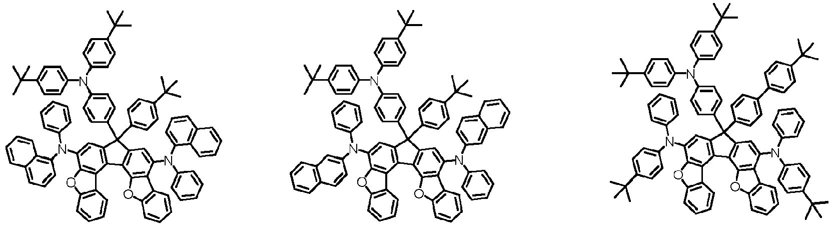

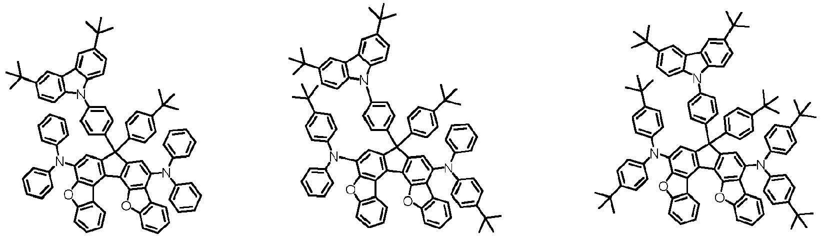

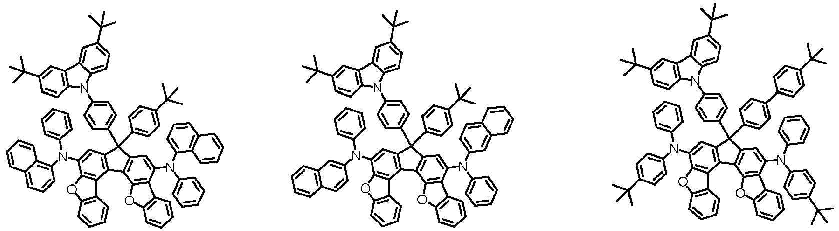

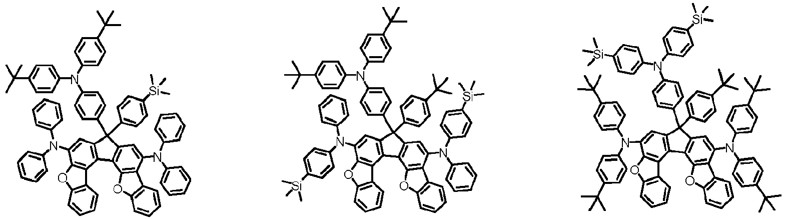

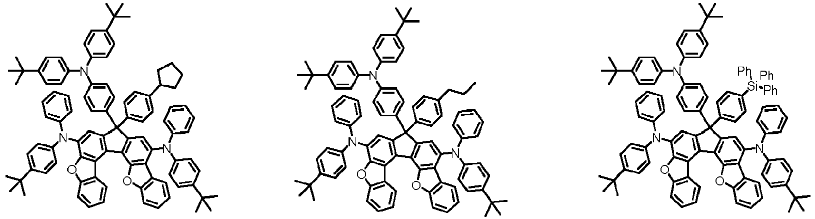

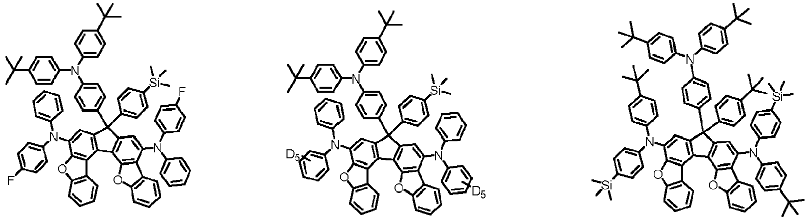

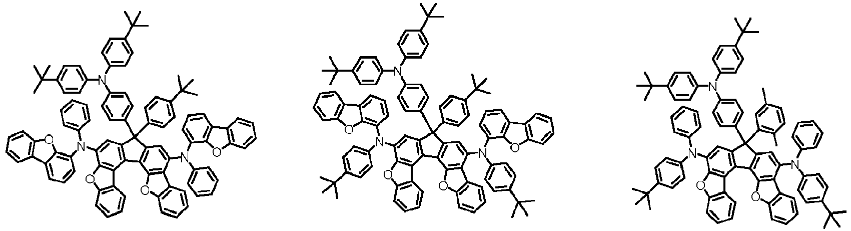

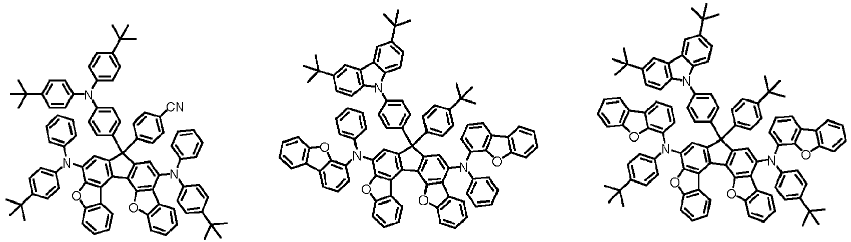

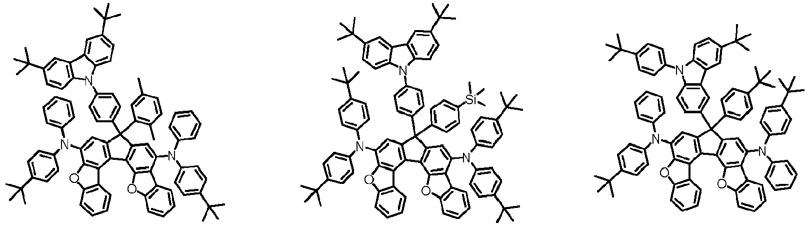

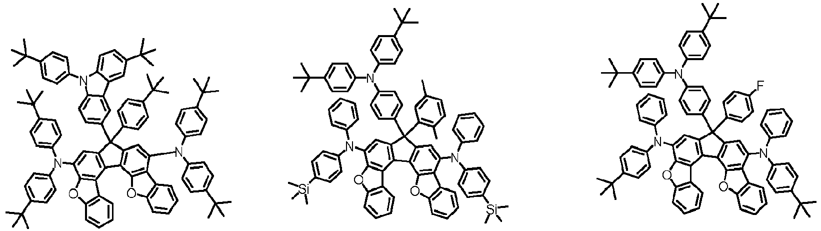

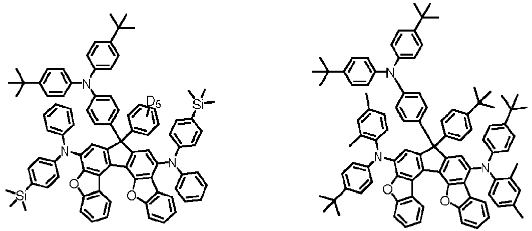

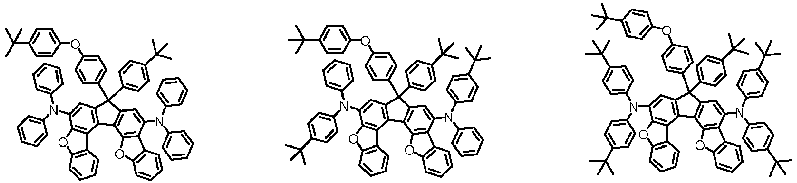

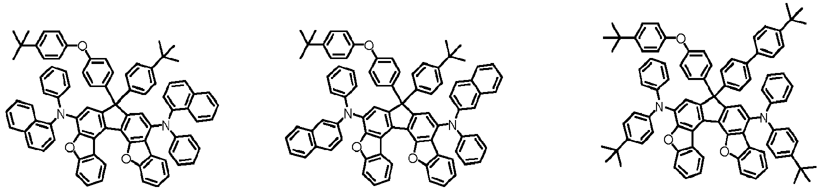

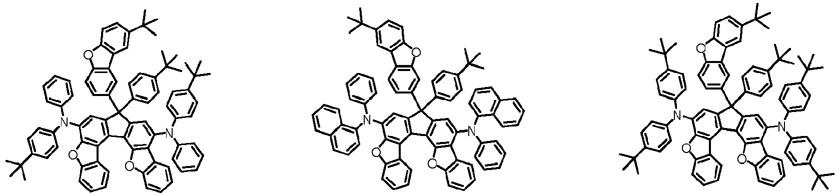

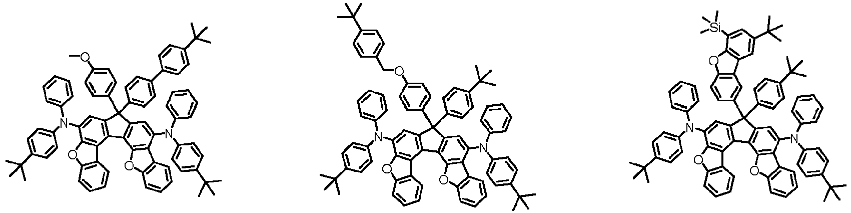

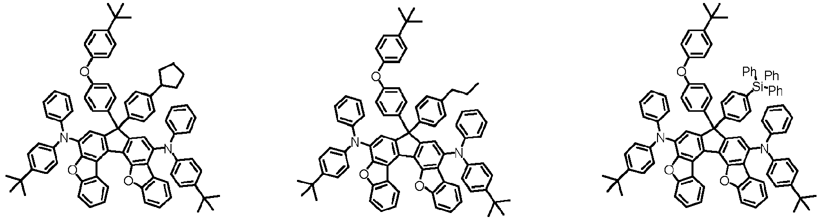

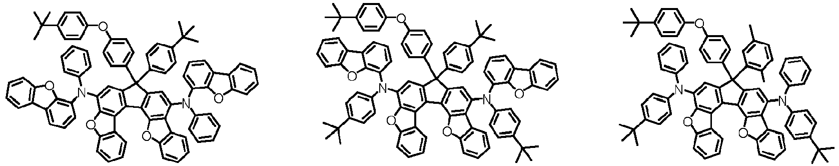

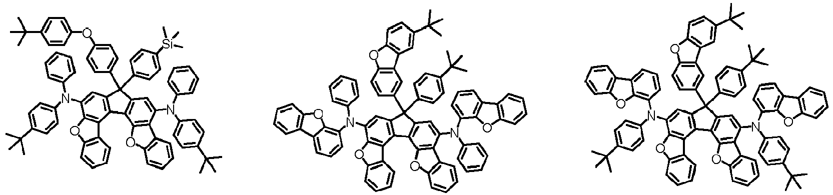

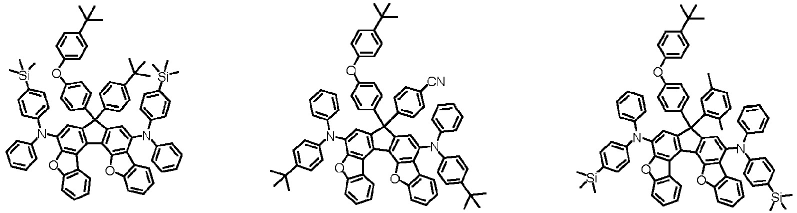

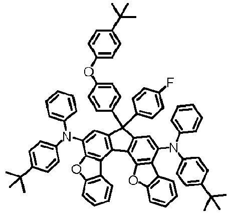

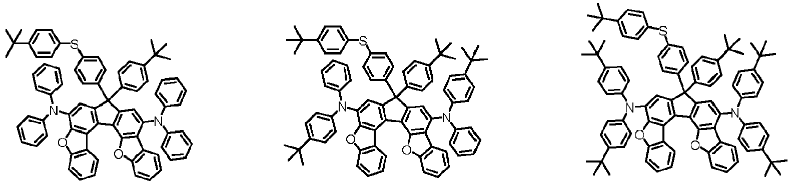

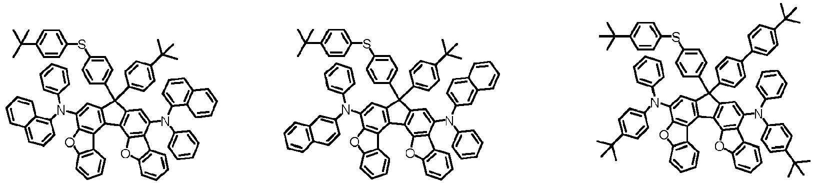

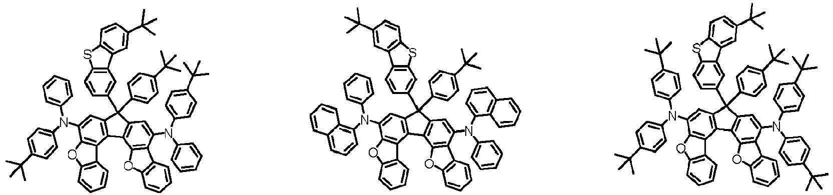

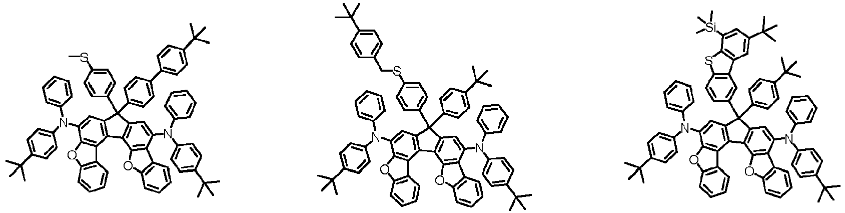

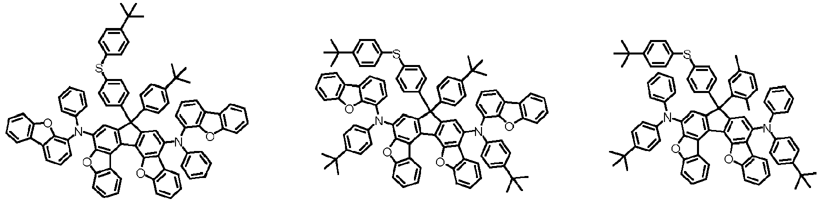

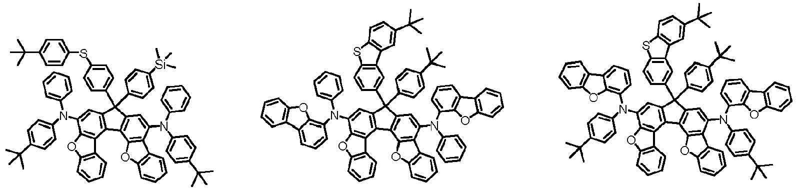

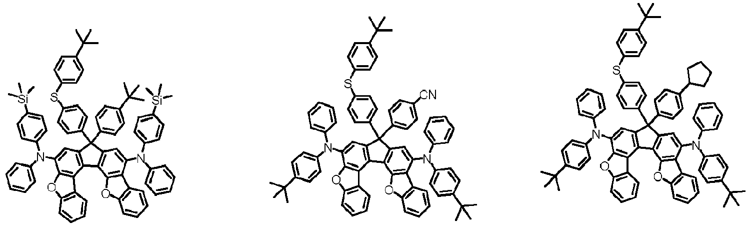

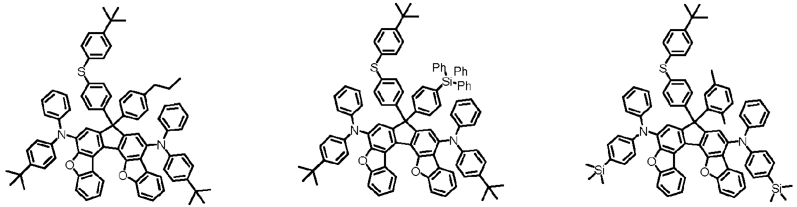

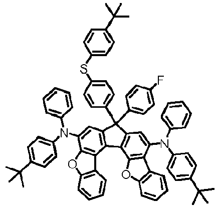

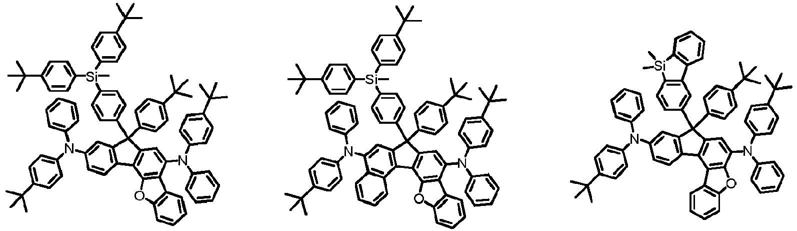

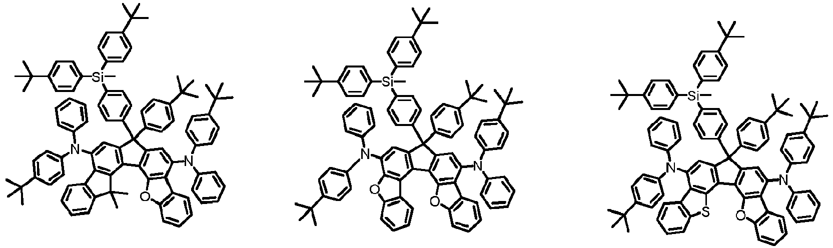

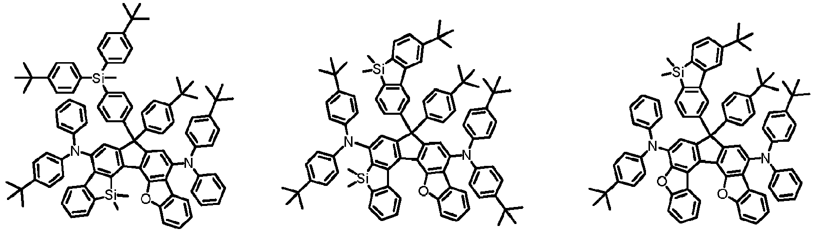

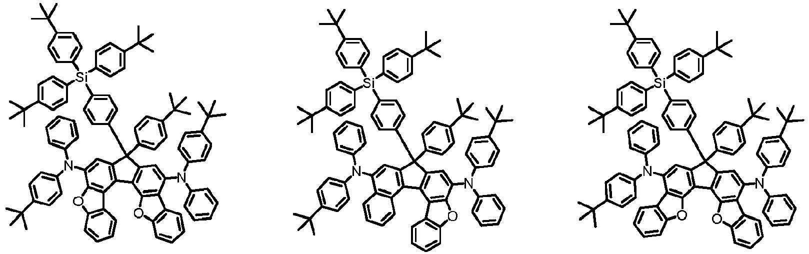

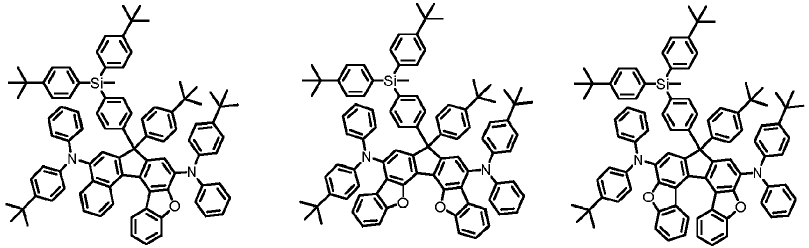

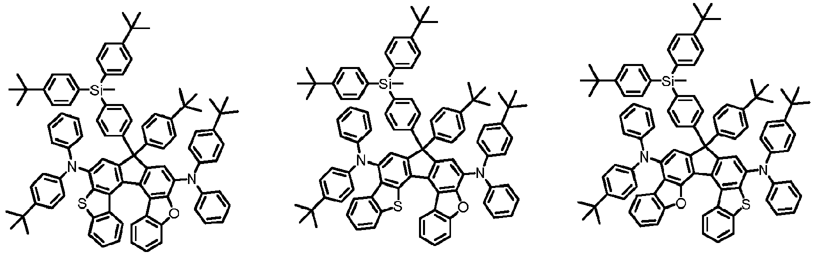

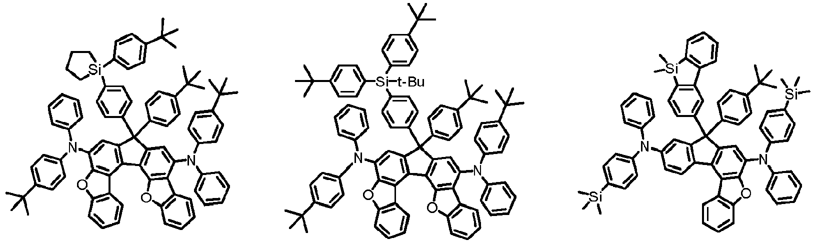

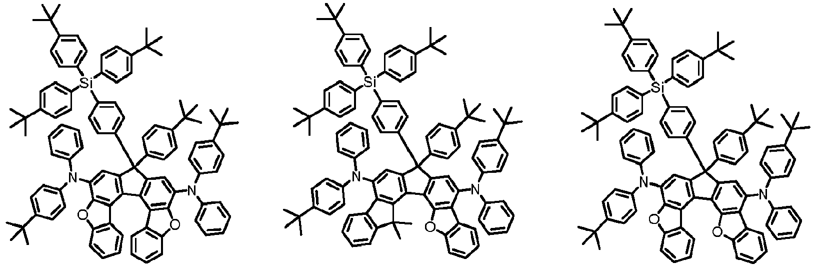

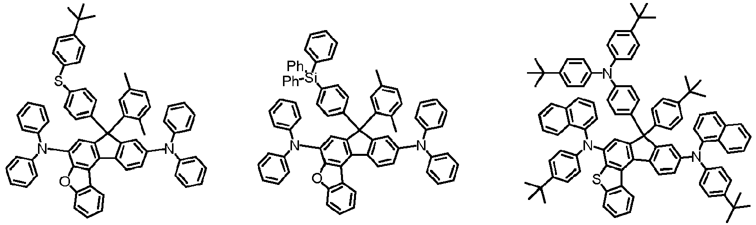

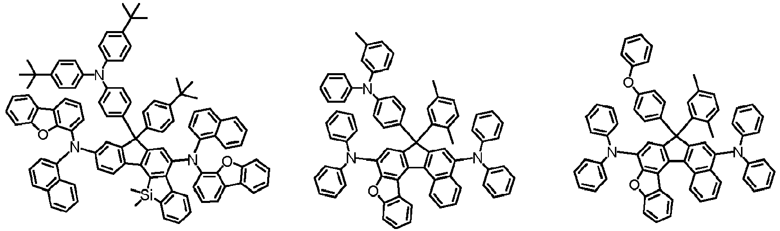

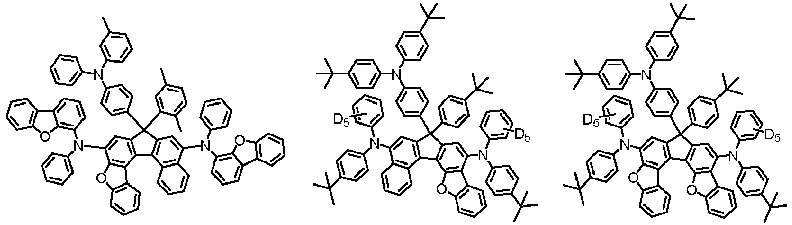

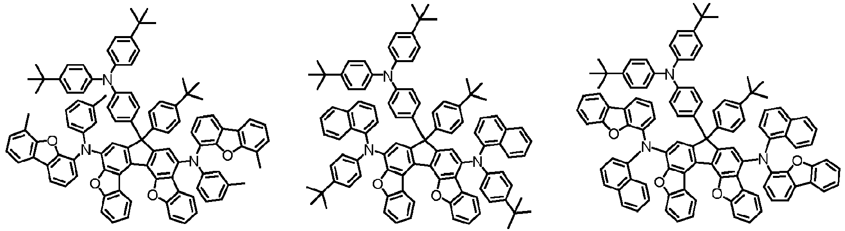

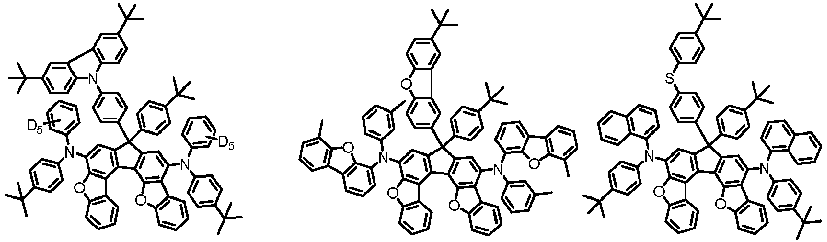

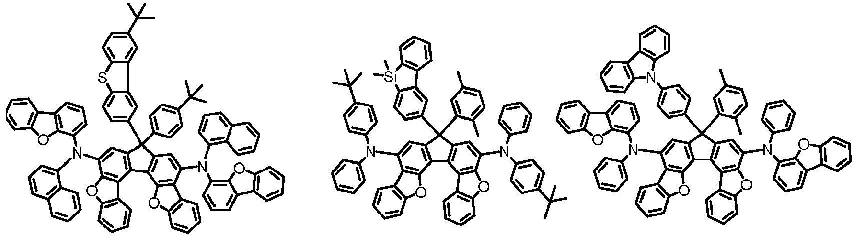

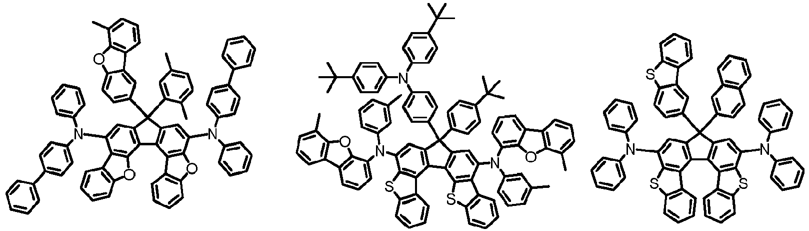

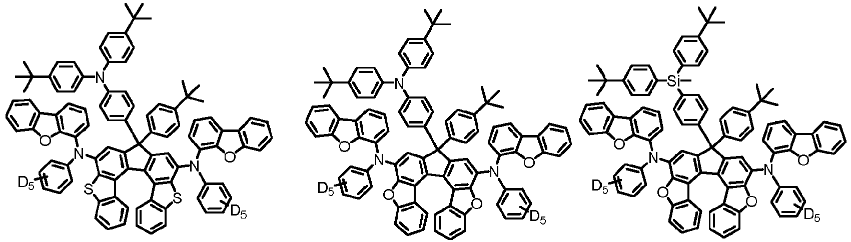

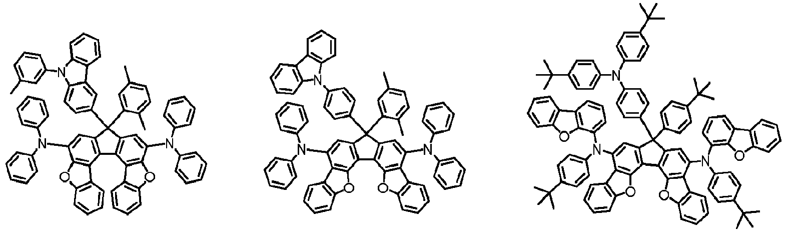

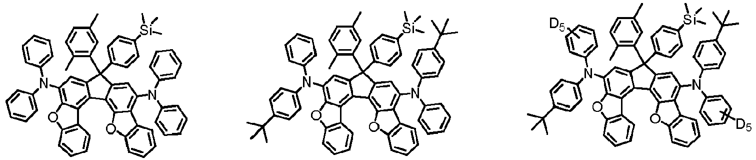

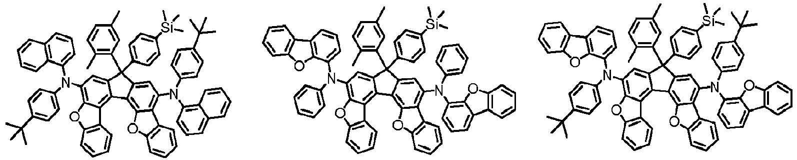

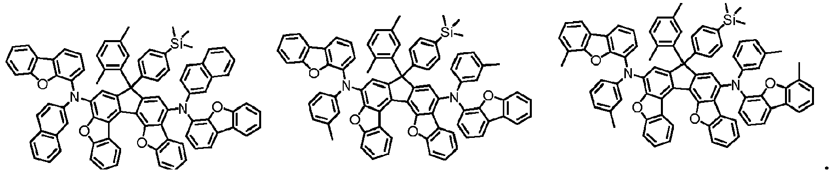

13. Organische lichtemittierende Vorrichtung, umfassend:
eine erste Elektrode; eine zweite Elektrode, die der ersten Elektrode gegenüberliegend vorgesehen ist; und
eine lichtemittierende Schicht, die zwischen der ersten Elektrode und der zweiten Elektrode vorgesehen ist,
worin die lichtemittierende Schicht die Verbindung gemäß irgendeinem der Ansprüche 1 bis 12 umfasst.

## Revendications

1. Composé représenté par la formule chimique 1 suivante : 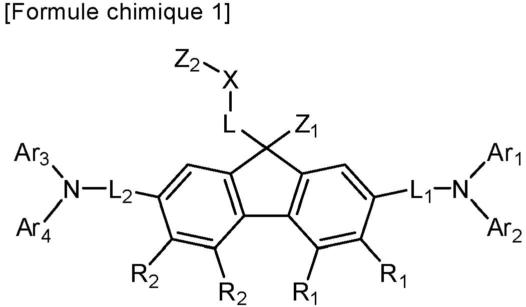 dans la formule chimique 1,
R₁ et R₂ sont chacun indépendamment un hydrogène, ou deux R₁ ou deux R₂ sont liés ensemble pour former un cycle aromatique en C₆₋₆₀ substitué ou non substitué ; un cycle non aromatique en C₆₋₆₀ substitué ou non substitué ; ou un cycle hétérocyclique en C₂₋₆₀ substitué ou non substitué contenant un quelconque ou plusieurs hétéroatomes sélectionnés parmi le groupe consistant en N, O, S et Si,
au moins un parmi les deux R₁ ou les deux R₂ sont liés ensemble pour former le cycle non aromatique en C₆₋₆₀ ; ou le cycle hétérocyclique en C₂₋₆₀,
L est un arylène en C₆₋₆₀ substitué ou non substitué ; ou un hétéroarylène en C₂₋₆₀ substitué ou non substitué contenant un quelconque ou plusieurs hétéroatomes sélectionnés parmi le groupe consistant en N, O et S,
X est O, S, NZ₃, ou SiZ₄Z₅,
Z₁ est un aryle en C₆₋₆₀ substitué ou non substitué,
Z₂ à Z₅ sont chacun indépendamment un alkyle en C₁₋₆₀ substitué ou non substitué ; un haloalkyle en C₁₋₆₀ substitué ou non substitué ; un cycloalkyle en C₃₋₆₀ substitué ou non substitué ; un aralkyle en C₇₋₆₀ substitué ou non substitué ; un aryle en C₆₋₆₀ substitué ou non substitué ; ou un hétéroaryle en C₂₋₆₀ substitué ou non substitué contenant un quelconque ou plusieurs hétéroatomes sélectionnés parmi le groupe consistant en N, O et S,
les L et Z₂ ; Z₂ et Z₃ ; ou Z₄ et Z₅ sont facultativement liés l'un à l'autre pour former un cycle hétérocyclique à 5 chaînons,
à condition que Z₁ et LX-Z₂ soient différents l'un de l'autre et ne soient pas liés l'un à l'autre,
L₁ et L₂ sont chacun indépendamment une liaison simple ; un arylène en C₆₋₆₀ substitué ou non substitué ; ou un hétéroarylène en C₂₋₆₀ substitué ou non substitué contenant un quelconque ou plusieurs hétéroatomes sélectionnés parmi le groupe consistant en N, O et S, et
Ar₁ à Ar₄ sont chacun indépendamment un aryle en C₆₋₆₀ substitué ou non substitué ; ou un hétéroaryle en C₂₋₆₀ substitué ou non substitué contenant un quelconque ou plusieurs hétéroatomes sélectionnés parmi le groupe consistant en N, O et S.

2. Composé selon la revendication 1,
dans lequel au moins un parmi les deux R₁ ou les deux R₂ sont liés ensemble pour former l'une quelconque des structures représentées par les formules chimiques 2d à 2g suivantes, les autres sont des hydrogènes, ou sont liés ensemble pour former l'une quelconque des structures représentées par les formules chimiques 2a à 2g suivantes : 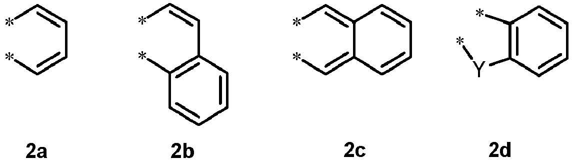 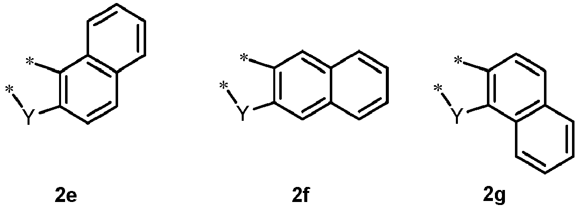 dans les formules chimiques 2a à 2g,
Y est O, S, CZ₆Z₇, ou SiZ₈Z₉,
Z₆ à Z₉ sont chacun indépendamment un alkyle en C₁₋₁₀, ou un aryle en C₆₋₂₀, et
* indique une position de liaison avec le carbone lié à R₁ ou R₂.

3. Composé selon la revendication 2,
dans lequel le composé représenté par la formule chimique 1 est représenté par l'une quelconque des formules chimiques 1-1, 1-3, et 1-5 suivantes. 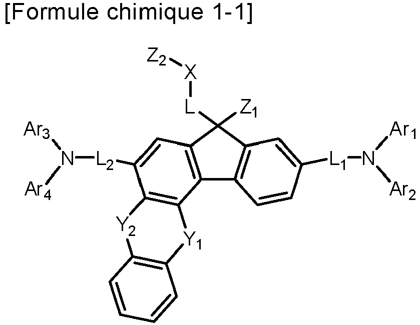 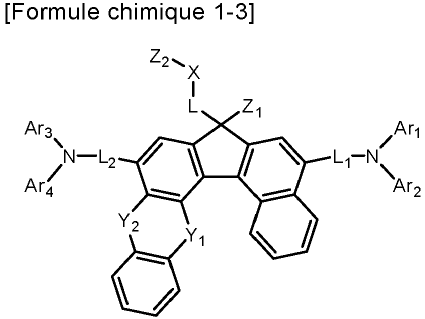 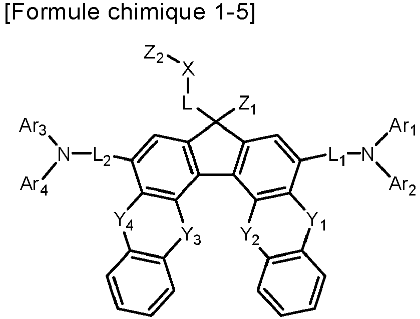 dans les formules chimiques 1-1, 1-3 et 1-5,
L, X, Z₁, Z₂, L₁, L₂ et Ar₁ à Ar₄ sont tels que définis dans la revendication 1,
un parmi Y₁ et Y₂ est une liaison simple, et l'autre est O, S, C(alkyle en C₁₋₄)₂, ou Si(alkyle en C₁₋₄)₂, et
un parmi Y₃ et Y₄ est une liaison simple, et l'autre est O, S, C(alkyle en C₁₋₄)₂, ou Si(alkyle en C₁₋₄)₂.

4. Composé selon la revendication 1,
dans lequel Z₁ est un phényle ou un biphénylyle,
dans lequel Z₁ est non substitué, ou substitué avec 1 à 5 substituants sélectionnés indépendamment parmi le groupe consistant en un deutérium, un halogène, un cyano, un alkyle en C₁₋₁₀, un cycloalkyle en C₃₋₁₀, Si(alkyle en C₁₋₁₀)₃ et Si(aryle en C₆₋₂₀)₃.

5. Composé selon la revendication 1,
dans lequel Z₁ est l'un quelconque sélectionné parmi le groupe consistant en :

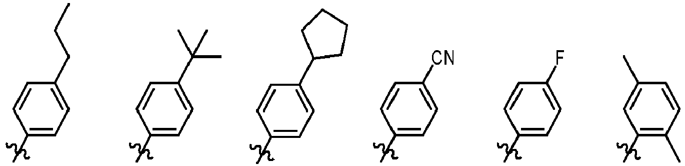

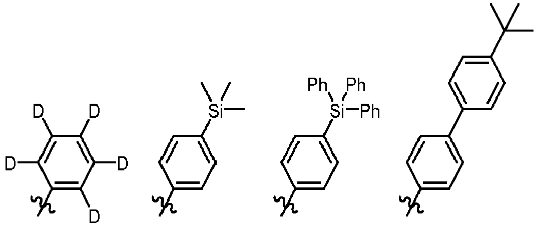

6. Composé selon la revendication 1,
dans lequel L-X-Z₂ est l'un quelconque sélectionné parmi le groupe consistant en : 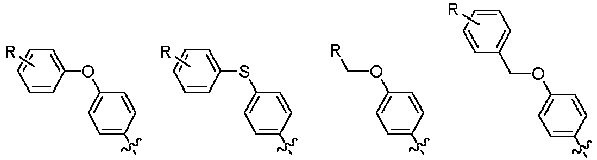 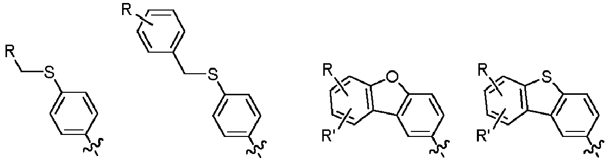 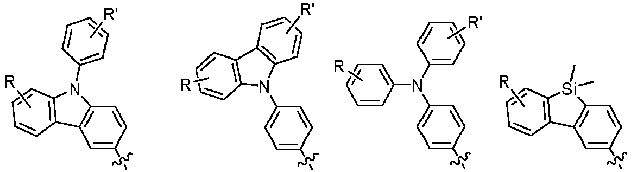 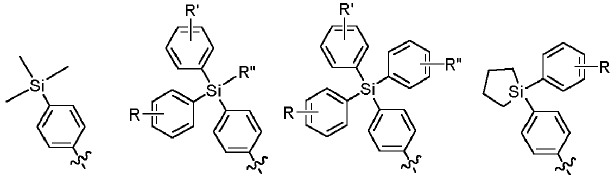 dans lequel,
R, R' et R" sont chacun indépendamment un hydrogène, un méthyle, un tert-butyle ou un Si(méthyle)₃.

7. Composé selon la revendication 1,
dans lequel L₁ et L₂ sont une liaison simple.

8. Composé selon la revendication 1,
dans lequel Ar₁ à Ar₄ sont chacun indépendamment un phényle, un naphtyle, un biphénylyle ou un dibenzofuranyle,
dans lequel Ar₁ à Ar₄ sont non substitués, ou substitués avec 1 à 5 substituants sélectionnés chacun indépendamment parmi le groupe consistant en un deutérium, un halogène, un alkyle en C₁₋₁₀ et Si(alkyle en C₁₋₁₀)₃.

9. Composé selon la revendication 1,
dans lequel Ar₁ à Ar₄ sont chacun indépendamment l'un quelconque sélectionné parmi le groupe consistant en :

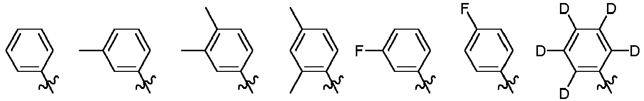

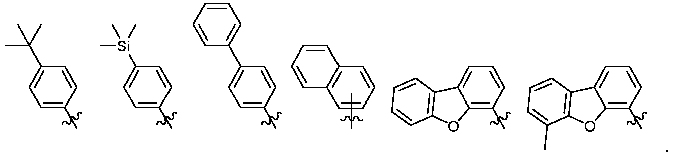

10. Composé selon la revendication 1,
dans lequel Ar₁ et Ar₄ sont identiques l'un à l'autre, et
Ar₂ et Ar₃ sont identiques l'un à l'autre.

11. Composé selon la revendication 1,
dans lequel le composé représenté par la formule chimique 1 est représenté par l'une quelconque des formules chimiques 2-1, 2-2, 2-4, 2-5, 2-7, 2-8, et 2-10 suivantes : 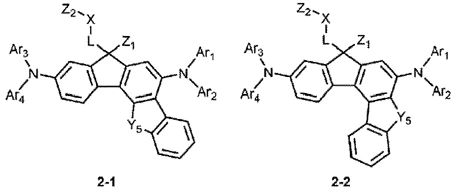 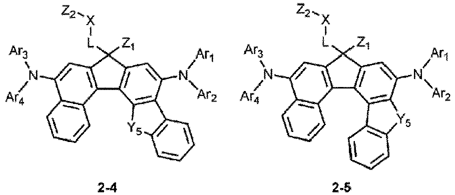 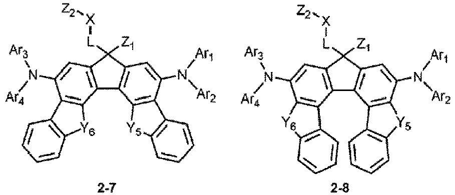 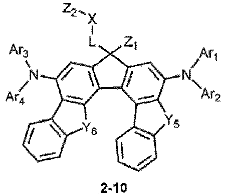 dans les formules chimiques 2-1, 2-2, 2-4, 2-5, 2-7, 2-8, et 2-10,
L, X, Z₁, Z₂ et Ar₁ à Ar₄ sont tels que définis dans la revendication 1, et
Y₅ et Y₆ sont chacun indépendamment O, S, un C(méthyle)₂, ou un Si(méthyle)₂.

12. Composé selon la revendication 1,
dans lequel le composé est l'un quelconque des composés suivants :

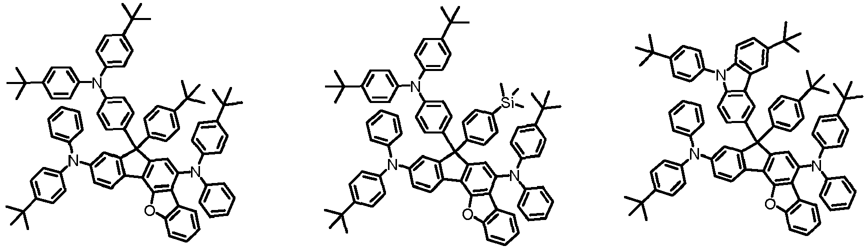

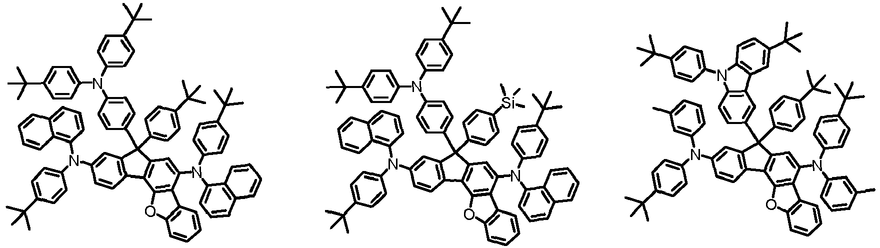

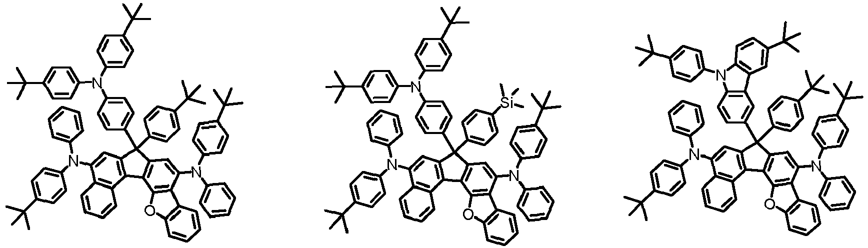

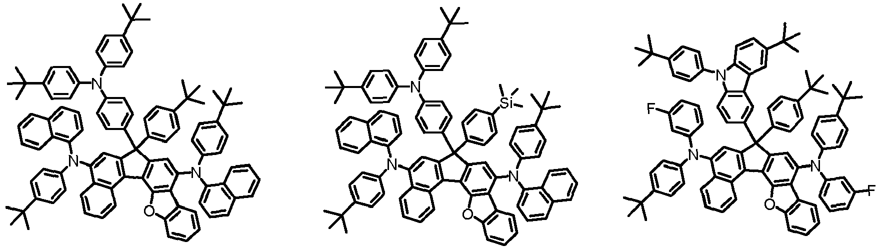

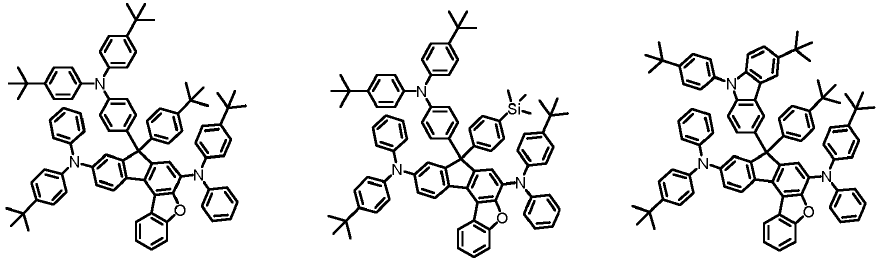

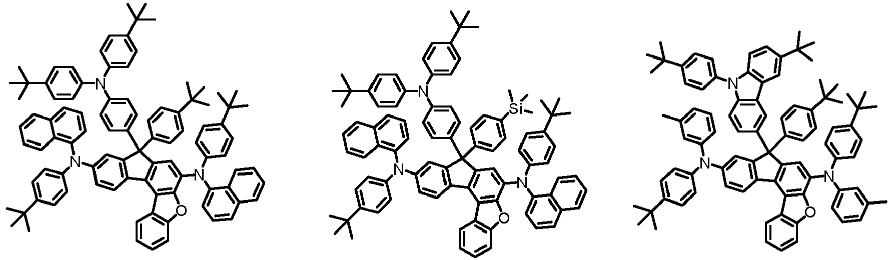

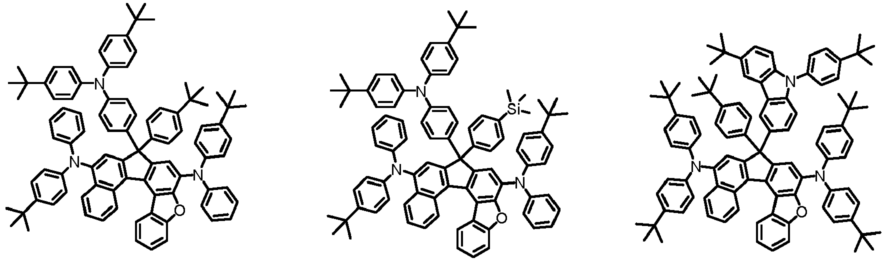

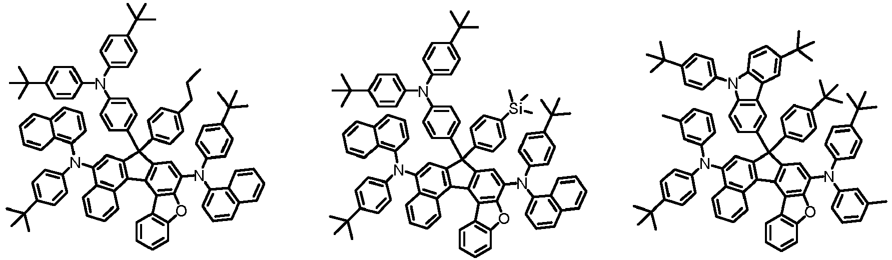

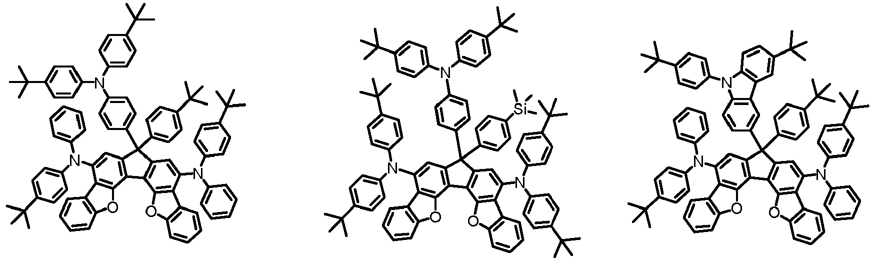

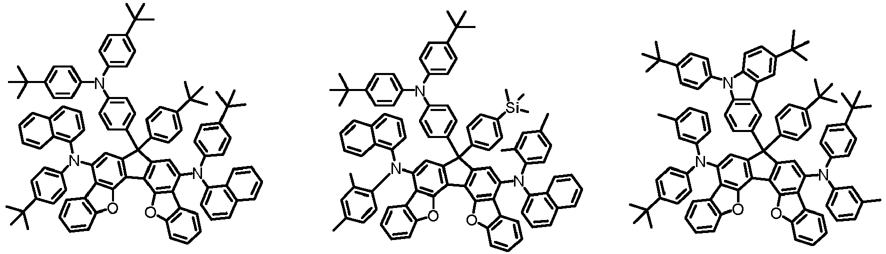

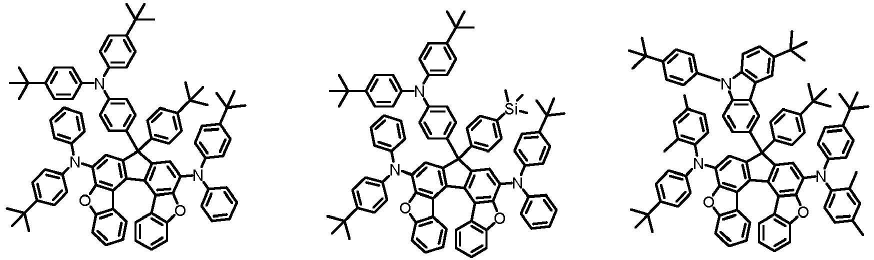

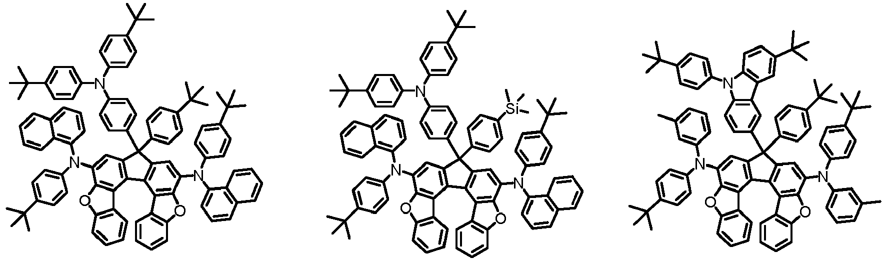

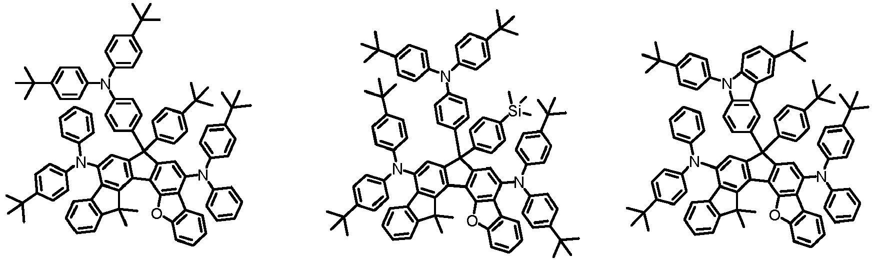

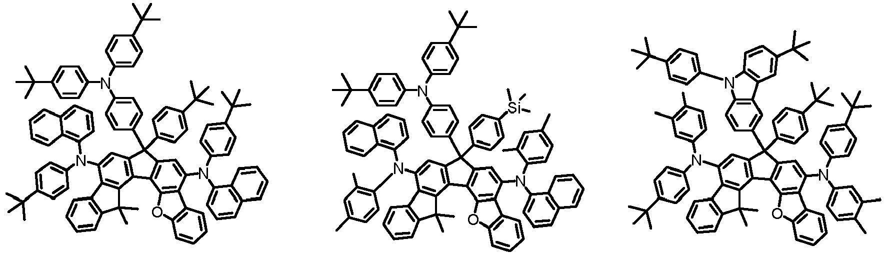

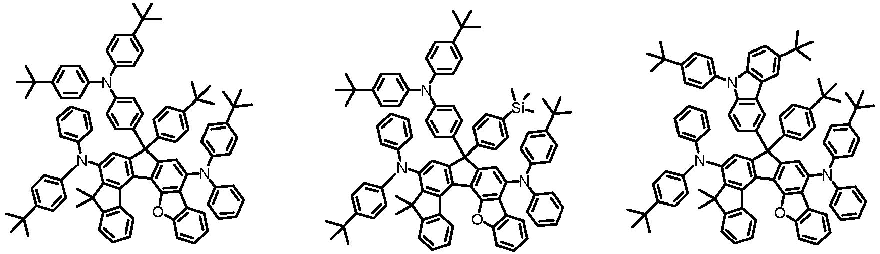

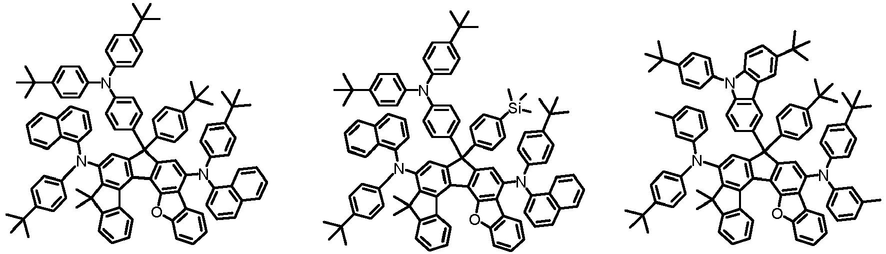

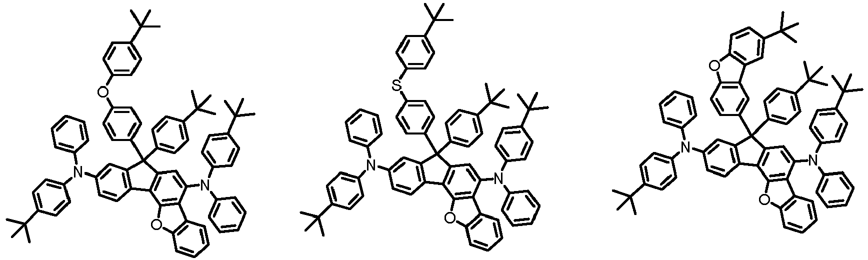

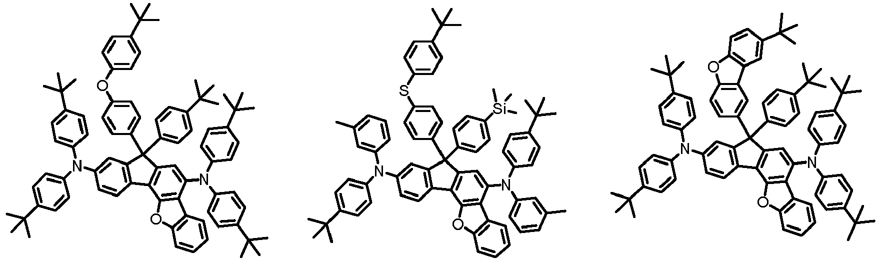

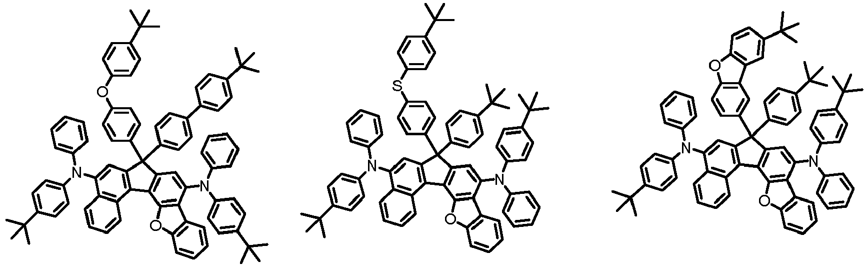

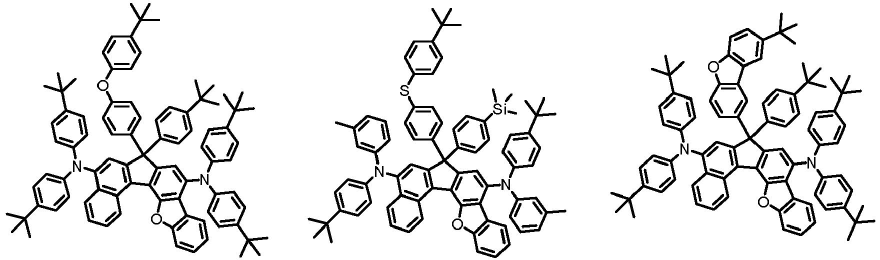

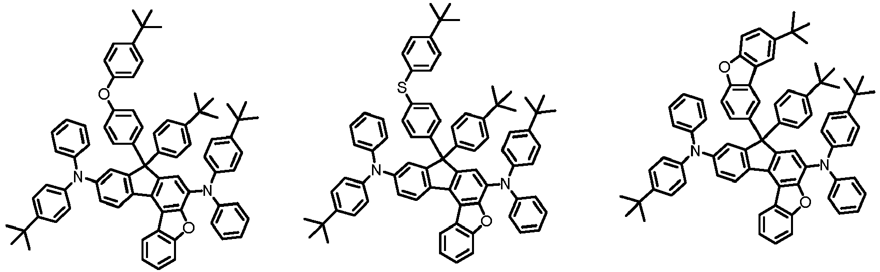

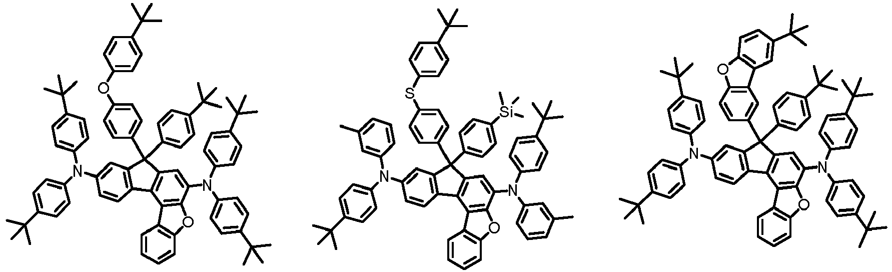

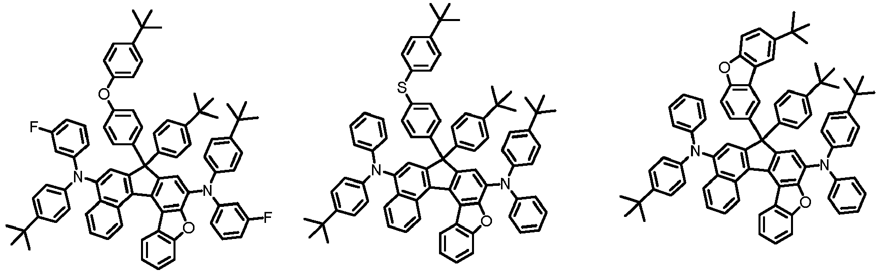

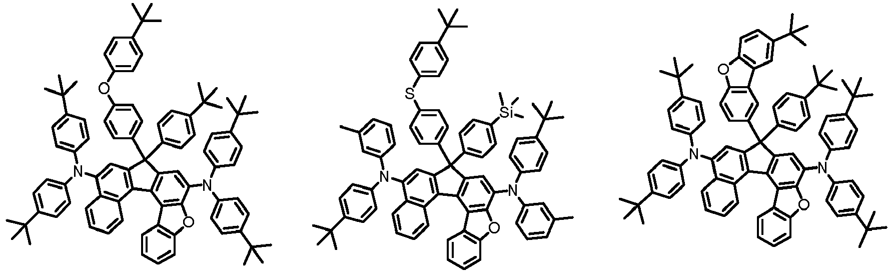

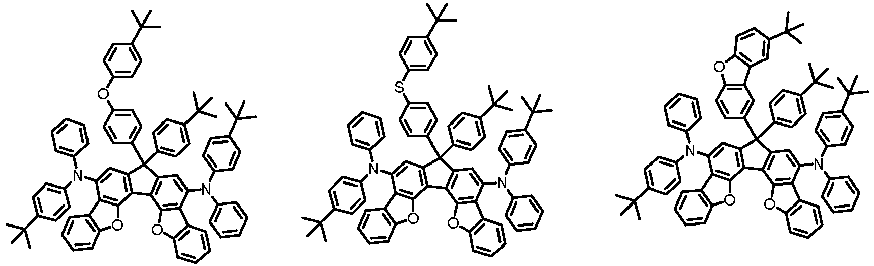

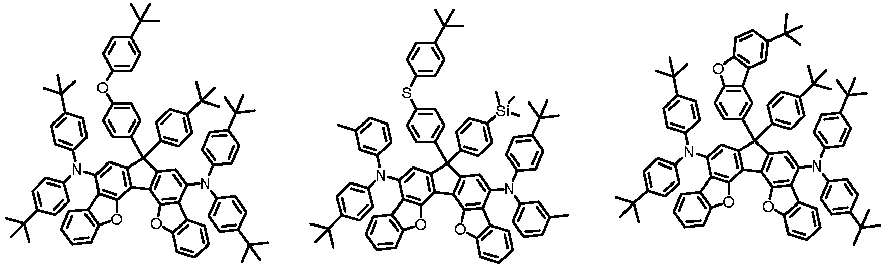

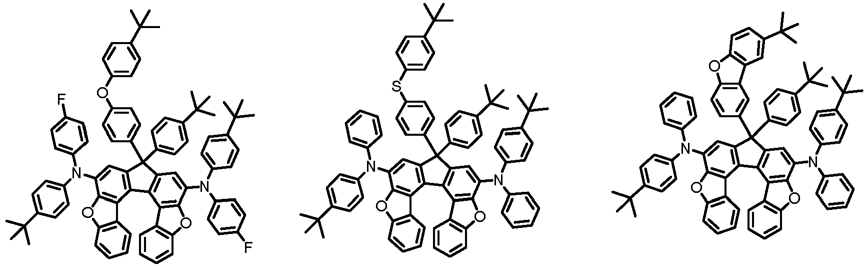

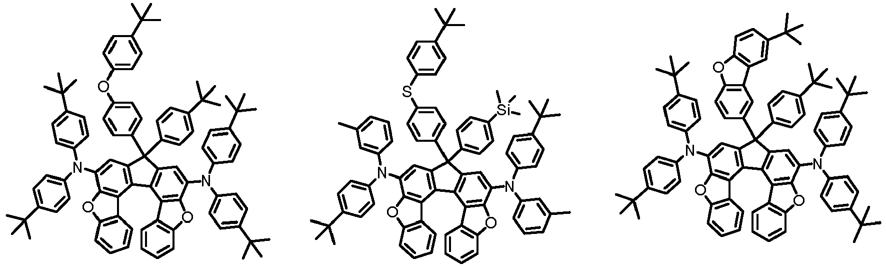

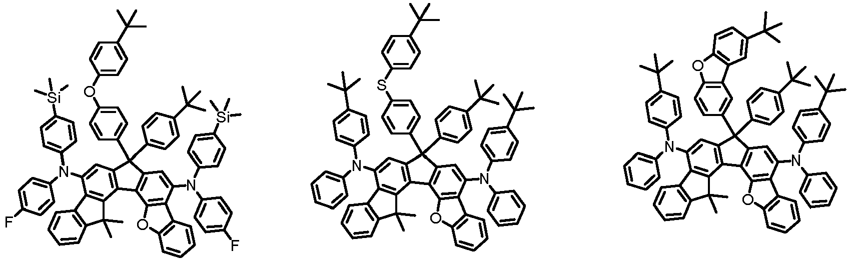

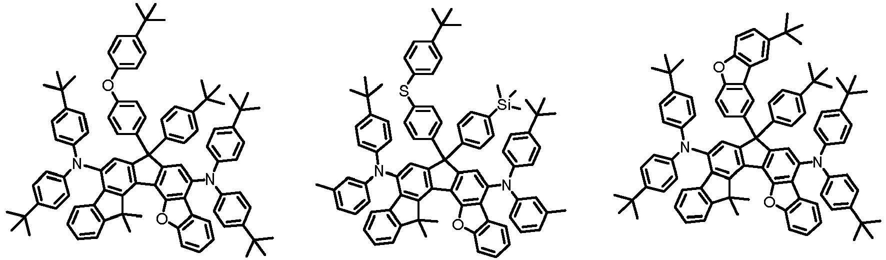

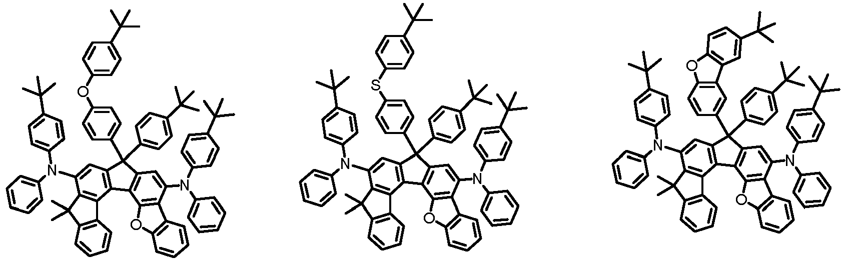

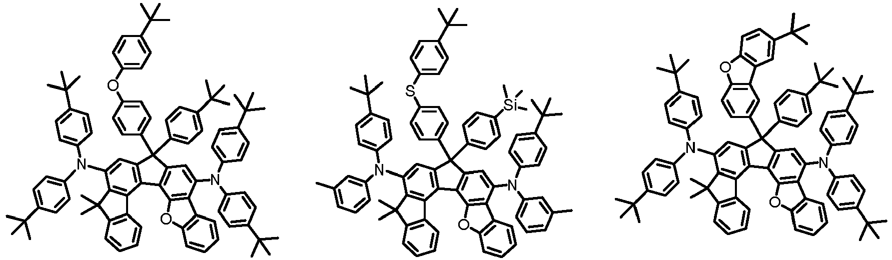

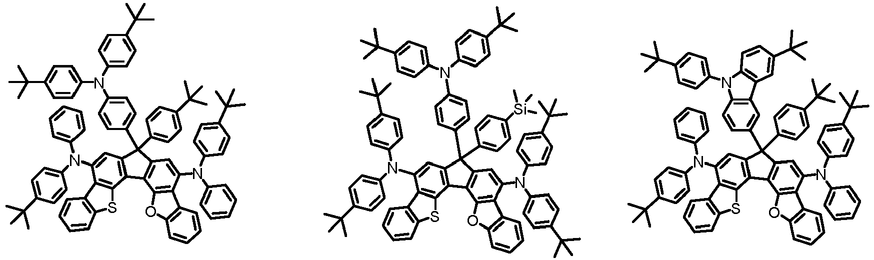

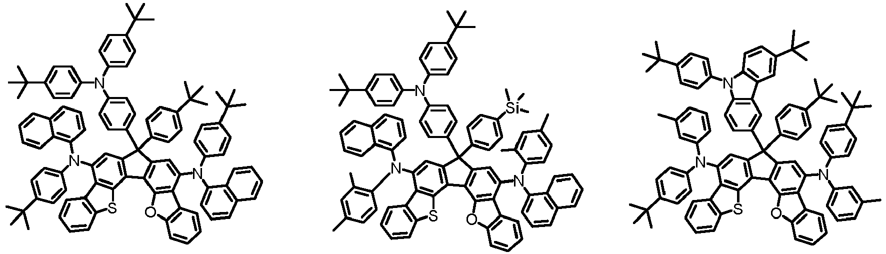

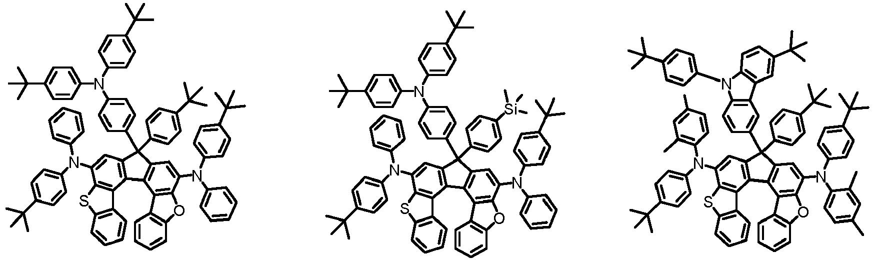

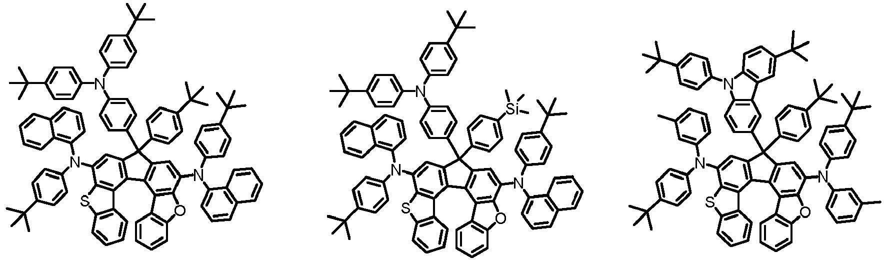

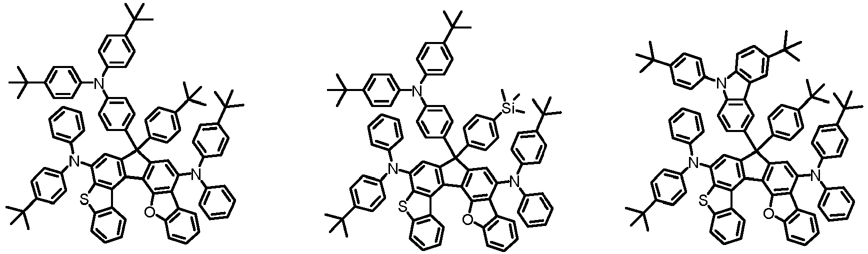

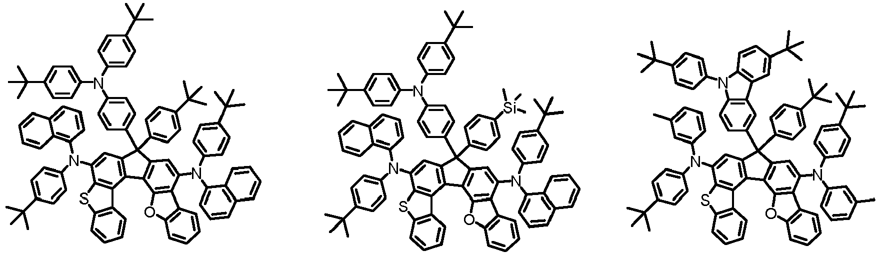

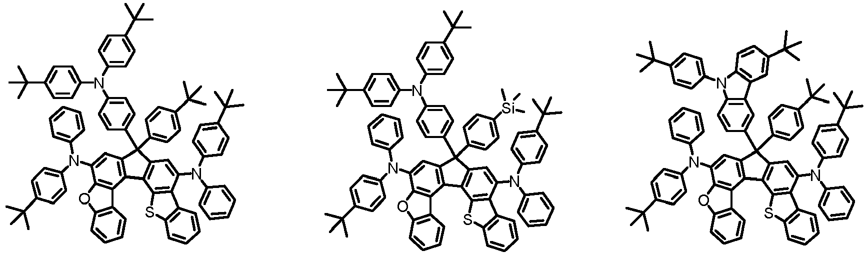

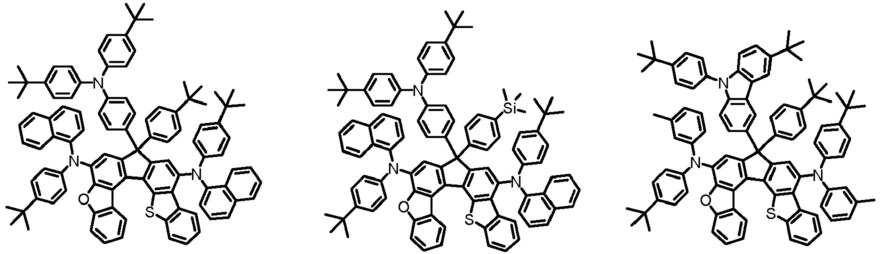

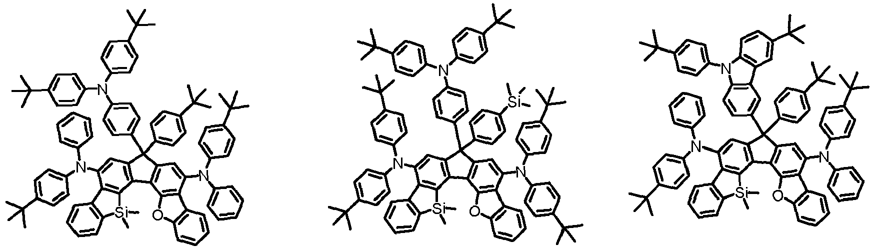

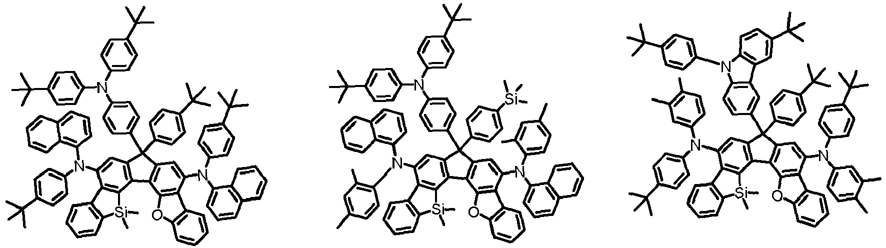

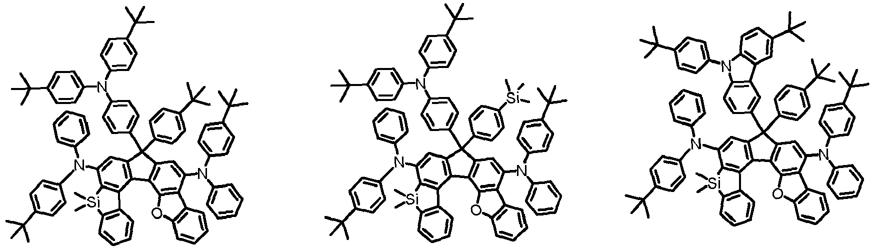

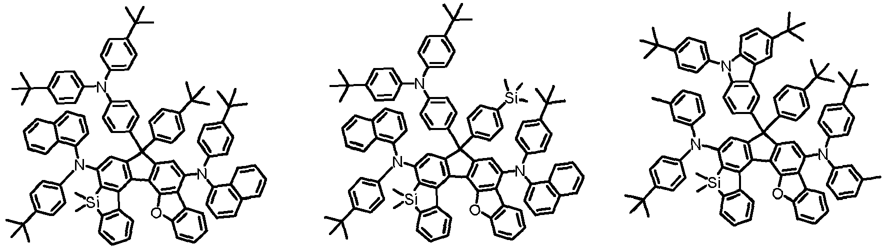

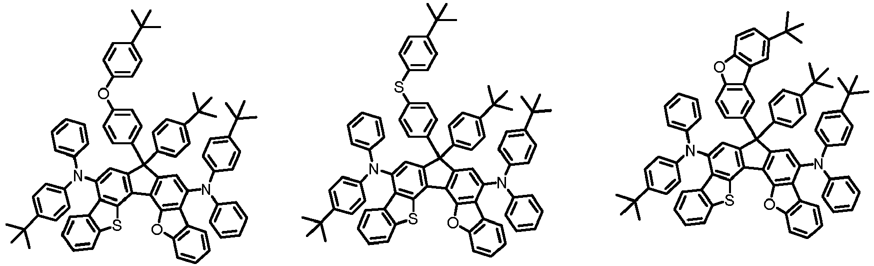

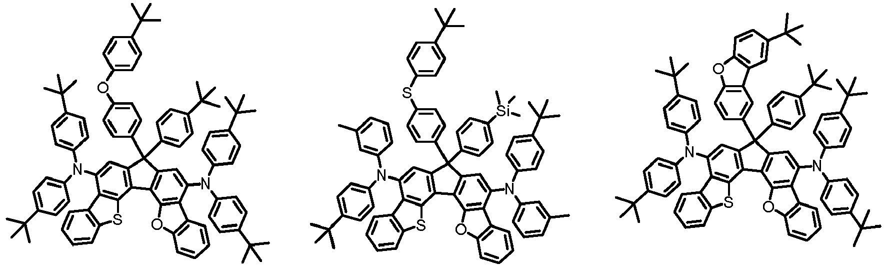

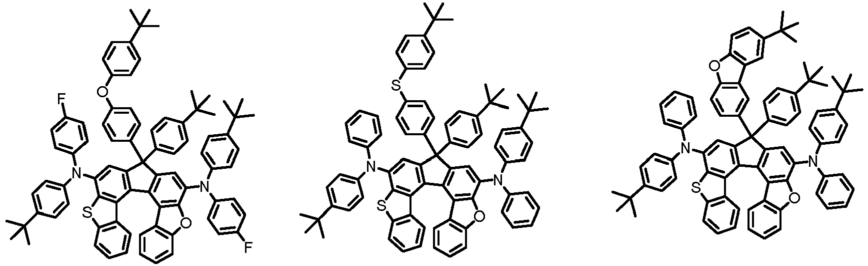

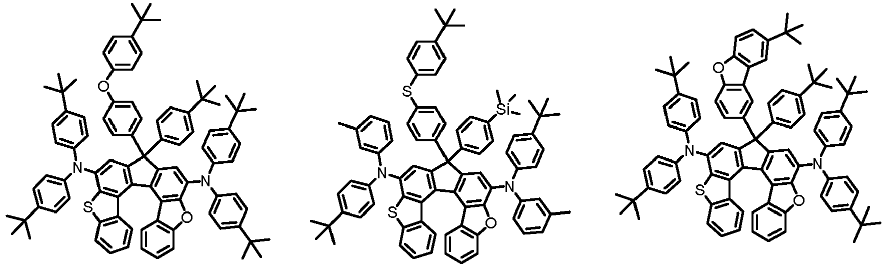

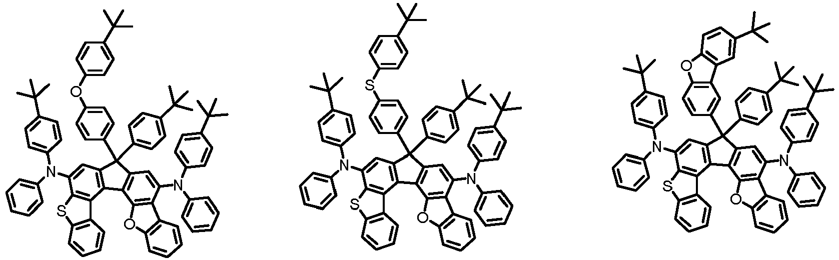

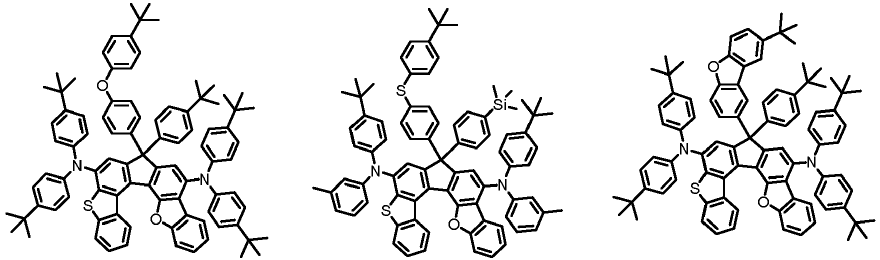

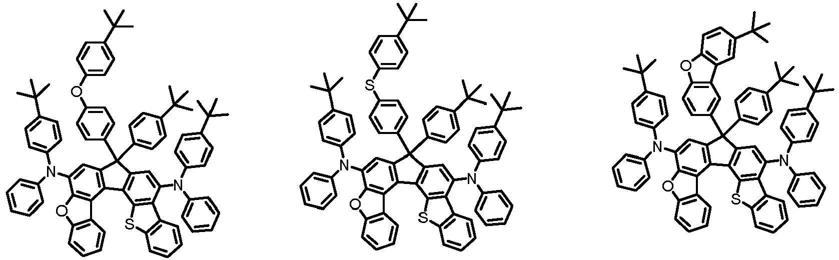

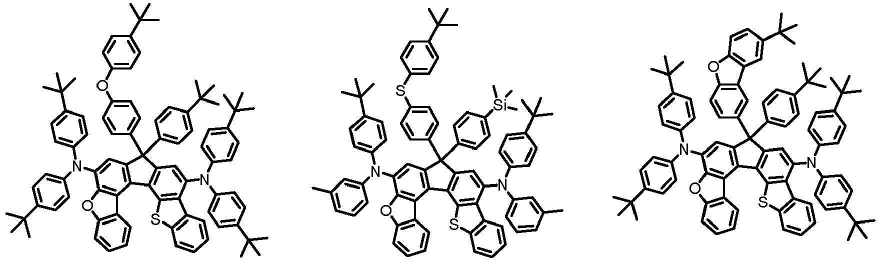

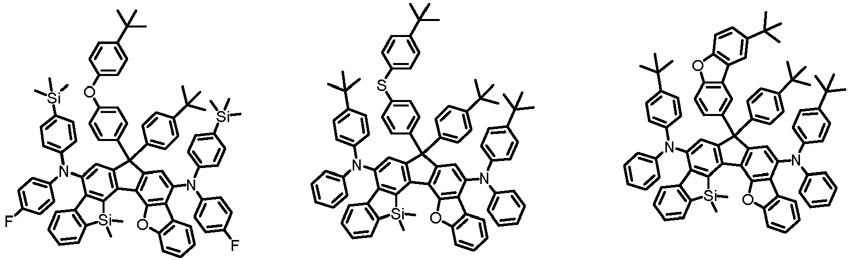

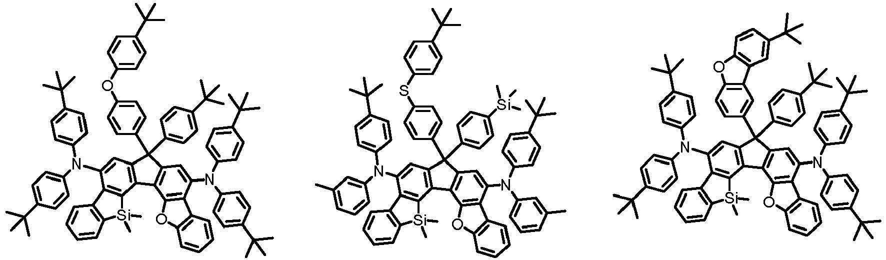

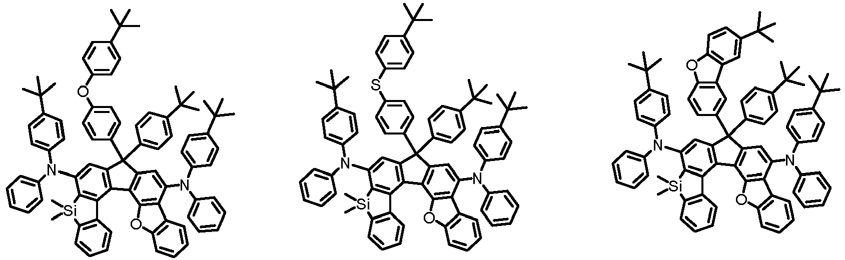

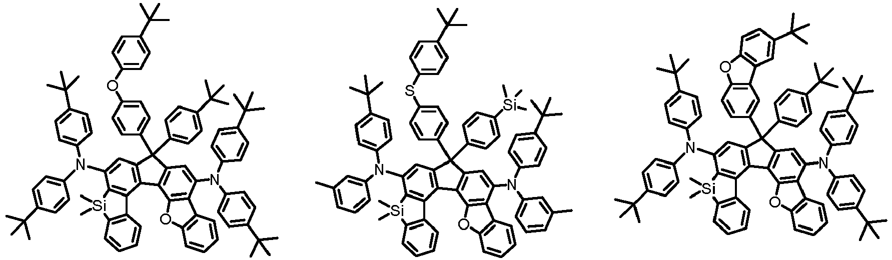

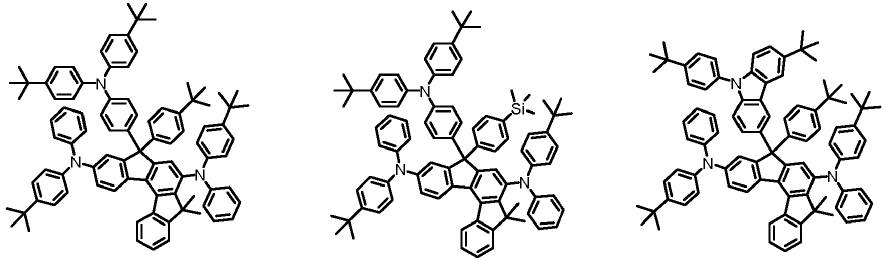

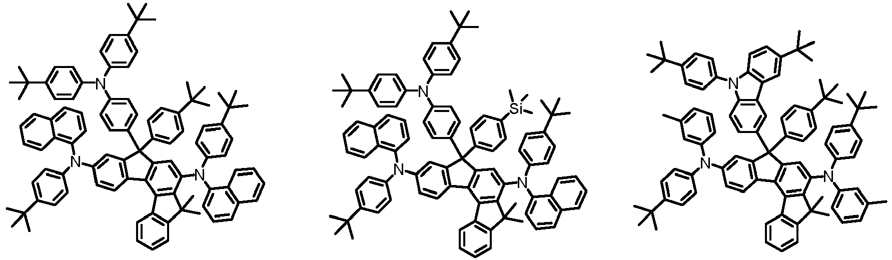

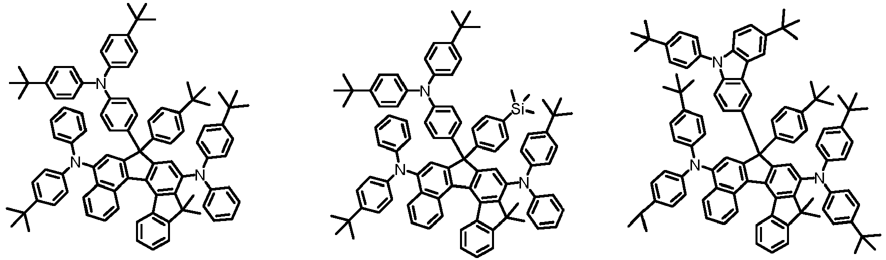

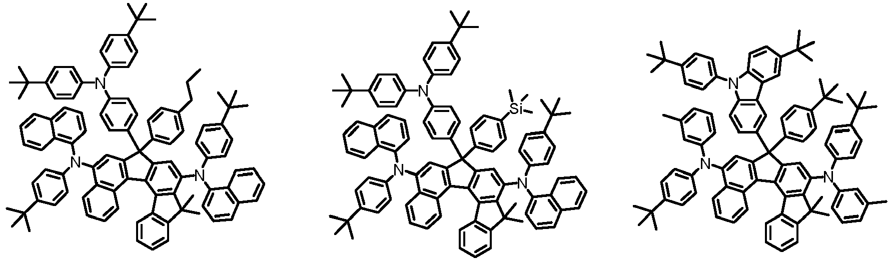

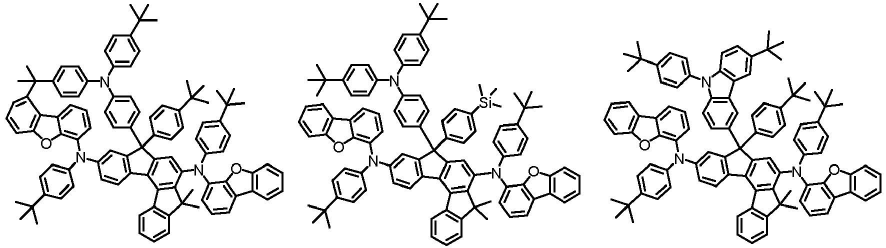

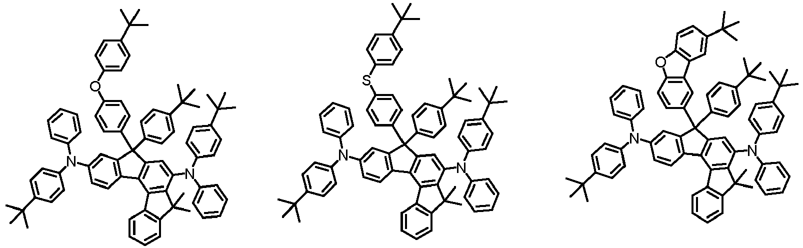

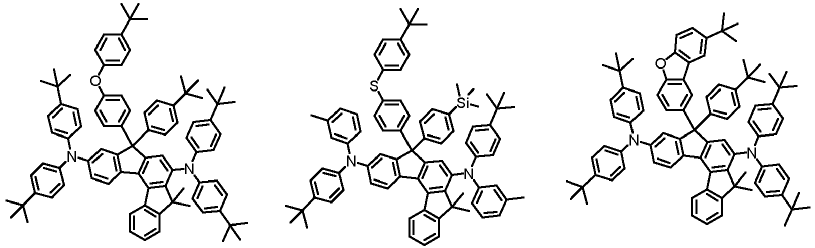

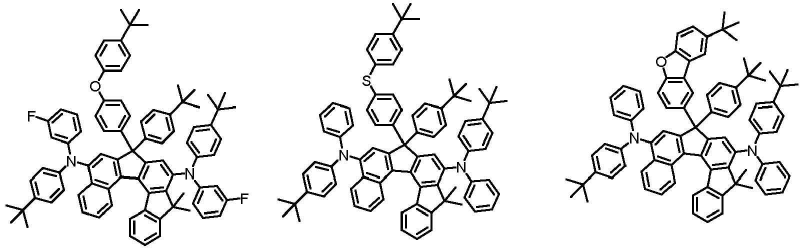

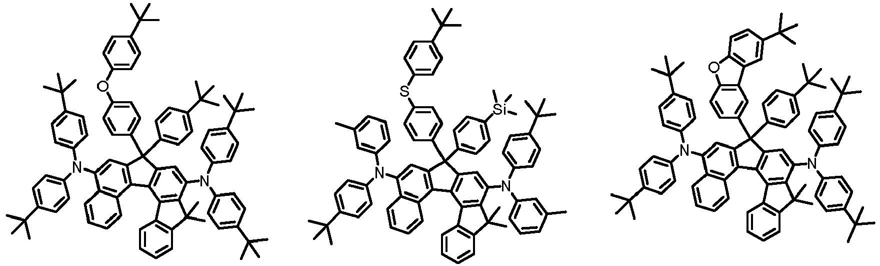

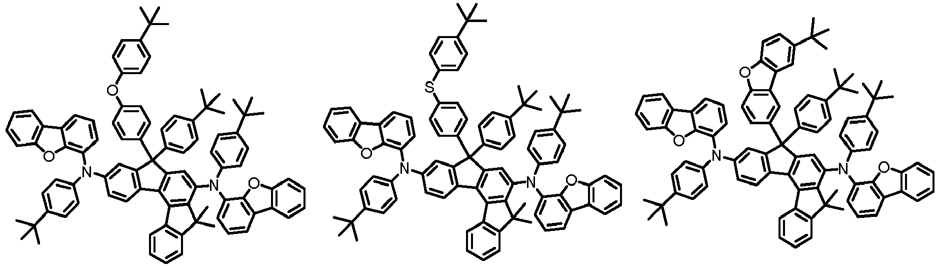

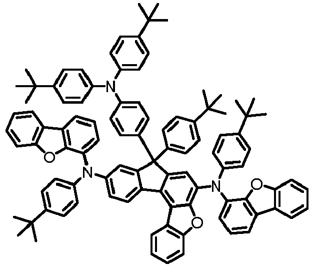

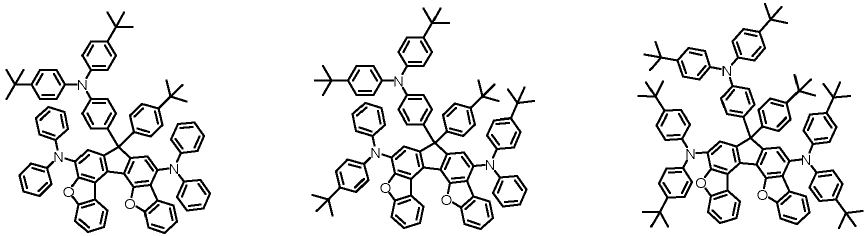

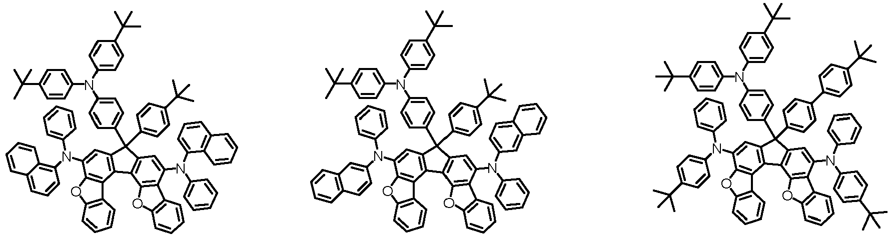

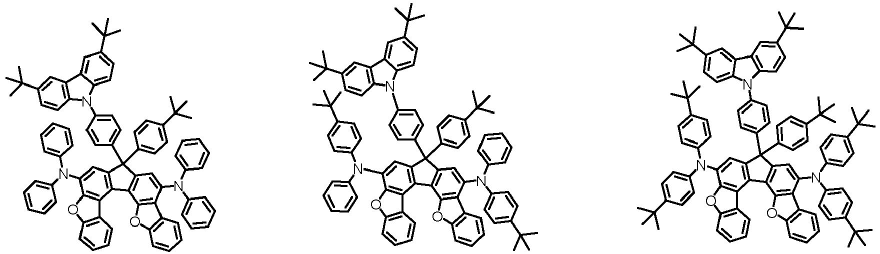

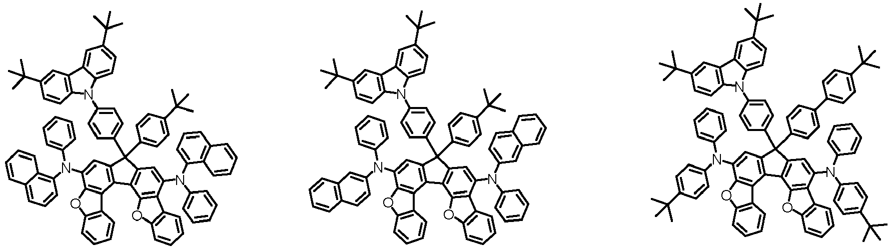

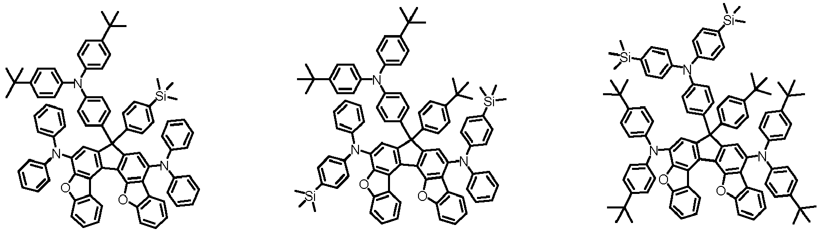

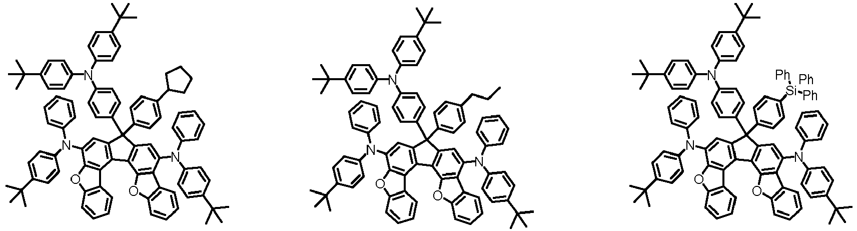

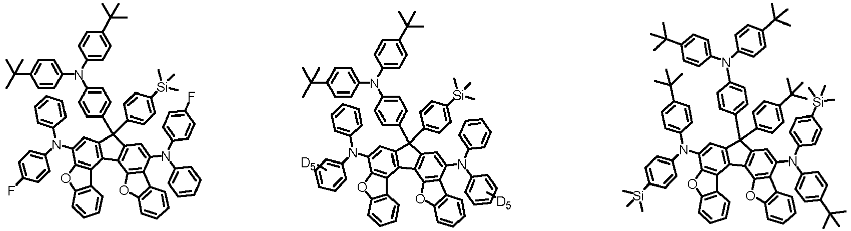

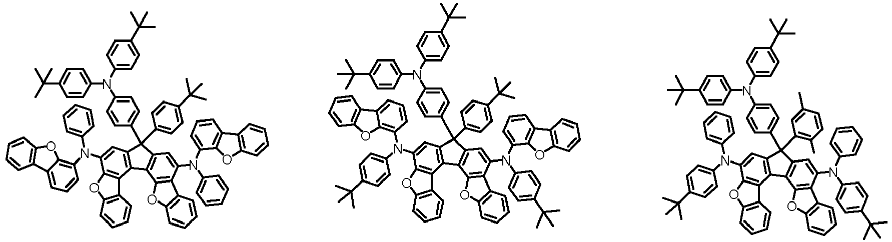

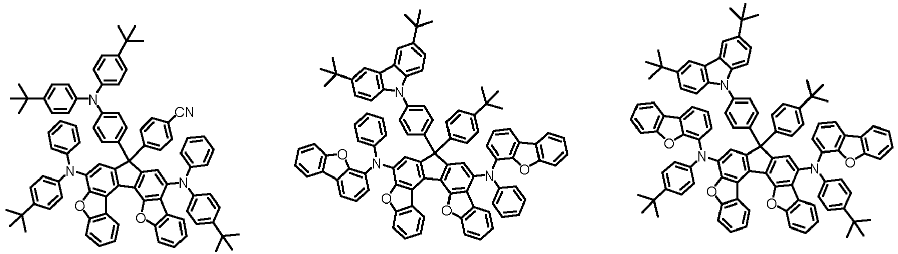

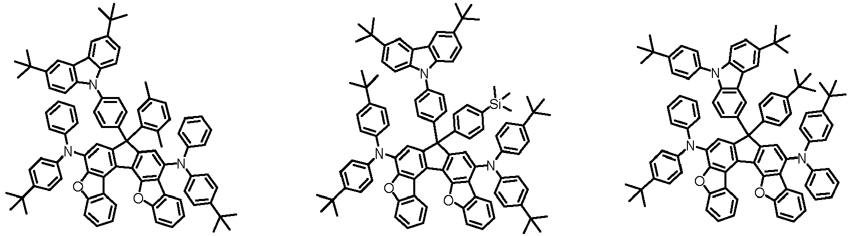

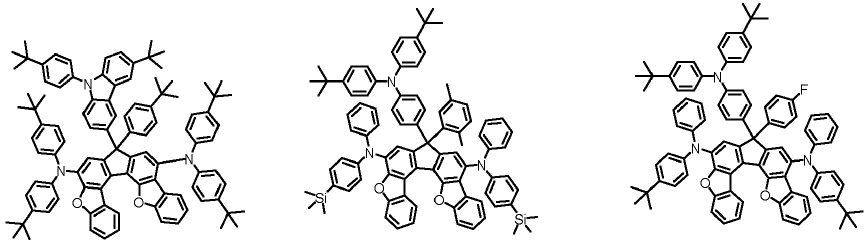

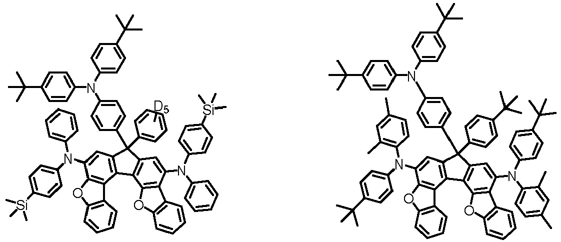

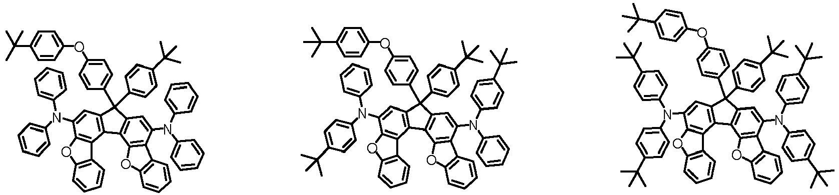

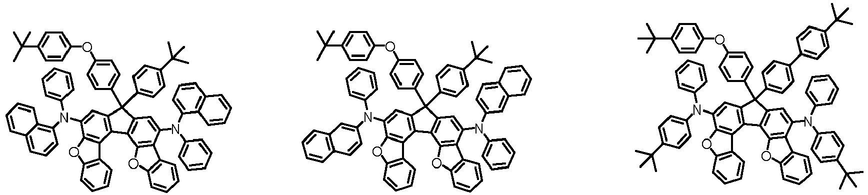

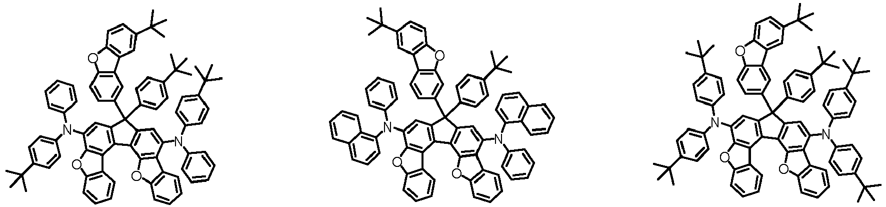

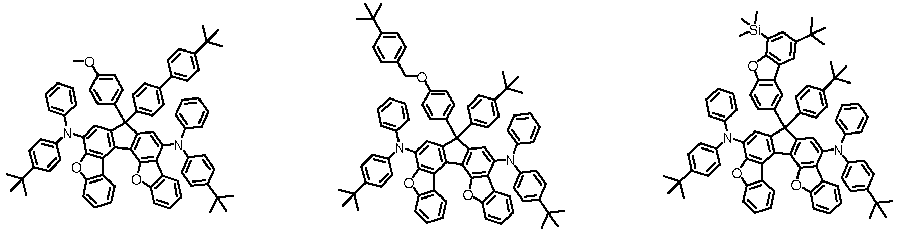

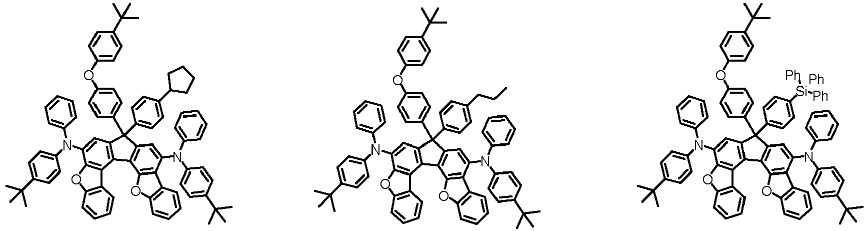

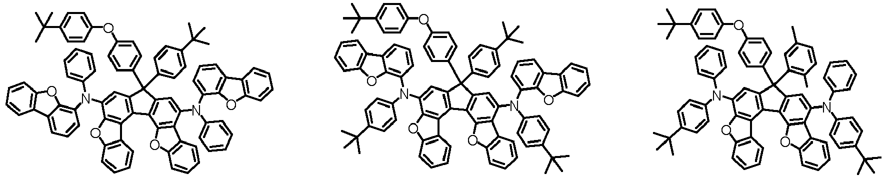

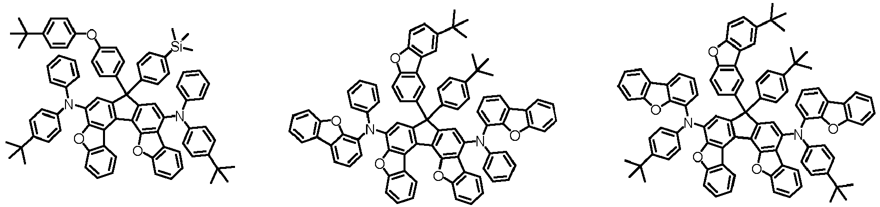

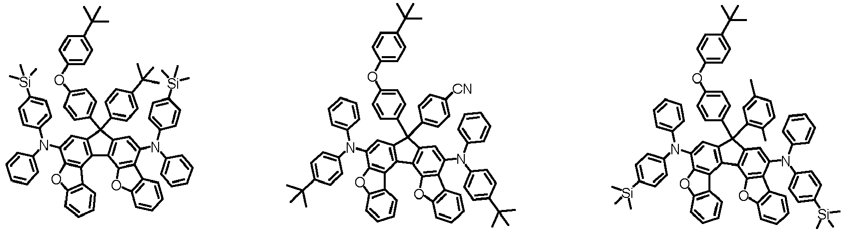

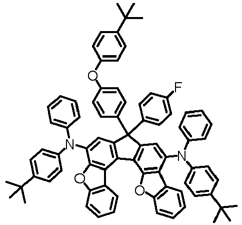

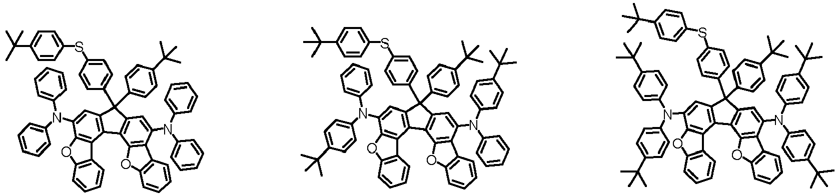

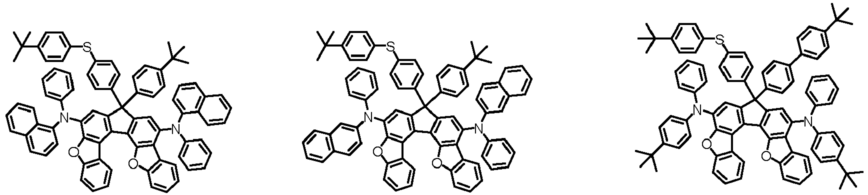

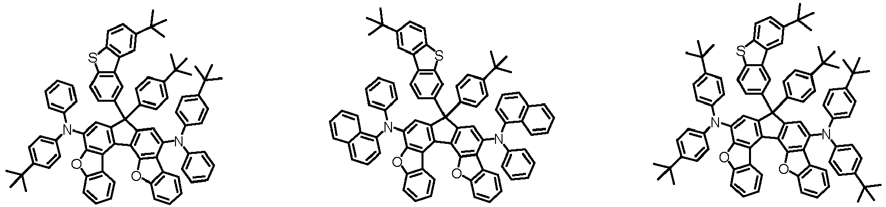

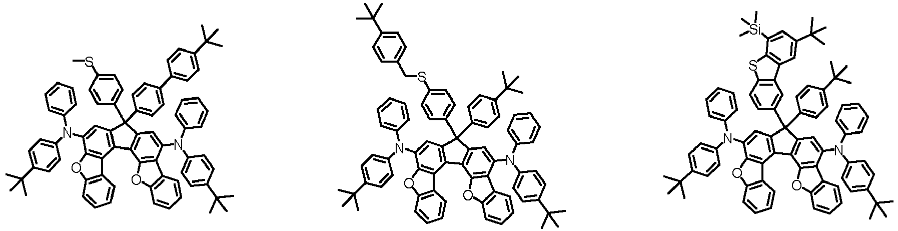

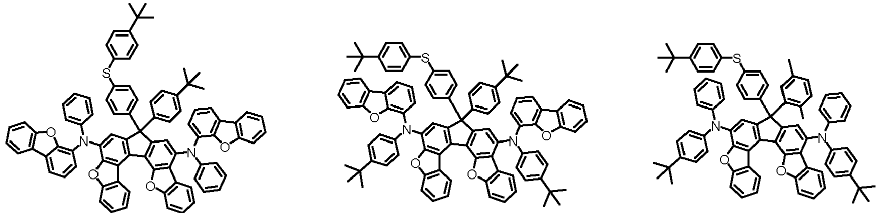

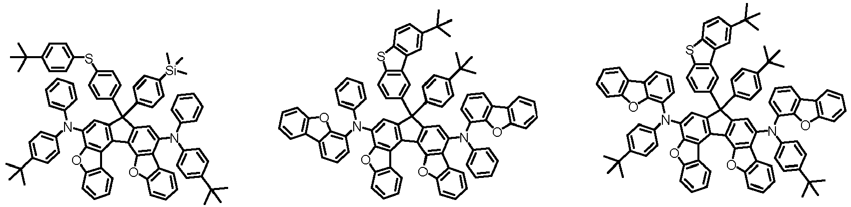

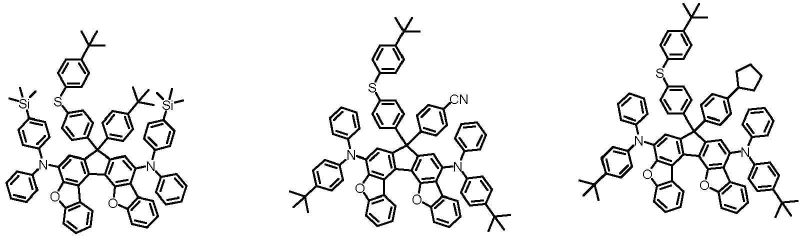

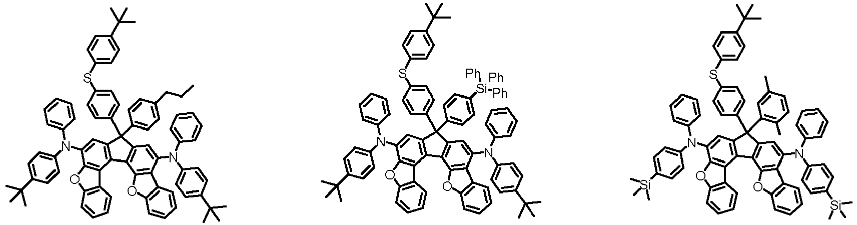

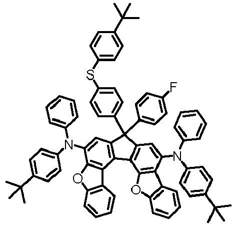

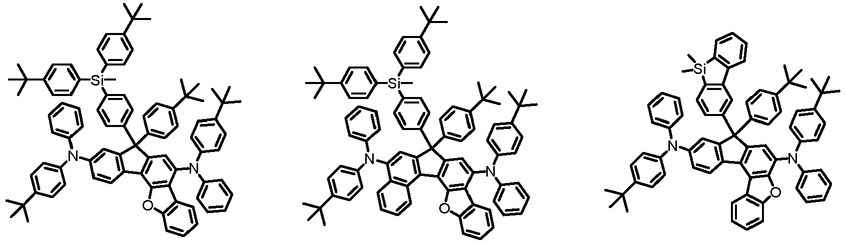

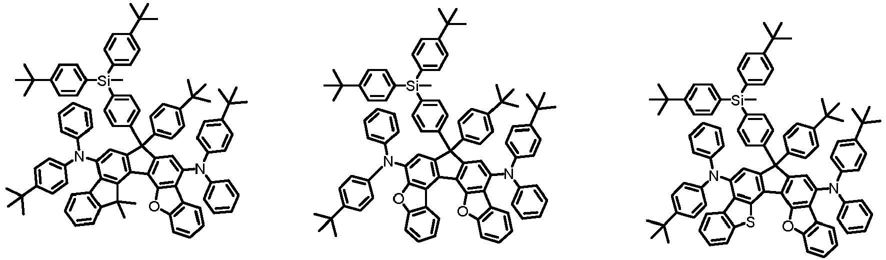

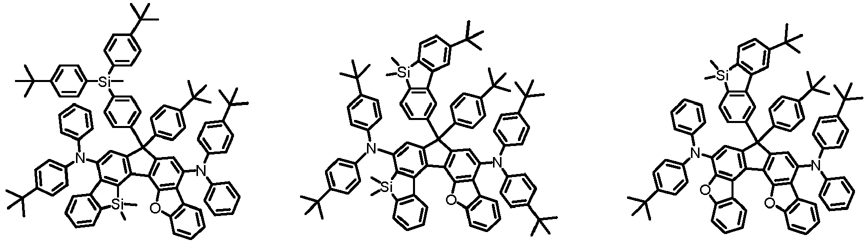

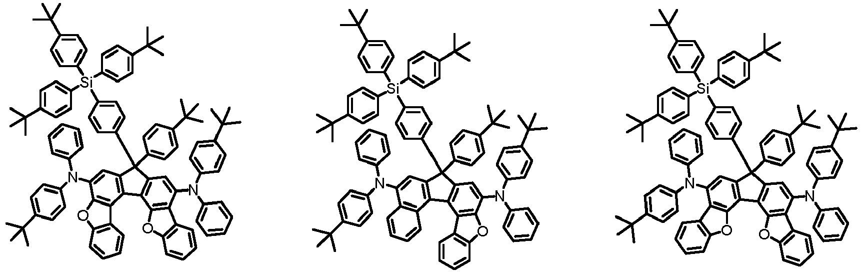

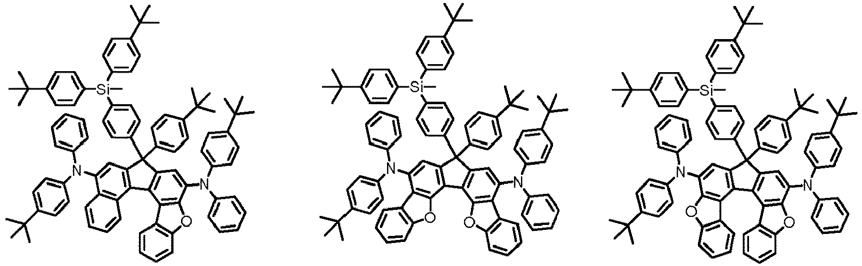

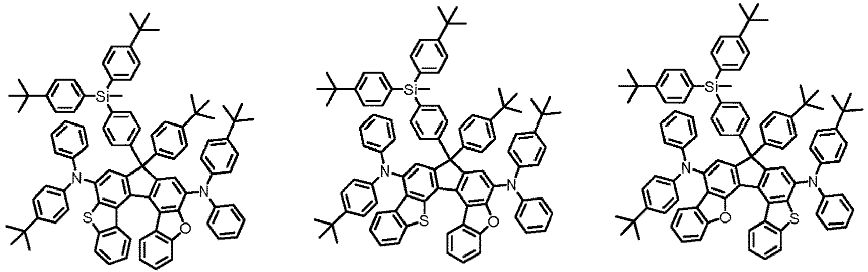

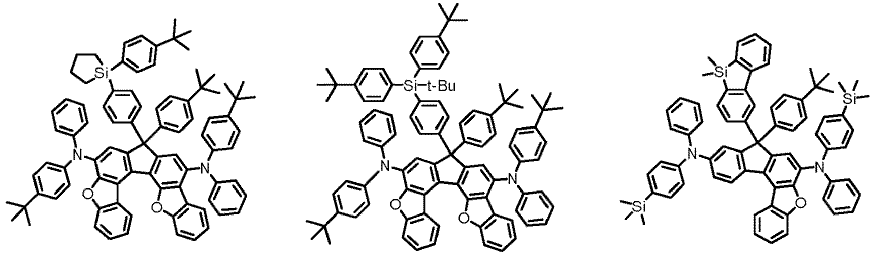

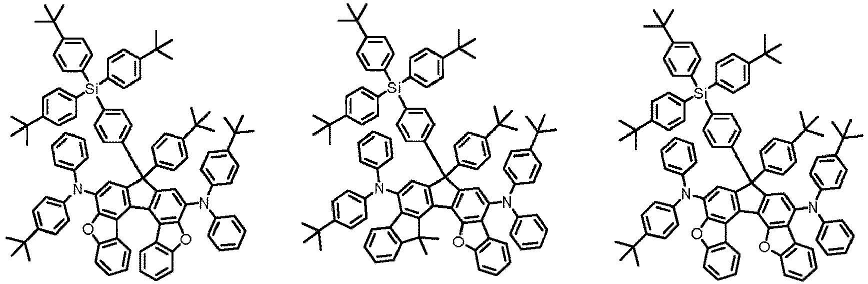

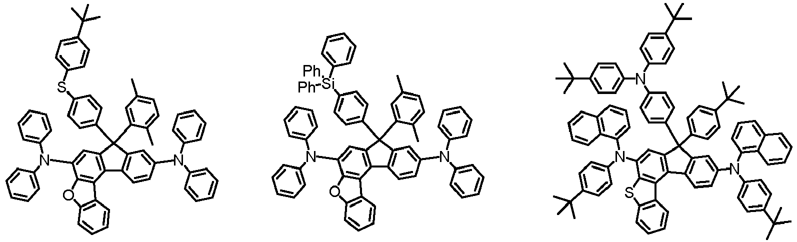

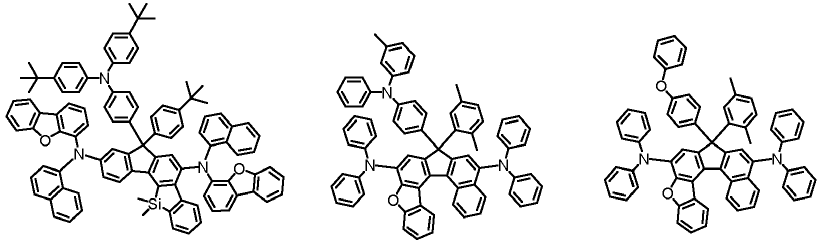

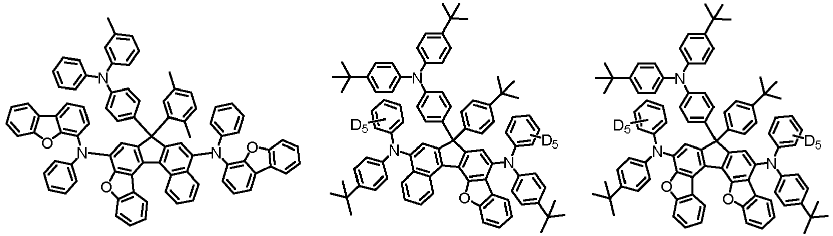

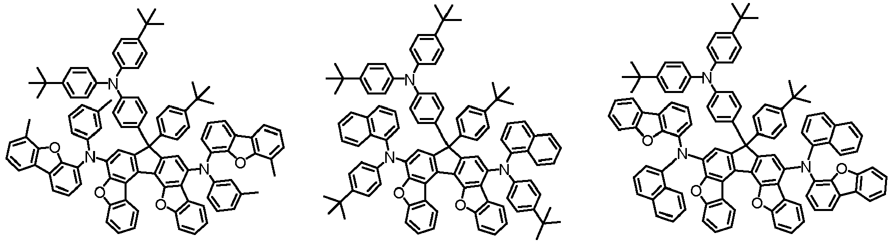

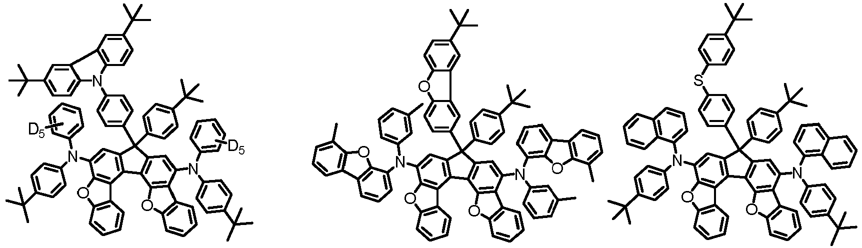

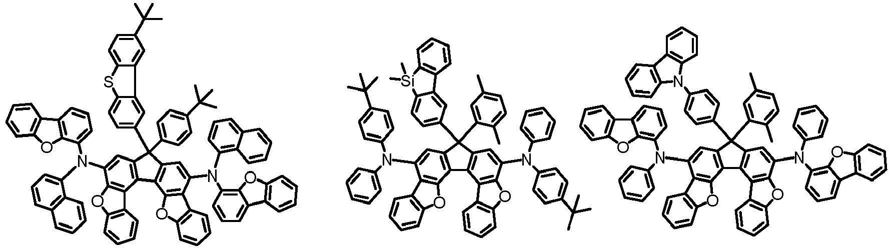

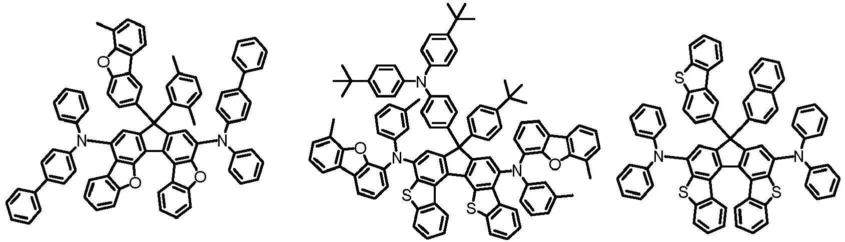

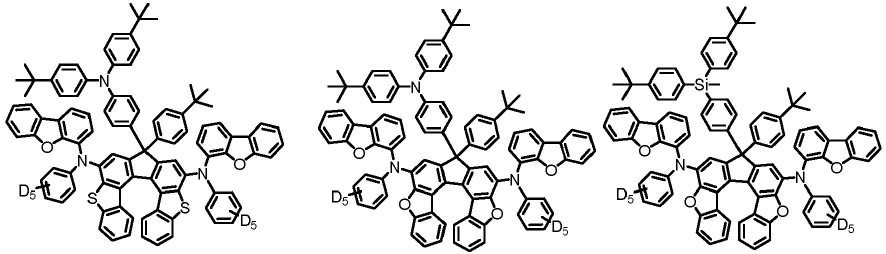

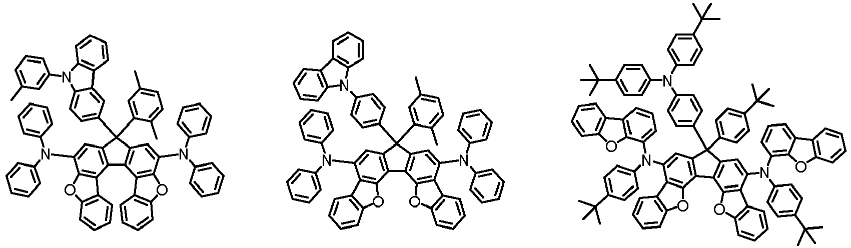

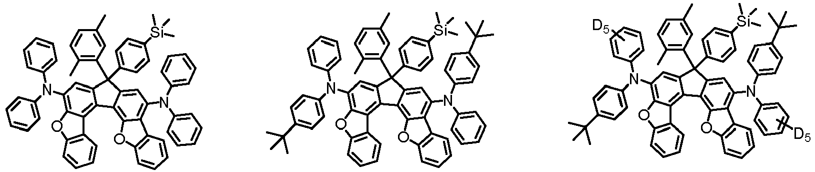

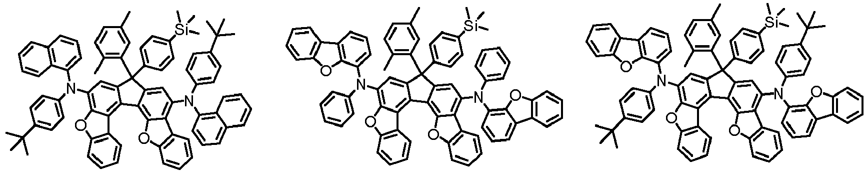

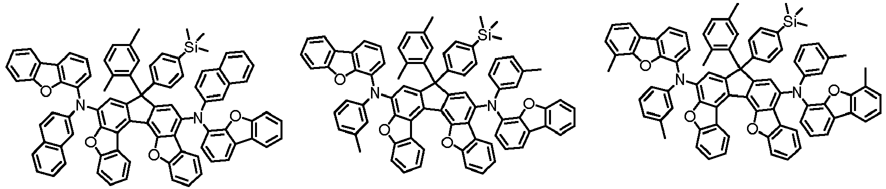

13. Dispositif électroluminescent organique comprenant : une première électrode ; une seconde électrode qui est prévue à l'opposé de la première électrode ; et une couche électroluminescente qui est prévue entre la première électrode et la seconde électrode, dans lequel la couche électroluminescente inclut le composé selon l'une quelconque des revendications 1 à 12.
